(19) Europäisches Patentamt
European Patent Office
Office européen des brevets

(11) **EP 3 305 781 A1**

(12) **EUROPEAN PATENT APPLICATION**

(43) Date of publication:
**11.04.2018 Bulletin 2018/15**

(21) Application number: **16192835.3**

(22) Date of filing: **07.10.2016**

(51) Int Cl.:
***C07D 403/12*** *(2006.01)*
***C07D 209/08*** *(2006.01)*
***C07D 405/12*** *(2006.01)*
***A61K 31/4155*** *(2006.01)*
***A61K 31/4439*** *(2006.01)*
***A61K 31/506*** *(2006.01)*
***C07D 231/40*** *(2006.01)*
***C07D 401/12*** *(2006.01)*
***C07D 409/12*** *(2006.01)*
***A61K 31/405*** *(2006.01)*
***A61K 31/4178*** *(2006.01)*
***C07C 235/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Deutsches Krebsforschungszentrum**
**69120 Heidelberg (DE)**

(72) Inventors:
- **MILLER, Aubry**
  **69126 Heidelberg (DE)**
- **SCHÜLER, Peter**
  **69493 Hirschberg (DE)**
- **DE VITA, Elena**
  **69121 Heidelberg (DE)**

(74) Representative: **Schiweck, Weinzierl & Koch**
**Patentanwälte Partnerschaft mbB**
**Landsberger Straße 98**
**80339 München (DE)**

(54) **CHEMICAL SUBSTANCES WHICH INHIBIT THE ENZYMATIC ACTIVITY OF HUMAN KALLIKREIN-RELATED PEPTIDASE 6 (KLK6)**

(57) The invention relates to compounds which are suitable for the treatment of a disease associated with kallikrein-like peptidase 6 overexpression and to pharmaceutical compositions containing such compounds. The invention further relates to a kit of parts comprising such compounds or pharmaceutical compositions.

**Figure 1**

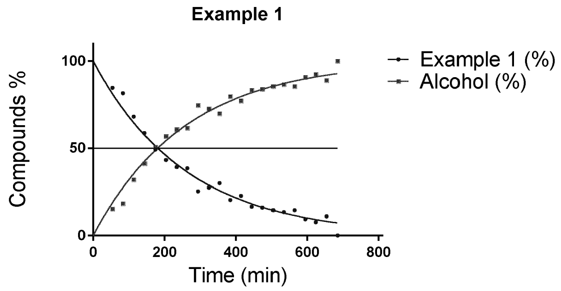


$t_{1/2}$ = 180.7 min ~ 3h

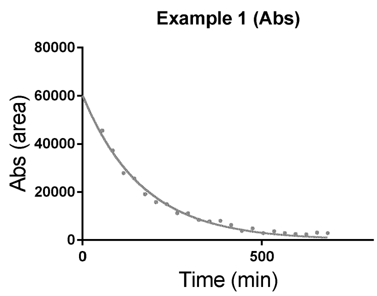


$t_{1/2}$ = 116.1min ~ 2h

EP 3 305 781 A1

## Description

## BACKGROUND

The human tissue kallikrein (KLK) family of secreted serine proteases comprises fifteen homologously related enzymes (Kallikrein-Related Peptidases, V. Magdolen et al. (ed), De Gruyter, Berlin (2012) Vol. 1 and 2). Human tissue kallikrein (KLK1), the parent member of the family and known since 1930, is now joined by fourteen kallikrein-related peptidases (KLK2-KLK15). The KLKs play important roles in a wide range of physiological processes, such as tooth enamel formation, skin desquamation, seminal liquefaction, neural plasticity and brain function, and immune response. The regulation of KLKs is fine-tuned by specific mechanisms such as zymogen activation cascades (KLKs are originally produced in inactive forms) and endogenous KLK inhibitors. Mis-regulation of KLK levels/activities has been observed in numerous diseases, and the use of KLKs as diagnostic and prognostic biomarkers, most notably KLK3 (PSA), has been extensively studied (Hernandez et al. Cancer, 2004, 101, 894; Schmitt et al. Thromb. Haemost. 2009, 101, 222). The investigation of KLKs as targets for pharmacological therapy is significantly more limited, but has been recently reviewed (Prassas et al. Nat. Rev. Drug Discov. 2015, 14, 183.)

KLK6 is most highly expressed in the central nervous system, and has been suggested as a potential therapeutic target in neurodegenerative diseases such as multiple sclerosis and Alzheimer's disease (Diamandis et al. Clin. Biochem. 2000, 33, 369; Yousef et al. Clin. Chim. Acta 2003, 329, 1; Shaw et al. Clin. Chem. 2007, 53, 1423; Blaber et al. Biochemistry, 2007, 46, 5209). In addition, a number of studies have shown a correlation between KLK6 levels and the proliferation (Kim et al. Cancer, 2011, 117, 2608), cell migration, and invasion of cancer cell lines (Krenzer et al. J. Invest. Dermatol. 2011, 131, 2281). There are only two reports of small molecule inhibitors of KLK6 (Liang et al. Bioorg. Med. Chem. Lett. 2012, 22, 2450; Liang et al. ACS Med. Chem. Lett. 2012, 3, 159.) Both publications report the X-ray crystal structure of inhibitors bound to KLK6 and demonstrate that the inhibitors are bound via hydrogen bonding of a basic residue to the side chain of Asp189, which sits in the active site of the enzyme. The inhibitors show only moderate potency and no selectivity over related enzymes was reported. Thus, there is still a great need for new inhibitor scaffolds which can potently and selectively inhibit KLK6. The present invention satisfies this need by providing the KLK6-inhibiting compounds of the formula I.

## SUMMARY

Accordingly, the present invention provides in a first aspect a compound for use in the treatment of a disease associated with kallikrein-like peptidase 6 overexpression, said compound having a structure according to Formula I

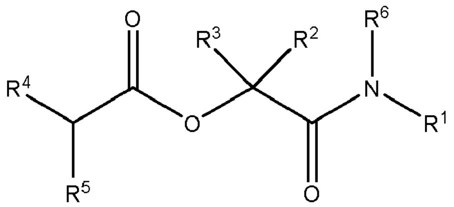

(I)

wherein,

$R^1$ is selected from the group consisting of substituted or unsubstituted alkyl, substituted or unsubstituted cycloalkyl, substituted or unsubstituted aryl, substituted or unsubstituted heteroaryl and substituted or unsubstituted heteroarylalkyl;
$R^2$ and $R^3$ are independently selected from the group consisting of hydrogen and substituted or unsubstituted alkyl; or $R^2$ and $R^3$ are substituted or unsubstituted alkyl and join together with the carbon atom to which they are attached to form a carbocyclic ring which is optionally substituted;
$R^4$ is selected from the group consisting of substituted or unsubstituted aryl, substituted or unsubstituted heteroaryl and substituted or unsubstituted heteroarylalkyl;
$R^5$ is hydrogen or substituted or unsubstituted alkyl;
$R^6$ is hydrogen or substituted or unsubstituted alkyl; or a pharmaceutically acceptable salt, solvate or hydrate thereof; with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In a second aspect, the present invention provides a pharmaceutical composition for use in the treatment of a disease associated with kallikrein-like peptidase 6 overexpression, wherein said composition comprises a compound having a structure according to Formula I

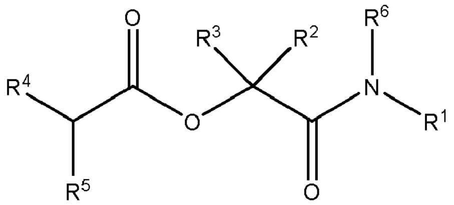
(I)

wherein,

$R^1$ is selected from the group consisting of substituted or unsubstituted alkyl, substituted or unsubstituted cycloalkyl, substituted or unsubstituted aryl, substituted or unsubstituted heteroaryl and substituted or unsubstituted heteroarylalkyl;
$R^2$ and $R^3$ are independently selected from the group consisting of hydrogen and substituted or unsubstituted alkyl; or $R^2$ and $R^3$ are substituted or unsubstituted alkyl and join together with the carbon atom to which they are attached to form a carbocyclic ring which is optionally substituted;
$R^4$ is selected from the group consisting of substituted or unsubstituted aryl, substituted or unsubstituted heteroaryl and substituted or unsubstituted heteroarylalkyl;
$R^5$ is hydrogen or substituted or unsubstituted alkyl;
$R^6$ is hydrogen or substituted or unsubstituted alkyl; or a pharmaceutically acceptable salt, solvate or hydrate thereof; with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In some embodiments said compound having a structure according to Formula I is characterized in that,
$R^1$ is selected from the group consisting of substituted or unsubstituted $(C_1-C_6)$alkyl, substituted or unsubstituted $(C_3-C_8)$cycloalkyl, substituted or unsubstituted $(C_6-C_{14})$aryl, substituted or unsubstituted $(C_3-C_{14})$heteroaryl and substituted or unsubstituted $(C_3-C_{14})$heteroaryl$(C_1-C_6)$alkyl; $R^2$ and $R^3$ are independently selected from the group consisting of hydrogen and substituted or unsubstituted $(C_1-C_6)$alkyl; or $R^2$ and $R^3$ are substituted or unsubstituted $(C_1-C_6)$alkyl and join together with the carbon atom to which they are attached to form a carbocyclic ring which is optionally substituted;
$R^4$ is selected from the group consisting of substituted or unsubstituted $(C_6-C_{14})$aryl, substituted or unsubstituted $(C_3-C_{14})$heteroaryl and substituted or unsubstituted $(C_3-C_{14})$heteroaryl$(C_1-C_6)$alkyl;
$R^5$ is hydrogen or substituted or unsubstituted $(C_1-C_6)$alkyl, preferably hydrogen; and
$R^6$ is hydrogen or substituted or unsubstituted $(C_1-C_6)$alkyl, preferably hydrogen.

In some embodiments said compound having a structure according to Formula I is characterized in that,
$R^1$ and $R^4$ are independently selected from the group consisting of substituted or unsubstituted $(C_6-C_{14})$aryl and substituted or unsubstituted $(C_3-C_{14})$ heteroaryl.

In some embodiments said compound having a structure according to Formula I is a compound having a structure according to Formula (II)

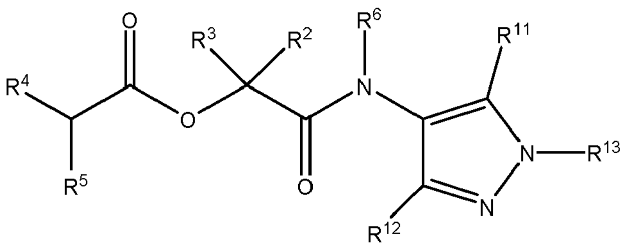
(II)

wherein,

$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of substituted or unsubstituted ($C_6$-$C_{14}$)aryl and substituted or unsubstituted ($C_3$-$C_{14}$) heteroaryl;
$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{11}$ and $R^{12}$ are independently selected from the group consisting of hydrogen and substituted or unsubstituted ($C_1$-$C_6$)alkyl;
$R^{13}$ is selected from the group consisting of substituted or unsubstituted ($C_1$-$C_6$)alkyl, substituted or unsubstituted ($C_6$-$C_{14}$)aryl, substituted or unsubstituted ($C_3$-$C_{14}$)heteroaryl($C_1$-$C_6$)alkyl and substituted or unsubstituted ($C_6$-$C_{14}$)aryl($C_1$-$C_6$)alkyl; or a pharmaceutically acceptable salt, solvate or hydrate thereof; with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

In some embodiments said compound having a structure according to Formula I is a compound having a structure according to Formula (III)

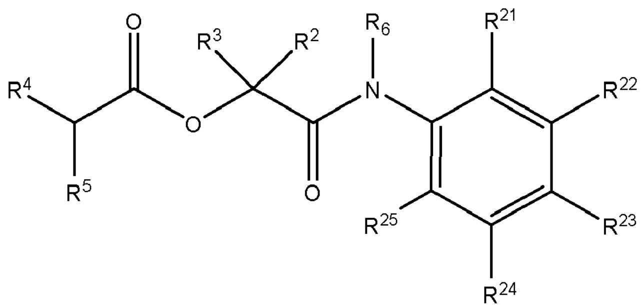
(III)

wherein,

$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of substituted or unsubstituted ($C_6$-$C_{14}$)aryl and substituted or unsubstituted ($C_3$-$C_{14}$) heteroaryl;
$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{21}$, $R^{22}$, $R^{23}$, $R^{24}$ and $R^{25}$ are independently selected from the group consisting of hydrogen, substituted or unsubstituted ($C_3$-$C_{14}$)heterocyclyl, substituted or unsubstituted amino, substituted or unsubstituted ($C_1$-$C_6$)alkyl, -$OR^{26}$, halogen and -C(O)$NR^{27}R^{28}$;
$R^{26}$ is selected from the group consisting of hydrogen, substituted or unsubstituted ($C_1$-$C_6$)alkyl and substituted or unsubstituted ($C_2$-$C_6$)alkenyl;
$R^{27}$ and $R^{28}$ are independently selected from the group consisting of hydrogen and substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

In some embodiments said compound having a structure according to Formula I is a compound having a

structure according to Formula (IV)

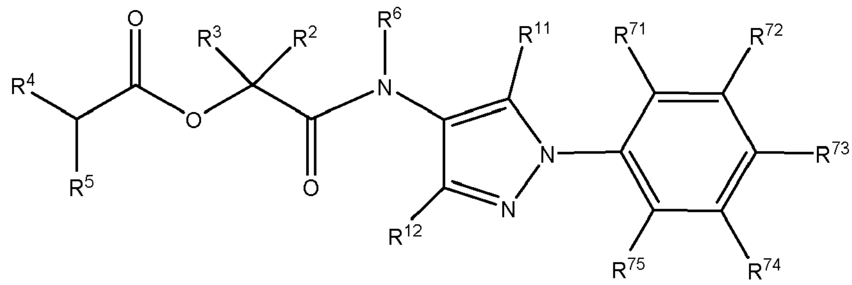

(IV)

wherein

$R^2$ and $R^3$ are independently selected from the group consisting of hydrogen and substituted or unsubstituted $(C_1\text{-}C_6)$alkyl; or $R^2$ and $R^3$ are substituted or unsubstituted $(C_1\text{-}C_6)$alkyl and join together with the carbon atom to which they are attached to form a carbocyclic ring which is optionally substituted;
$R^4$ is selected from the group consisting of substituted or unsubstituted $(C_6\text{-}C_{14})$aryl and substituted or unsubstituted $(C_3\text{-}C_{14})$ heteroaryl;
$R^5$ is hydrogen or substituted or unsubstituted $(C_1\text{-}C_6)$alkyl, preferably hydrogen;
$R^6$ is hydrogen or substituted or unsubstituted $(C_1\text{-}C_6)$alkyl, preferably hydrogen;
$R^{11}$ and $R^{12}$ are independently selected from the group consisting of hydrogen and substituted or unsubstituted $(C_1\text{-}C_6)$alkyl;
$R^{71}$, $R^{72}$, $R^{73}$, $R^{74}$, $R^{75}$ and $R^{76}$ are independently selected from the group consisting of hydrogen, $(C_1\text{-}C_6)$alkyl, $(C_1\text{-}C_6)$haloalkyl, $-NO_2$, halogen, amino, $-N(R^{78})((C_1\text{-}C_6)$alkyl-$OR^{77})$, cyano and - $OR^{77}$;
$R^{77}$ and $R^{78}$ are each independently selected from the group consisting of hydrogen, $(C_1\text{-}C_6)$haloalkyl and substituted or unsubstituted $(C_1\text{-}C_6)$alkyl, preferably hydrogen and/or substituted or unsubstituted $(C_1\text{-}C_6)$alkyl;
or a pharmaceutically acceptable salt, solvate or hydrate thereof;
with the proviso that

(i) $R^5$ is hydrogen, when one of $R^2$ and $R^3$ is substituted or unsubstituted $(C_1\text{-}C_6)$alkyl, or $R^2$ and $R^3$ are substituted or unsubstituted $(C_1\text{-}C_6)$alkyl and join together with the carbon atom to which they are attached to form a carbocyclic ring;
(ii) $R^2$ and $R^3$ are hydrogen, when $R^5$ is substituted or unsubstituted $(C_1\text{-}C_6)$alkyl.

In some embodiments said compound having a structure according to Formula (I), Formula (II), Formula (III) or Formula (IV) is characterized in that,
**[0011]** $R^4$ is selected from the group consisting of substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl.
In some embodiments said compound having a structure according to Formula (I), Formula (II), Formula (III) or Formula (IV) is characterized in that,
$R^4$ is selected from the group consisting of

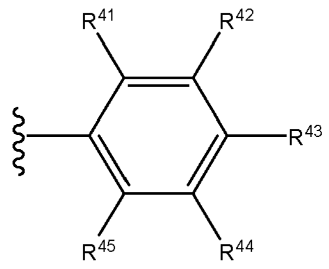

,

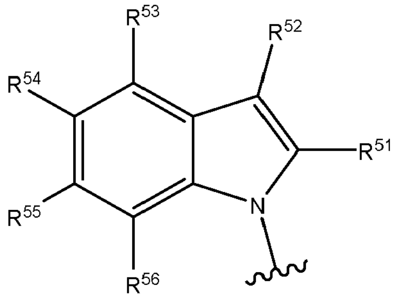

and

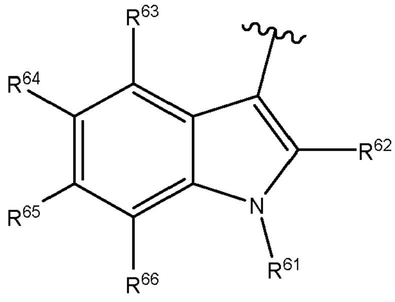

,

wherein,

$R^{41}$, $R^{42}$, $R^{43}$, $R^{44}$ and $R^{45}$ are independently selected from the group consisting of hydrogen, substituted or unsubstituted ($C_1$-$C_6$)alkyl, ($C_1$-$C_6$)haloalkyl, halogen, azido, -CN,-N($R^{46}$)C(=O)$R^{47}$, amino and $OR^{48}$;
$R^{46}$, $R^{47}$ and $R^{48}$ are independently selected from the group consisting of hydrogen, substituted or unsubstituted ($C_6$-$C_{14}$)aryl($C_1$-$C_6$)alkyl, ($C_1$-$C_6$)haloalkyl and substituted or unsubstituted ($C_1$-$C_6$)alkyl, preferably hydrogen and substituted or unsubstituted ($C_1$-$C_6$)alkyl;
$R^{51}$, $R^{52}$, $R^{53}$, $R^{54}$, $R^{55}$ and $R^{56}$ are independently selected from the group consisting of hydrogen, substituted or unsubstituted ($C_1$-$C_6$)alkyl, halogen and ($C_1$-$C_6$)haloalkyl, preferably substituted or unsubstituted ($C_1$-$C_6$)alkyl and ($C_1$-$C_6$)haloalkyl;
$R^{61}$, $R^{62}$, $R^{63}$, $R^{64}$, $R^{65}$ and $R^{66}$ are independently selected from the group consisting of hydrogen, substituted or unsubstituted ($C_1$-$C_6$)alkyl, halogen and ($C_1$-$C_6$)haloalkyl, preferably hydrogen, ($C_1$-$C_6$)haloalkyl and substituted or unsubstituted ($C_1$-$C_6$)alkyl.

In some embodiments said disease associated with kallikrein-like peptidase 6 overexpression is selected from the group consisting of cancer, immune disease and neuron injury, preferably wherein cancer is selected from the group consisting of Melanoma, Glioblastoma, Astrocytoma, Ovarian cancer, Colon carcinoma, Lung cancer, Gastric cancer, Gastroesophageal junction adenocarcinoma, Pancreatic ductal adenocarcinoma, Breast cancer and Laryngeal adenocarcinoma, preferably wherein the immune disease is an autoimmune disease, more preferably wherein the autoimmune disease is selected from the group consisting of Multiple Sclerosis, Psoriasis, Morbus Crohn and Colitis ulcerosa, even more preferably Multiple Sclerosis and Psoriasis, still more preferably Multiple Sclerosis, preferably wherein the neuron injury is Spinal Cord Injury (SCI).

In a third aspect, the present invention provides a compound having a structure according to Formula (IV)

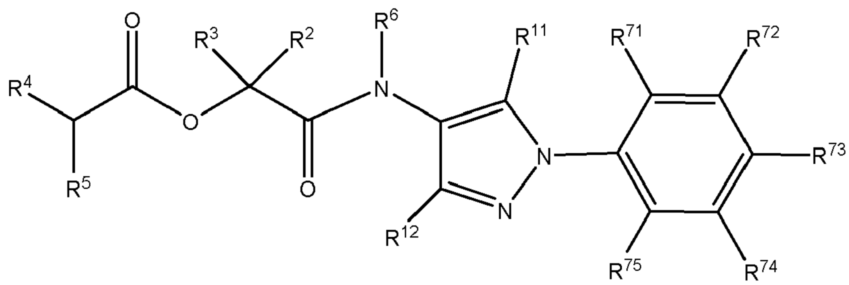

(IV)

wherein

$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of

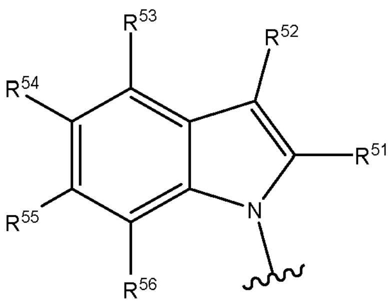

and

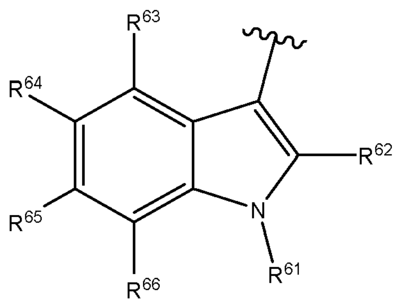

;

$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{11}$ and $R^{12}$ are independently selected from the group consisting of hydrogen and substituted or unsubstituted ($C_1$-$C_6$)alkyl;
$R^{51}$, $R^{52}$, $R^{53}$, $R^{54}$, $R^{55}$ and $R^{56}$ are independently selected from the group consisting of hydrogen, substituted or unsubstituted ($C_1$-$C_6$)alkyl, halogen and ($C_1$-$C_6$)haloalkyl, preferably substituted or unsubstituted ($C_1$-$C_6$)alkyl and ($C_1$-$C_6$)haloalkyl;

$R^{61}$, $R^{62}$, $R^{63}$, $R^{64}$, $R^{65}$ and $R^{66}$ are independently selected from the group consisting of hydrogen, substituted or unsubstituted ($C_1$-$C_6$)alkyl, halogen and ($C_1$-$C_6$)haloalkyl, preferably hydrogen, ($C_1$-$C_6$)haloalkyl and substituted or unsubstituted ($C_1$-$C_6$)alkyl;
$R^{71}$, $R^{72}$, $R^{73}$, $R^{74}$, $R^{75}$ and $R^{76}$ are independently selected from the group consisting of ($C_1$-$C_6$)haloalkyl, $-NO_2$, halogen, amino, $-N(R^{78})((C_1$-$C_6)$alkyl-$OR^{77})$, cyano and $-OR^{77}$;
$R^{77}$ and $R^{78}$ are each independently selected from the group consisting of hydrogen, ($C_1$-$C_6$)haloalkyl and substituted or unsubstituted ($C_1$-$C_6$)alkyl, preferably substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

In some embodiments said compound is selected from the group consisting of 2-((3,5-dimethyl-1-(m-tolyl)-1 H-pyrazol-4-yl)amino)-2-oxoethyl 2-(2-methyl-1H-indol-1-yl)acetate, 2-((3,5-dimethyl-1-(4-(trifluoromethyl)phenyl)-1 H-pyrazol-4-yl)amino)-2-oxoethyl 2-(2-methyl-1H-indol-1-yl)acetate, 2-((3,5-dimethyl-1-(2-nitrophenyl)-1H-pyrazol-4-yl)amino)-2-oxoethyl 2-(2-methyl-1H-indol-1-yl)acetate, 2-((1-(4-cyanophenyl)-3,5-dimethyl-1H-pyrazol-4-yl)amino)-2-oxoethyl 2-(2-methyl-1H-indol-1-yl)acetate, 2-((1-(4-methoxybenzyl)-3,5-dimethyl-1H-pyrazol-4-yl)amino)-2-oxoethyl 2-(2-methyl-1H-indol-1-yl)acetate, 2-((3,5-dimethyl-1-(2-(trifluoromethyl)phenyl)-1H-pyrazol-4-yl)amino)-2-oxoethyl 2-(2-methyl-1H-indol-1-yl)acetate, 2-((3,5-dimethyl-1-(p-tolyl)-1H-pyrazol-4-yl)amino)-2-oxoethyl 2-(2-methyl-1H-indol-1-yl)acetate, 2-((3,5-dimethyl-1-(4-nitrophenyl)-1H-pyrazol-4-yl)amino)-2-oxoethyl 2-(2-methyl-1H-indol-1-yl)acetate, 2-((3,5-dimethyl-1-phenyl-1H-pyrazol-4-yl)amino)-2-oxoethyl 2-(1H-indol-1-yl)acetate, 2-((3,5-dimethyl-1-(p-tolyl)-1H-pyrazol-4-yl)amino)-2-oxoethyl 2-(1 H-indol-1-yl)acetate, 2-((3,5-dimethyl-1-phenyl-1H-pyrazol-4-yl)amino)-2-oxoethyl 2-(3-methyl-1H-indol-1-yl)acetate, 2-((3,5-dimethyl-1-phenyl-1H-pyrazol-4-yl)amino)-2-oxoethyl 2-(2,3-dimethyl-1H-indol-1-yl)acetate, 2-((3,5-dimethyl-1-phenyl-1H-pyrazol-4-yl)amino)-2-oxoethyl 2-(2-(trifluoromethyl)-1H-indol-1-yl)acetate, 2-((3,5-dimethyl-1-phenyl-1 H-pyrazol-4-yl)amino)-2-oxoethyl 2-(1-methyl-1H-indol-3-yl)acetate, 2-((1-(4-((3-hydroxybutyl)amino)phenyl)-3,5-dimethyl-1H-pyrazol-4-yl)amino)-2-oxoethyl 2-(2-methyl-1 H-indol-1-yl)acetate, 2-((3,5-dimethyl-1-(p-tolyl)-1H-pyrazol-4-yl)amino)-2-oxoethyl 2-(2-(trifluoromethyl)-1H-indol-1-yl)acetate, 2-((3,5-dimethyl-1-(4-nitrophenyl)-1 H-pyrazol-4-yl)amino)-2-oxoethyl 2-(2-(trifluoromethyl)-1 H-indol-1-yl)acetate, 2-((1-(4-aminophenyl)-3,5-dimethyl-1H-pyrazol-4-yl)amino)-2-oxoethyl 2-(2-methyl-1 H-indol-1-yl)acetate, 2-((1-(2-aminophenyl)-3,5-dimethyl-1H-pyrazol-4-yl)amino)-2-oxoethyl 2-(2-methyl-1 H-indol-1-yl)acetate, 2-((3,5-dimethyl-1-phenyl-1 H-pyrazol-4-yl)amino)-2-oxoethyl 2-(4-fluoro-1 H-indol-1-yl)acetate, 2-((3,5-dimethyl-1-phenyl-1H-pyrazol-4-yl)amino)-2-oxoethyl 2-(6-chloro-1H-indol-1-yl)acetate, 2-((3,5-dimethyl-1-phenyl-1H-pyrazol-4-yl)amino)-2-oxoethyl 2-(5-chloro-1H-indol-1-yl)acetate, 2-((3,5-dimethyl-1-phenyl-1 H-pyrazol-4-yl)amino)-2-oxoethyl 2-(5-fluoro-1 H-indol-1-yl)acetate, 2-((3,5-dimethyl-1-phenyl-1 H-pyrazol-4-yl)amino)-2-oxoethyl 2-(5-chloro-2-methyl-1 H-indol-1-yl)acetate, 2-((3,5-dimethyl-1-phenyl-1 H-pyrazol-4-yl)amino)-2-oxoethyl 2-(7-chloro-1 H-indol-1-yl)acetate, 2-((3,5-dimethyl-1-phenyl-1 H-pyrazol-4-yl)amino)-2-oxoethyl 2-(2-isopropyl-1 H-indol-1-yl)acetate, 2-((3,5-dimethyl-1-phenyl-1 H-pyrazol-4-yl)amino)-2-oxoethyl 2-(7-chloro-2-methyl-1 H-indol-1-yl)acetate, 2-((3,5-dimethyl-1-phenyl-1 H-pyrazol-4-yl)amino)-2-oxoethyl 2-(2-methyl-1 H-indol-1-yl)propanoate, 2-((3,5-dimethyl-1-phenyl-1H-pyrazol-4-yl)amino)-2-oxoethyl 2-(2-ethyl-1H-indol-1-yl)acetate, 1-((3,5-dimethyl-1-phenyl-1H-pyrazol-4-yl)amino)-l-oxopropan-2-yl 2-(2-methyl-1H-indol-1-yl)acetate, 2-((3,5-dimethyl-1-phenyl-1 H-pyrazol-4-yl)amino)-2-oxoethyl 2-(2-methyl-1 H-indol-3-yl)acetate, 2-oxo-2-((1-phenyl-1H-pyrazol-4-yl)amino)ethyl 2-(2-methyl-1 H-indol-3-yl)acetate, 1-((3,5-dimethyl-1-phenyl-1H-pyrazol-4-yl)carbamoyl)cyclopropyl 2-(2-methyl-1H-indol-1-yl)acetate, 2-((3,5-dimethyl-1-phenyl-1H-pyrazol-4-yl)amino)-2-oxoethyl 2-(benzofuran-3-yl)acetate, 2-((3,5-dimethyl-1-phenyl-1H-pyrazol-4-yl)amino)-2-oxoethyl 2-(4-aminophenyl)acetate, 2-((3,5-dimethyl-1-phenyl-1H-pyrazol-4-yl)amino)-2-oxoethyl 2-(2-chlorophenyl)acetate, 2-((3,5-dimethyl-1-phenyl-1H-pyrazol-4-yl)amino)-2-oxoethyl 2-(4-cyanophenyl)acetate, 2-((3,5-dimethyl-1-phenyl-1H-pyrazol-4-yl)amino)-2-oxoethyl 2-(3-acetamidophenyl)acetate, 2-((3,5-dimethyl-1-phenyl-1 H-pyrazol-4-yl)amino)-2-oxoethyl 2-(2-bromophenyl)acetate, 2-((3,5-dimethyl-1-phenyl-1H-pyrazol-4-yl)amino)-2-oxoethyl 2-(m-tolyl)acetate, 2-((3,5-dimethyl-1-phenyl-1H-pyrazol-4-yl)amino)-2-oxoethyl 2-(2-iodophenyl)acetate, 2-((3,5-dimethyl-1-phenyl-1H-pyrazol-4-yl)amino)-2-oxoethyl 2-(3-aminophenyl)acetate, 2-((3,5-dimethyl-1-phenyl-1H-pyrazol-4-yl)amino)-2-oxoethyl 2-(3-cyanophenyl)acetate, 2-((3,5-dimethyl-1-phenyl-1H-pyrazol-4-yl)amino)-2-oxoethyl 2-(pyridin-3-yl)acetate, 2-((3,5-dimethyl-1-phenyl-1H-pyrazol-4-yl)amino)-2-oxoethyl 2-(pyridin-2-yl)acetate, 2-((3,5-dimethyl-1-phenyl-1 H-pyrazol-4-yl)amino)-2-oxoethyl 2-(5-chlorothiophen-2-yl)acetate, 2-((3,5-dimethyl-1-phenyl-1H-pyrazol-4-yl)amino)-2-oxoethyl 2-(2,5-dichlorothiophen-3-yl)acetate, 2-((3,5-dimethyl-1-phenyl-1H-pyrazol-4-yl)amino)-2-oxoethyl 2-(pyridin-4-yl)acetate, 2-((3,5-dimethyl-1-phenyl-1H-pyrazol-4-yl)amino)-2-oxoethyl 2-(2-(trifluoromethyl)phenyl)acetate, 2-((3,5-dimethyl-1-phenyl-1H-pyrazol-4-yl)amino)-2-oxoethyl 2-(3-fluorophenyl)acetate, 2-((3,5-dimethyl-1-phenyl-1 H-pyrazol-4-yl)amino)-2-oxoethyl 2-(2-chlorothiophen-3-yl)acetate,

2-((3,5-dimethyl-1-phenyl-1 H-pyrazol-4-yl)amino)-2-oxoethyl 2-(furan-2-yl)acetate, 2-((3,5-dimethyl-1-phenyl-1H-pyrazol-4-yl)amino)-2-oxoethyl 2-(2-(benzyloxy)phenyl)acetate, 2-((3,5-dimethyl-1-phenyl-1 H-pyrazol-4-yl)amino)-2-oxoethyl 2-(thiophen-3-yl)acetate, 2-((3,5-dimethyl-1-phenyl-1 H-pyrazol-4-yl)amino)-2-oxoethyl 2-(2-methyl-1 H-indol-3-yl)propanoate, 2-((3,5-dimethyl-1-phenyl-1H-pyrazol-4-yl)amino)-2-oxoethyl 2-(2-azidophenyl)acetate, 2-((3,5-dimethyl-1-phenyl-1H-pyrazol-4-yl)amino)-2-oxoethyl 2-(1H-indol-3-yl)propanoate, 2-((3,5-dimethyl-1-phenyl-1 H-pyrazol-4-yl)amino)-2-oxoethyl 3-(2-methyl-1 H-indol-3-yl)propanoate, 2-((3,5-dimethyl-1-phenyl-1 H-pyrazol-4-yl)(methyl)amino)-2-oxoethyl 2-(2-methyl-1 H-indol-3-yl)acetate, 2-(tert-butylamino)-2-oxoethyl 2-(2-methyl-1 H-indol-3-yl)acetate, 2-(cyclopentylamino)-2-oxoethyl 2-(2-methyl-1 H-indol-3-yl)acetate, 2-((3,5-dimethyl-1-phenyl-1 H-pyrazol-4-yl)amino)-2-oxoethyl 2-(3,5-dimethyl-1 H-pyrazol-1-yl)acetate, 2-((5-fluoropyridin-2-yl)amino)-2-oxoethyl 2-(2-methyl-1 H-indol-3-yl)acetate, 2-(((1H-imidazol-2-yl)methyl)amino)-2-oxoethyl 2-(2-methyl-1 H-indol-3-yl)acetate, 2-oxo-2-(pyrimidin-5-ylamino)ethyl 2-(2-methyl-1 H-indol-3-yl)acetate, 2-oxo-2-(phenylamino)ethyl 2-(2-methyl-1 H-indol-1-yl)acetate, 2-oxo-2-(o-tolylamino)ethyl 2-(2-methyl-1 H-indol-1-yl)acetate, 2-((4-morpholinophenyl)amino)-2-oxoethyl 2-(2-methyl-1 H-indol-1-yl)acetate, 2-((2-(allyloxy)phenyl)amino)-2-oxoethyl 2-(2-iodophenyl)acetate, 2-((2-chlorophenyl)amino)-2-oxoethyl 2-(2-methyl-1 H-indol-3-yl)acetate, 2-oxo-2-(p-tolylamino)ethyl 2-(2-methyl-1 H-indol-3-yl)acetate, 2-oxo-2-(m-tolylamino)ethyl 2-(2-methyl-1 H-indol-3-yl)acetate, 2-((4-morpholinophenyl)amino)-2-oxoethyl 2-(2-methyl-1 H-indol-3-yl)acetate, 2-((2-methoxyphenyl)amino)-2-oxoethyl 2-(2-methyl-1 H-indol-3-yl)acetate, 2-oxo-2-(o-tolylamino)ethyl 2-(2-methyl-1 H-indol-3-yl)acetate, 2-((3-methoxyphenyl)amino)-2-oxoethyl 2-(2-methyl-1 H-indol-3-yl)acetate, 2-((4-methoxyphenyl)amino)-2-oxoethyl 2-(2-methyl-1 H-indol-3-yl)acetate, 2-((3-carbamoylphenyl)amino)-2-oxoethyl 2-(2-methyl-1 H-indol-3-yl)acetate, 2-((3,5-dimethyl-1-phenyl-1H-pyrazol-4-yl)amino)-2-oxoethyl 2-(1H-indol-3-yl)acetate, 2-((3,5-dimethyl-1-phenyl-1 H-pyrazol-4-yl)amino)-2-oxoethyl 2-(1,2-dimethyl-1H-indol-3-yl)acetate, 2-((3,5-dimethyl-1-phenyl-1 H-pyrazol-4-yl)amino)-2-oxoethyl 2-(2-methylpyridin-3-yl)acetate and 2-((3,5-dimethyl-1-phenyl-1 H-pyrazol-4-yl)amino)-2-oxoethyl 3,3,3-trifluoro-2-(2-methyl-1H-indol-3-yl)propanoate
or a pharmaceutically acceptable salt, solvate or hydrate thereof.

In another aspect, the present invention relates to a pharmaceutical composition comprising said compound having a structure according to Formula (IV) or said compound selected from the group above and at least one pharmaceutically acceptable carrier.

In another aspect, the present invention relates to said compound having a structure according to Formula (IV) or said compound selected from the group above for use in medicine.

In another aspect, the present invention relates to said compound having a structure according to Formula (IV), said compound selected from the group above for use in medicine or said pharmaceutical composition for use in the treatment of a disease associated with kallikrein-like peptidase 6 overexpression, preferably wherein the disease associated with kallikrein-like peptidase 6 overexpression is selected from the group consisting of cancer, immune disease and neuron injury, preferably wherein cancer is selected from the group consisting of Melanoma, Glioblastoma, Astrocytoma, Ovarian cancer, Colon carcinoma, Lung cancer, Gastric cancer, Gastroesophageal junction adenocarcinoma, Pancreatic ductal adenocarcinoma, Breast cancer and Laryngeal adenocarcinoma, preferably wherein the immune disease is an autoimmune disease, more preferably wherein the autoimmune disease is selected from the group consisting of Multiple Sclerosis, Psoriasis, Morbus Crohn and Colitis ulcerosa, even more preferably Multiple Sclerosis and Psoriasis, still more preferably Multiple Sclerosis, preferably wherein the neuron injury is Spinal Cord Injury (SCI).

In another aspect, the present invention relates to a kit comprising said compound having a structure according to Formula (IV), said compound selected from the group above for use in medicine or said pharmaceutical composition and at least one pharmaceutically acceptable carrier.

Other features and advantages of the instant invention will be apparent from the following detailed description, figures and claims.

## FIGURES

**Figure 1:** Results of stability assay of Example Compound 1. "Example 1" in the drawing refers to Example Compound 1.

**Figure 2:** Results of stability assay of Example Compound 38. "Example 38" in the drawing refers to Example Compound 38.

**Figure 3:** Results of stability assay of Example Compound 81. "Example 81" in the drawing refers to Example Compound 81.

## DETAILED DESCRIPTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise. For example, if in one embodiment $R^1$ of the compounds of the invention is heteroaryl (such as pyrazolyl) and in another embodiment of the compounds of the invention $R^4$ is a substituted or unsubstituted phenyl ring, then in a preferred embodiment, $R^1$ of the compounds of the invention is heteroaryl (such as pyrazolyl) and $R^4$ is a substituted or unsubstituted phenyl ring.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

When used herein "consisting of' excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

*Compounds*

In order that the present invention may be more readily understood, certain terms are first defined. Additional definitions are set forth throughout the description.

As used herein and throughout the entire description, the term "alkyl" refers to a monoradical of a saturated straight or branched hydrocarbon. Preferably, the alkyl group comprises from 1 to 12 (such as 1 to 10) carbon atoms, i.e., 1, 2, 3, 4, 5, 6, 7, 8, 9, 10, 11, or 12 carbon atoms (such as 1, 2, 3, 4, 5, 6, 7, 8, 9, or 10 carbon atoms), more preferably 1 to 8 carbon atoms, such as 1 to 6 or 1 to 4 carbon atoms. In some embodiments, the alkyl group employed in the invention contains 1-20 carbon atoms ($C_{1-20}$ alkyl). In another embodiment, the alkyl group employed contains 1-15 carbon atoms ($C_{1-15}$ alkyl). In another embodiment, the alkyl group employed contains 1-10 carbon atoms ($C_{1-20}$

alkyl). In another embodiment, the alkyl group employed contains 1-8 carbon atoms ($C_{1-8}$ alkyl). In another embodiment, the alkyl group employed contains 1-6 carbon atoms ($C_{1-6}$ alkyl). In another embodiment, the alkyl group employed contains 1-5 carbon atoms ($C_{1-5}$ -alkyl). In another embodiment, the alkyl group employed contains 1-4 carbon atoms ($C_{1-4}$ alkyl). In another embodiment, the alkyl group employed contains 1-3 carbon atoms ($C_{1-3}$ alkyl). In another embodiment, the alkyl group employed contains 1-2 carbon atoms ($C_{1-2}$ alkyl). In another embodiment, the alkyl group employed is methyl. Examples of alkyl radicals include, but are not limited to, methyl, ethyl, propyl, iso-propyl, butyl, iso-butyl, tert-butyl, n-pentyl, iso-pentyl, sec-pentyl, neo-pentyl, 1,2-dimethyl-propyl, isoamyl, n-hexyl, iso-hexyl, sec-hexyl, n-heptyl, iso-heptyl, n-octyl, 2-ethyl-hexyl, n-nonyl, n-decyl, n-undecyl, n-dodecyl, and the like, which may bear one or more substituents. Alkyl group substituents include, but are not limited to, any of the substituents described herein, that result in the formation of a stable moiety. A substituted alkyl group can be substituted in any positions, provided that the resulting compound is sufficiently stable and is suitable as a pharmaceutical active compound. The prerequisite that a specific group and a compound of any one of formulas I, II, III and IV is sufficiently stable and suitable as a pharmaceutical active compound, applies in general with respect to all groups in the compounds of any one of formulas I, II, III and IV. If an alkyl group can be monosubstituted or polysubstituted by fluorine, it can be unsubstituted, i.e. not carry fluorine atoms, or substituted, for example by 1 , 2, 3, 4, 5, 6, 7, 8, 9, 10 or 11 fluorine atoms, preferably by 1 , 2, 3, 4 or 5 fluorine atoms, which can be present in any positions. For example, in a fluoro-substituted alkyl group one or more methyl groups can carry three fluorine atoms each and be present as trifluoromethyl groups, and/or one or more methylene groups ($CH_2$) can carry two fluorine atoms each and be present as difluoromethylene groups. The explanations with respect to the substitution of a group by fluorine also apply if the group additionally carries other substituents and/or is part of another group, for example of an alkyl-O-group. Examples of fluoro-substituted alkyl groups are trifluoromethyl, 2-fluoroethyl, 1 ,1-difluoroethyl, 2,2,2-trifluoroethyl, pentafluoroethyl, 3,3,3-trifluoropropyl, 2,2,3,3,3-pentafluoropropyl, 4,4,4-trifluorobutyl and heptafluoroisopropyl. Examples of fluoro-substituted alkyl-O- groups are trifluoromethoxy, 2,2,2-trifluoroethoxy, pentafluoroethoxy and 3,3,3-trifluoropropoxy. Examples of fluoro-substituted alkyl-$S(O)_m$- groups are trifluoromethanesulfanyl- ($CF_3$-S-, trifluoromethylsulfanyl-), trifluoromethanesulfinyl- ($CF_3$-S(O)-) and trifluoromethanesulfonyl- ($CF_3$-$S(O)_2$-). In some embodiments the alkyl group may be substituted by one or more identical or different substituents chosen from halogen, hydroxyl, cyano, ($C_1$-$C_6$)alkyl-O- and ($C_1$-$C_6$)alkyl-$S(O)_m$-. Examples of ($C_1$-$C_6$)alkyl-O- are methoxy, ethoxy, n-propoxy, isopropoxy, n-butoxy, isobutoxy, tert-butoxy and n-pentoxy. Examples of alkyl-$S(O)_m$- are methanesulfanyl- ($CH_3$-S-, methylsulfanyl), methanesulfinyl- ($CH_3$-S(O)-), methanesulfonyl- ($CH_3$-$S(O)_2$-), ethanesulfanyl- ($CH_3$-$CH_2$-S-, ethylsulfanyl-), ethanesulfinyl-($CH_3$-$CH_2$-S(O)-), ethanesulfonyl- ($CH_3$-$CH_2$-$S(O)_2$-), 1-methylethanesulfanyl- ($(CH_3)_2$CH-S-, 1-methylethylsulfanyl-), 1- methylethanesulfinyl- ($(CH_3)_2$CH-S(O)-) and 1-methylethanesulfonyl-($(CH_3)_2$CH- $S(O)_2$-). In one embodiment of the invention the number m is chosen from 0 and 2, wherein all numbers m are independent of each other and can be identical or different. For example, if the alkyl group is substituted by a $CF_3$-S- group, the $CF_3$-S- group is bonded to this alkyl group via the sulfur as it is symbolized by the terminal line (hyphen) next to the sulfur atom representing a free bond. In some embodiments the alkyl chain is a linear. In some embodiments the alkyl chain is branched. In some embodiments the alkyl chain is substituted. In some embodiment the alkyl chain is unsubstituted. In some embodiments the alkyl chain is linear and substituted or unsubstituted. In some embodiments the alkyl chain is branched and substituted or unsubstituted.

As used herein and throughout the entire description, the term term "alkylene" refers to a diradical of a saturated straight or branched hydrocarbon. Preferably, the alkylene comprises from 1 to 10 carbon atoms, i.e., 1, 2, 3, 4, 5, 6, 7, 8, 9, or 10 carbon atoms, more preferably 1 to 8 carbon atoms, such as 1 to 6 or 1 to 4 carbon atoms. Exemplary alkylene groups include methylene, ethylene (i.e., 1,1-ethylene, 1,2-ethylene), propylene (i.e., 1,1-propylene, 1,2-propylene (-$CH(CH_3)CH_2$-), 2,2-propylene (-$C(CH_3)_2$-), and 1,3-propylene), the butylene isomers (e.g., 1,1-butylene, 1,2-butylene, 2,2-butylene, 1,3-butylene, 2,3-butylene (cis or trans or a mixture thereof), 1,4-butylene, 1,1-iso-butylene, 1,2-iso-butylene, and 1,3-iso-butylene), the pentylene isomers (e.g., 1,1-pentylene, 1,2-pentylene, 1,3-pentylene, 1,4-pentylene, 1,5-pentylene, 1,1-iso-pentylene, 1,1-sec-pentyl, 1,1-neo-pentyl), the hexylenisomers (e.g., 1,1-hexylene, 1,2-hexylene, 1,3-hexylene, 1,4-hexylene, 1,5-hexylene, 1,6-hexylene, and 1,1-isohexylene), and the like. Alkylene groups may be cyclic or acyclic, branched or unbranched, substituted or unsubstituted. Alkylene group substituents include, but are not limited to, any of the substituents described herein, that result in the formation of a stable moiety.

As used herein and throughout the entire description, the term "haloalkyl" refers to an alkyl group substituted by one halogen substituent up to per halo-substitution. The halogen substituent is preferably fluorine. The haloalkyl is preferably a perfluoroalkyl. In some embodiments, the haloalkyl group employed in the invention contains 1-6 carbon atoms ($C_{1-6}$ haloalkyl). In another embodiment, the haloalkyl group employed contains 1-5 carbon atoms ($C_{1-5}$ haloalkyl). In another embodiment, the haloalkyl group employed contains 1-4 carbon atoms ($C_{1-4}$ haloalkyl). In another embodiment, the haloalkyl group employed contains 1-3 carbon atoms ($C_{1-3}$ haloalkyl). In another embodiment, the haloalkyl group employed contains 1-2 carbon atoms ($C_{1-2}$ haloalkyl). In another embodiment, the haloalkyl group employed contains 1-carbon atom ($C_1$ haloalkyl). In another embodiment, the haloalkyl group employed is trifluoromethyl. Exemplary fluoro-substituted $C_1$-$C_2$ alkyl includes -$CFH_2$, -$CF_2H$, -$CF_3$, $CH_2CH_2F$, -$CH_2CHF_2$, -$CHFCH_3$, -$CHFCH_3$, -$CF_2CHF_2$. Perfluoro-substituted $C_1$-$C_2$ haloalkyl, for example include -$CF_3$, and -$CF_2CF_3$.

As used herein and throughout the entire description, the term "alkenyl" refers to a monoradical of an unsaturated straight or branched hydrocarbon having at least one carbon-carbon double bond. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 to 4, i.e., 1, 2, 3, or 4, carbon-carbon double bonds. Preferably, the alkenyl group comprises from 2 to 10 carbon atoms, i.e., 2, 3, 4, 5, 6, 7, 8, 9, or 10 carbon atoms, more preferably 2 to 8 carbon atoms, such as 2 to 6 carbon atoms or 2 to 4 carbon atoms. Thus, in a preferred embodiment, the alkenyl group comprises from 2 to 10 carbon atoms and 1, 2, 3, 4, or 5 carbon-carbon double bonds, more preferably it comprises 2 to 8 carbon atoms and 1, 2, 3, or 4 carbon-carbon double bonds, such as 2 to 6 carbon atoms and 1, 2, or 3 carbon-carbon double bonds or 2 to 4 carbon atoms and 1 or 2 carbon-carbon double bonds. In certain embodiments, the alkenyl group employed in the invention contains 2-20 carbon atoms ($C_{2-20}$ alkenyl). In some embodiments, the alkenyl group employed in the invention contains 2-15 carbon atoms ($C_{2-15}$ alkenyl). In another embodiment, the alkenyl group employed contains 2-10 carbon atoms ($C_{2-10}$ alkenyl). In still other embodiments, the alkenyl group contains 2-8 carbon atoms ($C_{2-8}$ alkenyl). In yet other embodiments, the alkenyl group contains 2-6 carbons ($C_{2-6}$ alkenyl). In yet other embodiments, the alkenyl group contains 2-5 carbons ($C_{2-5}$ alkenyl). In yet other embodiments, the alkenyl group contains 2-4 carbons ($C_{2-4}$ alkenyl). In yet other embodiments, the alkenyl group contains 2-3 carbons ($C_{2-3}$ alkenyl). In yet other embodiments, the alkenyl group contains 2 carbons ($C_2$ alkenyl). The carbon-carbon double bond(s) may be in cis (Z) or trans (E) configuration. Exemplary alkenyl groups include vinyl, 1-propenyl, 2-propenyl (i.e., allyl), 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, and the like. If an alkenyl group is attached to a nitrogen atom, the double bond cannot be alpha to the nitrogen atom. Alkenyl group substituents include, but are not limited to, any of the substituents described herein, that result in the formation of a stable moiety. A substituted alkenyl group can be substituted in any positions, provided that the resulting compound is sufficiently stable and is suitable as a pharmaceutical active compound. The prerequisite that a specific group and a compound of any one of formulas I, II, III and IV is sufficiently stable and suitable as a pharmaceutical active compound, applies in general with respect to all groups in the compounds of any one of formulas I, II, III and IV. In some embodiments the alkenyl group may be substituted by one or more identical or different substituents chosen from halogen, hydroxyl, cyano, $(C_1$-$C_6)$alkyl-O- and $(C_1$-$C_6)$alkyl-S(O)$_m$-. In one embodiment of the invention the number m is chosen from 0 and 2, wherein all numbers m are independent of each other and can be identical or different. In some embodiments the alkenyl chain is a linear. In some embodiments the alkenyl chain is branched. In some embodiments the alkenyl chain is substituted. In some embodiment the alkenyl chain is unsubstituted. In some embodiments the alkenyl chain is linear and substituted or unsubstituted. In some embodiments the alkenyl chain is branched and substituted or unsubstituted. Alkenyl group substituents include, but are not limited to, any of the substituents described herein, that result in the formation of a stable moiety.

As used herein and throughout the entire description, the term "alkenylene" refers to a diradical of an unsaturated straight or branched hydrocarbon having at least one carbon-carbon double bond. Generally, the maximal number of carbon-carbon double bonds in the alkenylene group can be equal to the integer which is calculated by dividing the number of carbon atoms in the alkenylene group by 2 and, if the number of carbon atoms in the alkenylene group is uneven, rounding the result of the division down to the next integer. For example, for an alkenylene group having 9 carbon atoms, the maximum number of carbon-carbon double bonds is 4. Preferably, the alkenylene group has 1 to 4, i.e., 1, 2, 3, or 4, carbon-carbon double bonds. Preferably, the alkenylene group comprises from 2 to 10 carbon atoms, i.e., 2, 3, 4, 5, 6, 7, 8, 9, or 10 carbon atoms, more preferably 2 to 8 carbon atoms, such as 2 to 6 carbon atoms or 2 to 4 carbon atoms. Thus, in a preferred embodiment, the alkenylene group comprises from 2 to 10 carbon atoms and 1, 2, 3, 4, or 5 carbon-carbon double bonds, more preferably it comprises 2 to 8 carbon atoms and 1, 2, 3, or 4 carbon-carbon double bonds, such as 2 to 6 carbon atoms and 1, 2, or 3 carbon-carbon double bonds or 2 to 4 carbon atoms and 1 or 2 carbon-carbon double bonds. The carbon-carbon double bond(s) may be in cis (Z) or trans (E) configuration. Exemplary alkenylene groups include ethen-1,2-diyl, vinyliden, 1-propen-1,2-diyl, 1-propen-1,3-diyl, 1-propen-2,3-diyl, allyliden, 1-buten-1,2-diyl, 1-buten-1,3-diyl, 1-buten-1,4-diyl, 1-buten-2,3-diyl, 1-buten-2,4-diyl, 1-buten-3,4-diyl, 2-buten-1,2-diyl, 2-buten-1,3-diyl, 2-buten-1,4-diyl, 2-buten-2,3-diyl, 2-buten-2,4-diyl, 2-buten-3,4-diyl, and the like. If an alkenylene group is attached to a nitrogen atom, the double bond cannot be alpha to the nitrogen atom. Alkenylene groups may be cyclic or acyclic, branched or unbranched, substituted or unsubstituted. Alkenylene group substituents include, but are not limited to, any of the substituents described herein, that result in the formation of a stable moiety.

As used herein and throughout the entire description, the term "cycloalkyl" or "cycloaliphatic" or "carbocyclic" or "carbocycle" represents cyclic non-aromatic versions of "alkyl" and "alkenyl" with preferably 3 to 14 carbon atoms, such as 3 to 10 carbon atoms, i.e., 3, 4, 5, 6, 7, 8, 9, or 10 carbon atoms, more preferably 3 to 8 carbon atoms, even

more preferably 3 to 7 carbon atoms. In certain embodiments, the cycloalkyl group employed in the invention contains 3-14 carbon atoms ($C_{3-14}$ cycloalkyl). In certain embodiments, the cycloalkyl group employed in the invention contains 3-12 carbon atoms ($C_{3-12}$ cycloalkyl). In another embodiment, the cycloalkyl group employed in the invention contains 3-10 carbon atoms ($C_{3-10}$ cycloalkyl). In another embodiment, the cycloalkyl group employed in the invention contains 3-8 carbon atoms ($C_{3-8}$ cycloalkyl). In another embodiment, the cycloalkyl group employed in the invention contains 3-7 carbon atoms ($C_{3-7}$ cycloalkyl). In another embodiment, the cycloalkyl group employed in the invention contains 3-6 carbon atoms ($C_{3-6}$ cycloalkyl). In another embodiment, the cycloalkyl group employed in the invention contains 3-5 carbon atoms ($C_{3-5}$ cycloalkyl). In another embodiment, the cycloalkyl group employed in the invention contains 3-4 carbon atoms ($C_{3-4}$ cycloalkyl). In another embodiment, the cycloalkyl group employed in the invention contains 3 carbon atoms ($C_3$ cycloalkyl). Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The double bond in a cycloalkenyl group can be present in any position with respect to the carbon atom in position 1 via which the group is bonded to the reminder of the molecule, i.e. for example to the nitrogen atom in the compounds having a structure according to formula I, and cycloalkenyl can thus be cyclopent-1-enyl, cyclopent-2-enyl, cyclopent-3-enyl, cyclohex-1-enyl, cyclohex-2-enyl, cyclohex-3-enyl, cyclohept-1-enyl, cyclohept-2-enyl, cyclohept-3-enyl, cyclohept-4-enyl, for example. In preferred embodiments of the present invention, a cycloalkyl group, such as ($C_3$-$C_7$)-cycloalkyl, in the definition of any group is chosen from a subgroup of any two or more of the said specific cycloalkyl groups, for example from cyclopropyl and cyclobutyl, or from cyclopropyl, cyclobutyl and cyclopentyl, or from cyclopropyl, cyclopentyl and cyclohexyl, or from cyclopentyl and cyclohexyl, or from cyclopentyl, cyclohexyl and cycloheptyl. Similarly, in preferred embodiments a cycloalkenyl group is chosen from a subgroup of any two or more of the said specific cycloalkenyl groups, for example from cyclopentenyl and cyclohexenyl, or from cyclohexenyl and cycloheptenyl, or from cyclopent-1-enyl, cyclopent-2-enyl, cyclohex-1-enyl, cyclohex-2-enyl, cyclohept-1-enyl and cyclohept-2-enyl, or from cyclopent-2-enyl, cyclopent-3-enyl, cyclohex-2-enyl, cyclohex-3-enyl, cyclohept-2-enyl, cyclohept-3-enyl and cyclohept-4-enyl, or from cyclopent-2-enyl and cyclohex-2-enyl, or from cyclopent-2-enyl, cyclohex-2-enyl and cyclohept-2-enyl. Cycloalkyl groups and cycloalkenyl groups generally are optionally substituted by one or more ($C_1$-$C_4$)-alkyl substituents. I.e., they are unsubstituted, i.e. do not carry alkyl substituents, or substituted, for example by 1 , 2, 3 or 4 identical or different ($C_1$-$C_4$)-alkyl substituents, for example by methyl groups and/or ethyl groups and/or isopropyl groups and/or tert-butyl groups, in particular by methyl groups, which substituents can be present in any positions. Examples of alkyl-substituted cycloalkyl groups are 1-methyl-cyclopropyl, 2,2-dimethyl-cyclopropyl, 1-methyl-cyclopentyl, 2,3-dimethyl-cyclopentyl, 1-methyl-cyclohexyl, 4-methyl-cyclohexyl, 4-isopropyl-cyclohexyl, 4-tert-butyl- cyclohexyl and 3,3,5,5-tetramethyl-cyclohexyl. Examples of alkyl-substituted cycloalkenyl groups are 1-methyl-cyclopent-2-enyl, 2-methyl-cyclopent-2-enyl, 3- methyl-cyclopent-2-enyl, 3,4-dimethyl-cyclopent-3-enyl, 1-methyl-cyclohex-2-enyl, 2-methyl-cyclohex-2-enyl, 3-methyl-cyclohex-2-enyl, 4-methyl-cyclohex-2-enyl, 2- methyl-cyclohex-3-enyl, 3-methyl-cyclohex-3-enyl, 4-methyl-cyclohex-3-enyl, 2,3-dimethyl-cyclohex-2-enyl, 4,4-dimethyl-cyclohex-2-enyl, 3,4-dimethyl-cyclohex-3-enyl. Cycloalkyl groups and cycloalkenyl groups generally also are optionally substituted by one or more fluorine atoms. I.e., they are unsubstituted, i.e. do not carry fluorine atoms, or substituted, for example by 1 , 2, 3, 4, 5, 6, 7, 8, 9, 10 or 11 fluorine atoms, preferably by 1 , 2, 3, 4, 5 or 6 fluorine atoms. Cycloalkyl groups and cycloalkenyl groups can also be substituted simultaneously by fluorine and alkyl. The fluorine atoms can be present in any positions and can also be present in an alkyl substituent. Examples of fluoro-substituted cycloalkyl groups are 1-fluoro-cyclopropyl, 2,2- difluoro-cyclopropyl, 3,3-difluoro-cyclobutyl, 1-fluoro-cyclohexyl, 4,4-difluoro- cyclohexyl and 3,3,4,4,5,5-hexafluoro-cyclohexyl. Examples of fluoro-substituted cycloalkenyl groups are 1-fluoro-cyclopent-2-enyl, 1-fluoro-cyclohex-2-enyl, 4-fluoro- cyclohex-2-enyl, 4,4-difluoro-cyclohex-2-enyl. In one embodiment of the invention, cycloalkyl groups are not optionally substituted by substituents chosen from fluorine and ($C_1$-$C_4$)-alkyl. If a cycloalkyl group or cycloalkenyl group can be substituted by further substituents like hydroxy, it can be substituted by one or more such further substituents like hydroxy only and not by substituents chosen from fluorine and ($C_1$-$C_4$)-alkyl, or by one or more such further substituents and simultaneously by one or more substituents chosen from fluorine and ($C_1$-$C_4$)-alkyl. The number of such further substituents like hydroxy which can be present on a cycloalkyl or cycloalkenyl group, preferably is 1, 2 or 3, more preferably 1 or 2, for example 1. The total number of all substituents in a cycloalkyl group or cycloalkenyl group preferably is 1, 2, 3, 4, 5, 6, 7 or 8, more preferably 1 , 2, 3, 4 or 5, for example 1 , 2 or 3. Such further substituents like hydroxy can be present in any positions, provided that the resulting compound is sufficiently stable and is suitable as a subgroup in a pharmaceutical active compound. Preferably, a hydroxy substituent is not present in position 1 of a cycloalkenyl group or cycloalkyl group and in a cycloalkenyl group a hydroxy substituent is not present on a carbon atom which is part of the double bond. Examples of hydroxy-substituted cycloalkyl groups are 3-hydroxy-cyclobutyl, 2- hydroxy-cyclopentyl, 3-hydroxy-cyclopentyl, 3,4-dihydroxy-cyclopentyl, 2-hydroxy-cyclohexyl, 3-hydroxy-cyclohexyl, 4-hydroxy-cyclohexyl, 2,3-dihydroxy-cyclohexyl, 2,4-dihydroxy-cyclohexyl, 3,4-dihydroxy-cyclohexyl, 3,5-dihydroxy-cyclohexyl, 3,4,5- trihydroxy-cyclohexyl, 2-hydroxy-cycloheptyl, 3-hydroxy-cycloheptyl, 4-hydroxy- cycloheptyl. Examples of hydroxy-substituted cycloalkenyl groups are 5-hydroxy- cyclopent-2-enyl, 4-hydroxy-cyclohex-2-enyl, 5-hydroxy-cyclohex-2-enyl, 6-hydroxy- cyclohex-2-enyl, 6-hydroxy-cyclohex-3-enyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include $C_3$-$C_8$-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl. Cycloalkyl group substituents include, but are not limited to, any of the substituents described herein, that result in the formation of a stable moiety.

As used herein and throughout the entire description, the term "cyclopropylene" means a cyclopropyl group as defined above in which one hydrogen atom has been removed resulting in a diradical. The cyclopropylene may link two atoms or moieties via the same carbon atom (1,1-cyclopropylene, i.e., a geminal diradical) or via two carbon atoms (1,2-cyclopropylene).

As used herein and throughout the entire description, the term "aryl" or "aromatic ring" refers to an aromatic mono- or polycyclic ring system having 3-20 ring atoms, of which all the ring atoms are carbon, and which may be substituted or unsubstituted. In certain embodiments of the present invention, "aryl" refers to a mono, bi, or tricyclic $C_4$-$C_{20}$ aromatic ring system having one, two, or three aromatic rings which include, but are not limited to, phenyl, biphenyl, naphthyl, and the like, which may bear one or more substituents. Preferably, the aryl group contains 3 to 14 (e.g., 5 to 10, such as 5, 6, or 10) carbon atoms, more preferably 6 to 10 carbon atoms, which can be arranged in one ring (e.g., phenyl) or two or more condensed rings (e.g., naphthyl). Exemplary aryl groups include cyclopropenylium, cyclopentadienyl, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are substituted or unsubstituted phenyl and naphthyl. Even more preferred is substituted or unsubstituted phenyl In certain embodiments, the aryl group employed in the invention contains 3-20 carbon atoms ($C_{3-20}$ aryl). In certain embodiments, the aryl group employed in the invention contains 3-18 carbon atoms ($C_{3-18}$ aryl). In another embodiment, the aryl group employed in the invention contains 3-16 carbon atoms ($C_{3-16}$ aryl). In another embodiment, the aryl group employed in the invention contains 6-16 carbon atoms ($C_{6-16}$ aryl). In another embodiment, the aryl group employed in the invention contains 7-16 carbon atoms ($C_{7-16}$ aryl). In another embodiment, the aryl group employed in the invention contains 6-14 carbon atoms ($C_{6-14}$ aryl). In another embodiment, the aryl group employed in the invention contains 7-14 carbon atoms ($C_{7-14}$ aryl). In another embodiment, the aryl group employed in the invention contains 6-12 carbon atoms ($C_{6-12}$ aryl). ). In another embodiment, the aryl group employed in the invention contains 7-12 carbon atoms ($C_{7-12}$ aryl). In another embodiment, the aryl group employed in the invention contains 6-11 carbon atoms ($C_{6-11}$ aryl). In another embodiment, the aryl group employed in the invention contains 7-11 carbon atoms ($C_{7-11}$ aryl). In another embodiment, the aryl group employed in the invention contains 6-10 carbon atoms ($C_{6-10}$ aryl). In another embodiment, the aryl group employed in the invention contains 7-10 carbon atoms ($C_{7-10}$ aryl). In another embodiment, the aryl group employed in the invention contains 6-8 carbon atoms ($C_{6-8}$ aryl). In another embodiment, the aryl group employed in the invention contains 6 carbon atoms ($C_6$ aryl). In another embodiment, the aryl group employed in the invention contains 10 carbon atoms ($C_{10}$ aryl).

In substituted aryl groups, the substituents can be present in any positions. In monosubstituted phenyl groups, the substituent can be present in the 2-position, the 3-position or the 4-position. In disubstituted phenyl groups, the substituents can be present in 2, 3-position, 2,4-position, 2,5-position, 2,6-position, 3,4-position or 3,5- position. In trisubstituted phenyl groups, the substituents can be present in 2, 3,4- position, 2,3,5-position, 2,3,6-position, 2,4,5-position, 2,4,6-position or 3,4,5-position. If a phenyl group carries four substituents, of which one, two, three or four substituents can be fluorine atoms, for example, the unsubstituted ring carbon atom can be present in the 2-position, the 3-position or the 4-position. If a polysubstituted phenyl group or heteroaryl group carries different substituents, each substituent can be present in any suitable position, and the present invention comprises all positional isomers. The number of substituents in a substituted phenyl group can be 1, 2, 3, 4 or 5. Preferably, a substituted phenyl group, and likewise a substituted heteroaryl group, carries 1, 2 or 3, in particular 1 or 2, identical or different substituents. In a preferred embodiment the substituted phenyl group carries 1 substituent in 2, 3 or 4-position. In preferred embodiments of the invention, the substituents in substituted phenyl and heteroaryl groups are chosen from any subgroup of the substituents listed in the respective definition, for example by substituents chosen from halogen, substituted or unsubstituted ($C_1$-$C_6$)-alkyl, ($C_1$-$C_4$)-haloalkyl, -$NO_2$, cyano, halogen, substituted or unsubstituted ($C_1$-$C_6$)alkyl-O-, substituted or unsubstituted ($C_6$-$C_{14}$)aryl($C_1$-$C_6$)alkyl-O-, substituted or unsubstituted ($C_3$-$C_{14}$)heterocyclyl, substituted or unsubstituted amino, -C(O)$NH_2$, -C(O)NH(($C_1$-$C_6$)-alkyl), -C(O)N(($C_1$-$C_6$)-alkyl)$_2$, -NH(($C_1$-$C_6$)alkyl-O($C_1$-$C_6$)alkyl)), -NH(($C_1$-$C_6$)alkyl-OH), azido, -NH(C(=O)($C_1$-$C_6$)alkyl) and -N(($C_1$-$C_6$)alkyl)(C(=O)($C_1$-$C_6$)alkyl).

As used herein and throughout the entire description, the term "arylene" refers to an aryl biradical derived from an aryl group, as defined herein, by removal of two hydrogen atoms. Arylene groups may be substituted or unsubstituted. Arylene group substituents include, but are not limited to, any of the substituents described herein, that result in the formation of a stable moiety. Additionally, arylene groups may be incorporated as a linker group into an alkylene, alkenylene, alkynylene, heteroalkylene, heteroalkenylene, or heteroalkynylene group, as defined herein.

As used herein and throughout the entire description, the term "heteroaryl" or "heteroaromatic ring" means an aryl group as defined above in which one or more carbon atoms in the aryl group are replaced by heteroatoms of O, S, or N. Preferably, the heteroaryl group contains 3 to 14 carbon atoms. Preferably, heteroaryl refers to a five or six-membered aromatic monocyclic ring wherein 1, 2, or 3 carbon atoms are replaced by the same or different heteroatoms of O, N, or S. Alternatively, it means an aromatic bicyclic or tricyclic ring system wherein 1, 2, 3, 4, or 5 carbon atoms are replaced with the same or different heteroatoms of O, N, or S. Preferably, in each ring of the heteroaryl group the maximum number of O atoms is 1, the maximum number of S atoms is 1, and the maximum total number of O and S atoms is 2. In certain embodiments, the heteroaryl group employed in the invention is a five membered aromatic monocyclic ring wherein 1, 2, or 3 carbon atoms are replaced by the same or different heteroatoms of O, N, or S. In certain embodiments, the heteroaryl group employed in the invention is a five membered aromatic monocyclic ring wherein 1, 2, or 3 carbon atoms are replaced by the same or different heteroatoms of O. In certain embodiments, the heteroaryl group employed in the invention is a five membered aromatic monocyclic ring wherein 1, 2, or 3 carbon atoms are replaced by the same or different heteroatoms of O and N. In certain embodiments, the heteroaryl group employed in the invention is a five membered aromatic monocyclic ring wherein 1, 2, or 3 carbon atoms are replaced by the same or different heteroatoms of O and S. In certain embodiments, the heteroaryl group employed in the invention is a five membered aromatic monocyclic ring wherein 1, 2, or 3 carbon atoms are replaced by the same or different heteroatoms of N and S. In certain embodiments, the heteroaryl group employed in the invention is a six membered aromatic monocyclic ring wherein 1, 2, or 3 carbon atoms are replaced by the same or different heteroatoms of O, S or N. In certain embodiments, the heteroaryl group employed in the invention is a six membered aromatic monocyclic ring wherein 1, 2, or 3 carbon atoms are replaced by N. In certain embodiments, the heteroaryl group employed in the invention is an aromatic bicyclic system wherein 1, 2, 3, 4, or 5 carbon atoms are replaced with the same or different heteroatoms of O, N, or S. Exemplary heteroaryl groups include furanyl, thienyl, oxazolyl, isoxazolyl, oxadiazolyl (1,2,5-and 1,2,3-), pyrrolyl, imidazolyl, pyrazolyl, triazolyl (1,2,3-and 1,2,4-), tetrazolyl, thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), benzofuranyl (1- and 2-), indolyl, azaindolyl (4-, 5-6- and 7-), diazaindolyl, isoindolyl, benzothienyl (1- and 2-), 1H-indazolyl, benzimidazolyl, benzoxazolyl, indoxazinyl, benzisoxazolyl, benzothiazolyl, benzisothiazolyl, benzotriazolyl, quinolinyl, isoquinolinyl, benzodiazinyl, quinoxalinyl, quinazolinyl, benzotriazinyl (1,2,3- and 1,2,4-benzotriazinyl), pyridazinyl, phenoxazinyl, thiazolopyridinyl, pyrrolothiazolyl, phenothiazinyl, isobenzofuranyl, chromenyl, xanthenyl, phenoxathiinyl, pyrrolizinyl, indolizinyl, indazolyl, purinyl, quinolizinyl, phthalazinyl, naphthyridinyl (1,5-, 1,6-, 1,7-, 1,8-, and 2,6-), cinnolinyl, pteridinyl, carbazolyl, phenanthridinyl, acridinyl, perimidinyl, phenanthrolinyl (1,7-, 1,8-, 1,10-, 3,8-, and 4,7-), phenazinyl, oxazolopyridinyl, isoxazolopyridinyl, pyrrolooxazolyl, pyrrolopyrrolyl, and the like, which may bear one or more substituents. Exemplary 5- or 6-membered heteroaryl groups include furanyl, thienyl, oxazolyl, isoxazolyl, oxadiazolyl (1,2,5- and 1,2,3-), pyrrolyl, imidazolyl, pyrazolyl, triazolyl (1,2,3- and 1,2,4-), thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), and pyridazinyl. Exemplary bicyclic heteroaryl groups 7-azaindolyl, 6-azaindolyl, 5-azaindolyl, 4-azaindolyl, benzofuranyl and indolyl. Heteroaryl substituents include, but are not limited to, any of the substituents described herein, that result in the formation of a stable moiety.

In substituted heteroaryl groups, the substituents can be present in any positions, for example in a thiophen-2-yl group or a furan-2-yl group in the 3-position and/or in the 4-position and/or in the 5-position, in a thiophen-3-yl group or a furan-3-yl group in the 2-position and/or in the 4-position and/or in the 5-position, in a pyridin-2-yl group in the 3-position and/or in the 4-position and/or in the 5-position and/or in the 6- position, in a pyridin-3-yl group in the 2-position and/or in the 4-position and/or in the 5-position and/or in the 6-position, in a pyridin-4-yl group in the 2-position and/or in the 3-position and/or in the 5-position and/or in the 6-position, in a 1H-pyrazol-4-yl group in the 3-position and/ or in the 5-position and/or in the 1-position, in a 1H-pyrazol-1-yl group in the 3-position and/ or in the 5-position and/or in the 4-position, in a 1 H-indol-1-yl group in the 2-position and/or in the 3-position and/or in the 4-position and/or in the 5-position and/or in the 6-position and/or in the 7-position, in a 1 H-indol-3-yl group in the 1-position and/or in the 2-position and/or in the 4-position and/or in the 5-position and/or in the 6-position and/or in the 7-position. The substituted heteroaryl groups can be monosubstituted or polysubstituted, i.e. the carry more than one substituent. In preferred embodiments a 1 H-indol-1-yl group is substituted in 2-position and 3-position and/or in 6-position and 7-position. In other preferred embodiments a 1 H-indol-3-yl group is substituted in 1-position and 7-position and/or in 6-position and 7-position. Preferably, a substituted heteroaryl group is substituted by one, two or three, in particular one or two, for example one, identical or different substituents. If a ring nitrogen atom is present which can carry a hydrogen atom or a substituent, the substituent on this nitrogen atom can be a methyl group, an ethyl group, a propyl group or a tert-butyl group, for example, which groups can also be monosubstituted or polysubstituted by fluorine. Generally, suitable ring nitrogen atoms in an aromatic ring of a heteroaryl group, for example the nitrogen atom in a pyridinyl group or a nitrogen atom in a [1,2,5]oxadiazolyl group, can also carry an oxido substituent $-O^-$ and compounds of formula I, formula II, formula III and formula IV thus be present in the form of an N-oxide.

As used herein and throughout the entire description, the term "heteroarylene" refers to a biradical derived from a heteroaryl group, as defined herein, by removal of two hydrogen atoms. Heteroarylene groups may be substituted or

unsubstituted. Additionally, heteroarylene groups may be incorporated as a linker group into an alkylene, alkenylene, alkynylene, heteroalkylene, heteroalkenylene, or heteroalkynylene group, as defined herein. Heteroarylene group substituents include, but are not limited to, any of the substituents described herein, that result in the formation of a stable moiety.

As used herein and throughout the entire description, the terms "arylalkyl" and "heteroarylalkyl" are meant to include those radicals in which an aryl group and heteroaryl group, respectively, is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like). Preferably the Arylalkyl is a substituted or unsubstituted ($C_6$-$C_{14}$)aryl($C_1$-$C_6$)alkyl Preferably the Arylalkyl is a substituted or unsubstituted ($C_6$-$C_{10}$)aryl($C_1$-$C_6$)alkyl. Preferably the Heteroarylalkyl is a substituted or unsubstituted ($C_3$-$C_{14}$)heteroaryl($C_1$-$C_6$)alkyl. Preferably the Heteroarylalkyl is a substituted or unsubstituted ($C_3$-$C_{10}$)heteroaryl($C_1$-$C_6$)alkyl. In some embodiments the alkyl chain is a linear. In some embodiments the alkyl chain is branched. In some embodiments the alkyl chain is substituted. In some embodiments the alkyl chain is unsubstituted. In some embodiments the alkyl chain is linear and substituted or unsubstituted. In some embodiments the alkyl chain is branched and substituted or unsubstituted.

As used herein and throughout the entire description, the term "heterocyclyl" or "heterocyclic ring" or "heterocycle refers to a cyclic heteroaliphatic group. A heterocyclic group refers to a non-aromatic, partially unsaturated or fully saturated, 3- to 10-membered ring system, which includes single rings of 3 to 8 atoms in size, and bi- and tri-cyclic ring systems which may include aromatic five- or six-membered aryl or heteroaryl groups fused to a non-aromatic ring. The heterocyclic group may be substituted or unsubstituted. These heterocyclic rings include those having from one to three heteroatoms independently selected from oxygen, sulfur, and nitrogen, in which the nitrogen and sulfur heteroatoms may optionally be oxidized and the nitrogen heteroatom may optionally be quaternized. In certain embodiments, the term heterocyclic refers to a non-aromatic 5-, 6-, or 7-membered ring or polycyclic group wherein at least one ring atom is a heteroatom selected from O, S, and N (wherein the nitrogen and sulfur heteroatoms may be optionally oxidized), and the remaining ring atoms are carbon, the radical being joined to the rest of the molecule via any of the ring atoms. Heterocycyl groups include, but are not limited to, a bi-or tri-cyclic group, comprising fused five, six, or seven-membered rings having between one and three heteroatoms independently selected from the oxygen, sulfur, and nitrogen, wherein (i) each 5-membered ring has 0 to 2 double bonds, each 6-membered ring has 0 to 2 double bonds, and each 7-membered ring has 0 to 3 double bonds, (ii) the nitrogen and sulfur heteroatoms may be optionally oxidized, (iii) the nitrogen heteroatom may optionally be quaternized, and (iv) any of the above heterocyclic rings may be fused to an aryl or heteroaryl ring. Preferably, in each ring of the heterocyclyl group the maximum number of O atoms is 1, the maximum number of S atoms is 1, and the maximum total number of O and S atoms is 2. The term "heterocyclyl" is also meant to encompass partially or completely hydrogenated forms (such as dihydro, tetrahydro or perhydro forms) of the above-mentioned heteroaryl groups. Exemplary heterocyclyl groups include morpholino, isochromanyl, chromanyl, pyrrolidinyl, imidazolidinyl, pyrazolidinyl, piperidinyl, piperazinyl, indolinyl, isoindolinyl, di- and tetrahydrofuranyl, di- and tetrahydrothienyl, di- and tetrahydrooxazolyl, di- and tetrahydroisoxazolyl, di- and tetrahydrooxadiazolyl (1,2,5- and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydrothiazolyl, di- and tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), di- and tetrahydrobenzofuranyl (1- and 2-), di- and tetrahydroindolyl, di- and tetrahydroisoindolyl, di- and tetrahydrobenzothienyl (1- and 2), di- and tetrahydro-1H-indazolyl, di- and tetrahydrobenzimidazolyl, di- and tetrahydrobenzoxazolyl, di-and tetrahydroindoxazinyl, di- and tetrahydrobenzisoxazolyl, di- and tetrahydrobenzothiazolyl, di-and tetrahydrobenzisothiazolyl, di- and tetrahydrobenzotriazolyl, di- and tetrahydroquinolinyl, di-and tetrahydroisoquinolinyl, di- and tetrahydrobenzodiazinyl, di- and tetrahydroquinoxalinyl, di-and tetrahydroquinazolinyl, di- and tetrahydrobenzotriazinyl (1,2,3- and 1,2,4-), di- and tetrahydropyridazinyl, di- and tetrahydrophenoxazinyl, di- and tetrahydrothiazolopyridinyl (such as 4,5,6-7-tetrahydro[1,3]thiazolo[5,4-c]pyridinyl or 4,5,6-7-tetrahydro[1,3]thiazolo[4,5-c]pyridinyl, e.g., 4,5,6-7-tetrahydro[1,3]thiazolo[5,4-c]pyridin-2-yl or 4,5,6-7-tetrahydro[1,3]thiazolo[4,5-c]pyridin-2-yl), di- and tetrahydropyrrolothiazolyl (such as 5,6-dihydro-4H-pyrrolo[3,4-d][1,3]thiazolyl), di- and tetrahydrophenothiazinyl, di- and tetrahydroisobenzofuranyl, di- and tetrahydrochromenyl, di- and tetrahydroxanthenyl, di- and tetrahydrophenoxathiinyl, di- and tetrahydropyrrolizinyl, di- and tetrahydroindolizinyl, di- and tetrahydroindazolyl, di- and tetrahydropurinyl, di- and tetrahydroquinolizinyl, di- and tetrahydrophthalazinyl, di- and tetrahydronaphthyridinyl (1,5-, 1,6-, 1,7-, 1,8-, and 2,6-), di- and tetrahydrocinnolinyl, di- and tetrahydropteridinyl, di- and tetrahydrocarbazolyl, di- and tetrahydrophenanthridinyl, di- and tetrahydroacridinyl, di- and tetrahydroperimidinyl, di- and tetrahydrophenanthrolinyl (1,7-, 1,8-, 1,10-, 3,8-, and 4,7-), di- and tetrahydrophenazinyl, di- and tetrahydrooxazolopyridinyl, di- and tetrahydroisoxazolopyridinyl, di- and tetrahydropyrrolooxazolyl, and di- and tetrahydropyrrolopyrrolyl. Exemplary 5- or 6-memered heterocyclyl groups include morpholino, pyrrolidinyl, imidazolidinyl, pyrazolidinyl, piperidinyl, piperazinyl, di- and tetrahydrofuranyl, di- and tetrahydrothienyl, di- and tetrahydrooxazolyl, di- and tetrahydroisoxazolyl, di- and tetrahydrooxadiazolyl (1,2,5- and 1,2,3-), dihydropyrrolyl, dihydroimidazolyl, dihydropyrazolyl, di-and tetrahydrotriazolyl (1,2,3- and 1,2,4-), di- and tetrahydrothiazolyl, di- and tetrahydroisothiazolyl, di- and

tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), di- and tetrahydropyridazinyl and the like, which may bear one or more substituents. In preferred embodiments the heterocyclyl group is morpholino, wherein the morpholino moiety is bound to the remainder of the molecule via the N-atom. In some embodiments, the heterocyclyl group is a substituted or unsubstituted ($C_3$-$C_{14}$)heterocyclyl group, wherein 1, 2, 3, 4, or 5 carbon atoms are replaced with the same or different heteroatoms of O, N, or S. Heterocyclyl group substituents include, but are not limited to, any of the substituents described herein, that result in the formation of a stable moiety. A substituted heterocyclyl group can be substituted in any positions, provided that the resulting compound is sufficiently stable and is suitable as a pharmaceutical active compound. The prerequisite that a specific group and a compound of any one of formulas I, II, III and IV is sufficiently stable and suitable as a pharmaceutical active compound, applies in general with respect to all groups in the compounds of any one of formulas I, II, III and IV. In some embodiments the heterocyclyl group may be substituted by one or more identical or different substituents chosen from halogen, hydroxyl, $-NO_2$, cyano, ($C_1$-$C_6$)alkyl-O- and ($C_1$-$C_6$)alkyl-$S(O)_m$-. In one embodiment of the invention the number m is chosen from 0 and 2, wherein all numbers m are independent of each other and can be identical or different.

As used herein and throughout the entire description, the term "halogen" or "halo" means fluoro, chloro, bromo, or iodo.

As used herein and throughout the entire description, the term "cyano" mean -CN.

As used herein and throughout the entire description, the term "azido" means $N_3$.

As used herein and throughout the entire description, the term "amino" includes unsubstituted amino (i.e., the group $-NH_2$) and substituted amino (i.e., mono- or disubstituted amino, wherein one or two of the hydrogen atoms have been replaced with a group other than hydrogen). Thus, the term "amino" means the group $-N(R^a)(R^b)$, wherein $R^a$ and $R^b$ are, in each case, independently selected from the group consisting of -H, alkyl, alkenyl, alkynyl, cycloalkyl, aryl, heteroaryl, and heterocyclyl, or $R^a$ and $R^b$ may join together with the nitrogen atom to which they are attached to form the group $-N=CR^cR^d$, wherein each of the alkyl, alkenyl, alkynyl, cycloalkyl, aryl, heteroaryl, and heterocyclyl groups is optionally substituted with one or more (such as 1 to the maximum number of hydrogen atoms bound to the alkyl, alkenyl, alkynyl, cycloalkyl, aryl, heteroaryl, or heterocyclyl group, e.g., 1, 2, 3, 4, 5, 6, 7, 8, 9, or up to 10, such as between 1 to 5, 1 to 4, or 1 to 3, or 1 or 2) independently selected $R^g$; $R^c$ and $R^d$ are independently selected from the group consisting of -H, alkyl, alkenyl, alkynyl, cycloalkyl, aryl, heteroaryl, heterocyclyl, and $-NH_yR^e_{2-y}$, or $R^c$ and $R^d$ may join together with the atom to which they are attached to form a ring which is optionally substituted with one or more (such as 1 to the maximum number of hydrogen atoms bound to the ring, e.g., 1, 2, 3, 4, 5, 6, 7, 8, 9, or up to 10, such as between 1 to 5, 1 to 4, or 1 to 3, or 1 or 2) independently selected $R^g$, wherein each of the alkyl, alkenyl, alkynyl, cycloalkyl, aryl, heteroaryl, and heterocyclyl groups is optionally substituted with one or more (such as 1 to the maximum number of hydrogen atoms bound to the alkyl, alkenyl, alkynyl, cycloalkyl, aryl, heteroaryl, or heterocyclyl group, e.g., 1, 2, 3, 4, 5, 6, 7, 8, 9, or up to 10, such as between 1 to 5, 1 to 4, or 1 to 3, or 1 or 2) independently selected $R^g$; y is an integer from 0 to 2; $R^e$ and $R^g$ are each independently selected from the group consisting of alkyl, alkenyl, alkynyl, cycloalkyl, aryl, heteroaryl, and heterocyclyl. In some embodiments $R^a$ and $R^b$ are, in each case, independently selected from the group consisting of hydrogen, alkyl, cycloalkyl, carboxyalkyl, alkoxycarbonyl, aryl, heteroaryl and heterocyclyl. Unless otherwise constrained by the definition, all substituents may optionally be further substituted by 1, 2 or 3 substituents selected from alkyl, carboxy, carboxyalkyl, aminocarbonyl, hydroxy, alkoxy, halogen, $CF_3$, amino, substituted amino, cyano, and $-S(O)_pR^h$, where $R^h$ is alkyl, aryl, or heteroaryl and p is 0, 1 or 2.

As used herein and throughout the entire description, the term "optionally substituted" or "substituted" indicates that one or more (such as 1 to the maximum number of hydrogen atoms bound to a group, e.g., 1, 2, 3, 4, 5, 6, 7, 8, 9, or up to 10, such as between 1 to 5, 1 to 4, or 1 to 3, or 1 or 2) hydrogen atom(s) may be replaced with a group different from hydrogen such as ($C_1$-$C_6$)alkyl, ($C_1$-$C_6$)heteroalkyl, ($C_1$-$C_6$)haloalkyl; ($C_2$-$C_6$)alkenyl, ($C_2$-$C_6$)alkynyl, ($C_3$-$C_8$)cycloalkyl, ($C_6$-$C_{10}$)aryl, ($C_6$-$C_{10}$)aryl($C_1$-$C_6$)alkyl, ($C_3$-$C_{10}$)heteroaryl, ($C_3$-$C_{10}$)heteroaryl($C_1$-$C_6$)alkyl, halogen, -CN, $-NO_2$, $-OR^{161}$, $-N(R^{162})(R^{163})$, $-N(R^{161})(OR^{161})$, $-S(O)_{0-2}R^{161}$, $-S(O)_{1-2}OR^{161}$, $-OS(O)_{1-2}R^{161}$, $-OS(O)_{1-2}OR^{161}$, $-S(O)_{1-2}N(R^{162})(R^{163})$, $-OS(O)_{1-2}N(R^{162})(R^{163})$, $-N(R^{161})S(O)_{1-2}R^{161}$, $-NR^{161}S(O)_{1-2}OR^{161}$, $-NR^{161}S(O)_{1-2}N(R^{162})(R^{163})$, $-C(=W)R^{161}$, $-C(=W)WR^{161}$, $-WC(=W)R^{161}$, and $-WC(=W)WR^{161}$; wherein $R^{161}$, $R^{162}$, and $R^{163}$ are independently selected from the group consisting of -H, alkyl, alkenyl, alkynyl, cycloalkyl, aryl, heteroaryl, and heterocyclyl, preferably wherein $R^{161}$, $R^{162}$, and $R^{163}$ are independently selected from the group consisting of -H, $C_{1-6}$ alkyl, $C_{2-6}$ alkenyl, $C_{2-6}$ alkynyl, 3- to 7-membered cycloalkyl, 5- or 6-membered aryl, 5-or 6-membered heteroaryl, and 3- to 7-membered heterocyclyl; $R^{164}$ is independently selected from the group consisting of -H, alkyl, alkenyl, alkynyl, cycloalkyl, aryl, heteroaryl, heterocyclyl, and $-OR^{161}$; W is independently selected from O, S, and $N(R^{164})$.

As used herein and throughout the entire description, the term "optional" or "optionally" that the subsequently described event, circumstance or condition may or may not occur, and that the description includes instances where said event, circumstance, or condition occurs and instances in which it does not occur.

As used herein and throughout the entire description, the term "solvate" refers to an addition complex of a

dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

As used herein and throughout the entire description, the term "pharmaceutically acceptable salt" refers to a salt that retains the desired biological activity of the parent compound and does not impart any undesired toxicological effects (see e.g., Berge, S. M., et al. (1977) J. Pharm. Sci. 66: 1-19). Physiologically acceptable salts of the compounds of the present invention are in particular salts with a nontoxic salt component and preferably are pharmaceutically utilizable salts. They can contain inorganic or organic salt components. Such salts can be formed, for example, from compounds of the present invention which contain an acidic group, for example a carboxylic acid group (HO-CO-) or a sulfonic acid group ($HO-S(O)_2-$) and nontoxic inorganic or organic bases. Suitable bases are, for example, alkali metal compounds or alkaline earth metal compounds, such as sodium hydroxide, potassium hydroxide, sodium carbonate or sodium hydrogencarbonate, or ammonia, organic amino compounds and quaternary ammonium hydroxides. Reactions of compounds of the present invention with bases for the preparation of the salts are in general carried out according to customary procedures in a solvent or diluent. On account of the physiological and chemical stability, advantageous salts of acidic groups are in many cases sodium, potassium, magnesium or calcium salts or ammonium salts which can also carry one or more organic groups on the nitrogen atom. Compounds of the present invention which contain a basic, i.e. protonatable, group, for example an amino group or another basic heterocycle, can be present in the form of their acid addition salts with physiologically acceptable acids, for example as salt with hydrogen chloride, hydrogen bromide, phosphoric acid, sulfuric acid, acetic acid, benzoic acid, methanesulfonic acid, p-toluenesulfonic acid, which in general can be prepared from the compounds of the present invention by reaction with an acid in a solvent or diluent according to customary procedures. As usual, in particular in the case of acid addition salts of a compound containing two or more basic groups, in an obtained salt the ratio of the salt components can deviate upward or downward from the stoichiometric ratio, such as the molar ratio 1:1 or 1:2 in the case of the acid addition salt of a compound of the present invention containing one or two basic groups with a monovalent acid, and vary depending on the applied conditions. The present invention comprises also salts containing the components in a non-stoichiometric ratio, and an indication that an acid addition salt of a compound of the present invention contains an acid in equimolar amount, for example, also allows for a lower or higher amount of acid in the obtained salt, for example about 0.8 or about 1.1 mol of acid per mol of compound of the present invention. If the compounds of the present invention simultaneously contain an acidic and a basic group in the molecule, the invention also includes internal salts (betaines, zwitterions) in addition to the salt forms mentioned. The present invention also comprises all salts of the compounds of the present invention which, because of low physiological tolerability, are not directly suitable for use as a pharmaceutical, but are suitable as intermediates for chemical reactions or for the preparation of physiologically acceptable salts, for example by means of anion exchange or cation exchange. A subject of the present invention also are solvates of the compounds of the present invention and their salts, such as hydrates and adducts with alcohols like ($C_1$-$C_4$)-alkanols, in particular physiologically acceptable solvates, as well as active metabolites of compounds of the present invention.

As used herein and throughout the entire description, the term "pharmaceutically acceptable" may in particular mean approved by a regulatory agency or other generally recognized pharmacopoeia for use in animals, and more particularly in humans.

The present invention comprises all stereoisomeric forms of the compounds of the formula I, for example, all possible enantiomers and diastereomers including cis/trans isomers. The invention likewise comprises mixtures of two or more stereoisomeric forms, for example mixtures of enantiomers and/or diastereomers including cis/trans isomers, in all ratios. Asymmetric centers contained in the compounds of the formula I for example in unsubstituted or substituted alkyl groups or in the stereogenic carbons $CR^4R^5$ and $CR^2R^3$, in case that $R^4$ and $R^5$ or $R^2$ and $R^3$ are not equal chemical groups, i.e. not both methyl, for example, depicted in formula I, can all independently of one another have the S configuration or the R configuration. The invention relates to enantiomers, both the levorotatory and the dextrorotatory antipode, in enantiomerically pure form and substantially enantiomerically pure form and in the form of racemates and in the form of mixtures of the two enantiomers in all ratios. The invention likewise relates to diastereomers in the form of pure and substantially pure diastereomers and in the form of mixtures of two or more diastereomers in all ratios. The invention also comprises all cis/trans isomers of the compounds of the formula I, for example, in pure form and substantially pure form and in the form of mixtures of the cis isomer and the trans isomer in all ratios. Cis/trans isomerism can occur in substituted cycloalkane rings for example. The preparation of individual stereoisomers, if desired, can be carried out by resolution of a mixture according to customary methods, for example by chromatography or crystallization, or by use of stereochemically uniform starting compounds in the synthesis or by stereoselective reactions. Optionally, before a separation of stereoisomers a derivatization can be carried out. The separation of a mixture of stereoisomers can be carried out at the stage of the compound of the formula I or at the stage of an intermediate in the course of the synthesis. The invention also comprises all tautomeric forms of the compounds of the present invention.

In one aspect, the present invention provides compounds having a structure according to formula I, in any of

their stereoisomeric forms or a mixture of stereoisomeric forms in any ratio, and the physiologically acceptable salts thereof, and the physiologically acceptable solvates or hydrates of any of them,

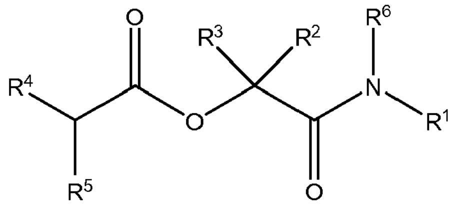

(I)

wherein,

$R^1$ is selected from the group consisting of substituted or unsubstituted alkyl, substituted or unsubstituted cycloalkyl, substituted or unsubstituted aryl, substituted or unsubstituted heteroaryl and substituted or unsubstituted heteroarylalkyl;
$R^2$ and $R^3$ are independently selected from the group consisting of hydrogen and substituted or unsubstituted alkyl; or $R^2$ and $R^3$ are substituted or unsubstituted alkyl and join together with the carbon atom to which they are attached to form a carbocyclic ring which is optionally substituted;
$R^4$ is selected from the group consisting of substituted or unsubstituted aryl, substituted or unsubstituted heteroaryl and substituted or unsubstituted heteroarylalkyl;
$R^5$ is hydrogen or substituted or unsubstituted alkyl;
$R^6$ is hydrogen or substituted or unsubstituted alkyl;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In some embodiments $R^1$ is selected from the group consisting of substituted or unsubstituted ($C_1$-$C_6$)alkyl, substituted or unsubstituted ($C_3$-$C_8$)cycloalkyl, substituted or unsubstituted ($C_6$-$C_{14}$)aryl, substituted or unsubstituted ($C_3$-$C_{14}$)heteroaryl and substituted or unsubstituted ($C_3$-$C_{14}$)heteroaryl($C_1$-$C_6$)alkyl, in other embodiments from substituted or unsubstituted ($C_3$-$C_8$)cycloalkyl, substituted or unsubstituted ($C_6$-$C_{14}$)aryl and substituted or unsubstituted ($C_3$-$C_{14}$)heteroaryl, in other embodiments from unsubstituted ($C_3$-$C_8$)cycloalkyl, substituted or unsubstituted ($C_6$-$C_{14}$)aryl, substituted or unsubstituted ($C_3$-$C_{14}$)heteroaryl, in other embodiments from substituted or unsubstituted ($C_6$-$C_{14}$)aryl and substituted or unsubstituted ($C_3$-$C_{14}$)heteroaryl. In some embodiments $R^1$ is substituted or unsubstituted ($C_6$-$C_{14}$)aryl, preferably wherein aryl is selected from the group of substituted or unsubstituted naphthyl and substituted or unsubstituted phenyl, more preferably wherein aryl is substituted or unsubstituted phenyl, in other embodiments substituted or unsubstituted ($C_3$-$C_{14}$)heteroaryl. The alkyl, cycloalkyl, aryl, heteroaryl and heteroarylalkyl may be substituted by one or more identical or different substituents selected from the group consisting of substituted or unsubstituted ($C_3$-$C_{14}$)heterocyclyl, substituted or unsubstituted amino, substituted or unsubstituted ($C_1$-$C_6$)alkyl, substituted or unsubstituted ($C_1$-$C_6$)haloalkyl, -$OR^{26}$, halogen and -C(O)$NR^{27}R^{28}$, substituted or unsubstituted ($C_6$-$C_{14}$)aryl, substituted or unsubstituted ($C_3$-$C_{14}$)heteroaryl($C_1$-$C_6$)alkyl, substituted or unsubstituted ($C_6$-$C_{14}$)aryl($C_1$-$C_6$)alkyl, cyano, -$NO_2$ and azido, preferably ($C_3$-$C_{14}$)heterocyclyl, substituted or unsubstituted amino, ($C_1$-$C_6$)alkyl, ($C_1$-$C_6$)haloalkyl, -$OR^{26}$, halogen and -C(O)$NR^{27}R^{28}$, substituted or unsubstituted ($C_6$-$C_{14}$)aryl, substituted or unsubstituted ($C_3$-$C_{14}$)heteroaryl($C_1$-$C_6$)alkyl, substituted or unsubstituted ($C_6$-$C_{14}$)aryl($C_1$-$C_6$)alkyl, cyano, -$NO_2$ and azido, wherein $R^{26}$, $R^{27}$ and $R^{28}$ are independently selected from the group consisting of hydrogen, substituted or unsubstituted ($C_1$-$C_6$)alkyl and substituted or unsubstituted ($C_2$-$C_6$)alkenyl, preferably from hydrogen and substituted or unsubstituted ($C_1$-$C_6$)alkyl. Preferably the number of substituents in a substituted group $R^1$ is one, two, three or four, more preferably one, two or three, for example one or two. The substituents in a substituted group $R^1$ can be present on carbon atoms in any positions as indicated above with respect to substituted alkyl, cycloalkyl, aryl, heteroaryl and heteroarylalkyl groups in general. In some embodiments $R^1$ is selected from the group consisting of 3,5-dimethyl-1-phenyl-1H-pyrazol-4-yl, 3,5-dimethyl-1-(m-tolyl)-1 H-pyrazol-4-yl, 3,5-dimethyl-1-(4-(trifluoromethyl)phenyl)-1 H-pyrazol-4-yl, 3,5-dimethyl-1-(2-nitrophenyl)-1H-pyrazol-4-yl, 1-(4-cyanophenyl)-3,5-dimethyl-1H-pyrazol-4-yl, 1-(4-methoxybenzyl)-3,5-dimethyl-1 H-pyrazol-4-yl, 3,5-dimethyl-1-(2-(trifluoromethyl)phenyl)-1 H-pyrazol-4-yl, 3,5-dimethyl-1-(p-tolyl)-1H-pyrazol-4-yl, 3,5-dimethyl-1-(4-nitrophenyl)-1H-pyrazol-4-yl, 3,5-dimethyl-1-phenyl-1H-pyrazol-4-yl, 1-(4-((3-hydroxybutyl)amino)phenyl)-3,5-dimethyl-1H-pyrazol-4-yl, 1-(4-aminophenyl)-3,5-dimethyl-1 H-pyrazol-4-yl, 1-(2-aminophenyl)-3,5-dimethyl-1 H-pyrazol-4-yl, 1-phenyl-1H-pyrazol-4-yl, 1,3,5-trimethyl-1 H-pyrazol-4-yl, phenyl, 2-methylphenyl, 4-morpholinophenyl, 2-(allyloxy)phenyl, 2-chlorophenyl, 3-methylphenyl, 2-methoxyphenyl, 3-methoxyphenyl, 4-methoxyphenyl, 4-methylphenyl, 3-carbamoylphenyl, *tert*-butyl, cyclopentyl, 5-fluoropyridin-2-yl, 1H-imidazol-2-yl and pyrimidin-5-yl, preferably 3,5-dimethyl-1-phenyl-1H-pyrazol-4-yl, 3,5-dimethyl-1-(m-tolyl)-1H-pyrazol-4-yl, 3,5-dimethyl-1-(4-(trifluoromethyl)phenyl)-1H-pyrazol-4-yl, 3,5-dimethyl-1-(2-nitrophenyl)-1H-pyrazol-4-yl, 1-(4-cyanophenyl)-3,5-dimethyl-1H-pyrazol-4-yl, 1-(4-methoxybenzyl)-3,5-dimethyl-1H-pyrazol-4-yl, 3,5-dimethyl-1-(2-(trifluoromethyl)phenyl)-1H-pyrazol-4-yl, 3,5-dimethyl-1-(p-tolyl)-1H-pyrazol-4-yl, 3,5-dimethyl-1-(4-nitrophenyl)-1H-pyrazol-4-yl, 3,5-dimethyl-1-phenyl-1H-pyrazol-4-yl, 1-(4-((3-hydroxybutyl)amino)phenyl)-3,5-dimethyl-1H-pyrazol-4-yl, 1-(4-aminophenyl)-3,5-dimethyl-1 H-pyrazol-4-yl, 1-(2-aminophenyl)-3,5-dimethyl-1 H-pyrazol-4-yl, phenyl, 2-methylphenyl, 4-morpholinophenyl, 2-(allyloxy)phenyl, 2-chlorophenyl, 3-methylphenyl, 2-methoxyphenyl, 3-methoxyphenyl, 4-methoxyphenyl, 4-methylphenyl, 3-carbamoylphenyl and 1-phenyl-1 H-pyrazol-4-yl. In some embodiments $R^1$ is selected from the group consisting of 3,5-dimethyl-1-phenyl-1 H-pyrazol-4-yl, 3,5-dimethyl-1-(m-tolyl)-1 H-pyrazol-4-yl, 3,5-dimethyl-1-(4-(trifluoromethyl)phenyl)-1 H-pyrazol-4-yl, 3,5-dimethyl-1-(2-nitrophenyl)-1 H-pyrazol-4-yl, 1-(4-cyanophenyl)-3,5-dimethyl-1 H-pyrazol-4-yl, 1-(4-methoxybenzyl)-3,5-dimethyl-1 H-pyrazol-4-yl, 3,5-dimethyl-1-(2-(trifluoromethyl)phenyl)-1H-pyrazol-4-yl, 3,5-dimethyl-1-(p-tolyl)-1 H-pyrazol-4-yl, 3,5-dimethyl-1-(4-nitrophenyl)-1 H-pyrazol-4-yl, 3,5-dimethyl-1-phenyl-1 H-pyrazol-4-yl, 1-(4-((3-hydroxybutyl)amino)phenyl)-3,5-dimethyl-1 H-pyrazol-4-yl, 1-(4-aminophenyl)-3,5-dimethyl-1 H-pyrazol-4-yl and 1-(2-aminophenyl)-3,5-dimethyl-1H-pyrazol-4-yl. In some embodiments $R^1$ is 3,5-dimethyl-1-phenyl-1H-pyrazol-4-yl. In some embodiments $R^1$ is selected from the group consisting of phenyl, 2-methylphenyl, 4-morpholinophenyl, 2-(allyloxy)phenyl, 2-chlorophenyl, 3-methylphenyl, 2-methoxyphenyl, 3-methoxyphenyl, 4-methoxyphenyl, 4-methylphenyl and 3-carbamoylphenyl. In some embodiments $R^1$ is selected from the group consisting of 5-fluoropyridin-2-yl, 1H-imidazol-2-yl and pyrimidin-5-yl. In some embodiments $R^1$ is 1-phenyl-1H-pyrazol-4-yl. In some embodiments $R^1$ is 1,3,5-trimethyl-1 H-pyrazol-4-yl. In some embodiments $R^1$ is *tert*-butyl and/or cyclopentyl.

In some embodiments $R^2$ and $R^3$ are independently selected from the group consisting of hydrogen and substituted or unsubstituted $(C_1\text{-}C_6)$alkyl, preferably hydrogen. In some embodiments $R^2$ is hydrogen and $R^3$ is substituted or unsubstituted $(C_1\text{-}C_6)$alkyl, preferably unsubstituted $(C_1\text{-}C_6)$alkyl; more preferably methyl. In some embodiments $R^2$ and $R^3$ are methyl, in other embodiments $R^2$ is methyl and $R^3$ is substituted or unsubstituted $(C_1\text{-}C_6)$alkyl, preferably substituted or unsubstituted $(C_1\text{-}C_4)$alkyl. In some embodiments $R^2$ and $R^3$ are substituted or unsubstituted $(C_1\text{-}C_6)$alkyl and join together with the carbon atom to which they are attached to form a carbocyclic ring which is optionally substituted, preferably unsubstituted $(C_1\text{-}C_6)$alkyl. Said carbocyclic ring may be a cycloalkane ring. The $(C_1\text{-}C_6)$alkyl group may be substituted by one or more identical or different substituents selected from the group consisting of halogen, cyano, -O-(($C_1$-$C_4$)-alkyl) and ($C_1$-$C_4$)-alkyl, preferably halogen, more preferably fluorine. Preferably the number of substituents in a substituted group $R^2$ and/or $R^3$ is one, two, three or four, more preferably one, two or three, for example one or two. The substituents in a substituted group $R^2$ and/or $R^3$ can be present on carbon atoms in any positions as indicated above with respect to substituted alkyl group in general. The said cycloalkane ring, like a cycloalkane ring in the compounds of, for example, formula I in general, can carry one or more ($C_1$-$C_4$)-alkyl groups, for example one, two, three or four methyl groups, and/or one or more, for example one, two, three or four fluorine atoms. Preferably the said cycloalkane ring is a cyclopropane, cyclobutane, cyclopentane or cyclohexane ring which can all be unsubstituted or substituted by alkyl and/or fluorine as indicated. In one embodiment of the invention the said cycloalkane ring is a cyclopropane ring which can be unsubstituted or substituted by alkyl and/or fluorine as indicated. In some embodiments $R^5$ is hydrogen, when one of $R^2$ and $R^3$ is substituted or unsubstituted $(C_1\text{-}C_6)$alkyl, or $R^2$ and $R^3$ are substituted or unsubstituted $(C_1\text{-}C_6)$alkyl and join together with the carbon atom to which they are attached to form a carbocyclic ring, in other embodiments $R^2$ and $R^3$ are hydrogen, when $R^5$ is substituted or unsubstituted $(C_1\text{-}C_6)$alkyl.

In some embodiments $R^4$ is selected from the group consisting of substituted or unsubstituted $(C_6\text{-}C_{14})$aryl, substituted or unsubstituted $(C_3\text{-}C_{14})$heteroaryl and substituted or unsubstituted $(C_3\text{-}C_{14})$heteroaryl$(C_1\text{-}C_6)$alkyl, in other embodiments from substituted or unsubstituted $(C_6\text{-}C_{14})$aryl and substituted or unsubstituted $(C_3\text{-}C_{14})$ heteroaryl, in other embodiments $R^4$ is substituted or unsubstituted $(C_6\text{-}C_{14})$aryl, in other embodiments $R^4$ is substituted or unsubstituted $(C_3\text{-}C_{14})$ heteroaryl. In some embodiments $R^4$ is selected from the group consisting of substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, in other embodiments from substituted or unsubstituted phenyl, substituted or unsubstituted indolyl, and substituted or unsubstituted pyridyl, in other embodiments from substituted or unsubstituted phenyl and substituted or unsubstituted indolyl, in other embodiments $R^4$ is substituted or unsubstituted phenyl, in other embodiments $R^4$ is substituted or unsubstituted indolyl, preferably 1H-indol-3-yl and/or 1H-indol-1-yl, in other embodiments $R^4$ is pyridyl, preferably 2-pyridyl, 3-pyridyl and/or 4-pyridyl, more preferably 1-pyridyl. The phenyl, naphthyl, indolyl, thiophenyl, benzofuranyl and pyridyl group may be substituted by one or more identical or different substituents selected from the group consisting of substituted or unsubstituted $(C_1\text{-}C_6)$alkyl, $(C_1\text{-}C_6)$haloalkyl, halogen, azido, -CN, - $N(R^{46})C(=O)R^{47}$, amino and $OR^{48}$, preferably substituted or unsubstituted $(C_1\text{-}C_6)$alkyl, halogen, $(C_1\text{-}C_6)$haloalkyl, and $OR^{48}$, more preferably substituted or unsubstituted $(C_1\text{-}C_6)$alkyl, halogen and $(C_1\text{-}C_6)$haloalkyl, in other embodiments from $(C_1\text{-}C_6)$alkyl, $(C_1\text{-}C_6)$haloalkyl, halogen, azido, - CN, $-N(R^{46})C(=O)R^{47}$,

amino and $OR^{48}$, preferably $(C_1-C_6)$alkyl, halogen, $(C_1-C_6)$haloalkyl, and $OR^{48}$, more preferably $(C_1-C_6)$alkyl, halogen and $(C_1-C_6)$haloalkyl, wherein $R^{46}$, $R^{47}$ and $R^{48}$ are independently selected from the group consisting of hydrogen, substituted or unsubstituted $(C_6-C_{14})$aryl$(C_1-C_6)$alkyl, $(C_1-C_6)$haloalkyl and substituted or unsubstituted $(C_1-C_6)$alkyl, preferably hydrogen and substituted or unsubstituted $(C_1-C_6)$alkyl, more preferably hydrogen and $(C_1-C_6)$alkyl. Preferably the number of substituents in a substituted group $R^4$ is one, two, three or four, more preferably one, two or three, for example one or two. The substituents in a substituted group $R^4$ can be present on carbon atoms in any positions as indicated above with respect to substituted phenyl, naphthyl, indolyl, thiophenyl, benzofuranyl and pyridyl group in general. In some embodiments $R^4$ is selected from the group consisting of

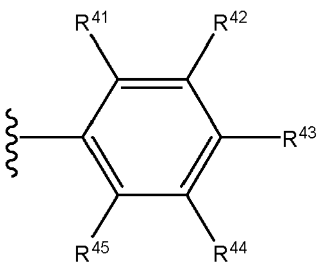 , 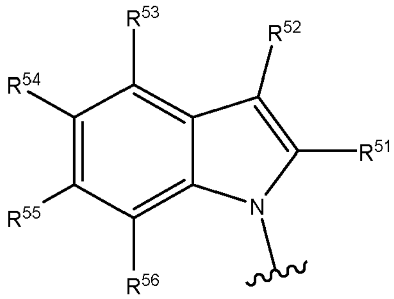 and 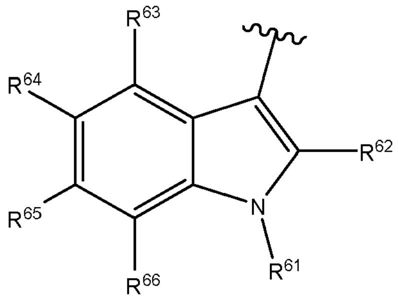 ,

in other embodiments from

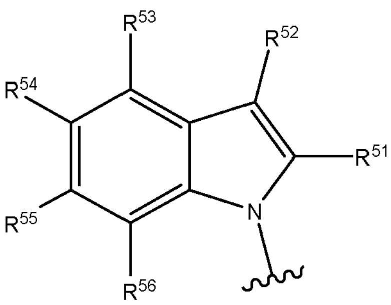 and 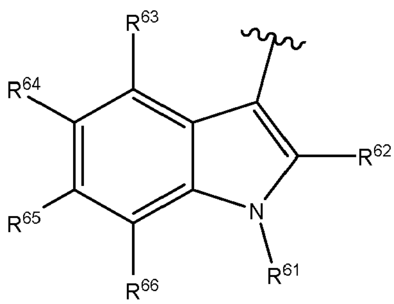 ,

in other embodiments $R^4$ is

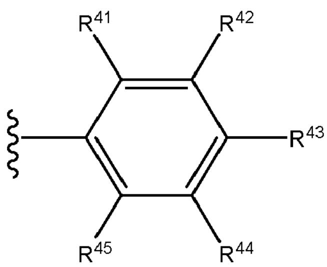 ,

other embodiments $R^4$ is

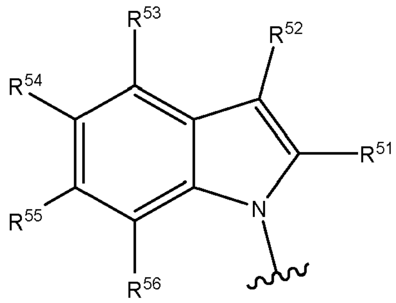

, (

other embodiments $R^4$ is

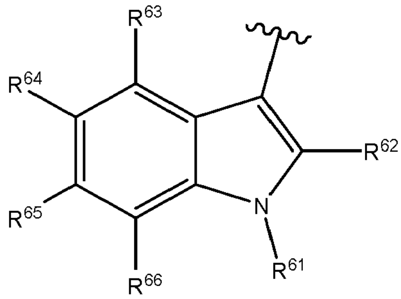

In some embodiments $R^4$ is selected from the group consisting of phenyl, 2-chlorophenyl, 4-aminophenyl, 4-cyanophenyl, 3-acetamidophenyl, 2-bromophenyl, 3-methylphenyl, 2-methylphenyl, 2-iodophenyl, 4-methoxyphenyl, 3-methoxyphenyl, 2-methoxyphenyl, 2-aminophenyl, 2-cyanophenyl, 2-benzyloxyphenyl, 2-azidophenyl, 1H-indol-1yl, 2-methyl-1 H-indol-1yl, 3-methyl-1H-indol-1yl, 2,3-dimethyl-1H-indol-1yl, 2-(trifluoromethyl)-1H-indol-1yl, 4-fluoro-1 H-indol-1 yl, 6-chloro-1H-indol-1yl, 5-chloro-1H-indol-1yl, 5-fluoro-1H-indol-1yl, 5-chloro, 2-methyl-1H-indol-1yl, 7-chloro-1H-indol-1yl, 2-isopropyl-1H-indol-1yl, 7-chloro-2-methyl-1 H-indol-1yl, 2-ethyl-1H-indol-1yl, 1H-indol-3yl, 1-methyl-1H-indol-3yl, 2-methyl-1H-indol-3yl, 3,5-dimethyl-1 H-pyrazol, benzofuran-2-yl, naphthalene-1-yl, pyridine-3-yl, pyridine-2-yl, pyridine-4-yl, thiophen-2-yl, 5-chlorothiophen-2-yl, 2,5-dichlorothiophen-3-yl, 2-chlorothiophen-3-yl, thiophen-3-yl and furan-2-yl, in other embodiments from phenyl, 2-chlorophenyl, 4-aminophenyl, 4-cyanophenyl, 3-acetamidophenyl, 2-bromophenyl, 3-methylphenyl, 2-methylphenyl, 2-iodophenyl, 4-methoxyphenyl, 3-methoxyphenyl, 2-methoxyphenyl, 2-aminophenyl, 2-cyanophenyl, 2-benzyloxyphenyl, 2-azidophenyl, 1H-indol-1yl, 2-methyl-1H-indol-1yl, 3-methyl-1H-indol-1yl, 2,3-dimethyl-1H-indol-1yl, 2-(trifluoromethyl)-1H-indol-1yl, 4-fluoro-1H-indol-1yl, 7-chloro-2-methyl-1 H-indol-1 yl, 6-chloro-1H-indol-1yl, 5-chloro-1H-indol-1yl, 5-fluoro-1H-indol-1yl, 5-chloro, 2-methyl-1H-indol-1yl, 7-chloro-1H-indol-1yl, 2-isopropyl-1H-indol-1yl, 2-ethyl-1H-indol-1yl, 1-methyl-1H-indol-3yl, 2-methyl-1H-indol-3yl, benzofuran-2-yl, naphthalene-1-yl, pyridine-3-yl, pyridine-2-yl, pyridine-4-yl, thiophen-2-yl, 5-chlorothiophen-2-yl, 2,5-dichlorothiophen-3-yl, 2-chlorothiophen-3-yl, thiophen-3-yl and furan-2-yl, in other embodiments from phenyl, 4-aminophenyl, 4-cyanophenyl, 2-methylphenyl, 2-iodophenyl, 4-methoxyphenyl, 2-methoxyphenyl, 2-aminophenyl, 2-cyanophenyl, 1H-indol-1yl, 2-methyl-1H-indol-1yl, 2,3-dimethyl-1H-indol-1yl, 2-(trifluoromethyl)-1 H-indol-1 yl, 6-chloro-1 H-indol-1 yl, 5-chloro, 2-methyl-1H-indol-1yl, 7-chloro-1H-indol-1yl, 2-isopropyl-1H-indol-1yl, 7-chloro-2-methyl-1H-indol-1yl, 2-ethyl-1H-indol-1yl, 2-methyl-1H-indol-3yl, pyridine-2-yl, thiophen-2-yl, 2,5-dichlorothiophen-3-yl and 2-chlorothiophen-3-yl, in other embodiments from 2-iodophenyl, 2-methyl-1H-indol-1yl, 2-(trifluoromethyl)-1H-indol-1yl, 2-ethyl-1H-indol-1yl, 7-chloro-2-methyl-1H-indol-1yl and 2-methyl-1H-indol-3yl. In some embodiments $R^4$ is selected from the group consisting of phenyl, 2-chlorophenyl, 4-aminophenyl, 4-cyanophenyl, 3-acetamidophenyl, 2-bromophenyl, 3-methylphenyl, 2-methylphenyl, 2-iodophenyl, 4-methoxyphenyl, 3-methoxyphenyl, 2-methoxyphenyl, 2-aminophenyl, 2-cyanophenyl, 2-benzyloxyphenyl and 2-azidophenyl, preferably phenyl, 2-chlorophenyl, 3-acetamidophenyl, 2-bromophenyl, 3-methylphenyl, 2-methylphenyl, 2-iodophenyl, 3-methoxyphenyl, 2-methoxyphenyl, 2-aminophenyl, 2-cyanophenyl, 2-benzyloxyphenyl and 2-azidophenyl. In some embodiments $R^4$ is selected from the group consisting of 1H-indol-1yl, 2-methyl-1 H-indol-1 yl, 3-methyl-1 H-indol-1 yl, 2,3-dimethyl-1H-indol-1yl, 2-(trifluoromethyl)-1H-indol-1yl, 4-fluoro-1H-indol-1yl, 6-chloro-1H-indol-1yl, 5-chloro-1H-indol-1yl, 5-fluoro-1H-indol-1yl, 5-chloro-2-methyl-1H-indol-1yl, 7-chloro-1H-indol-1yl, 2-isopropyl-1H-indol-1yl, 7-chloro-2-methyl-1H-indol-1yl and 2-ethyl-1H-indol-1yl, preferably - (trifluoromethyl)-1H-indol-1yl, 2-methyl-1H-indol-1yl, 7-chloro-2-methyl-1H-indol-1yl and 2-ethyl-1 H-indol-1 yl. In some embodiments $R^4$ is selected from the group consisting of 1H-indol-3yl, 1-methyl-1 H-indol-3yl and 2-methyl-1H-indol-3yl, preferably 1-methyl-1H-indol-3yl.

In some embodiments $R^4$ is selected from the group consisting of pyridine-3-yl, pyridine-2-yl and pyridine-4-yl. In some embodiments $R^4$ is selected from the group consisting of 3,5-dimethyl-1 H-pyrazol, benzofuran-2-yl, naphthalene-1-yl, pyridine-3-yl, pyridine-2-yl, pyridine-4-yl, thiophen-2-yl, 5-chlorothiophen-2-yl, 2,5-dichlorothiophen-3-yl, 2-chlorothiophen-3-yl, thiophen-3-yl and furan-2-yl, preferably thiophen-2-yl, 5-chlorothiophen-2-yl, 2,5-dichlorothiophen-3-yl, 2-chlorothiophen-3-yl, thiophen-3-yl, and furan-2-yl.

$R^{41}$, $R^{42}$, $R^{43}$, $R^{44}$ and $R^{45}$ are independently selected from the group consisting of hydrogen, substituted or unsubstituted $(C_1-C_6)$alkyl, $(C_1-C_6)$haloalkyl, halogen, azido, -CN, - $N(R^{46})C(=O)R^{47}$, amino and $OR^{48}$, preferably unsubstituted $(C_1-C_6)$alkyl, $(C_1-C_6)$haloalkyl and halogen, more preferably unsubstituted $(C_1-C_6)$alkyl and halogen. In a preferred embodiment the halogen is iodine, in another preferred embodiment the $(C_1-C_6)$haloalkyl is $-CF_3$. In some preferred embodiments $R^{41}$, $R^{42}$, $R^{43}$, $R^{44}$ and $R^{45}$ are hydrogen. In some preferred embodiments only one of $R^{41}$, $R^{42}$, $R^{43}$, $R^{44}$ and $R^{45}$ is substituted by a moiety as indicated above and the other substituents are hydrogen. In some embodiments $R^{43}$ is substituted by a moiety as indicated above and $R^{41}$, $R^{42}$, $R^{44}$ and $R^{45}$ are hydrogen. In some embodiments at least one of $R^{41}$ and $R^{45}$ is substituted by a moiety as indicated above and the other substituents are hydrogen. In some embodiments at least one of $R^{42}$ and $R^{44}$ is substituted by a moiety as indicated above and the other substituents are hydrogen. In some embodiments $R^{42}$ is substituted by a moiety as indicated above and $R^{41}$, $R^{43}$, $R^{44}$ and $R^{45}$ are hydrogen. In some embodiments $R^{41}$ is substituted by a moiety as indicated above and $R^{42}$, $R^{43}$, $R^{44}$ and $R^{45}$ are hydrogen. In some embodiments the $(C_1-C_6)$alkyl group may be substituted by one or more identical or different substituents selected from the group consisting of halogen, cyano, $-O-((C_1-C_4)$-alkyl) and $(C_1-C_4)$-alkyl, preferably halogen, more preferably fluorine. Preferably the number of substituents in a substituted group $R^{41}$, $R^{42}$, $R^{43}$, $R^{44}$ and $R^{45}$ is one, two, three or four, more preferably one, two or three, for example one or two. The substituents in a substituted group $R^{41}$, $R^{42}$, $R^{43}$, $R^{44}$ and $R^{45}$ can be present on carbon atoms in any positions as indicated above with respect to substituted alkyl group in general.

$R^{46}$, $R^{47}$ and $R^{48}$ are independently selected from the group consisting of hydrogen, substituted or unsubstituted $(C_6-C_{14})$aryl$(C_1-C_6)$alkyl, $(C_1-C_6)$haloalkyl and substituted or unsubstituted $(C_1-C_6)$alkyl, preferably hydrogen and substituted or unsubstituted $(C_1-C_6)$alkyl. In some embodiments the $(C_1-C_6)$alkyl and $(C_6-C_{14})$aryl$(C_1-C_6)$alkyl group may be substituted by one or more identical or different substituents selected from the group consisting of halogen, cyano, - $O-((C_1-C_4)$-alkyl) and $(C_1-C_4)$-alkyl, preferably halogen, more preferably fluorine. Preferably the number of substituents in a substituted group $R^{46}$, $R^{47}$ and $R^{48}$ is one, two, three or four, more preferably one, two or three, for example one or two. The substituents in a substituted group $R^{46}$, $R^{47}$ and $R^{48}$ can be present on carbon atoms in any positions as indicated above with respect to substituted $(C_1-C_6)$alkyl and $(C_6-C_{14})$aryl$(C_1-C_6)$alkyl group in general.

$R^{51}$, $R^{52}$, $R^{53}$, $R^{54}$, $R^{55}$ and $R^{56}$ are independently selected from the group consisting of hydrogen, substituted or unsubstituted $(C_1-C_6)$alkyl, halogen and $(C_1-C_6)$haloalkyl, preferably substituted or unsubstituted $(C_1-C_6)$alkyl and $(C_1-C_6)$haloalkyl. In a preferred embodiment the halogen is chlorine or fluorine, preferably chlorine, in another preferred embodiment the $(C_1-C_6)$haloalkyl is $-CF_3$, in another preferred embodiment the $(C_1-C_6)$alkyl is methyl. In some preferred embodiments $R^{51}$, $R^{52}$, $R^{53}$, $R^{54}$, $R^{55}$ and $R^{56}$ are hydrogen. In some preferred embodiments only one of $R^{51}$, $R^{52}$, $R^{53}$, $R^{54}$, $R^{55}$ and $R^{56}$ is substituted by a moiety as indicated above and the other substituents are hydrogen. In some embodiments $R^{51}$ is substituted by a moiety as indicated above and $R^{52}$, $R^{53}$, $R^{54}$, $R^{55}$ and $R^{56}$ are hydrogen. In some embodiments $R^{54}$ is substituted by a moiety as indicated above and $R^{51}$, $R^{52}$, $R^{53}$, $R^{55}$ and $R^{56}$ are hydrogen. In some embodiments $R^{55}$ is substituted by a moiety as indicated above and $R^{51}$, $R^{52}$, $R^{53}$, $R^{54}$ and $R^{56}$ are hydrogen. In some embodiments $R^{56}$ is substituted by a moiety as indicated above and $R^{51}$, $R^{52}$, $R^{53}$, $R^{54}$ and $R^{55}$ are hydrogen. In some embodiments the $(C_1-C_6)$alkyl group may be substituted by one or more identical or different substituents selected from the group consisting of halogen, cyano, - $O-((C_1-C_4)$-alkyl) and $(C_1-C_4)$-alkyl, preferably halogen, more preferably fluorine. Preferably the number of substituents in a substituted group $R^{51}$, $R^{52}$, $R^{53}$, $R^{54}$, $R^{55}$ and $R^{56}$ is one, two, three or four, more preferably one, two or three, for example one or two. The substituents in a substituted group $R^{51}$, $R^{52}$, $R^{53}$, $R^{54}$, $R^{55}$ and $R^{56}$ can be present on carbon atoms in any positions as indicated above with respect to substituted alkyl group in general.

$R^{61}$, $R^{62}$, $R^{63}$, $R^{64}$, $R^{65}$ and $R^{66}$ are independently selected from the group consisting of hydrogen, substituted or unsubstituted $(C_1-C_6)$alkyl, halogen and $(C_1-C_6)$haloalkyl, preferably hydrogen, $(C_1-C_6)$haloalkyl and substituted or unsubstituted $(C_1-C_6)$alkyl. In a preferred embodiment the halogen is chlorine or fluorine, preferably chlorine, in another preferred embodiment the $(C_1-C_6)$haloalkyl is $-CF_3$, in another preferred embodiment the $(C_1-C_6)$alkyl is methyl. In some preferred embodiments $R^{61}$, $R^{62}$, $R^{63}$, $R^{64}$, $R^{65}$ and $R^{66}$ are hydrogen. In some preferred embodiments only one of $R^{61}$, $R^{62}$, $R^{63}$, $R^{64}$, $R^{65}$ and $R^{66}$ is substituted by a moiety as indicated above and the other substituents are hydrogen. In some embodiments $R^{62}$ is substituted by a moiety as indicated above and $R^{61}$, $R^{63}$, $R^{64}$, $R^{65}$ and $R^{66}$ are hydrogen. In some embodiments the $(C_1-C_6)$alkyl group may be substituted by one or more identical or different substituents selected from the group consisting of halogen, cyano, $-O-((C_1-C_4)$-alkyl) and $(C_1-C_4)$-alkyl, preferably halogen, more preferably fluorine. Preferably the number of substituents in a substituted group $R^{61}$, $R^{62}$, $R^{63}$, $R^{64}$, $R^{65}$ and $R^{66}$ is one, two, three or four, more preferably one, two or three, for example one or two. The substituents in a substituted group $R^{61}$, $R^{62}$, $R^{63}$, $R^{64}$, $R^{65}$ and $R^{66}$ can be present on carbon atoms in any positions as indicated above with respect to substituted

alkyl group in general.

In some embodiments $R^5$ is hydrogen or substituted or unsubstituted $(C_1\text{-}C_6)$alkyl, preferably hydrogen. In some embodiments $R^5$ is methyl. In some embodiments $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring, in other embodiments $R^2$ and $R^3$ are hydrogen, when $R^5$ is substituted or unsubstituted alkyl. In some embodiments the $(C_1\text{-}C_6)$alkyl group may be substituted by one or more identical or different substituents selected from the group consisting of halogen, cyano, -O-($(C_1\text{-}C_4)$-alkyl) and $(C_1\text{-}C_4)$-alkyl, preferably halogen, more preferably fluorine. Preferably the number of substituents in a substituted group $R^5$ is one, two, three or four, more preferably one, two or three, for example one or two. The substituents in a substituted group $R^5$ can be present on carbon atoms in any positions as indicated above with respect to substituted alkyl group in general.

In some embodiments $R^6$ is hydrogen or substituted or unsubstituted $(C_1\text{-}C_6)$alkyl, preferably hydrogen. In some embodiments $R^6$ is methyl. In some embodiments the $(C_1\text{-}C_6)$alkyl group may be substituted by one or more identical or different substituents selected from the group consisting of halogen, cyano, -O-($(C_1\text{-}C_4)$-alkyl) and $(C_1\text{-}C_4)$-alkyl, preferably halogen, more preferably fluorine. Preferably the number of substituents in a substituted group $R^6$ is one, two, three or four, more preferably one, two or three, for example one or two. The substituents in a substituted group $R^6$ can be present on carbon atoms in any positions as indicated above with respect to substituted alkyl group in general.

In some embodiments the compound of Formula (I) is a compound having a structure according to Formula (II)

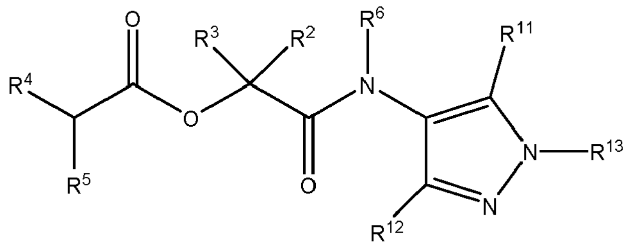

(II)

wherein $R^2$, $R^3$, $R^4$, $R^5$, $R^6$, $R^{11}$, $R^{12}$ and $R^{13}$ are defined as above and below.

$R^{11}$ and $R^{12}$ are each independently selected from the group consisting of hydrogen and substituted or unsubstituted $(C_1\text{-}C_6)$alkyl. in some embodiments $R^{11}$ and $R^{12}$ are substituted or unsubstituted $(C_1\text{-}C_6)$alkyl, preferably $(C_1\text{-}C_6)$alkyl, more preferably methyl. In some embodiments $R^{11}$ is hydrogen and $R^{12}$ is substituted or unsubstituted $(C_1\text{-}C_6)$alkyl, preferably $(C_1\text{-}C_6)$alkyl, more preferably methyl. In some embodiments $R^{12}$ is hydrogen and $R^{11}$ is substituted or unsubstituted $(C_1\text{-}C_6)$alkyl, preferably $(C_1\text{-}C_6)$alkyl, more preferably methyl. In some embodiments the $(C_1\text{-}C_6)$alkyl group may be substituted by one or more identical or different substituents selected from the group consisting of halogen, cyano, -O-($(C_1\text{-}C_4)$-alkyl) and $(C_1\text{-}C_4)$-alkyl, preferably halogen, more preferably fluorine. Preferably the number of substituents in a substituted group $R^{11}$ and $R^{12}$ is one, two, three or four, more preferably one, two or three, for example one or two. The substituents in a substituted group $R^{11}$ and $R^{12}$ can be present on carbon atoms in any positions as indicated above with respect to substituted alkyl group in general.

$R^{13}$ is selected from the group consisting of substituted or unsubstituted $(C_1\text{-}C_6)$alkyl, substituted or unsubstituted $(C_6\text{-}C_{14})$aryl, substituted or unsubstituted $(C_3\text{-}C_{14})$heteroaryl$(C_1\text{-}C_6)$alkyl and substituted or unsubstituted $(C_6\text{-}C_{14})$aryl$(C_1\text{-}C_6)$alkyl. In some embodiments $R^{13}$ is selected from the group consisting of substituted or unsubstituted $(C_1\text{-}C_6)$alkyl, substituted or unsubstituted $(C_6\text{-}C_{14})$aryl and substituted or unsubstituted $(C_6\text{-}C_{14})$aryl$(C_1\text{-}C_6)$alkyl, in other embodiments from $(C_1\text{-}C_6)$alkyl, substituted or unsubstituted $(C_6\text{-}C_{14})$aryl and substituted or unsubstituted $(C_6\text{-}C_{14})$aryl$(C_1\text{-}C_6)$alkyl. In some embodiments $R^{13}$ is substituted or unsubstituted $(C_1\text{-}C_6)$alkyl, preferably $(C_1\text{-}C_6)$alkyl. In some embodiments $R^{13}$ is substituted or unsubstituted $(C_6\text{-}C_{14})$aryl, preferably substituted or unsubstituted phenyl, more preferably substituted phenyl. In some embodiments $R^{13}$ is substituted or unsubstituted $(C_3\text{-}C_{14})$heteroaryl$(C_1\text{-}C_6)$alkyl. In some embodiments $R^{13}$ is substituted or unsubstituted $(C_6\text{-}C_{14})$aryl$(C_1\text{-}C_6)$alkyl, preferably substituted $(C_6\text{-}C_{14})$aryl$(C_1\text{-}C_6)$alkyl, more preferably substituted benzyl. The $(C_1\text{-}C_6)$alkyl, $(C_6\text{-}C_{14})$aryl, $(C_3\text{-}C_{14})$heteroaryl$(C_1\text{-}C_6)$alkyl and $(C_6\text{-}C_{14})$aryl$(C_1\text{-}C_6)$alkyl group may be substituted by one or more identical or different substituents selected from the group consisting of $(C_1\text{-}C_6)$alkyl, $(C_1\text{-}C_6)$haloalkyl, $-NO_2$, halogen, amino, $-N(R^{78})((C_1\text{-}C_6)\text{alkyl-}OR^{77})$, cyano and $-OR^{77}$, preferably $(C_1\text{-}C_6)$haloalkyl, $-NO_2$, halogen, amino, $-N(R^{78})((C_1\text{-}C_6)\text{alkyl-}OR^{77})$, cyano and $-OR^{77}$, wherein $R^{77}$ and $R^{78}$ are each independently selected from the group consisting of hydrogen substituted or unsubstituted $(C_1\text{-}C_6)$haloalkyl and substituted or unsubstituted $(C_1\text{-}C_6)$alkyl, preferably hydrogen and/or substituted or unsubstituted $(C_1\text{-}C_6)$alkyl, more preferably substituted or unsubstituted $(C_1\text{-}C_6)$alkyl, even more preferably $(C_1\text{-}C_6)$alkyl. Preferably the number of substituents in a substituted group $R^{13}$ is one, two, three or four, more preferably one, two or three, for example one or two. The substituents in a substituted group $R^{13}$ can be present on carbon atoms in any positions as indicated above with

respect to substituted ($C_1$-$C_6$)alkyl, ($C_6$-$C_{14}$)aryl, ($C_3$-$C_{14}$)heteroaryl($C_1$-$C_6$)alkyl and ($C_6$-$C_{14}$)aryl($C_1$-$C_6$)alkyl group in general.

In some embodiments the compound of Formula (I) is a compound having a structure according to Formula (III)

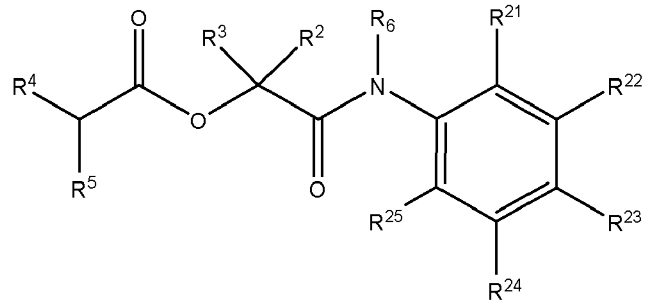

(III)

wherein $R^2$, $R^3$, $R^4$, $R^5$, $R^6$, $R^{21}$, $R^{22}$, $R^{23}$, $R^{24}$ and $R^{25}$ are defined as above and below.

$R^{21}$, $R^{22}$, $R^{23}$, $R^{24}$ and $R^{25}$ are independently selected from the group consisting of hydrogen, substituted or unsubstituted ($C_3$-$C_{14}$)heterocyclyl, substituted or unsubstituted amino, substituted or unsubstituted ($C_1$-$C_6$)alkyl, -$OR^{26}$, halogen and -C(O)$NR^{27}R^{28}$. In some embodiments the ($C_1$-$C_6$)alkyl is methyl, in other embodiments halogen is fluorine or chlorine, preferably chlorine, in other embodiments amino is morpholino, preferably wherein the morpholino moiety is bound to the remainder molecule via the nitrogen atom, in other embodiments ($C_3$-$C_{14}$)heterocyclyl is morpholino, preferably wherein the morpholino moiety is bound to the remainder molecule via the nitrogen atom. In some preferred embodiments $R^{21}$, $R^{22}$, $R^{23}$, $R^{24}$ and $R^{25}$ are hydrogen. In some preferred embodiments only one of $R^{21}$, $R^{22}$, $R^{23}$, $R^{24}$ and $R^{25}$ is substituted by a moiety as indicated above and the other substituents are hydrogen. In some embodiments $R^{21}$ is substituted by a moiety as indicated above and $R^{22}$, $R^{23}$, $R^{24}$ and $R^{25}$ are hydrogen. In some embodiments $R^{22}$ is substituted by a moiety as indicated above and $R^{21}$, $R^{23}$, $R^{24}$ and $R^{25}$ are hydrogen. In some embodiments $R^{23}$ is substituted by a moiety as indicated above and $R^{21}$, $R^{22}$, $R^{24}$ and $R^{25}$ are hydrogen. In some embodiments the ($C_1$-$C_6$)alkyl and ($C_3$-$C_{14}$)heterocyclyl group may be substituted by one or more identical or different substituents selected from the group consisting of halogen, cyano, -O-(($C_1$-$C_4$)-alkyl) and ($C_1$-$C_4$)-alkyl, preferably halogen, more preferably fluorine. Preferably the number of substituents in a substituted group $R^{21}$, $R^{22}$, $R^{23}$, $R^{24}$ and $R^{25}$ is one, two, three or four, more preferably one, two or three, for example one or two. The substituents in a substituted group $R^{21}$, $R^{22}$, $R^{23}$, $R^{24}$ and $R^{25}$ can be present on carbon atoms in any positions as indicated above with respect to substituted alkyl group in general.

$R^{26}$ is selected from the group consisting of hydrogen, substituted or unsubstituted ($C_1$-$C_6$)alkyl and substituted or unsubstituted ($C_2$-$C_6$)alkenyl. In some embodiments $R^{26}$ is selected from the group consisting of hydrogen, ($C_1$-$C_6$)alkyl and ($C_2$-$C_6$)alkenyl, in other embodiments $R^{26}$ is hydrogen or ($C_1$-$C_6$)alkyl. In some embodiments ($C_1$-$C_6$)alkyl is methyl. In some embodiments ($C_2$-$C_6$)alkenyl is allyl. In some embodiments the ($C_1$-$C_6$)alkyl and ($C_2$-$C_6$)alkenyl group may be substituted by one or more identical or different substituents selected from the group consisting of halogen, cyano, -O-(($C_1$-$C_4$)-alkyl) and ($C_1$-$C_4$)-alkyl, preferably halogen, more preferably fluorine. Preferably the number of substituents in a substituted group $R^{26}$ is one, two, three or four, more preferably one, two or three, for example one or two. The substituents in a substituted group $R^{26}$ can be present on carbon atoms in any positions as indicated above with respect to substituted alkyl group in general.

$R^{27}$ and $R^{28}$ are independently selected from the group consisting of hydrogen and substituted or unsubstituted ($C_1$-$C_6$)alkyl. In some embodiments $R^{27}$ and $R^{28}$ are hydrogen. In some embodiments $R^{27}$ is hydrogen and $R^{28}$ is substituted or unsubstituted ($C_1$-$C_6$)alkyl, preferably ($C_1$-$C_6$)alkyl. In some embodiments $R^{27}$ and $R^{28}$ are substituted or unsubstituted ($C_1$-$C_6$)alkyl, preferably ($C_1$-$C_6$)alkyl. In some embodiments the ($C_1$-$C_6$)alkyl group may be substituted by one or more identical or different substituents selected from the group consisting of halogen, cyano, -O-(($C_1$-$C_4$)-alkyl) and ($C_1$-$C_4$)-alkyl, preferably halogen, more preferably fluorine. Preferably the number of substituents in a substituted group $R^{27}$ and $R^{28}$ is one, two, three or four, more preferably one, two or three, for example one or two. The substituents in a substituted group $R^{27}$ and $R^{28}$ can be present on carbon atoms in any positions as indicated above with respect to substituted alkyl group in general.

In some embodiments the compound of Formula (I) is a compound having a structure according to Formula (IV)

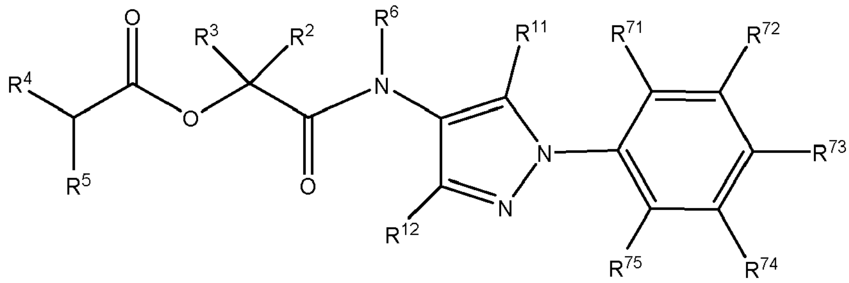

(IV)

wherein $R^2$, $R^3$, $R^4$, $R^5$, $R^6$, $R^{11}$, $R^{12}$, $R^{71}$, $R^{72}$, $R^{73}$, $R^{74}$, $R^{75}$ and $R^{76}$ are defined as above and below.

$R^{71}$, $R^{72}$, $R^{73}$, $R^{74}$, $R^{75}$ and $R^{76}$ are independently selected from the group consisting of hydrogen, $(C_1\text{-}C_6)$alkyl, $(C_1\text{-}C_6)$haloalkyl, $-NO_2$, halogen, amino, $-N(R^{78})((C_1\text{-}C_6)$alkyl-$OR^{77})$, cyano and - $OR^{77}$. In some embodiments $R^{71}$, $R^{72}$, $R^{73}$, $R^{74}$, $R^{75}$ and $R^{76}$ are independently selected from the group consisting of $(C_1\text{-}C_6)$haloalkyl, $-NO_2$, halogen, amino, $-N(R^{78})((C_1\text{-}C_6)$alkyl-$OR^{77})$, cyano and $-OR^{77}$. In some embodiments $(C_1\text{-}C_6)$alkyl is methyl. In some embodiments $(C_1\text{-}C_6)$haloalkyl is $-CF_3$. In some embodiments $R^{71}$, $R^{72}$, $R^{73}$, $R^{74}$, $R^{75}$ and $R^{76}$ are independently selected from the group consisting of hydrogen, $(C_1\text{-}C_6)$alkyl, $(C_1\text{-}C_6)$haloalkyl, $-NO_2$, amino, and cyano, in other embodiments from hydrogen, methyl, $-CF_3$, $NO_2$, amino, and cyano. In some preferred embodiments $R^{71}$, $R^{72}$, $R^{73}$, $R^{74}$, $R^{75}$ and $R^{76}$ are hydrogen. In some preferred embodiments only one of $R^{71}$, $R^{72}$, $R^{73}$, $R^{74}$, $R^{75}$ and $R^{76}$ is substituted by a moiety as indicated above, preferably from the group consisting of $(C_1\text{-}C_6)$alkyl, $(C_1\text{-}C_6)$haloalkyl, $-NO_2$, amino, and cyano, more preferably from methyl, $-CF_3$, $NO_2$, amino, and cyano, and the other substituents are hydrogen. In some embodiments $R^{11}$ is substituted by a moiety as indicated above and $R^{72}$, $R^{73}$, $R^{74}$, $R^{75}$ and $R^{76}$ are hydrogen. In some embodiments $R^{72}$ is substituted by a moiety as indicated above and $R^{71}$, $R^{73}$, $R^{74}$, $R^{75}$ and $R^{76}$ are hydrogen. In some embodiments $R^{73}$ is substituted by a moiety as indicated above and $R^{71}$, $R^{72}$, $R^{74}$, $R^{75}$ and $R^{76}$ are hydrogen.

$R^{77}$ and $R^{78}$ are each independently selected from the group consisting of hydrogen, $(C_1\text{-}C_6)$haloalkyl and substituted or unsubstituted $(C_1\text{-}C_6)$alkyl, preferably hydrogen and/or substituted or unsubstituted $(C_1\text{-}C_6)$alkyl. In some embodiments $R^{77}$ and $R^{78}$ are hydrogen. In some embodiments $R^{77}$ and $R^{78}$ are $(C_1\text{-}C_6)$haloalkyl and substituted or unsubstituted $(C_1\text{-}C_6)$alkyl, preferably $(C_1\text{-}C_6)$haloalkyl and $(C_1\text{-}C_6)$alkyl, more preferably $(C_1\text{-}C_6)$alkyl. In some embodiments $R^{78}$ is hydrogen and $R^{77}$ is $C_1\text{-}C_6)$haloalkyl or substituted or unsubstituted $(C_1\text{-}C_6)$alkyl, preferably $C_1\text{-}C_6)$haloalkyl or $(C_1\text{-}C_6)$alkyl, more preferably methyl. In some embodiments $(C_1\text{-}C_6)$alkyl is methyl. In some embodiments $(C_1\text{-}C_6)$haloalkyl is $-CF_3$. In some embodiments the $(C_1\text{-}C_6)$alkyl group may be substituted by one or more identical or different substituents selected from the group consisting of halogen, cyano, $-O-((C_1\text{-}C_4)$-alkyl) and $(C_1\text{-}C_4)$-alkyl, preferably halogen, more preferably fluorine. Preferably the number of substituents in a substituted group $R^{77}$ and $R^{78}$ is one, two, three or four, more preferably one, two or three, for example one or two. The substituents in a substituted group $R^{77}$ and $R^{78}$ can be present on carbon atoms in any positions as indicated above with respect to substituted alkyl group in general.

In preferred compounds of the invention any one or more structural elements such as groups, substituents and numbers are defined as in any of the preferred definitions of the elements or in any specified embodiment and/or can have one or more of the specific meanings which are mentioned as examples of elements, wherein all combinations of one or more preferred definitions and embodiments and/or specific meanings are a subject of the present invention. Also with respect to all preferred compounds of the formula I, formula II, formula III and formula IV all their stereoisomeric forms and mixtures of stereoisomeric forms in all ratios, and their physiologically acceptable salts, and the physiologically acceptable solvates of any of them, are a subject of the present invention. Similarly, also with respect to all specific compounds disclosed herein, such as the example compounds, which represent embodiments of the invention wherein the various groups and numbers in the general definition of the compounds of the formula I, formula II, formula III and formula IV have the specific meanings present in the respective specific compound, all their stereoisomeric forms and mixtures of stereoisomeric forms in all ratios, and their physiologically acceptable salts, and the physiologically acceptable solvates of any of them, are a subject of the present invention. In particular, a subject of the invention are all specific compounds disclosed herein, independently thereof whether they are disclosed as a free compound and/or as a specific salt, both in the form of the free compound and in the form of all its physiologically acceptable salts, and if a specific salt is disclosed, additionally in the form of this specific salt, and the physiologically acceptable solvates thereof.

In some embodiments the compound of Formula (I) is characterized in that,

$R^1$ is selected from the group consisting of substituted or unsubstituted $(C_6\text{-}C_{14})$aryl and substituted or unsubstituted $(C_3\text{-}C_{14})$ heteroaryl, preferably substituted or unsubstituted phenyl and substituted or unsubstituted $(C_3\text{-}C_{10})$ heteroaryl;

$R^2$ and $R^3$ are independently selected from the group consisting of hydrogen and substituted or unsubstituted $(C_1\text{-}C_6)$alkyl; or $R^2$ and $R^3$ are substituted or unsubstituted $(C_1\text{-}C_6)$alkyl and join together with the carbon atom to which they are attached to form a carbocyclic ring which is optionally substituted;

$R^4$ is selected from the group consisting of substituted or unsubstituted ($C_6$-$C_{14}$)aryl, substituted or unsubstituted ($C_3$-$C_{14}$)heteroaryl and substituted or unsubstituted ($C_3$-$C_{14}$)heteroaryl($C_1$-$C_6$)alkyl, preferably substituted or unsubstituted ($C_6$-$C_{14}$)aryl and substituted or unsubstituted ($C_3$-$C_{14}$)heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted ($C_1$-$C_6$)alkyl, preferably hydrogen; and
$R^6$ is hydrogen or substituted or unsubstituted ($C_1$-$C_6$)alkyl, preferably hydrogen;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In some embodiments the compound of Formula (I) is characterized in that,
$R^1$ is selected from the group consisting of substituted or unsubstituted ($C_1$-$C_6$)alkyl, substituted or unsubstituted ($C_3$-$C_8$)cycloalkyl, substituted or unsubstituted ($C_6$-$C_{14}$)aryl, substituted or unsubstituted ($C_3$-$C_{14}$)heteroaryl and substituted or unsubstituted ($C_3$-$C_{14}$)heteroaryl($C_1$-$C_6$)alkyl; $R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of substituted or unsubstituted ($C_6$-$C_{14}$)aryl and substituted or unsubstituted ($C_3$-$C_{14}$) heteroaryl, preferably substituted or unsubstituted phenyl and substituted or unsubstituted ($C_3$-$C_{10}$) heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted ($C_1$-$C_6$)alkyl, preferably hydrogen; and
$R^6$ is hydrogen or substituted or unsubstituted ($C_1$-$C_6$)alkyl, preferably hydrogen;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In some embodiments the compound of Formula (I) is characterized in that,
$R^1$ is selected from the group consisting of substituted or unsubstituted ($C_6$-$C_{14}$)aryl and substituted or unsubstituted ($C_3$-$C_{14}$) heteroaryl, preferably substituted or unsubstituted phenyl and substituted or unsubstituted ($C_3$-$C_{10}$) heteroaryl;
$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of substituted or unsubstituted ($C_6$-$C_{14}$)aryl and substituted or unsubstituted ($C_3$-$C_{14}$) heteroaryl, preferably substituted or unsubstituted phenyl and substituted or unsubstituted ($C_3$-$C_{10}$) heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted ($C_1$-$C_6$)alkyl, preferably hydrogen; and
$R^6$ is hydrogen or substituted or unsubstituted ($C_1$-$C_6$)alkyl, preferably hydrogen;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

In some embodiments the compound of Formula (II) is characterized in that,
$R^2$ and $R^3$ are independently selected from the group consisting of hydrogen and substituted or unsubstituted $(C_1-C_6)$alkyl; or $R^2$ and $R^3$ are substituted or unsubstituted $(C_1-C_6)$alkyl and join together with the carbon atom to which they are attached to form a carbocyclic ring which is optionally substituted;
$R^4$ is selected from the group consisting of substituted or unsubstituted $(C_6-C_{14})$aryl and substituted or unsubstituted $(C_3-C_{14})$ heteroaryl, preferably substituted or unsubstituted phenyl and substituted or unsubstituted $(C_3-C_{10})$ heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted $(C_1-C_6)$alkyl, preferably hydrogen;
$R^6$ is hydrogen or substituted or unsubstituted $(C_1-C_6)$alkyl, preferably hydrogen;
$R^{11}$ and $R^{12}$ are independently selected from the group consisting of hydrogen and substituted or unsubstituted $(C_1-C_6)$alkyl;
$R^{13}$ is selected from the group consisting of substituted or unsubstituted $(C_1-C_6)$alkyl, substituted or unsubstituted $(C_6-C_{14})$aryl, substituted or unsubstituted $(C_3-C_{14})$heteroaryl$(C_1-C_6)$alkyl and substituted or unsubstituted $(C_6-C_{14})$aryl$(C_1-C_6)$alkyl, preferably substituted or unsubstituted $(C_1-C_6)$alkyl, substituted or unsubstituted phenyl, substituted or unsubstituted $(C_3-C_{10})$heteroaryl$(C_1-C_6)$alkyl and substituted or unsubstituted benzyl;
or a pharmaceutically acceptable salt, solvate or hydrate thereof;
with the proviso that

(i) $R^5$ is hydrogen, when one of $R^2$ and $R^3$ is substituted or unsubstituted $(C_1-C_6)$alkyl, or $R^2$ and $R^3$ are substituted or unsubstituted $(C_1-C_6)$alkyl and join together with the carbon atom to which they are attached to form a carbocyclic ring;
(ii) $R^2$ and $R^3$ are hydrogen, when $R^5$ is substituted or unsubstituted $(C_1-C_6)$alkyl.

In some embodiments the compound of Formula (III) is characterized in that,
$R^2$ and $R^3$ are independently selected from the group consisting of hydrogen and substituted or unsubstituted $(C_1-C_6)$alkyl; or $R^2$ and $R^3$ are substituted or unsubstituted $(C_1-C_6)$alkyl and join together with the carbon atom to which they are attached to form a carbocyclic ring which is optionally substituted;
$R^4$ is selected from the group consisting of substituted or unsubstituted $(C_6-C_{14})$aryl and substituted or unsubstituted $(C_3-C_{14})$ heteroaryl, preferably substituted or unsubstituted phenyl and substituted or unsubstituted $(C_3-C_{10})$ heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted $(C_1-C_6)$alkyl, preferably hydrogen;
$R^6$ is hydrogen or substituted or unsubstituted $(C_1-C_6)$alkyl, preferably hydrogen;
$R^{21}$, $R^{22}$, $R^{23}$, $R^{24}$ and $R^{25}$ are independently selected from the group consisting of hydrogen, substituted or unsubstituted $(C_3-C_{14})$heterocyclyl, substituted or unsubstituted amino, substituted or unsubstituted $(C_1-C_6)$alkyl, $-OR^{26}$, halogen and $-C(O)NR^{27}R^{28}$, wherein only one of $R^{21}$, $R^{22}$, $R^{23}$, $R^{24}$ and $R^{25}$ is not hydrogen;
$R^{26}$ is selected from the group consisting of hydrogen, substituted or unsubstituted $(C_1-C_6)$alkyl and substituted or unsubstituted $(C_2-C_6)$alkenyl;
$R^{27}$ and $R^{28}$ are independently selected from the group consisting of hydrogen and substituted or unsubstituted $(C_1-C_6)$alkyl;
or a pharmaceutically acceptable salt, solvate or hydrate thereof;
with the proviso that

(i) $R^5$ is hydrogen, when one of $R^2$ and $R^3$ is substituted or unsubstituted $(C_1-C_6)$alkyl, or $R^2$ and $R^3$ are substituted or unsubstituted $(C_1-C_6)$alkyl and join together with the carbon atom to which they are attached to form a carbocyclic ring;
(ii) $R^2$ and $R^3$ are hydrogen, when $R^5$ is substituted or unsubstituted $(C_1-C_6)$alkyl.

In some embodiments the compound of Formula (IV) is characterized in that,
$R^2$ and $R^3$ are independently selected from the group consisting of hydrogen and substituted or unsubstituted $(C_1-C_6)$alkyl; or $R^2$ and $R^3$ are substituted or unsubstituted $(C_1-C_6)$alkyl and join together with the carbon atom to which they are attached to form a carbocyclic ring which is optionally substituted;
$R^4$ is selected from the group consisting of substituted or unsubstituted $(C_6-C_{14})$aryl and substituted or unsubstituted

($C_3$-$C_{14}$) heteroaryl, preferably substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl, more preferably substituted or unsubstituted phenyl, substituted or unsubstituted indolyl and substituted or unsubstituted pyridyl, even more preferably substituted or unsubstituted phenyl and substituted or unsubstituted indolyl;
$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{11}$ and $R^{12}$ are independently selected from the group consisting of hydrogen and substituted or unsubstituted ($C_1$-$C_6$)alkyl;
$R^{71}$, $R^{72}$, $R^{73}$, $R^{74}$, $R^{75}$ and $R^{76}$ are independently selected from the group consisting of hydrogen, ($C_1$-$C_6$)alkyl, ($C_1$-$C_6$)haloalkyl, -$NO_2$, halogen, amino, -N($R^{78}$)(($C_1$-$C_6$)alkyl-O$R^{77}$), cyano and - O$R^{77}$;
$R^{77}$ and $R^{78}$ are each independently selected from the group consisting of hydrogen, ($C_1$-$C_6$)haloalkyl and substituted or unsubstituted ($C_1$-$C_6$)alkyl, preferably hydrogen and/or substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

In some embodiments the compound of Formula (IV) is characterized in that,
$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of

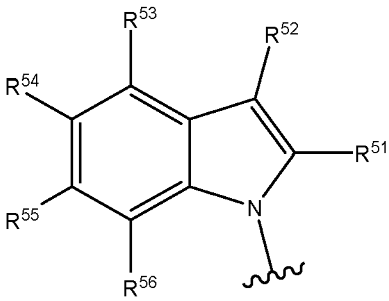
and
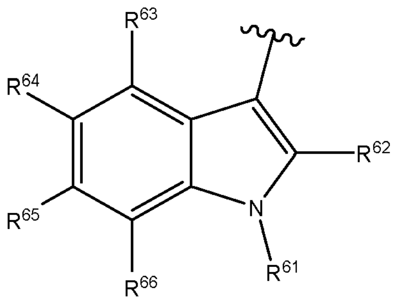
;

$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{11}$ and $R^{12}$ are independently selected from the group consisting of hydrogen and substituted or unsubstituted ($C_1$-$C_6$)alkyl;
$R^{51}$, $R^{52}$, $R^{53}$, $R^{54}$, $R^{55}$ and $R^{56}$ are independently selected from the group consisting of hydrogen, substituted or unsubstituted ($C_1$-$C_6$)alkyl, halogen and ($C_1$-$C_6$)haloalkyl, preferably substituted or unsubstituted ($C_1$-$C_6$)alkyl and ($C_1$-$C_6$)haloalkyl;
$R^{61}$, $R^{62}$, $R^{63}$, $R^{64}$, $R^{65}$ and $R^{66}$ are independently selected from the group consisting of hydrogen, substituted or unsubstituted ($C_1$-$C_6$)alkyl, halogen and ($C_1$-$C_6$)haloalkyl, preferably hydrogen, ($C_1$-$C_6$)haloalkyl and substituted or unsubstituted ($C_1$-$C_6$)alkyl;
$R^{71}$, $R^{72}$, $R^{73}$, $R^{74}$, $R^{75}$ and $R^{76}$ are independently selected from the group consisting of ($C_1$-$C_6$)haloalkyl, -$NO_2$, halogen, amino, -N($R^{78}$)(($C_1$-$C_6$)alkyl-O$R^{77}$), cyano and -O$R^{77}$;
$R^{77}$ and $R^{78}$ are each independently selected from the group consisting of hydrogen, ($C_1$-$C_6$)haloalkyl and substituted or unsubstituted ($C_1$-$C_6$)alkyl, preferably substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;

(ii) $R^2$ and $R^3$ are hydrogen, when $R^5$ is substituted or unsubstituted ($C_1$-$C_6$)alkyl.

In some embodiments the compound of Formula (I) is selected from the list consisting of 2-((3,5-dimethyl-1-(m-tolyl)-1H-pyrazol-4-yl)amino)-2-oxoethyl 2-(2-methyl-1H-indol-1-yl)acetate, 2-((3,5-dimethyl-1-(4-(trifluoromethyl)phenyl)-1 H-pyrazol-4-yl)amino)-2-oxoethyl 2-(2-methyl-1H-indol-1-yl)acetate, 2-((3,5-dimethyl-1-(2-nitrophenyl)-1H-pyrazol-4-yl)amino)-2-oxoethyl 2-(2-methyl-1H-indol-1-yl)acetate, 2-((1-(4-cyanophenyl)-3,5-dimethyl-1H-pyrazol-4-yl)amino)-2-oxoethyl 2-(2-methyl-1H-indol-1-yl)acetate, 2-((1-(4-methoxybenzyl)-3,5-dimethyl-1H-pyrazol-4-yl)amino)-2-oxoethyl 2-(2-methyl-1H-indol-1-yl)acetate, 2-((3,5-dimethyl-1-(2-(trifluoromethyl)phenyl)-1H-pyrazol-4-yl)amino)-2-oxoethyl2-(2-methyl-1H-indol-1-yl)acetate, 2-((3,5-dimethyl-1-(p-tolyl)-1H-pyrazol-4-yl)amino)-2-oxoethyl 2-(2-methyl-1H-indol-1-yl)acetate, 2-((3,5-dimethyl-1-(4-nitrophenyl)-1H-pyrazol-4-yl)amino)-2-oxoethyl 2-(2-methyl-1H-indol-1-yl)acetate, 2-((3,5-dimethyl-1-phenyl-1H-pyrazol-4-yl)amino)-2-oxoethyl 2-(1H-indol-1-yl)acetate, 2-((3,5-dimethyl-1-(p-tolyl)-1H-pyrazol-4-yl)amino)-2-oxoethyl 2-(1 H-indol-1-yl)acetate, 2-((3,5-dimethyl-1-phenyl-1H-pyrazol-4-yl)amino)-2-oxoethyl 2-(3-methyl-1H-indol-1-yl)acetate, 2-((3,5-dimethyl-1-phenyl-1H-pyrazol-4-yl)amino)-2-oxoethyl 2-(2,3-dimethyl-1H-indol-1-yl)acetate, 2-((3,5-dimethyl-1-phenyl-1H-pyrazol-4-yl)amino)-2-oxoethyl 2-(2-(trifluoromethyl)-1H-indol-1-yl)acetate, 2-((3,5-dimethyl-1-phenyl-1 H-pyrazol-4-yl)amino)-2-oxoethyl 2-(1-methyl-1H-indol-3-yl)acetate, 2-((1-(4-((3-hydroxybutyl)amino)phenyl)-3,5-dimethyl-11H-pyrazol-4-yl)amino)-2-oxoethyl 2-(2-methyl-1 H-indol-1-yl)acetate, 2-((3,5-dimethyl-1-(p-tolyl)-1H-pyrazol-4-yl)amino)-2-oxoethyl 2-(2-(trifluoromethyl)-1H-indol-1-yl)acetate, 2-((3,5-dimethyl-1-(4-nitrophenyl)-1 H-pyrazol-4-yl)amino)-2-oxoethyl 2-(2-(trifluoromethyl)-1H-indol-1-yl)acetate, 2-((1-(4-aminophenyl)-3,5-dimethyl-1H-pyrazol-4-yl)amino)-2-oxoethyl 2-(2-methyl-1 H-indol-1-yl)acetate, 2-((1-(2-aminophenyl)-3,5-dimethyl-1H-pyrazol-4-yl)amino)-2-oxoethyl 2-(2-methyl-1 H-indol-1-yl)acetate, 2-((3,5-dimethyl-1-phenyl-1 H-pyrazol-4-yl)amino)-2-oxoethyl 2-(4-fluoro-1H-indol-1-yl)acetate, 2-((3,5-dimethyl-1-phenyl-1H-pyrazol-4-yl)amino)-2-oxoethyl 2-(6-chloro-1H-indol-1-yl)acetate, 2-((3,5-dimethyl-1-phenyl-1H-pyrazol-4-yl)amino)-2-oxoethyl 2-(5-chloro-1H-indol-1-yl)acetate, 2-((3,5-dimethyl-1-phenyl-1 H-pyrazol-4-yl)amino)-2-oxoethyl 2-(5-fluoro-1H-indol-1-yl)acetate, 2-((3,5-dimethyl-1-phenyl-1 H-pyrazol-4-yl)amino)-2-oxoethyl 2-(5-chloro-2-methyl-1 H-indol-1-yl)acetate, 2-((3,5-dimethyl-1-phenyl-1 H-pyrazol-4-yl)amino)-2-oxoethyl 2-(7-chloro-1 H-indol-1-yl)acetate, 2-((3,5-dimethyl-1-phenyl-1 H-pyrazol-4-yl)amino)-2-oxoethyl 2-(2-isopropyl-1 H-indol-1-yl)acetate, 2-((3,5-dimethyl-1-phenyl-1 H-pyrazol-4-yl)amino)-2-oxoethyl 2-(7-chloro-2-methyl-1 H-indol-1-yl)acetate, 2-((3,5-dimethyl-1-phenyl-1 H-pyrazol-4-yl)amino)-2-oxoethyl 2-(2-methyl-1 H-indol-1-yl)propanoate, 2-((3,5-dimethyl-1-phenyl-1H-pyrazol-4-yl)amino)-2-oxoethyl 2-(2-ethyl-1H-indol-1-yl)acetate, 1-((3,5-dimethyl-1-phenyl-1H-pyrazol-4-yl)amino)-1-oxopropan-2-yl 2-(2-methyl-1H-indol-1-yl)acetate, 2-((3,5-dimethyl-1-phenyl-1 H-pyrazol-4-yl)amino)-2-oxoethyl 2-(2-methyl-1 H-indol-3-yl)acetate, 2-oxo-2-((1-phenyl-1H-pyrazol-4-yl)amino)ethyl 2-(2-methyl-1H-indol-3-yl)acetate, 1-((3,5-dimethyl-1-phenyl-1H-pyrazol-4-yl)carbamoyl)cyclopropyl 2-(2-methyl-1H-indol-1-yl)acetate, 2-((3,5-dimethyl-1-phenyl-1H-pyrazol-4-yl)amino)-2-oxoethyl 2-(benzofuran-3-yl)acetate, 2-((3,5-dimethyl-1-phenyl-1H-pyrazol-4-yl)amino)-2-oxoethyl 2-(4-aminophenyl)acetate, 2-((3,5-dimethyl-1-phenyl-1H-pyrazol-4-yl)amino)-2-oxoethyl 2-(2-chlorophenyl)acetate, 2-((3,5-dimethyl-1-phenyl-1H-pyrazol-4-yl)amino)-2-oxoethyl 2-(4-cyanophenyl)acetate, 2-((3,5-dimethyl-1-phenyl-1H-pyrazol-4-yl)amino)-2-oxoethyl 2-(3-acetamidophenyl)acetate, 2-((3,5-dimethyl-1-phenyl-1 H-pyrazol-4-yl)amino)-2-oxoethyl 2-(2-bromophenyl)acetate, 2-((3,5-dimethyl-1-phenyl-1H-pyrazol-4-yl)amino)-2-oxoethyl 2-(m-tolyl)acetate, 2-((3,5-dimethyl-1-phenyl-1H-pyrazol-4-yl)amino)-2-oxoethyl 2-(2-iodophenyl)acetate, 2-((3,5-dimethyl-1-phenyl-1H-pyrazol-4-yl)amino)-2-oxoethyl 2-(3-aminophenyl)acetate, 2-((3,5-dimethyl-1-phenyl-1H-pyrazol-4-yl)amino)-2-oxoethyl 2-(3-cyanophenyl)acetate, 2-((3,5-dimethyl-1-phenyl-1H-pyrazol-4-yl)amino)-2-oxoethyl 2-(pyridin-3-yl)acetate, 2-((3,5-dimethyl-1-phenyl-1H-pyrazol-4-yl)amino)-2-oxoethyl 2-(pyridin-2-yl)acetate, 2-((3,5-dimethyl-1-phenyl-1H-pyrazol-4-yl)amino)-2-oxoethyl 2-(5-chlorothiophen-2-yl)acetate, 2-((3,5-dimethyl-1-phenyl-1H-pyrazol-4-yl)amino)-2-oxoethyl 2-(2,5-dichlorothiophen-3-yl)acetate, 2-((3,5-dimethyl-1-phenyl-1H-pyrazol-4-yl)amino)-2-oxoethyl 2-(pyridin-4-yl)acetate, 2-((3,5-dimethyl-1-phenyl-1H-pyrazol-4-yl)amino)-2-oxoethyl 2-(2-(trifluoromethyl)phenyl)acetate, 2-((3,5-dimethyl-1-phenyl-1H-pyrazol-4-yl)amino)-2-oxoethyl 2-(3-fluorophenyl)acetate, 2-((3,5-dimethyl-1-phenyl-1 H-pyrazol-4-yl)amino)-2-oxoethyl 2-(2-chlorothiophen-3-yl)acetate, 2-((3,5-dimethyl-1-phenyl-1H-pyrazol-4-yl)amino)-2-oxoethyl 2-(furan-2-yl)acetate, 2-((3,5-dimethyl-1-phenyl-1H-pyrazol-4-yl)amino)-2-oxoethyl 2-(2-(benzyloxy)phenyl)acetate, 2-((3,5-dimethyl-1-phenyl-1 H-pyrazol-4-yl)amino)-2-oxoethyl 2-(thiophen-3-yl)acetate, 2-((3,5-dimethyl-1-phenyl-1 H-pyrazol-4-yl)amino)-2-oxoethyl 2-(2-methyl-1 H-indol-3-yl)propanoate, 2-((3,5-dimethyl-1-phenyl-1H-pyrazol-4-yl)amino)-2-oxoethyl 2-(2-azidophenyl)acetate, 2-((3,5-dimethyl-1-phenyl-1H-pyrazol-4-yl)amino)-2-oxoethyl 2-(1H-indol-3-yl)propanoate, 2-((3,5-dimethyl-1-phenyl-1 H-pyrazol-4-yl)amino)-2-oxoethyl 3-(2-methyl-1 H-indol-3-yl)propanoate, 2-((3,5-dimethyl-1-phenyl-1 H-pyrazol-4-yl)(methyl)amino)-2-oxoethyl 2-(2-methyl-1 H-indol-3-yl)acetate, 2-(tert-butylamino)-2-oxoethyl 2-(2-methyl-1 H-indol-3-yl)acetate, 2-(cyclopentylamino)-2-oxoethyl 2-(2-methyl-1 H-indol-3-yl)acetate, 2-((3,5-dimethyl-1-phenyl-1 H-pyrazol-4-yl)amino)-2-oxoethyl 2-(3,5-dimethyl-1 H-pyrazol-1-yl)acetate, 2-((5-fluoropyridin-2-yl)amino)-2-oxoethyl 2-(2-methyl-1 H-indol-3-yl)acetate, 2-(((1H-imidazol-2-yl)methyl)amino)-2-oxoethyl 2-(2-methyl-1 H-indol-3-yl)acetate, 2-oxo-2-(pyrimidin-5-ylamino)ethyl 2-(2-methyl-1 H-indol-3-yl)acetate, 2-oxo-2-(phenylamino)ethyl 2-(2-methyl-1 H-indol-1-yl)acetate, 2-oxo-2-(o-tolylamino)ethyl 2-(2-methyl-1 H-indol-1-yl)acetate, 2-((4-morpholinophenyl)amino)-2-oxoethyl 2-(2-methyl-1 H-indol-1-yl)acetate, 2-((2-(allyloxy)phenyl)amino)-2-oxoethyl 2-(2-iodophenyl)acetate, 2-((2-chlorophenyl)amino)-2-oxoethyl 2-(2-methyl-1 H-indol-3-yl)acetate, 2-oxo-2-(p-tolylamino)ethyl 2-(2-methyl-1 H-indol-3-yl)acetate, 2-oxo-2-(m-tolylamino)ethyl 2-(2-methyl-1 H-indol-3-yl)acetate, 2-((4-morpholinophenyl)amino)-2-oxoethyl 2-(2-methyl-1 H-indol-3-yl)acetate, 2-((2-methoxyphenyl)amino)-2-oxoethyl 2-(2-methyl-1 H-indol-3-yl)acetate, 2-oxo-2-(o-tolylamino)ethyl 2-(2-methyl-1 H-indol-3-yl)acetate, 2-((3-methoxyphenyl)amino)-2-oxoethyl 2-(2-methyl-1 H-indol-3-yl)acetate, 2-((4-methoxyphenyl)amino)-2-oxoethyl 2-(2-methyl-1 H-indol-3-yl)acetate, 2-((3-carbamoylphenyl)amino)-2-oxoethyl 2-(2-methyl-1 H-indol-3-yl)acetate, 2-((3,5-dimethyl-1-phenyl-1 H-pyrazol-4-yl)amino)-2-oxoethyl 2-(1H-indol-3-yl)acetate, 2-((3,5-dimethyl-1-phenyl-1 H-pyrazol-4-yl)amino)-2-oxoethyl 2-(1,2-dimethyl-1 H-indol-3-yl)acetate, 2-((3,5-dimethyl-1-phenyl-1 H-pyrazol-4-yl)amino)-2-oxoethyl 2-(2-methylpyridin-3-yl)acetate and 2-((3,5-dimethyl-1-phenyl-1 H-pyrazol-4-yl)amino)-2-oxoethyl 3,3,3-trifluoro-2-(2-methyl-1 H-indol-3-yl)propanoate
or a pharmaceutically acceptable salt, solvate or hydrate thereof.

In some embodiments the compound of Formula (I) is selected from the list consisting of 2-((3,5-dimethyl-1-(4-(trifluoromethyl)phenyl)-1 H-pyrazol-4-yl)amino)-2-oxoethyl 2-(2-methyl-1 H-indol-1-yl)acetate; 2-((3,5-dimethyl-1-(2-nitrophenyl)-1H-pyrazol-4-yl)amino)-2-oxoethyl 2-(2-methyl-1 H-indol-1-yl)acetate; 2-((1-(4-cyanophenyl)-3,5-dimethyl-1 H-pyrazol-4-yl)amino)-2-oxoethyl 2-(2-methyl-1 H-indol-1-yl)acetate; 2-((3,5-dimethyl-1-(2-(trifluoromethyl)phenyl)-1H-pyrazol-4-yl)amino)-2-oxoethyl 2-(2-methyl-1H-indol-1-yl)acetate; 2-((3,5-dimethyl-1-(4-nitrophenyl)-1H-pyrazol-4-yl)amino)-2-oxoethyl 2-(2-methyl-1 H-indol-1-yl)acetate; 2-((1-(4-((3-hydroxybutyl)amino)phenyl)-3,5-dimethyl-1H-pyrazol-4-yl)amino)-2-oxoethyl 2-(2-methyl-1 H-indol-1-yl)acetate; 2-((1-(4-aminophenyl)-3,5-dimethyl-1H-pyrazol-4-yl)amino)-2-oxoethyl 2-(2-methyl-1H-indol-1-yl)acetate and 2-((1-(2-aminophenyl)-3,5-dimethyl-1H-pyrazol-4-yl)amino)-2-oxoethyl 2-(2-methyl-1H-indol-1-yl)acetate
or a pharmaceutically acceptable salt, solvate or hydrate thereof.

In some embodiments the compound of Formula (I) is selected from the list consisting of 2-((3,5-dimethyl-1-phenyl-1H-pyrazol-4-yl)amino)-2-oxoethyl 2-(2-methyl-1H-indol-1-yl)acetate, 2-((3,5-dimethyl-1-phenyl-1 H-pyrazol-4-yl)amino)-2-oxoethyl 2-(2-methyl-1 H-indol-3-yl)acetate and 2-((3,5-dimethyl-1-phenyl-1H-pyrazol-4-yl)amino)-2-oxoethyl 2-(2-iodophenyl)acetate
or a pharmaceutically acceptable salt, solvate or hydrate thereof.

In some embodiments the compound of Formula (I) is 2-((3,5-dimethyl-1-phenyl-1 H-pyrazol-4-yl)amino)-2-oxoethyl 2-(2-methyl-1H-indol-1-yl)acetate or a pharmaceutically acceptable salt, solvate or hydrate thereof. In some embodiments the compounds of Formula (I) is 2-((3,5-dimethyl-1-phenyl-1 H-pyrazol-4-yl)amino)-2-oxoethyl 2-(2-methyl-1H-indol-3-yl)acetate or a pharmaceutically acceptable salt, solvate or hydrate thereof. In some embodiments the compounds of Formula (I) is 2-((3,5-dimethyl-1-phenyl-1 H-pyrazol-4-yl)amino)-2-oxoethyl 2-(2-iodophenyl)acetate or a pharmaceutically acceptable salt, solvate or hydrate thereof.

In some embodiments compound of Formula (I) is selected from the list consisting of 2-((3,5-dimethyl-1-(m-tolyl)-1H-pyrazol-4-yl)amino)-2-oxoethyl 2-(2-methyl-1 H-indol-1-yl)acetate, 2-((3,5-dimethyl-1-(4-(trifluoromethyl)phenyl)-1 H-pyrazol-4-yl)amino)-2-oxoethyl 2-(2-methyl-1H-indol-1-yl)acetate, 2-((3,5-dimethyl-1-(2-nitrophenyl)-1H-pyrazol-4-yl)amino)-2-oxoethyl 2-(2-methyl-1H-indol-1-yl)acetate, 2-((1-(4-cyanophenyl)-3,5-dimethyl-1H-pyrazol-4-yl)amino)-2-oxoethyl 2-(2-methyl-1H-indol-1-yl)acetate, 2-((1-(4-methoxybenzyl)-3,5-dimethyl-1H-pyrazol-4-yl)amino)-2-oxoethyl 2-(2-methyl-1H-indol-1-yl)acetate, 2-((3,5-dimethyl-1-(2-(trifluoromethyl)phenyl)-1H-pyrazol-4-yl)amino)-2-oxoethyl 2-(2-methyl-1H-indol-1-yl)acetate, 2-((3,5-dimethyl-1-(p-tolyl)-1H-pyrazol-4-yl)amino)-2-oxoethyl 2-(2-methyl-1H-indol-1-yl)acetate, 2-((3,5-dimethyl-1-(4-nitrophenyl)-1H-pyrazol-4-yl)amino)-2-oxoethyl 2-(2-methyl-1H-indol-1-yl)acetate, 2-((3,5-dimethyl-1-phenyl-1 H-pyrazol-4-yl)amino)-2-oxoethyl 2-(1H-indol-1-yl)acetate, 2-((3,5-dimethyl-1-(p-tolyl)-1H-pyrazol-4-yl)amino)-2-oxoethyl 2-(1 H-indol-1-yl)acetate, 2-((3,5-dimethyl-1-phenyl-1H-pyrazol-4-yl)amino)-2-oxoethyl 2-(3-methyl-1H-indol-1-yl)acetate, 2-((3,5-dimethyl-1-phenyl-1 H-pyrazol-4-yl)amino)-2-oxoethyl 2-(2,3-dimethyl-1 H-indol-1-yl)acetate, 2-((3,5-dimethyl-1-phenyl-1H-pyrazol-4-yl)amino)-2-oxoethyl 2-(2-(trifluoromethyl)-1H-indol-1-yl)acetate, 2-((3,5-dimethyl-1-phenyl-1 H-pyrazol-4-yl)amino)-2-oxoethyl 2-(1-methyl-1H-indol-3-yl)acetate, 2-((1-(4-((3-hydroxybutyl)amino)phenyl)-3,5-dimethyl-1H-pyrazol-4-yl)amino)-2-oxoethyl 2-(2-methyl-1 H-indol-1-yl)acetate, 2-((3,5-dimethyl-1-(p-tolyl)-1H-pyrazol-4-yl)amino)-2-oxoethyl 2-(2-(trifluoromethyl)-1H-indol-1-yl)acetate, 2-((3,5-dimethyl-1-(4-nitrophenyl)-1 H-pyrazol-4-yl)amino)-2-oxoethyl 2-(2-(trifluoromethyl)-1H-indol-1-yl)acetate, 2-((1-(4-aminophenyl)-3,5-dimethyl-1H-pyrazol-4-yl)amino)-2-oxoethyl 2-(2-methyl-1 H-indol-1-yl)acetate, 2-((1-(2-aminophenyl)-3,5-dimethyl-1H-pyrazol-4-yl)amino)-2-oxoethyl 2-(2-methyl-1 H-indol-1-yl)acetate, 2-((3,5-dimethyl-1-phenyl-1 H-pyrazol-4-yl)amino)-2-oxoethyl 2-(4-fluoro-1H-indol-1-yl)acetate, 2-((3,5-dimethyl-1-phenyl-1H-pyrazol-4-yl)amino)-2-oxoethyl 2-(6-chloro-1H-indol-1-yl)acetate, 2-((3,5-dimethyl-1-phenyl-1H-pyrazol-4-yl)amino)-2-oxoethyl 2-(5-chloro-1H-indol-1-yl)acetate, 2-((3,5-dimethyl-1-phenyl-1 H-pyrazol-4-yl)amino)-2-oxoethyl 2-(5-fluoro-1H-indol-1-yl)acetate, 2-((3,5-dimethyl-1-phenyl-1 H-pyrazol-4-yl)amino)-2-oxoethyl 2-(5-chloro-2-methyl-1H-indol-1-yl)acetate, 2-((3,5-dimethyl-1-phenyl-1 H-pyrazol-4-yl)amino)-2-oxoethyl 2-(7-chloro-1 H-indol-1-yl)acetate, 2-((3,5-dimethyl-1-phenyl-1 H-pyrazol-4-yl)amino)-2-oxoethyl 2-(2-isopropyl-1 H-indol-1-yl)acetate, 2-((3,5-dimethyl-1-phenyl-1 H-pyrazol-4-yl)amino)-2-oxoethyl 2-(7-chloro-2-methyl-1 H-indol-1-yl)acetate, 2-((3,5-dimethyl-1-phenyl-1 H-pyrazol-4-yl)amino)-2-oxoethyl 2-(2-methyl-1 H-indol-1-yl)propanoate, 2-((3,5-dimethyl-1-phenyl-1H-pyrazol-4-yl)amino)-2-oxoethyl 2-(2-ethyl-1H-indol-1-yl)acetate, 1-((3,5-dimethyl-1-phenyl-1H-pyrazol-4-yl)amino)-1-oxopropan-2-yl 2-(2-methyl-1H-indol-1-yl)acetate,

2-((3,5-dimethyl-1-phenyl-1 H-pyrazol-4-yl)amino)-2-oxoethyl 2-(2-methyl-1 H-indol-3-yl)acetate, 2-oxo-2-((1-phenyl-1H-pyrazol-4-yl)amino)ethyl 2-(2-methyl-1 H-indol-3-yl)acetate, 1-((3,5-dimethyl-1-phenyl-1H-pyrazol-4-yl)carbamoyl)cyclopropyl 2-(2-methyl-1H-indol-1-yl)acetate, 2-((3,5-dimethyl-1-phenyl-1H-pyrazol-4-yl)amino)-2-oxoethyl 2-(benzofuran-3-yl)acetate, 2-((3,5-dimethyl-1-phenyl-1H-pyrazol-4-yl)amino)-2-oxoethyl 2-(4-aminophenyl)acetate, 2-((3,5-dimethyl-1-phenyl-1H-pyrazol-4-yl)amino)-2-oxoethyl 2-(2-chlorophenyl)acetate, 2-((3,5-dimethyl-1-phenyl-1H-pyrazol-4-yl)amino)-2-oxoethyl 2-(4-cyanophenyl)acetate, 2-((3,5-dimethyl-1-phenyl-1H-pyrazol-4-yl)amino)-2-oxoethyl 2-(3-acetamidophenyl)acetate, 2-((3,5-dimethyl-1-phenyl-1 H-pyrazol-4-yl)amino)-2-oxoethyl 2-(2-bromophenyl)acetate, 2-((3,5-dimethyl-1-phenyl-1H-pyrazol-4-yl)amino)-2-oxoethyl 2-(m-tolyl)acetate, 2-((3,5-dimethyl-1-phenyl-1H-pyrazol-4-yl)amino)-2-oxoethyl 2-(2-iodophenyl)acetate, 2-((3,5-dimethyl-1-phenyl-1H-pyrazol-4-yl)amino)-2-oxoethyl 2-(3-aminophenyl)acetate, 2-((3,5-dimethyl-1-phenyl-1H-pyrazol-4-yl)amino)-2-oxoethyl 2-(3-cyanophenyl)acetate, 2-((3,5-dimethyl-1-phenyl-1H-pyrazol-4-yl)amino)-2-oxoethyl 2-(pyridin-3-yl)acetate, 2-((3,5-dimethyl-1-phenyl-1H-pyrazol-4-yl)amino)-2-oxoethyl 2-(pyridin-2-yl)acetate, 2-((3,5-dimethyl-1-phenyl-1 H-pyrazol-4-yl)amino)-2-oxoethyl 2-(5-chlorothiophen-2-yl)acetate, 2-((3,5-dimethyl-1-phenyl-1H-pyrazol-4-yl)amino)-2-oxoethyl 2-(2,5-dichlorothiophen-3-yl)acetate, 2-((3,5-dimethyl-1-phenyl-1H-pyrazol-4-yl)amino)-2-oxoethyl 2-(pyridin-4-yl)acetate, 2-((3,5-dimethyl-1-phenyl-1H-pyrazol-4-yl)amino)-2-oxoethyl 2-(2-(trifluoromethyl)phenyl)acetate, 2-((3,5-dimethyl-1-phenyl-1H-pyrazol-4-yl)amino)-2-oxoethyl 2-(3-fluorophenyl)acetate, 2-((3,5-dimethyl-1-phenyl-1 H-pyrazol-4-yl)amino)-2-oxoethyl 2-(2-chlorothiophen-3-yl)acetate, 2-((3,5-dimethyl-1-phenyl-1 H-pyrazol-4-yl)amino)-2-oxoethyl 2-(furan-2-yl)acetate, 2-((3,5-dimethyl-1-phenyl-1H-pyrazol-4-yl)amino)-2-oxoethyl 2-(2-(benzyloxy)phenyl)acetate, 2-((3,5-dimethyl-1-phenyl-1 H-pyrazol-4-yl)amino)-2-oxoethyl 2-(thiophen-3-yl)acetate, 2-((3,5-dimethyl-1-phenyl-1 H-pyrazol-4-yl)amino)-2-oxoethyl 2-(2-methyl-1 H-indol-3-yl)propanoate, 2-((3,5-dimethyl-1-phenyl-1H-pyrazol-4-yl)amino)-2-oxoethyl 2-(2-azidophenyl)acetate, 2-((3,5-dimethyl-1-phenyl-1H-pyrazol-4-yl)amino)-2-oxoethyl 2-(1H-indol-3-yl)propanoate, 2-((3,5-dimethyl-1-phenyl-1 H-pyrazol-4-yl)amino)-2-oxoethyl 3-(2-methyl-1 H-indol-3-yl)propanoate and 2-((3,5-dimethyl-1-phenyl-1 H-pyrazol-4-yl)(methyl)amino)-2-oxoethyl 2-(2-methyl-1 H-indol-3-yl)acetate
or a pharmaceutically acceptable salt, solvate or hydrate thereof.

In some embodiments compound of Formula (I) is selected from the list consisting of 2-oxo-2-(phenylamino)ethyl 2-(2-methyl-1 H-indol-1-yl)acetate, 2-oxo-2-(o-tolylamino)ethyl 2-(2-methyl-1 H-indol-1-yl)acetate, 2-((4-morpholinophenyl)amino)-2-oxoethyl 2-(2-methyl-1 H-indol-1-yl)acetate, 2-((2-(allyloxy)phenyl)amino)-2-oxoethyl 2-(2-iodophenyl)acetate, 2-((2-chlorophenyl)amino)-2-oxoethyl 2-(2-methyl-1 H-indol-3-yl)acetate, 2-oxo-2-(p-tolylamino)ethyl 2-(2-methyl-1 H-indol-3-yl)acetate, 2-oxo-2-(m-tolylamino)ethyl 2-(2-methyl-1 H-indol-3-yl)acetate, 2-((4-morpholinophenyl)amino)-2-oxoethyl 2-(2-methyl-1 H-indol-3-yl)acetate, 2-((2-methoxyphenyl)amino)-2-oxoethyl 2-(2-methyl-1 H-indol-3-yl)acetate, 2-oxo-2-(o-tolylamino)ethyl 2-(2-methyl-1 H-indol-3-yl)acetate, 2-((3-methoxyphenyl)amino)-2-oxoethyl 2-(2-methyl-1 H-indol-3-yl)acetate, 2-((4-methoxyphenyl)amino)-2-oxoethyl 2-(2-methyl-1 H-indol-3-yl)acetate and 2-((3-carbamoylphenyl)amino)-2-oxoethyl 2-(2-methyl-1 H-indol-3-yl)acetate
or a pharmaceutically acceptable salt, solvate or hydrate thereof.

A selection of compounds within the scope of, or use within the methods, of the present invention is listed in the following Table 1.

**Table 1**

| Compound number | Structure |
|---|---|
| **1** | 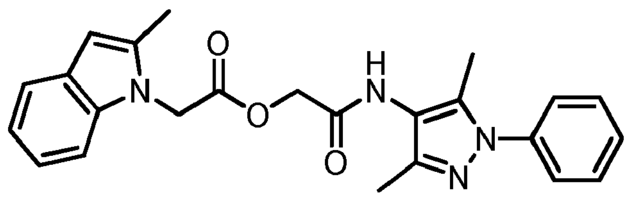 |
| **2** | 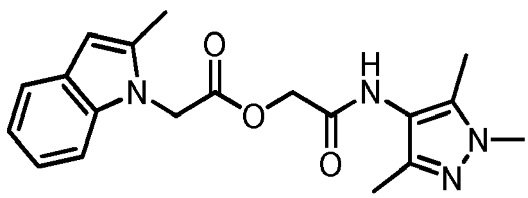 |

(continued)

| Compound number | Structure |
|---|---|
| **3** | 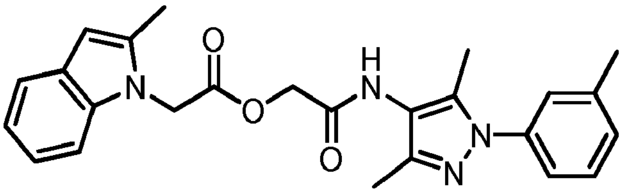 |
| **4** | 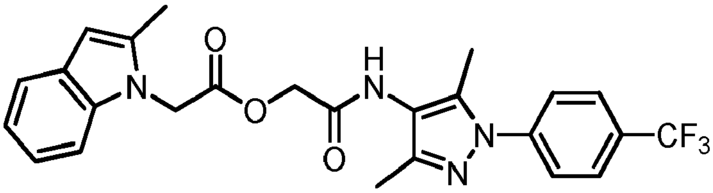 |
| **5** | 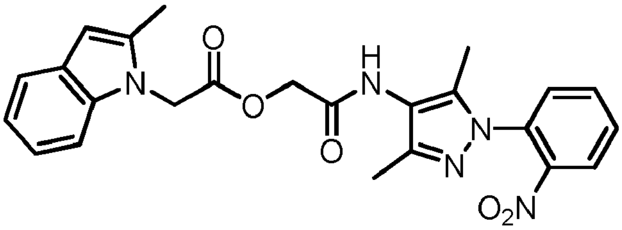 |
| **6** | 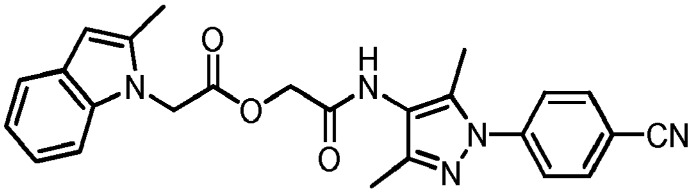 |
| **7** | 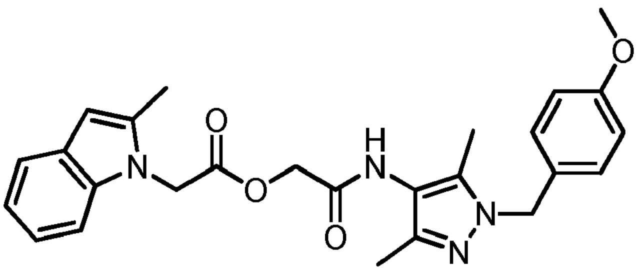 |
| **8** | 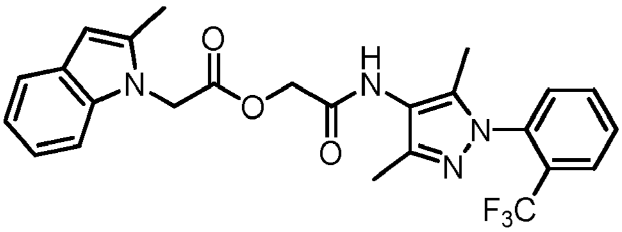 |
| **9** | 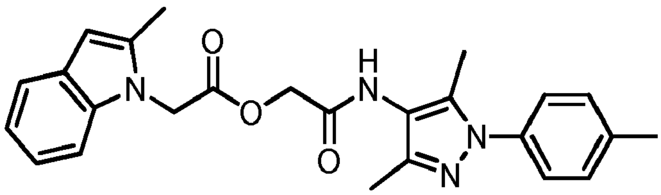 |

(continued)

| Compound number | Structure |
|---|---|
| **10** | 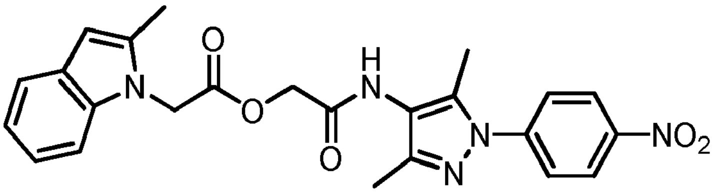 |
| **11** | 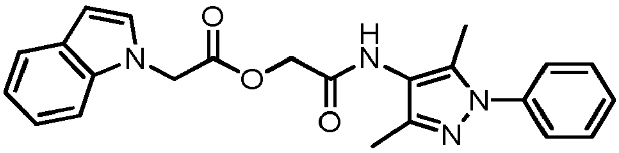 |
| **12** | 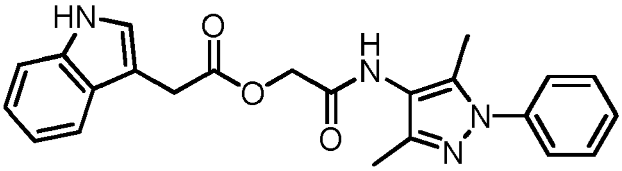 |
| **13** | 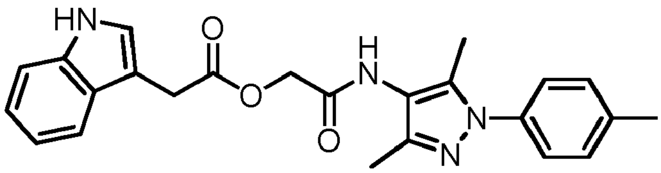 |
| **14** | 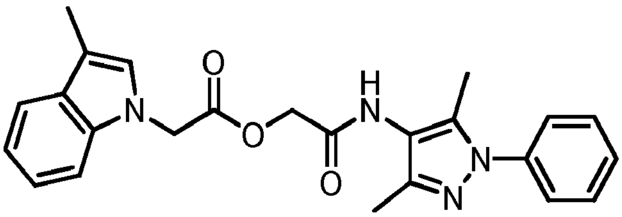 |
| **15** | 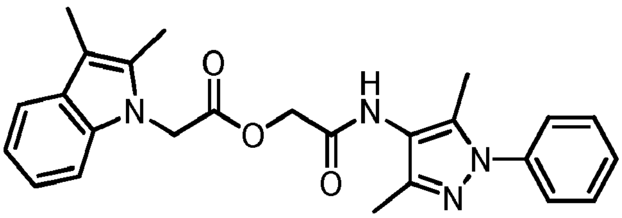 |
| **16** | 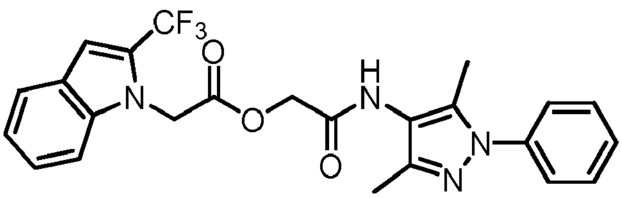 |

(continued)

| Compound number | Structure |
|---|---|
| **17** | 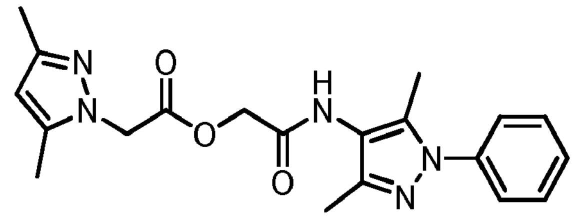 |
| **18** | 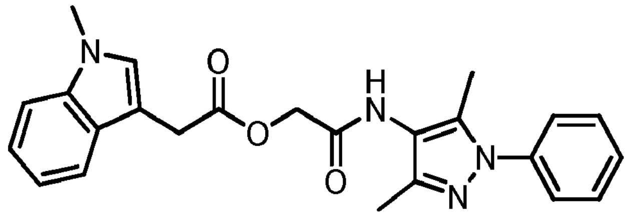 |
| **19** | 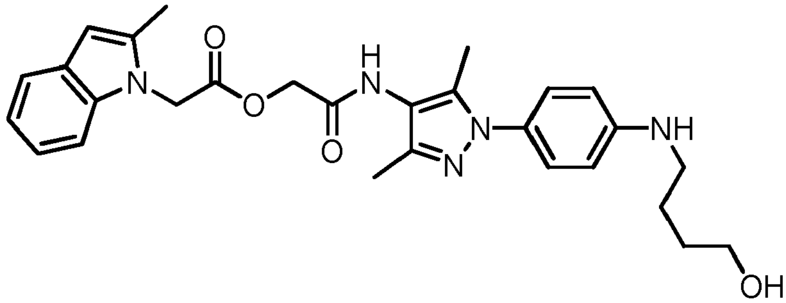 |
| **20** | 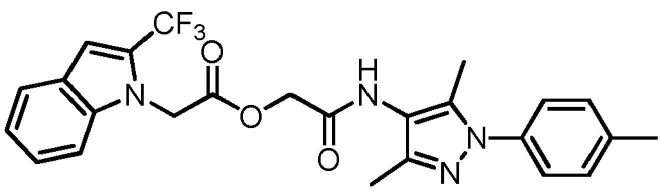 |
| **21** | 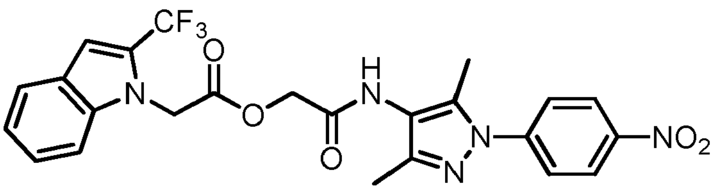 |
| **22** | 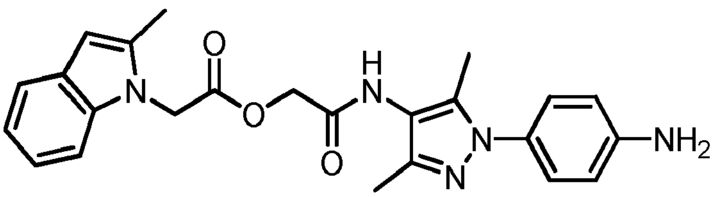 |

(continued)

| Compound number | Structure |
|---|---|
| **23** | 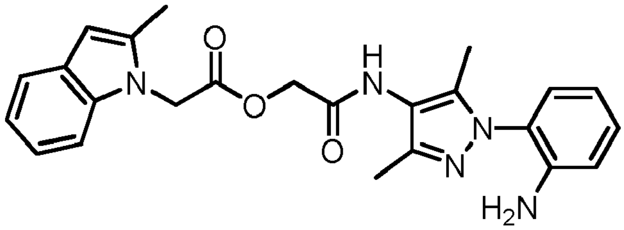 |
| **24** | 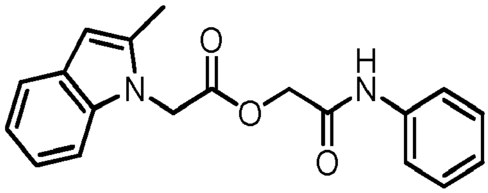 |
| **25** | 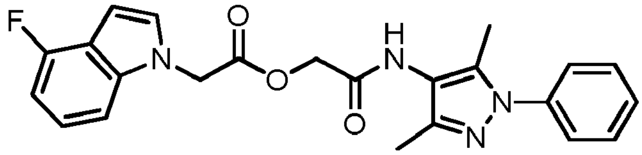 |
| **26** | 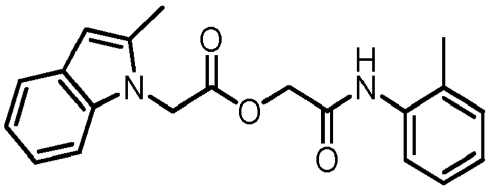 |
| **27** | 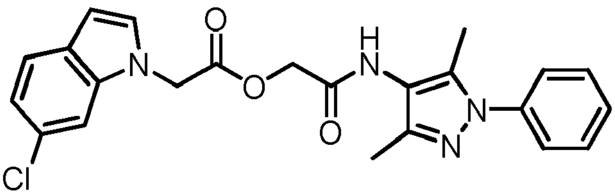 |
| **28** | 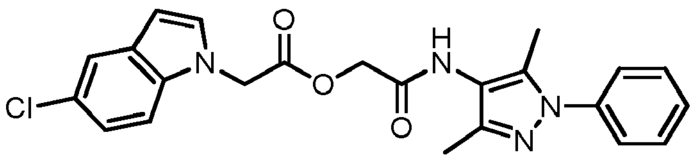 |
| **29** | 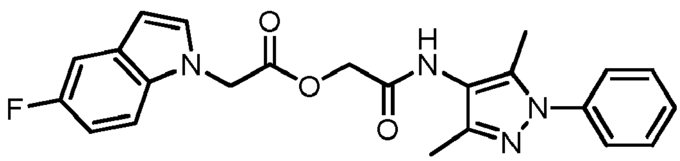 |
| **30** | 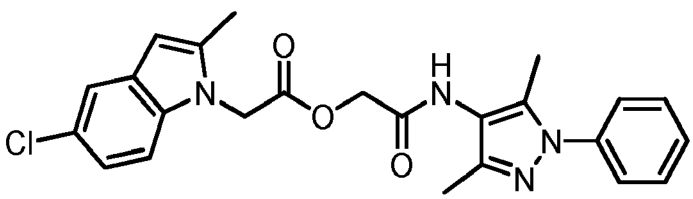 |

(continued)

| Compound number | Structure |
|---|---|
| **31** | 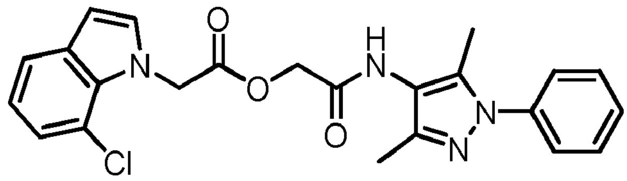 |
| **32** | 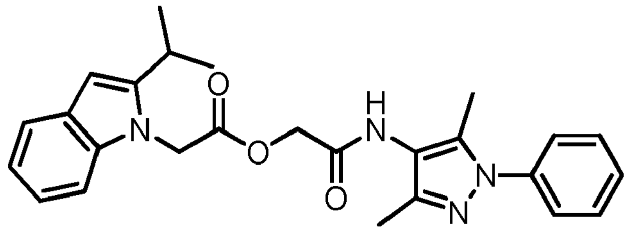 |
| **33** | 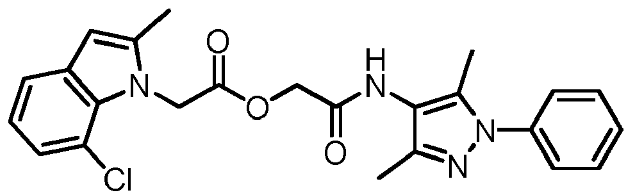 |
| **34** | 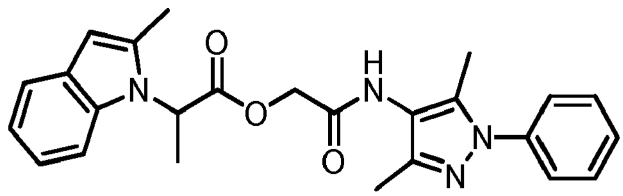 |
| **35** | 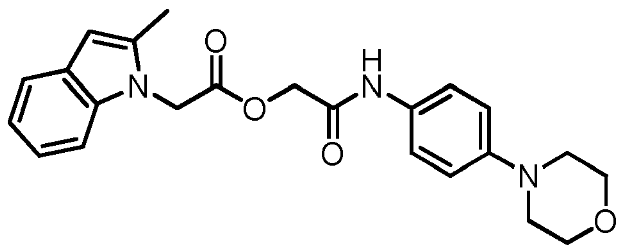 |
| **36** | 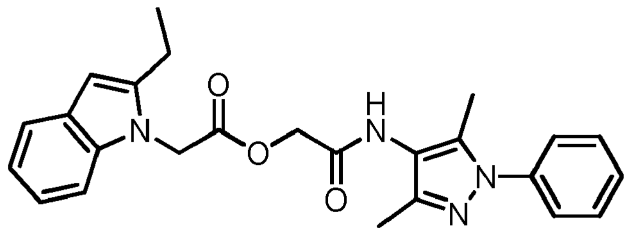 |
| **37** | 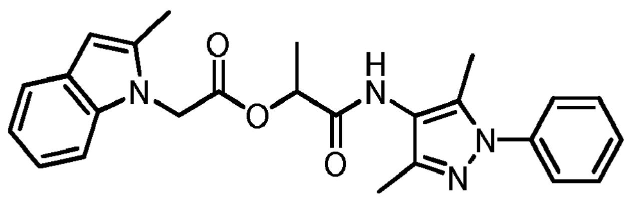 |

(continued)

| Compound number | Structure |
|---|---|
| **38** | 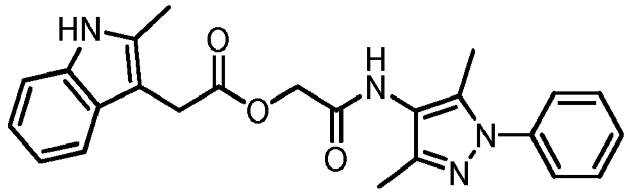 |
| **39** | 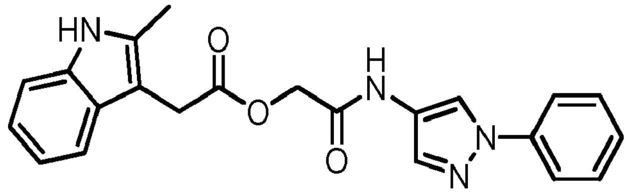 |
| **40** | 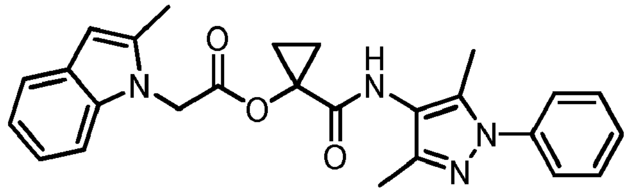 |
| **41** | 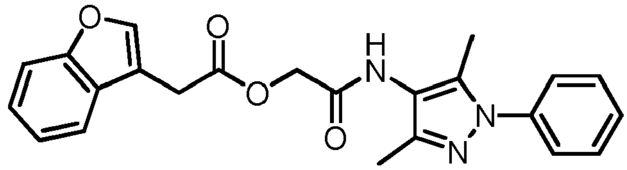 |
| **42** | 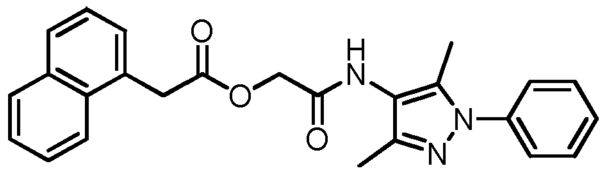 |
| **43** | 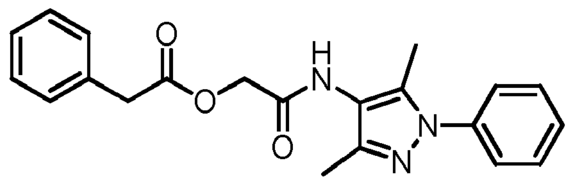 |
| **44** | 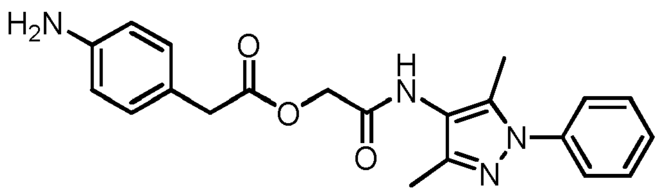 |
| **45** | 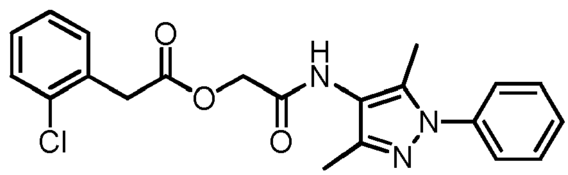 |

(continued)

| Compound number | Structure |
|---|---|
| **46** | 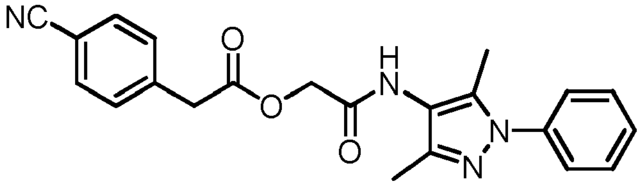 |
| **47** | 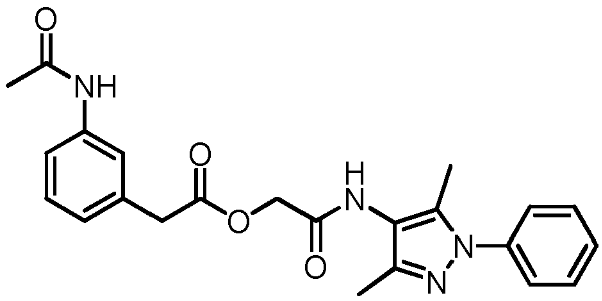 |
| **48** | 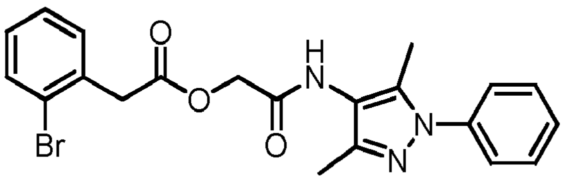 |
| **49** | 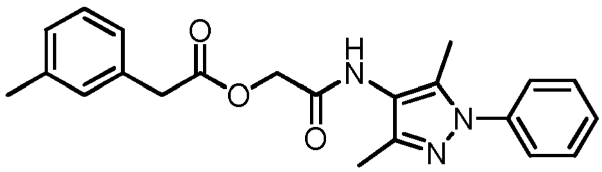 |
| **50** | 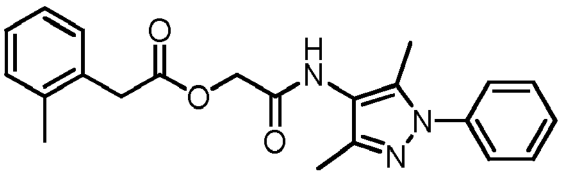 |
| **51** | 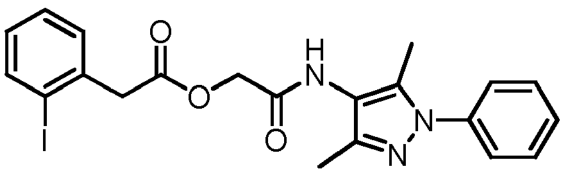 |
| **52** | 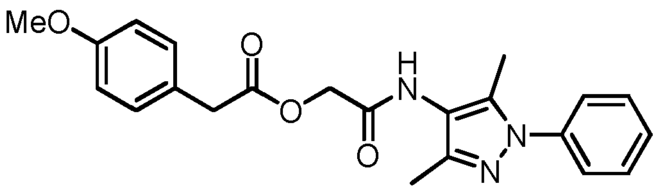 |

(continued)

| Compound number | Structure |
|---|---|
| **53** | 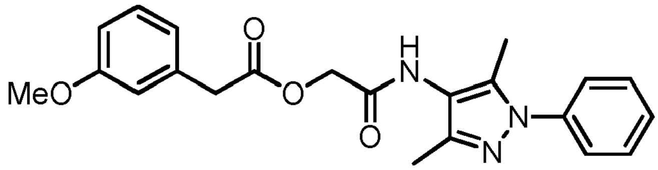 |
| **54** | 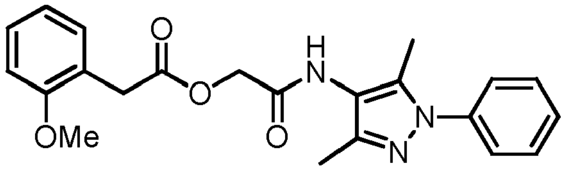 |
| **55** | 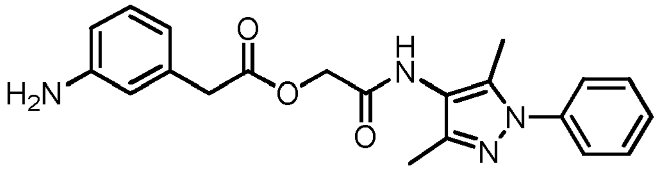 |
| **56** | 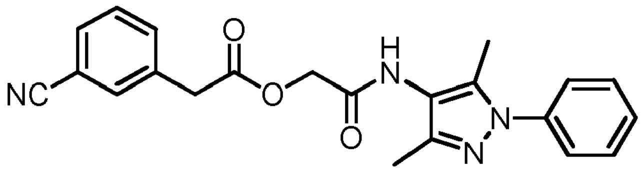 |
| **57** | 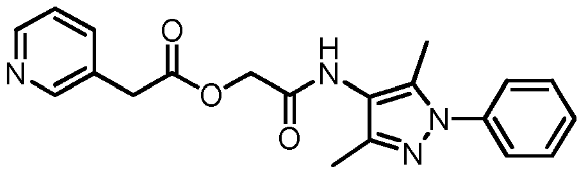 |
| **58** | 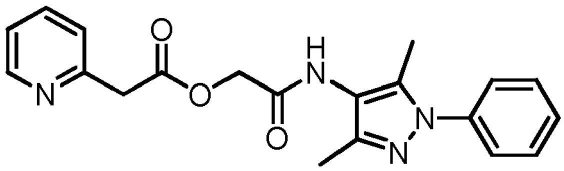 |
| **59** | 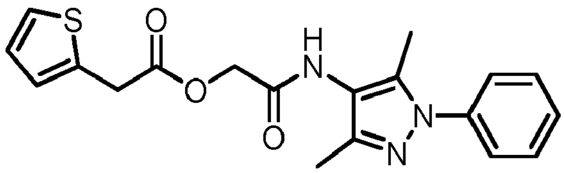 |
| **60** | 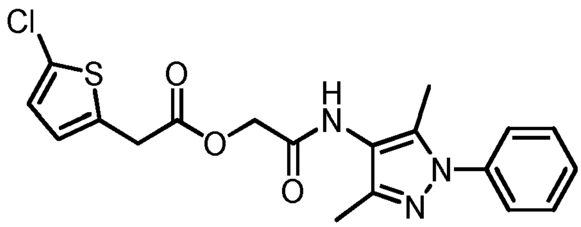 |

(continued)

| Compound number | Structure |
|---|---|
| **61** | 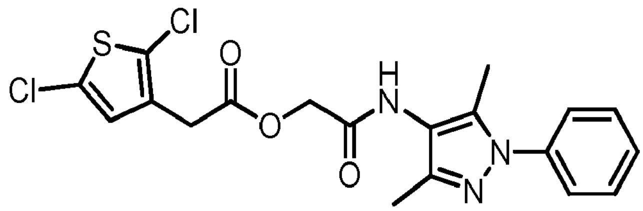 |
| **62** | 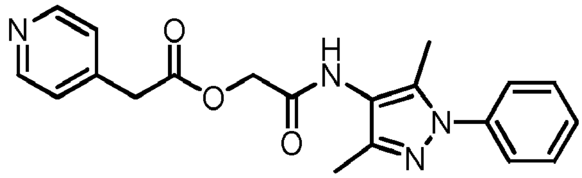 |
| **63** | 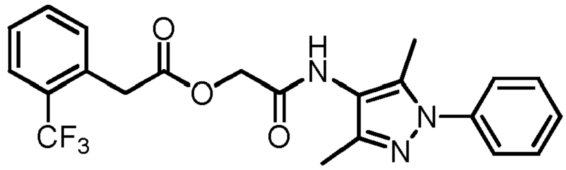 |
| **64** | 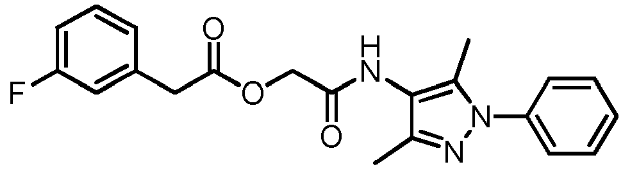 |
| **65** | 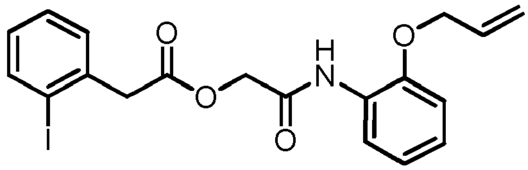 |
| **66** | 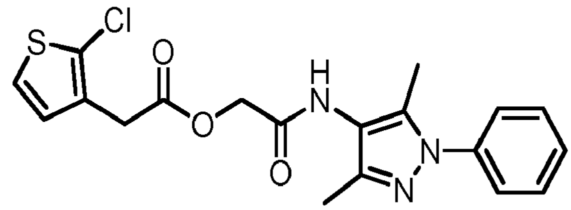 |
| **67** | 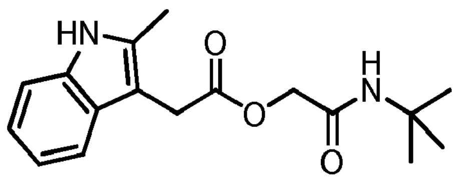 |
| **68** | 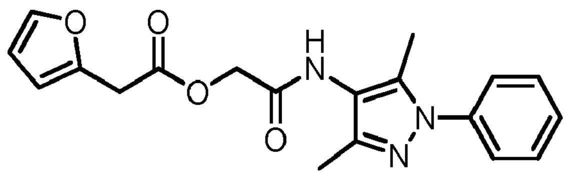 |

(continued)

| Compound number | Structure |
|---|---|
| **69** | 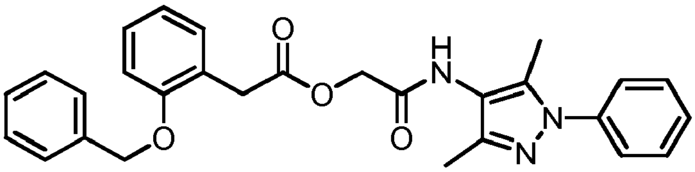 |
| **70** | 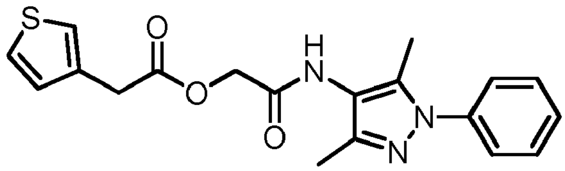 |
| **71** | 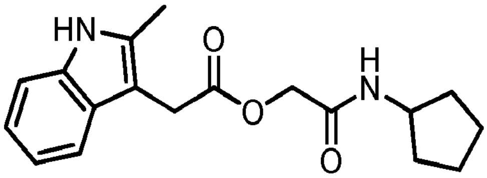 |
| **72** | 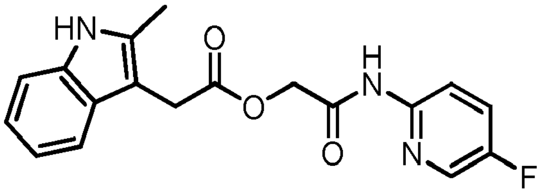 |
| **73** | 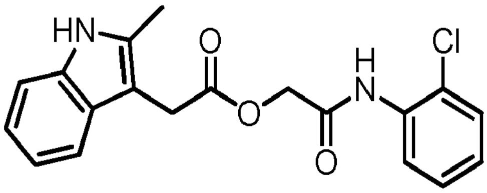 |
| **74** | 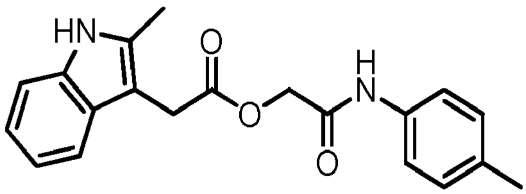 |
| **75** | 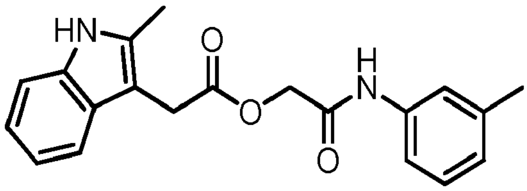 |

(continued)

| Compound number | Structure |
|---|---|
| **76** | 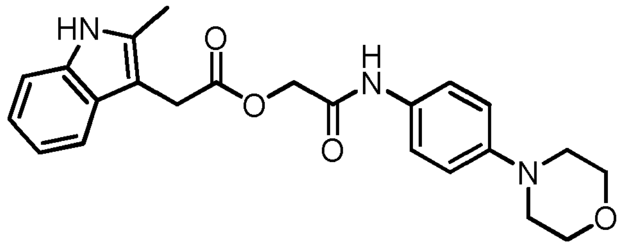 |
| **77** | 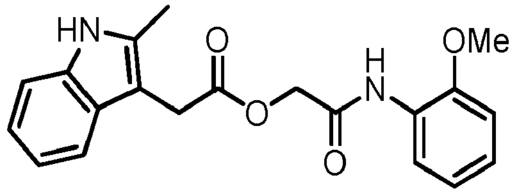 |
| **78** | 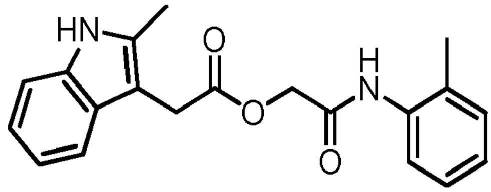 |
| **79** | 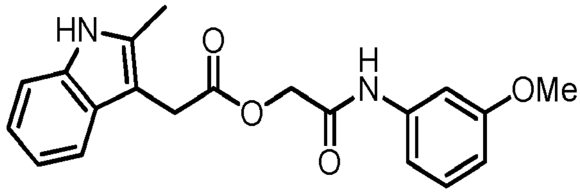 |
| **80** | 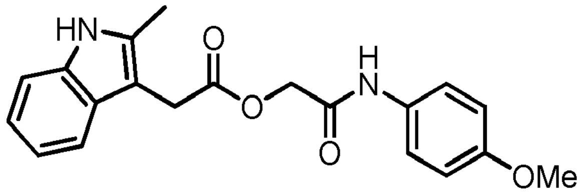 |
| **81** | 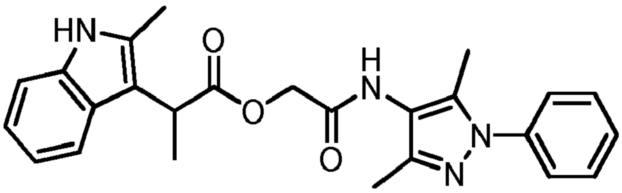 |
| **82** | 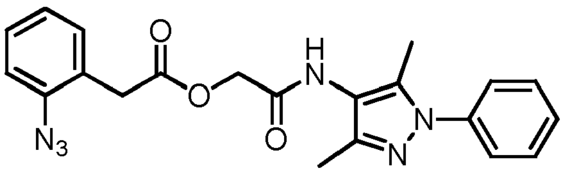 |

(continued)

| Compound number | Structure |
|---|---|
| **83** | 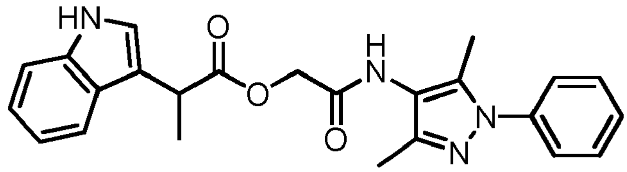 |
| **84** | 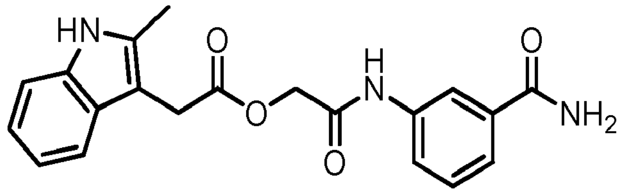 |
| **85** | 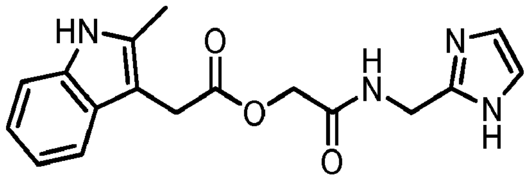 |
| **86** | 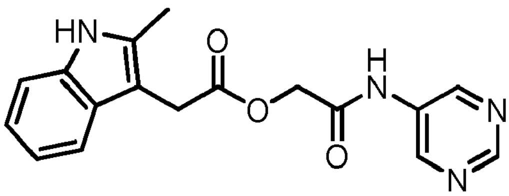 |
| **87** | 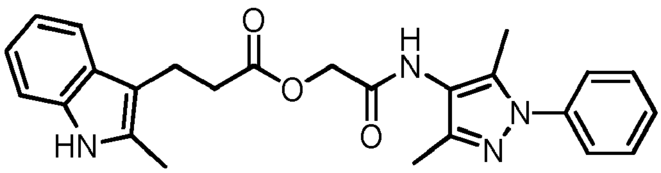 |
| **88** | 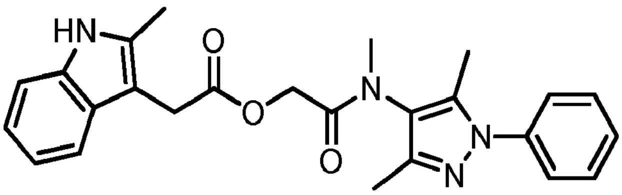 |
| **89** | 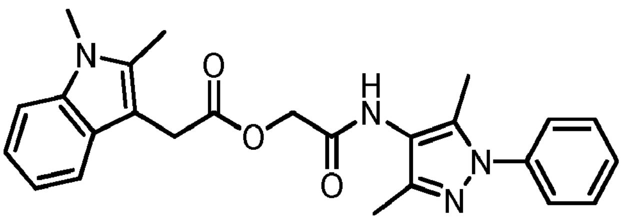 |
| **90** | 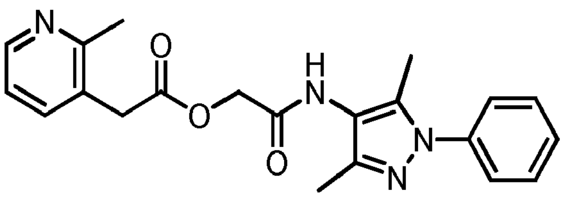 |

(continued)

| Compound number | Structure |
|---|---|
| 91 | 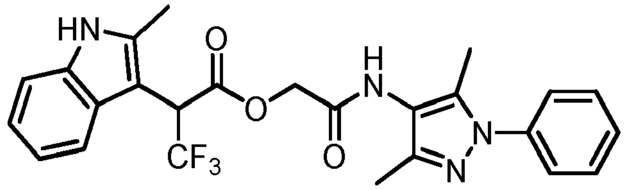 |

The compound numbering in Table 1 is equal to the numbering of the Example Compounds in the Examples section herein below.

In a further aspect, the present invention provides a compound of the invention (in particular those specified above with respect to any of formulas (I), (II), (III) and (IV), as well as the compounds of Table 1 for use as medicament.

As it is evident from the examples, the inventors have found that the compounds of the invention inhibit the enzymatic activity of human kallikrein-like peptidase 6 (KLK6). In some embodiments the compounds of the invention are selective inhibitors of human kallikrein-like peptidase 6. In some embodiments, the compounds of the present invention exhibit pharmacological properties (bioavailability, toxicity, side effects, dosing, patient compliance, compatibility, stability, half-life, etc.), which are in at least one aspect superior to the pharmacological properties of known kalikrein inhibitors.

The compounds of the present invention can be prepared as described below or prepared by methods analogous thereto, which are readily known and available to a person of ordinary skill in the art of organic synthesis. The synthetic access to the compounds according to the invention, i.e. the compounds of formulas (I), (II), (III) and (IV) is further illustrated by the example compounds in the description.

For example, the preparation of compounds of the formula I, including their salts, solvates or hydrates can be carried out by the conversion of a primary amine of the formula V to an amide of the formula IX via reaction with compounds of the formulas VI, VII, or VIII as exemplarily shown in the below reaction scheme.

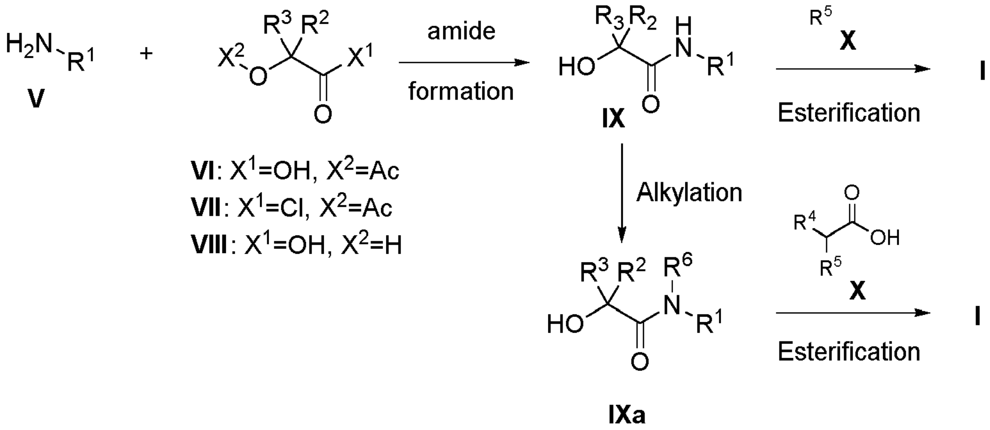


In the first instance, condensation of V and VI can be e.g. accomplished with peptide coupling reagents such as e.g. N,N'-dicyclohexylcarbodiimide (DCC), N,N'-diisopropylcarbodiimide (DIC), or 1-ethyl-3-(3-dimethylaminopropyl)carbodiimide (EDCI) in the presence of a catalyst like 4-dimethylaminopyridine (DMAP) in dichloromethane (DCM) as solvent. The hydroxyl functionality is then revealed by cleavage of the acetyl (Ac) group via saponification of the acetyl ester bond with e.g. sodium methoxide (NaOMe) in methanol (MeOH) to give compounds of the formula IX. In the second instance, reaction of V with acid chloride VII is e.g. conducted in the presence of a base like e.g. triethylamine ($Et_3N$) or diisopropylethylamine ($i$-$Pr_2NEt$) in DCM. Instead of a chloride any other suitable leaving group known to a person of ordinary skill may be used, such as other halides. Saponification of the acetyl ester can then be conducted with NaOMe as described above to give compounds of the formula IX. In the third instance, the reaction of V and VIII to directly give IX is performed e.g. with EDCI, 1-hydroxybenzotriazole (HOBT), and $i$-$Pr_2NEt$ in dimethylformamide (DMF). As appreciated by a person of ordinary skill, in these transformations other hydroxyl protecting groups than acetyl (Ac) may be used in formulas VI and VII, i.e. protecting groups stable under the reaction conditions and cleavable under different reaction conditions to allow the preparation of compounds of the formula IX, such as e.g. a benzyl group or a

silyl group. Compounds of the formula IX can then be converted into compounds of the formula I via esterification with compounds of the formula X as shown in the reaction scheme above. This can be e.g. conducted with dehydrating agents like e.g. DCC or EDCI in the presence of DMAP and *i*-$Pr_2NEt$ in DCM. It can also be conducted by e.g. first converting the carboxylic acid moiety in X into the corresponding acid chloride by the action of oxalyl chloride with catalytic amounts of DMF in DCM. Subsequent reaction with IX in the presence of a base like $Et_3N$, pyridine, or *i*-$Pr_2NEt$ and catalytic amounts of DMAP in a solvent like DCM or tetrahydrofuran (THF) provides compounds of the formula I. Compounds of the formula IX can also be converted to compounds of the formula IXa via alkylation of the amide nitrogen as shown in the above reaction scheme with e.g. an alkyl halide in DMF with sodium hydride (NaH) as a base. Compounds of the formula IXa can then be reacted with compounds of the formula X to provide compounds of the formula I by way of esterification using e.g. the same methods described above. In these syntheses of compounds of the formula (I) the R groups, in particular $R^1$, $R^2$, $R^3$, $R^4$, $R^5$ and $R^6$, are defined as described throughout this specification. These syntheses are equally applicable to the preparation of compounds of the formulas II, III and IV.

In some instances, compounds of the formulas I, II, IV and V as described herein have an $R^1$ group that is a substituted pyrazole, wherein a nitrogen atom of the pyrazole is bound to an aryl or a heteroaryl group. In these cases, compounds of the formula XIII can be e.g. prepared from a nitro substituted pyrazole XI via Ullmann coupling or nucleophilic aromatic substitution ($S_NAr$) reactions as exemplarily shown for an aryl halide XII in the below reaction scheme.

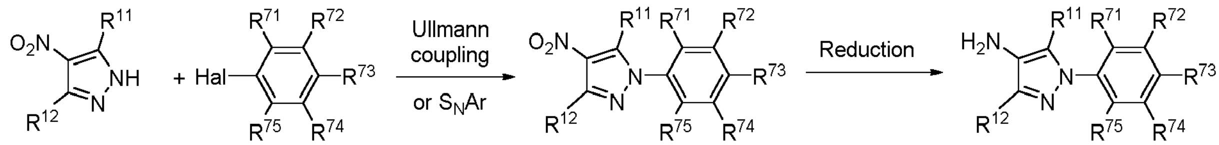


XI XII XIII V

Hal = F, Cl, Br, I,
preferably I in case
of Ullmann coupling,
preferably F in case
of $S_NAr$

In the first instance, using an Ullman coupling, a pyrazole XI is e.g. heated with an aryl halide or heteroaryl halide (preferably an iodide), an alkali carbonate (e.g. potassium carbonate), catalytic amounts of cuprous iodide (CuI), and the ligand trans-N,N'-di-methylcyclohexane-1,2-diamine to 180 °C in a microwave reactor to give compounds of the formula XIII. In the second instance, using an $S_NAr$ reaction, XI may be e.g. heated with an electron poor aryl halide or heteroaryl halide (preferably a fluoride) in the presence of an alkali *tert*-butoxide (e.g. potassium *tert*-butoxide) in dimethylsulfoxide (DMSO) to give compounds of the formula XIII. As appreciated by a person of ordinary skill, other suitable leaving groups may be used instead of a halide, such as a tosylate. Compounds of the formula XIII can be converted to compounds of the formula V by way of reduction using e.g. hydrogenation with hydrogen ($H_2$) (at atmospheric or elevated pressures) with 10% Pd/C as a catalyst in ethanol (EtOH) as a solvent, or stannous chloride ($SnCl_2$) in EtOH. Compounds of formula V can then be used to prepare compounds of the formula I as described herein above. In these syntheses of compounds of the formula V the R groups, in particular $R^{11}$, $R^{12}$, $R^{71}$, $R^{72}$, $R^{73}$, $R^{74}$ and $R^{75}$, are defined as described throughout this specification.

Compounds of the formula V can also be prepared via $S_NAr$ reaction of an aminopyrazole XIV with an electron-poor aryl halide or heteroaryl halide (preferably a fluoride) as exemplarily shown for an aryl halide in the below reaction scheme.

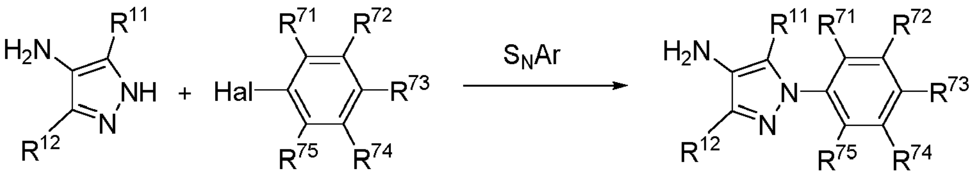


XIV XII V

Hal = F, Cl, Br, I, preferably **F**

In this case XIV can be e.g. heated with the electron-poor aryl halide or heteroaryl halide (preferably a fluoride) in the presence of an alkali *tert*-butoxide (e.g. potassium tert-butoxide) in dimethylsulfoxide (DMSO) to give compounds of the formula V. As appreciated by a person of ordinary skill, other suitable leaving groups may be used instead of a

halide, such as a tosylate. In these syntheses of compounds of the formula V the R groups, in particular $R^{11}$, $R^{12}$, $R^{71}$, $R^{72}$, $R^{73}$, $R^{74}$ and $R^{75}$, are defined as described throughout this specification. Compounds of formula V can then be used to prepare compounds of the formula I as described herein above.

In some instances, compounds of the formulas I, II and V as described herein have an $R^1$ group that is a substituted pyrazole, wherein a nitrogen atom of the pyrazole is bound to an alkyl, arylalkyl or heteroarylalkyl group. In these cases, compounds of the formula XVI can be prepared reacting a pyrazole XI with a halide XV, preferably a bromide or an iodide, in a nucleophilic substitution ($S_N$) reaction as exemplarily shown in the below reaction scheme.

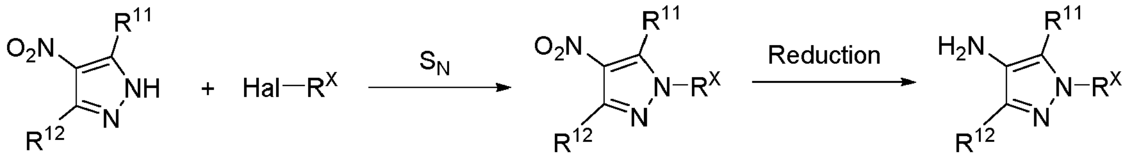


XI XV XVI V

Hal = F, Cl, Br, I,
preferably Br, I;
$R^X$ = alkyl, arylalkyl
or heteroarylalkyl

In this instance, XI may be heated with the halide XV in the presence of an alkali carbonate, e.g. potassium carbonate, in DMF to give compounds of formula XVI. As appreciated by a person of ordinary skill, other suitable leaving groups may be used instead of a halide, such as a tosylate. Compounds of the formula XVI can be converted into compounds of the formula V by way of reduction using e.g. hydrogenation with $H_2$ (at atmospheric or elevated pressures) with 10% Pd/C as a catalyst in EtOH as a solvent, or $SnCl_2$ in EtOH. In these syntheses of compounds of the formula V the R groups, in particular $R^{11}$ and $R^{12}$, are defined as described throughout this specification. Compounds of formula V can then be used to prepare compounds of the formula I as described herein above.

Compounds of the formula I can also be prepared as exemplarily shown in the below reaction scheme by condensation of acids of the formula X with alcohols of the formula XVII using e.g. dehydrating agents like DCC or EDCI in the presence of DMAP and *i*-$Pr_2$NEt in DCM.

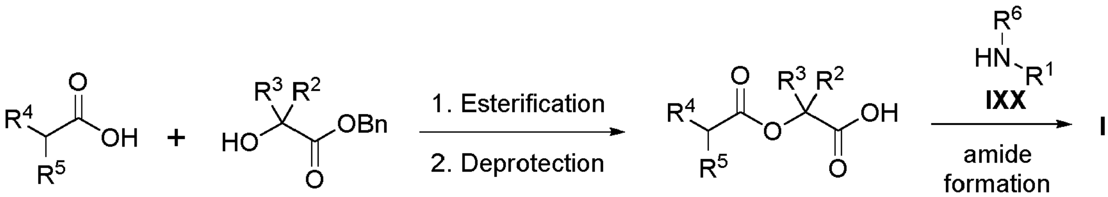


X XVII XVIII

Removal of the benzyl protecting group (Bn) in such substances e.g. with $H_2$ in EtOAc with 10% Pd/C as a catalyst gives acids of the formula XVIII. As appreciated by a person of ordinary skill, in these transformations other carboxylic acid protecting groups than benzyl (Bn) may be used, i.e. protecting groups stable under the reaction conditions and cleavable under different reaction conditions to allow the preparation of compounds of the formula XVIII, such as e.g. a methyl group or a tert-butyl group. The compounds of the formula XVIII can then be converted into compounds of the formula I via amide formation with amines of the formula IXX e.g. with dehydrating agents like DCC or EDCI in DCM, or e.g. via conversion of the carboxylic acid moiety in XVIII to the corresponding carboxylic acid chloride with oxalyl chloride and catalytic amounts of DMF in DCM before reacting with amine IXX in DCM with a base like $Et_3N$ or *i*-$Pr_2$NEt. In these syntheses of compounds of the formula I the R groups, in particular $R^1$, $R^2$, $R^3$, $R^4$, $R^5$ and $R^6$, are defined as described throughout this specification.

In some instances, compounds of the formula I may bear aryl nitro groups or aryl amino groups on $R^1$, $R^4$, $R^5$ or $R^6$. Compounds of the formula I which contain aryl nitro groups on $R^1$, $R^4$, $R^5$, or $R^6$ can be converted into compounds with aryl amino groups of the formula I e.g. via hydrogenation with $H_2$ and 10% Pd/C in EtOAc as exemplarily shown in the below reaction scheme. These substances can be further derivatized to the corresponding N-acyl substances e.g. via the action of acetic anhydride and pyridine in DCM.

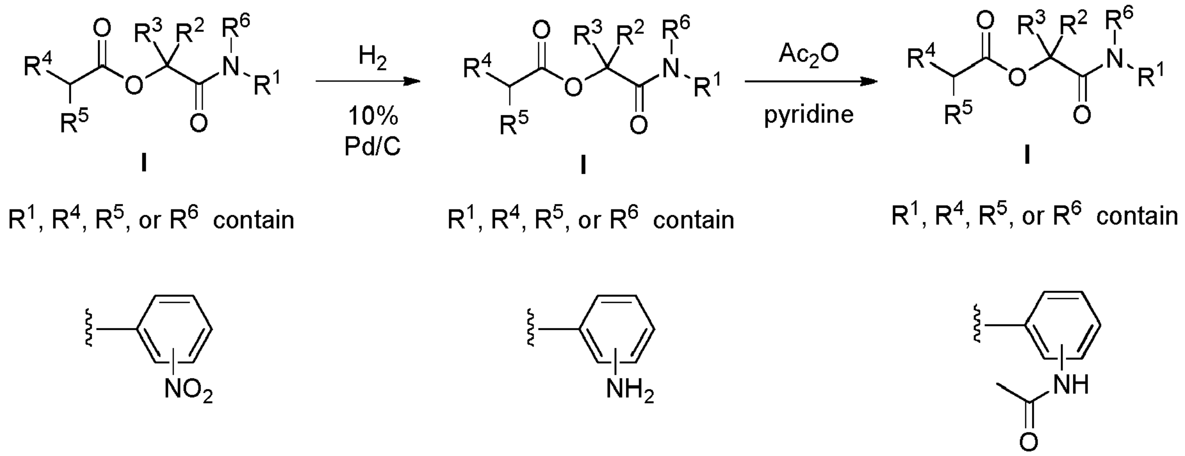


In any one of the syntheses described herein additional functional groups can be present in the compounds in protected form or in the form of a precursor group which is later converted into the final group.

The starting compounds and building blocks for the synthesis of the compounds of the formula I are commercially available or can be prepared according to procedures described in the literature or analogously to such procedures.

A person of ordinary skill will appreciate that other routes of synthesis may be employed as well. In particular, other routes of synthesis may in fact be applied to certain embodiments of the compounds disclosed herein. Likewise, other suitable reaction conditions than those described herein regarding e.g. reagents, temperature, solvents, reaction time and the like are known to a person of ordinary skill and/or can be readily selected. The person of ordinary skill is referred to general textbooks, such as March's Advanced Organic Chemistry (Michael B. Smith & Jerry March, Wiley-Interscience, 2000), The Practice of Medicinal Chemistry (Camile G. Wermuth, Academia Press, 2003) and Protective Groups in Organic Synthesis (Theodora W. Greene & Peter G.M. Wuts; John Wiley & Sons Inc, 1999).

*Pharmaceutical compositions*

In a further aspect, the present invention provides a pharmaceutical composition comprising a compound as specified above under the heading "Compounds" and one or more pharmaceutically acceptable excipients.

The compounds described in present invention (in particular those specified above such as those of formula (I), (II), (III) and/or (IV), as well as the compounds of Table 1) are preferably administered to a patient in need thereof via a pharmaceutical composition. In one embodiment, the pharmaceutical composition comprises a compound as described above (e.g. having the general formula (I), (II), (III) and/or (IV), as well as the compounds of Table 1 or a hydrate, solvate, salt, complex, racemic mixture, diastereomer, enantiomer, or tautomer thereof or an isotopically enriched form of any of the foregoing) and one or more pharmaceutically acceptable excipients.

The pharmaceutical composition may be administered to an individual by any route, such as enterally or parenterally.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral,intracerebroventricular, subarachnoid, intraspinal, epidural and intrasternal administration (such as by injection and/or infusion) as well as topical administration (e.g., epicutaneous, inhalational, or through mucous membranes (such as buccal, sublingual or vaginal)).

The compounds used in the present invention are generally applied in "pharmaceutically acceptable amounts" and in "pharmaceutically acceptable preparations". Such compositions may contain salts, buffers, preserving agents, carriers and optionally other therapeutic agents. "Pharmaceutically acceptable salts" comprise, for example, acid addition salts which may, for example, be formed by mixing a solution of compounds with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, Berge et al., "Pharmaceutical Salts", J. Pharm. Sci., 66, pp. 1-19 (1977)).

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, emulsifiers, buffers, flavoring agents, colorants, or antioxidants.

The compositions described in the present invention may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)). Depending on the route of administration, the active compound, i.e., the compound used in the present invention, either alone or in combination with one or more additional active compounds, may be coated in a material to protect the active compound(s) from the action of acids and other natural conditions that may inactivate the active compound.

Examples of suitable aqueous and non-aqueous carriers which may be employed in the pharmaceutical compositions used according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, vegetable oils (such as olive oil), and injectable organic esters (such as ethyl oleate). Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions used according to the present invention is contemplated.

The compositions may also contain adjuvants such as preservatives, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds, which may be used in a suitable hydrated form, and/or the pharmaceutical compositions used according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999.).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound used in the present invention by certain routes of administration, it may be necessary to coat the compound with, or co-administer the compound with, a material to prevent its inactivation. For example, the compound may be administered to an individual in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7: 27(1984)).

Pharmaceutical compositions typically are sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

An injectable composition should be sterile and fluid to the extent that the composition is deliverable by syringe. In addition to water, the carrier can be an isotonic buffered saline solution, ethanol, polyol (for example, glycerol, propylene glycol, and liquid polyethylene glycol, and the like), and suitable mixtures thereof. Sterile injectable solutions can be prepared by incorporating the active compound in the required amount in an appropriate solvent with one or a combination of ingredients enumerated above, as required, followed by sterilization microfiltration.

Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying

(lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic/pharmaceutical formulations, compositions used according to the present invention include those suitable for enteral administration (such as oral or rectal) or parenteral administration (such as nasal, topical (including vaginal, buccal and sublingual)). The compositions may conveniently be presented in unit dosage form and may be prepared by any methods known in the art of pharmacy. The amount of active ingredient (in particular, the amount of a compound used according to the present invention) which can be combined with a carrier material to produce a pharmaceutical composition (such as a single dosage form) will vary depending upon the individual being treated, and the particular mode of administration. The amount of active ingredient which can be combined with a carrier material to produce a single dosage form will generally be that amount of the composition which produces a therapeutic effect.

Generally, out of 100% (for the pharmaceutical formulations/compositions), the amount of active ingredient (in particular, the amount of the compound used according to the present invention, optionally together with other therapeutically active agents, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active ingredient, e.g., a compound used according to the present invention, in a unit dosage form and/or when administered to an individual or used in therapy, may range from about 0.1 mg to about 1000 mg (for example, from about 1 mg to about 500 mg, such as from about 10 mg to about 200 mg) per unit, administration or therapy. In certain embodiments, a suitable amount of such active ingredient may be calculated using the mass or body surface area of the individual, including amounts of between about 1 mg/kg and 10 mg/kg (such as between about 2

mg/kg and 5 mg/kg), or between about 1 mg/$m^2$ and about 400 mg/$m^2$ (such as between about 3 mg/$m^2$ and about 350 mg/$m^2$ or between about 10 mg/$m^2$ and about 200 mg/$m^2$).

Actual dosage levels of the active ingredients in the pharmaceutical compositions used according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start with doses of the compounds used according to the present invention at levels lower than that required in order to achieve the desired therapeutic effect and gradually increase the dosage until the desired effect is achieved. In general, a suitable daily dose of a composition used according to the present invention will be that amount of the compound which is the lowest dose effective to produce a therapeutic effect. Such an effective dose will generally depend upon the factors described above. It is preferred that administration be oral, intravenous, intramuscular, intraperitoneal, or subcutaneous, preferably administered proximal to the site of the target. If desired, the effective daily dose of a pharmaceutical composition may be administered as two, three, four, five, six or more sub-doses administered separately at appropriate intervals throughout the day, optionally, in unit dosage forms. While it is possible for a compound used according to the present invention to be administered alone, it is preferable to administer the compound as a pharmaceutical formulation/composition.

For oral administration, the pharmaceutical composition used according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate. Preparations for oral administration can be suitably formulated to give controlled release of the pharmaceutical composition of the invention.

In one embodiment, the compound is orally administered in a concentration of at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound is parenterally administered (e.g., intravenously, intramuscularly, or subcutaneously), in a concentration of at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The pharmaceutical composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.

The pharmaceutical composition used according to the present invention can be formulated for parenteral administration by injection, for example, by bolus injection or continuous infusion. In one embodiment, the compounds or compositions used according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In yet another embodiment, the compounds or compositions used according to the present invention are administered by maintenance therapy, such as, e.g., once a week for a period of 6 months or more.

Formulations for injection can be presented in units dosage form (e.g., in phial, in multi-dose container), and with an added preservative. The pharmaceutical composition used according to the present invention can take such forms as suspensions, solutions or emulsions in oily or aqueous vehicles, and can contain formulatory agents such as

suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Compositions used according to the present invention which are suitable for vaginal administration also include pessaries, tampons, creams, gels, pastes, foams or spray formulations containing such carriers as are known in the art to be appropriate. Dosage forms for the topical or transdermal administration of compositions used according to the present invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

Therapeutic/pharmaceutical compositions can be administered with medical devices known in the art. For example, in a preferred embodiment, a therapeutic/pharmaceutical composition used according to the present invention can be administered with a needleless hypodermic injection device, such as the devices disclosed in US 5,399,163; US 5,383,851; US 5,312,335; US 5,064,413; US 4,941,880; US 4,790,824; or US 4,596,556. Examples of well-known implants and modules useful in the present invention include those described in: US 4,487,603, which discloses an implantable micro-infusion pump for dispensing medication at a controlled rate; US 4,486,194, which discloses a therapeutic device for administering medicants through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,196, which discloses an osmotic drug delivery system.

Many other such implants, delivery systems, and modules are known to those skilled in the art. In certain embodiments, the compounds used according to the present invention can be formulated to ensure proper distribution *in vivo.* For example, the blood-brain barrier (BBB) excludes many highly hydrophilic compounds. To ensure that the compounds used according to the present invention cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, and thus enhance targeted drug delivery (see, e.g., V.V. Ranade (1989) J. Clin. Pharmacol. 29: 685). Exemplary targeting moieties include folate or biotin (see, e.g., US 5,416,016 to Low *et al*.); mannosides (Umezawa et al., (1988) Biochem. Biophys. Res. Commun. 153: 1038); antibodies (P.G. Bloeman et al. (1995) FEBS Lett. 357: 140; M. Owais et al. (1995) Antimicrob. Agents Chemother. 39: 180); and surfactant protein A receptor (Briscoe et al. (1995) Am. J. Physiol. 1233: 134).

In one embodiment, the compounds used according to the present invention are formulated in liposomes. In a more preferred embodiment, the liposomes include a targeting moiety. In a most preferred embodiment, the compounds in the liposomes are delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for therapy/treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective tumor response.

A "therapeutically effective dosage" for therapy/treatment can also be measured by its ability to stabilize the progression of a condition, disorder or disease. The ability of a compound to inhibit KLK6 can be evaluated by using an appropriate assay known to the skilled practitioner, such as the *in vitro* assay described herein measuring the KLK6 activity/inhibition. Alternatively, the properties of a compound described in the present invention can be evaluated by examining the ability of the compound in appropriate animal model systems known to the skilled practitioner. A therapeutically effective amount of a compound used according to the present invention can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the individual’s size, the severity of the individual’s symptoms, and the particular composition or route of administration selected.

The pharmaceutical composition used according to the invention can also, if desired, be presented in a pack, or dispenser device which can contain one or more unit dosage forms containing the active compound. The pack can for example comprise metal or plastic foil, such as blister pack. The pack or dispenser device can be accompanied with instruction for administration.

*Therapeutic and other applications*

In further aspects, the present application provides a compound as specified above under the heading "Compounds" or a pharmaceutical composition as specified above under the heading "Pharmaceutical compositions" for use in therapy.

Generally, the present invention demonstrates that the compounds having a structure according to formula (I), (II), (III) and/or (IV), or the compounds of Table 1 as described herein under the heading "Compounds" or a pharmaceutical composition as specified above under the heading "Pharmaceutical compositions" are capable of inhibiting the enzymatic activity of human kallikrein-like peptidase 6 (KLK6).

Thus, in one aspect, the present invention is directed to a compound having a structure according to formula (I), (II), (III) and/or (IV), or a compound of Table 1 and hydrates, solvates, salts, complexes, racemic mixtures, diastereomers, enantiomers, and tautomers thereof and isotopically enriched forms of any of the foregoing, for use in the treatment of a disease associated with kallikrein-like peptidase 6 overexpression. Optionally, the method comprises the step of administering at least one additional active compound to the individual. The at least one additional active compound can be administered together with, before or after the compound having a structure according to formula (I), (II), (III) and/or (IV), or the compound of Table 1.

As used herein and throughout the entire description, the term "disease associated with kallikrein-like peptidase 6 overexpression" means any pathological state or disease which occurs cumulatively by an overexpression of KLK6 or correlates to an overexpression of KLK6. It has been shown KLK6 overexpression correlates to pathologies such as Melanoma and Cutaneous malignant melanoma (Krenzer, S., Peterziel, H., Mauch, C., Blaber, S. I., Blaber, M., Angel, P., and Hess, J., (2011), Expression and Function of the Kallikrein-Related Peptidase 6 in the Human Melanoma Microenvironment, J. Invest. Dermatol., 131(11): 2281-2288. doi:10.1038/jid.2011.190), Glioblastoma (Drucker, K. L., Paulsen, A. R., Giannini, C., Decker, P. A., Blaber, S. I., Blaber, M., Uhm, J. H., O'Neill, B. P., Jenkins, R. B., and Scarisbrick, I. A., 2013, Clinical significance and novel mechanism of action of kallikrein 6 in glioblastoma, Neuro-Oncology, 15(3): 305-318. doi:10.1093/neuonc/nos313), Astrocytoma (Drucker, K. L., Gianinni, C., Decker, P. A., Diamandis, E. P., and Scarisbrick, I. A., (2015), Prognostic significance of multiple kallikreins in high-grade astrocytoma, BMC Cancer, 15: 565. doi: 10.1186/s12885-015-1566-5), Ovarian cancer (Shan, S. J. C., Scorilas A., Katsaros, D., and Diamandis, E. P., (2007), Transcriptional upregulation of human tissue kallikrein 6 in ovarian cancer: clinical and mechanistic aspects, British Journal of Cancer, 96: 362-372. doi:10.1038/sj.bjc.6603556), Colon carcinoma (Henkhaus, R. S., Gerner, E. W., and Ignatenko, N. A., (2008), Kallikrein 6 is a mediator of K-RAS-dependent migration of colon carcinoma cells, Biol Chem., 389(6): 757-764. doi:10.1515/BC.2008.087), Lung cancer (Heuzé-Vourc'h, N., Planque, C., Guyetant, S., Coco, C., Brillet, B., Blechet, C., Parent, C., Laurent, B., Reverdiau, P., Jourdan, M-L., Courty, Y., (2009), High kallikrein-related peptidase 6 in non-small cell lung cancer cells: an indicator of tumour proliferation and poor prognosis, J. Cell. Mol. Med., 13(9B): 4014-4022. doi:10.1111/j.1582-4934.2009.00763.x), Gastric cancer (Hu, C. J., Chen, K. X., Zheng, J. F., Chen, Y. J., (2013), Expression and biological significance of human kallikrein 6 in gastric cancer tissues, Contemp. Oncol., 17(1): 64-67. doi: 10.5114/wo.2013.33776; and Nagahara, H., Mimori, K., Utsunomiya, T., Barnard, G. F., Ohira, M., Hirakawa, K., and Mori, M., (2005), Clinicopathologic and Biological Significance of Kallikrein 6 Overexpression in Human Gastric Cancer, Clin Cancer Res. ;11 (19): 6800-6806. doi:10.1158/1078-0432.CCR-05-0943), Gastroesophageal junction adenocarcinoma (Grin, A., Samaan, S, Tripathi, M., Rotondo, F., Kovacs, K., Bassily, M. N., Yousef, G. M., (2015), Evaluation of human tissue kallikrein-related peptidases 6 and 10 expression in early gastroesophageal adenocarcinoma, Human Pathology, 46: 541-548. doi:10.1016/j.humpath.2014.12.005), Pancreatic ductal adenocarcinoma (Rückert, F., Hennig, M., Petraki, C. D., Wehrum, D., Distler, M., Denz, A., Schröder, M., Dawelbait, G., Kalthoff4, H., Saeger, H-D., Diamandis, E. P., Pilarsky, C., and Grützmann, R., (2008), Co-expression of KLK6 and KLK10 as prognostic factors for survival in pancreatic ductal adenocarcinoma, British Journal of Cancer, 99: 1484 - 1492. doi:10.1038/sj.bjc.6604717), Breast cancer (Sidiropoulosa, K. G., Dinga, Q., Pampalakisc, G., Whitea, N. M. A., Boulosa, P., Sotiropoulouc, G., Yousef G. M., (2016), KLK6-regulated miRNA networks activate oncogenic pathways in breast cancer subtypes, Mol. Oncology, Article in Press. 10.1016/j.molonc.2016.03.008), Laryngeal adenocarcinoma (Zhang, Y., Zhang, Z., Yang, L., Xu, B., Li, W., Tang, P., Zhang, Z., Han, N., Gao, Y., Cheng, S., and Xiao, T., (2014), Identification of Human Tissue Kallikrein 6 as a Potential Marker of Laryngeal Cancer Based on the Relevant Secretory/Releasing Protein Database, Disease Markers, Article ID 594093, 8 pages. doi:10.1155/2014/594093), Multiple Sclerosis (Scarisbrick, I. A., Yoon, H., Panos, M., Larson, N., Blaber, S. I., Blaber, M., and Rodrigues, M., (2012), Kallikrein 6 Regulates Early CNS Demyelination in a Viral Model of Multiple Sclerosis, Brain Pathol., 22(5): 709-722. doi:10.1111/j.1750-3639.2012.00577.x), Psoriasis (Lundberg, K. C., Fritz, Y., Johnston, A., Foster, A. M., Baliwag, J., Gudjonsson, J. E., Schlatzer, D., Gokulrangan, G., McCormick, T. S., Chance, M. R., and Ward, N. L., (2015), Proteomics of Skin Proteins in Psoriasis: From Discovery and Verification in a Mouse Model to Confirmation in Humans, Mol. And Cell. Proteomics, 14(1): 109-119. doi: 10.1074/mcp.M114.042242; and Komatsu, N., Saijoh, K., Kuk, C., Shirasaki, F., Takehara, K., and Diamandis, E. P., (2007), Aberrant human tissue kallikrein levels in the stratum corneum and serum of patients with psoriasis: dependence on phenotype, severity and therapy, British Journal of Dermatology, 156: 875-883.

doi: 10.1111/j.1365-2133.2006.07743.x) and/or Neuron injuries (e. g. Spinal Cord Injury, SCI) (Yoon, H., Radulovic, M., Wu, J., Blaber, S. I., Blaber, M., Fehlings, M. G., and Scarisbrick, I. A., (2013), Kallikrein 6 Signals through PAR1 and PAR2 to Promote Neuron Injury and Exacerbate Glutamate Neurotoxicity, J Neurochem, 127(2): 283-298. doi:10.1111/jnc.12293; and Scarisbrick, I. A., Sabharwal, P., Cruz, H., Larsen, N., Vandell,A. G., Blaber, S. I., Ameenuddin, S., Papke, L. M., Fehlings, M. G., Reeves, R. K., Blaber, M., Windebank, A. J., and Rodriguez, M., (2006), Dynamic role of kallikrein 6 in traumatic spinal cord injury, European Journal of Neuroscience, (24): 1457-1469).

Tests to determine a disease associated with kallikrein-like peptidase 6 overexpression by measuring the amount of KLK6 protein in a sample from a subject are known to the person skilled in the art. On a general basis, the amount of KLK6 protein in a sample can be determined using different assays or detection methods known to those skilled in the art, such as UV/VIS spectroscopy, colorimetric assays, immunoassays, mass spectrometry and radiolabelling, just to name some. A variety of quantitative immunoassays such as quantitative enzyme-linked immunosorbent assays (ELISAs) for the detection of human KLK6 have been described in the art, inter alia in Xi, Y., Zhang, Y., Fang, J., Wilson, J. J., Luo, S., and Huang, R-P, Development of Monoclonal Antibodies and Characterization of an ELISA Platform Against Kallikrein-Related Peptidase 6 as a Tumor Biomarker, Monoclonal Antibodies in Immunodiagnosis and Immunotherapy, 2015, 34(5): 346-353. DOI: 10.1089/mab.2015.0019, and are commercially provided (e.g. human KLK6 ELISA Kit, Catalog-No. EHKLK6, Thermofischer, human KLK6 ELISA, Catalog-No. RAB0704-1KT, Sigma Aldrich, or human KLK6 ELISA, CODE: ELH-KLK6-1, RayBiotech). Alternatively, immunohistochemistry (IHC) methods can be applied for determining the expression of KLK6 protein directly in discrete tissue components. In this regard, IHC combines anatomical, immunological and biochemical techniques to identify KLK6 protein with specific anti-KLK6-antibodies tagged with a visible label. IHC makes it possible to visualize the distribution and localization of KLK6 within cells and in the proper tissue context. While there are multiple approaches and permutations in IHC methodology, all of the steps involved are separated into two groups: sample preparation and labeling. The immunohistochemical analysis of KLK6 with commercial antibodies has been described for example in Krenzer, S., Peterziel, H., Mauch, C., Blaber, S. I., Blaber, M., Angel, P., and Hess, J., (2011), Expression and Function of the Kallikrein-Related Peptidase 6 in the Human Melanoma Microenvironment, J. Invest. Dermatol., 131(11): 2281-2288. doi:10.1038/jid.2011.190. Following this reference, paraffin sections can, for example, be stained according to the manufacturer's instructions (Vectastain Elite ABC Kit and ImmPRESS Kit, Vector Laboratories, Lörrach, Germany). Primary and secondary antibodies are listed in Table 2.

**Table 2:** Summary of the antibodies used in the method of Krenzer et al.

| Name | Number | Assay | Antigen | Company | Dilution |
|---|---|---|---|---|---|
| anti-human KLK5 | AF1108 | IHC | KLK5 | R&D systems, Minneapolis, USA | 1:200 |
| anit-human KLK6 | AF2008 | IHC | KLK6 | R&D systems, Minneapolis, USA | 1:100 |
| anti-human KLK7 | AF2624 | IHC | KLK7 | R&D systems, Minneapolis, USA | 1:200 |
| anti-human S100 | SI691R06 | IHC | S-100 | DCS, Hamburg, Germany | ready to use |
| biotinylated horse-anti-mouse IgG | BA-2000 | IHC | | Vector, Burlingame, CA | 1:200 |
| biotinylated horse-anti-goat IgG | BA-9500 | IHC | | Vector, Burlingame, CA | 1:200 |
| anti-human PAR-1 | sc-13503 | Western Blot | PAR-1 | Santa Cruz Biotechnology, Santa Cruz, CA | 1:500 |
| anti-human ß-actin | sc-1615 | Western Blot | ß-actin | Santa Cruz Biotechnology, Santa Cruz, CA | 1:2000 |
| anti-goat IgG, HRP-linked antibody | sc-2020 | Western Blot | | Santa Cruz Biotechnology, Santa Cruz, CA | 1:5000 |
| anti-mouse IgG, HRP-linked antibody | #7076 | Western Blot | | Cell Signaling Technology, Boston, USA | 1:2000 |
| anti human PAR-1 | AF3855 | FACS analysis | PAR-1 | R&D systems, Minneapolis, USA | 2.5 μg/ml |
| anti-goat Alexa 488 | A11055 | FACS analysis | | Invitrogen, Karlsruhe, Germany | 1:200 |

(continued)

| Name | Number | Assay | Antigen | Company | Dilution |
|---|---|---|---|---|---|
| anti-human CD45 | M0701 | IF | CD45 | Dako Cytomation | 1:100 |
| anti-human mast cell tryptase | M7052 | IF | mast cell tryptase | Dako Cytomation | 1:100 |
| anti-human CD34 | NCL-END | IF | CD34 | Novocastra Laboratories | 1:50 |
| anti-human CD1a | M3571 | IF | CD1a | Dako Cytomation | 1:50 |
| anti-human CD68 | M0876 | IF | CD68 | Dako Cytomation | 1:100 |

As substrates, liquid DABþ Substrates or AECþ Substrate Chromogen (Dako, Hamburg, Germany), or VectorRed and Histogreen (Vector Laboratories) can, for example, be used. All tissue sections were counterstained with hematoxylin (AppliChem, Darmstadt, Germany). In addition to commercially available antibodies, newly made can of course also be employed in the detection of KKL6. Such "homemade" antibodies for use in a KLK6 detection method as described herein can be produced, for example by using the method described for the production of MPS-specific antibodies by Scarisbrick, I. A., Asakura, K., Blaber, S., Blaber, M., Isackson, P. J., Bieto, T., Rodrigues, M., and Windebank, A., Preferential expression of myelencephalon specific protease by oligodendrocytes of the adult rat spinal cord white matter. Glia. 2000;30:219-230. Following this reference an antibody recognizing rat MSP was, for example, reported to be generated against a peptide corresponding to amino acids 108-131 of the predicted amino acid sequence of rat MSP (Scarisbrick et al., 1997b). For this purpose, the following experimental protocol was used.

Unconjugated peptide was produced at the Mayo Protein Core Facility (431 Peptide Synthesizer, Applied Biosystems). The peptide was emulsified in equal ratios of Freund's complete adjuvant (FCA) and 0.01 M phosphate-buffered saline (PBS), to a final concentration of 2 mg/ml. Balb/c mice (Jackson Laboratories, Bar Harbor, ME) were immunized in the rear footpad with 50 ml of the FCA emulsion. At 7-day intervals over a period of 49 days, each mouse was given an intravenous (IV) boost of 50 mg peptide dissolved in 1 ml PBS. At 56 days, the mice were bled and the sera were screened for their ability to produce immunostaining in spinal cord tissue sections in a pattern resembling the known distribution of MSP mRNA (Scarisbrick et al., 1997b). Unless otherwise specified, all reagents were obtained from Sigma Chemical Company (St. Louis, MO). Splenic lymphocytes of mice whose sera produced strong cellular labeling mirroring the expression pattern of MSP mRNA in the spinal cord white matter were fused with myeloma cells. The protocol used for generation of B-cell hybridomas was previously described by Faedas de St. Groth and Scheidegger (1980). Briefly, spleens were removed from mice 3 days after intravenous boosting with antigen. Single-cell suspensions were prepared and red blood cells lysed with ammonium chloride buffer. Splenic lymphocytes and F/O myeloma cells (nonsecreting myeloma derived from sp2/0 Balb/c myeloma cells) were mixed at a 5:1 ratio and centrifuged to form a pellet. The cell pellet was resuspended in 1 ml of a 50% solution of polyethylene glycol (PEG) 1540, then incubated at 37°C for 90 s. Cells were washed and resuspended in fresh media, and 100-ml aliquots were added to the wells of microtiter plates. After 24 h, 100 ml culture medium supplemented with 1 M hypoxanthine (HT), 4 mM aminopterin, and 0.16 M thymidine (HAT), were added to each well. Every 3- 4 days thereafter, 100 ml culture medium was replaced with 100 ml fresh medium containing HAT, HT, and complete medium without HAT, successively over a period of approximately 14 days. Upon reaching 75% confluency, the culture supernatants were screened by immunohistochemisty for the presence of specific relevant antibody, as above. The hybridomas of interest were then cloned in limiting dilution cultures at 1 cell per microtiter well. Balb/c spleen cells served as feeder layer cells for fusion and cloning microtiter plates (3 x 10^5 per well). Fused cells were grown in 96-well plates in Iscove's modified Dulbecco's medium, containing 10.0% fetal bovine serum (FBS). Clones producing a pattern of immunolabeling resembling the known distribution of MSP mRNA were subcloned at 0.3 cells per microtiter well. The supernatants of subclones were screened as above and selected for production of ascites or hybridoma cell culture supernatants. For generation of ascites, Balb/c mice were primed with an intraperitoneal injection of 0.5 ml prisane oil (Sigma, T-7640); 3-14 days later, 1-2 3x10^6 hybridoma cells in 0.5 ml PBS were injected intraperitoneally. Daily collections of ascites fluid began after 7-10 days. Ascites fluid was diluted with equal amounts of PBS. Ascites and cell culture supernatants were isotyped as IgG or IgM, using a mouse monoclonal antibody isotyping kit (IsoStrip, Boehringer-Mannheim, Indianapolis, IN).

The antibodies produces by the method of Scarisbrick et al. can be successfully applied to histochemistry staining as disclosed by Drucker, K. L., Paulsen, A. R., Giannini, C., Decker, P. A., Blaber, S. I., Blaber, M., Uhm, J. H., O'Neill, B. P., Jenkins, R. B., and Scarisbrick, I. A., 2013, Clinical significance and novel mechanism of action of kallikrein 6 in glioblastoma, Neuro-Oncology, 15(3): 305-318. doi:10.1093/neuonc/nos313. In this regard, KLK6 immunoreactivity (IR) can be detected using a KLK6-specific monoclonal antibody (MSP-3-3) as described in detail in Scarisbrick et al. Briefly, deparaffinized sections were treated with citrate solution (BioGenex), and purified primary antibody was applied

to sections at a concentration of 10 mg/mL for 18 h at 48C. After sequential application of biotinylated secondary antibody (Jackson Laboratories), followed by peroxidaseconjugated streptavidin, each for 1 h at 208C (DAKO), IR was visualized using 3’,3’-diaminobenzadine tetrahydrochloride as the substrate. All immunostained sections were counterstained with Gills hematoxylin and imaged with a Bliss digital imaging system that pairs an Axioplan microscope (Zeiss) with a slide scanner (Bacus Laboratories). KLK6 IR in each tumor core was evaluated independently by 2 observers without knowledge of tumor type or demographic information. In each case, a score was assigned for staining intensity (range 1-3: low, medium, high) and for percent of the tumor core stained (range 1-4 in 25% increments). The final IR score for each tumor was assessed as the product of staining intensity and percent stained. Scores across multiple cores from the same tumor and across the 2 independent observers were averaged. Additional highresolution images were prepared using 100X-oil immersion objective and an Olympus BX51 microscope fitted with an Olympus DP71 camera.

Further, there exist methods in the art suitable for detecting KLK6 expression under *in vivo* conditions. Such methods may be suitable for the *in vivo* diagnosis of a disease associated with an increased KLK6 expression in a subject, thereby using e.g. labelled anti-KLK6-antibodies having high molecular specificity to KLK6. Such anti-KLK6-antibodies may be applied to analyze KLK6 levels at local site, and can be applied in combination with standard *in vivo* molecular imaging techniques, such as single photon emission tomography (SPECT), positron emission tomography (PET), optical imaging, ultrasound and photoacoustic imaging, which offer unique molecular sensitivity.

The "sample" used for determining or measuring the amount of KLK6 by using any of the methods described herein refers to a sample obtained from a subject as described herein. To this end, it is envisaged to compare the obtained results to the results of a reference sample taken from a reference subject. The reference subject as described herein is preferably a subject not suffering from a disease associate with KLK6-overexpression. Hence, an increased amount of KLK6 protein as compared to a reference sample is indicative for a disease associate with KLK6-overexpression, whereas no difference as compared to the reference sample indicates that said subject has no disease associate with KLK6-overexpression. In this context, the sample obtained from the subject of the present invention may be any biological sample taken from said subject and being appropriate to determine the amount of KLK6 protein and thus for diagnosing a disease associate with KLK6-overexpression. Non-limiting examples of useful samples may include blood samples, tissues samples, body fluid samples, skin samples or any other samples known to those skilled in the art for use in *in vitro* diagnosis.

Equally when applying *in vivo* molecular imaging techniques in combination with labelled anti-KLK6-antibodies having high molecular specificity to KLK6, KLK6 protein can be detecting using said non-invasive molecular imaging method, thereby collecting imaging data and comparing the imaging data to reference imaging data of the reference subject. Here, an increased signal in the imaging data from the subject as compared to reference imaging data is indicative for a disease associate with KLK6-overexpression, whereas no difference in the imaging signal as compared to reference imaging data indicates that said subject has no disease associate with KLK6-overexpression.

The terms "disease", "disorder" and "condition", when used in the context of treatment or therapy (including prophylactic therapy), are used herein interchangeably and refer to any pathological state, including cancer, in particular those pathological states (including cancer forms) described herein.

In a further aspect, the present invention provides a pharmaceutical composition for use in the treatment of a disease associated with kallikrein-like peptidase 6 overexpression, said composition comprising a compound having a structure according to formula (I), (II), (III) and/or (IV), or a compound of Table 1, and one or more excipients, and optionally at least one additional active compound. The at least one additional active compound can be administered together with, before or after the compound having a structure according to formula (I), (II), (III) and/or (IV), or the compound of Table 1.

In a further aspect, the present invention provides a method for treating and/or preventing a condition, disorder or disease that is associated with kallikrein-like peptidase 6 overexpression, said method comprising administering a therapeutically effective amount of a compound as described herein (in particular a therapeutically effective amount of a compound having a structure according to formula (I), (II), (III) and/or (IV), or a compound of Table 1 to an individual in need thereof. Optionally, the method comprises the step of administering at least one additional active compound to the individual. The at least one additional active compound can be administered together with, before or after the compound having a structure according to formula (I), (II), (III) and/or (IV), or the compound of Table 1.

As used herein and throughout the entire description, the term "Subject" or "individual" are used interchangeably and mean animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like. The subject is preferably a mammal, more preferably a human.

As used herein and throughout the entire description, the term "amount effective" in the context of a composition or dosage form for administration to a subject refers to an amount of the composition or dosage form sufficient to provide a benefit in the treatment of disease associated with kallikrein-like peptidase 6 overexpression, to delay or minimize symptoms associated with disease associated with kallikrein-like peptidase 6 overexpression, or to cure or ameliorate the disease or cause thereof. In particular, a therapeutically effective amount means an amount sufficient to provide a

therapeutic benefit *in vivo.* Used in connection with an amount of a compound of the invention, the term preferably encompasses a non-toxic amount that improves overall therapy, reduces or avoids symptoms or causes of disease, or enhances the therapeutic efficacy of or synergies with another therapeutic agent.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

In some embodiments the disease associated with kallikrein-like peptidase 6 overexpression is selected from the group consisting of cancer, immune disease and neuron injury. Accordingly, in some embodiments the disease associated with kallikrein-like peptidase 6 overexpression is cancer. In some embodiments the disease associated with kallikrein-like peptidase 6 overexpression is an immune disease. In some embodiments the disease associated with kallikrein-like peptidase 6 overexpression is neuron injury. In other embodiments the disease associated with kallikrein-like peptidase 6 overexpression is cancer, preferably selected from the group consisting of Melanoma, Glioblastoma, Astrocytoma, Ovarian cancer, Colon carcinoma,

Lung cancer, Gastric cancer, Gastroesophageal junction adenocarcinoma, Pancreatic ductal adenocarcinoma, Breast cancer and Laryngeal adenocarcinoma. Accordingly, in some embodiments the disease associated with kallikrein-like peptidase 6 overexpression is Melanoma. In some embodiments the disease associated with kallikrein-like peptidase 6 overexpression is Glioblastoma. In some embodiments the disease associated with kallikrein-like peptidase 6 overexpression is Astrocytoma. In some embodiments the disease associated with kallikrein-like peptidase 6 overexpression is Ovarian cancer. In some embodiments the disease associated with kallikrein-like peptidase 6 overexpression is Astrocytoma. In some embodiments the disease associated with kallikrein-like peptidase 6 overexpression is Colon carcinoma. In some embodiments the disease associated with kallikrein-like peptidase 6 overexpression is Lung cancer. In some embodiments the disease associated with kallikrein-like peptidase 6 overexpression is Gastric cancer. In some embodiments the disease associated with kallikrein-like peptidase 6 overexpression is Gastroesophageal junction adenocarcinoma. In some embodiments the disease associated with kallikrein-like peptidase 6 overexpression is Pancreatic ductal adenocarcinoma. In some embodiments the disease associated with kallikrein-like peptidase 6 overexpression is Breast cancer. In some embodiments the disease associated with kallikrein-like peptidase 6 overexpression is Pancreatic ductal adenocarcinoma. In some embodiments the disease associated with kallikrein-like peptidase 6 overexpression is Laryngeal adenocarcinoma. In other embodiments the disease associated with kallikrein-like peptidase 6 overexpression is an immune disease, preferably the immune disease is an autoimmune disease, more preferably the autoimmune disease is selected from the group consisting of Multiple Sclerosis, Psoriasis, Morbus Crohn and Colitis ulcerosa, even more preferably Multiple Sclerosis and Psoriasis, still more preferably Multiple Sclerosis. Accordingly, in some embodiments the disease associated with kallikrein-like peptidase 6 overexpression is an autoimmune disease. In some embodiments the disease associated with kallikrein-like peptidase 6 overexpression is Multiple Sclerosis. In some embodiments the disease associated with kallikrein-like peptidase 6 overexpression is Psoriasis. In some embodiments the disease associated with kallikrein-like peptidase 6 overexpression is Morbus Crohn. In some embodiments the disease associated with kallikrein-like peptidase 6 overexpression is Colitis ulcerosa. In other embodiments the disease associated with kallikrein-like peptidase 6 overexpression is neuron injury, preferably Spinal Cord Injury (SCI). Accordingly, in some embodiments the disease associated with kallikrein-like peptidase 6 overexpression is Spinal Cord Injury (SCI).

As used herein, a "cancer disease" or "cancer" includes a disease characterized by aberrantly regulated cellular growth, proliferation, differentiation, adhesion, and/or migration. By "cancer cell" is meant an abnormal cell that grows by a rapid, uncontrolled cellular proliferation and continues to grow after the stimuli that initiated the new growth cease.

Generally, the amount of a compound according to the invention as described herein (in particular the amount of a compound having a structure according to formula (I), (II), (III) and/or (IV), or a compound of Table 1) administered daily to an individual may be at most 100 mg/kg (such as at most 50 mg/kg, at most 40 mg/kg, at most 30 mg/kg, at most 20 mg/kg, at most 10 mg/kg, at most 9 mg/kg, at most 8 mg/kg, at most 7 mg/kg, at most 6 mg/kg, at most 5 mg/kg, at most 4 mg/kg, at most 3 mg/kg, at most 2 mg/kg, or at most 1 mg/kg), depending on factors such as the condition of the subject to be treated and the mode of administration. For example, the amount of a compound according to the invention as described herein (in particular the amount of a compound having a structure according to formula (I), (II), (III) and/or (IV), or a compound of Table 1) administered daily to an individual may range from about 0.01 mg/kg to 100 mg/kg (such as from about 0.05 mg/kg to 50 mg/kg, from about 0.1 mg/kg to 40 mg/kg, from about 0.2 mg/kg to 30 mg/kg, from about 0.3 mg/kg to 20 mg/kg, from about 0.4 mg/kg to 10 mg/kg, from about 0.5 mg/kg to 9 mg/kg, from about 0.6 mg/kg to 8 mg/kg, from about 0.7 mg/kg to 7 mg/kg, from about 0.8 mg/kg to 6 mg/kg, from about 0.9 mg/kg

to 5 mg/kg, from about 1 mg/kg to 4 mg/kg" or from about 2 mg/kg to 3 mg/kg) depending on factors such as the condition of the subject to be treated and the mode of administration.

In one embodiment, the compound according to the invention as described herein (in particular the compound having a structure according to formula (I), (II), (III) and/or (IV), or the compound of Table 1) is orally administered in a concentration of at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound according to the invention as described herein (in particular the the compound having a structure according to formula (I), (II), (III) and/or (IV), or the compound of Table 1) is parenterally administered (e.g., intravenously, intramuscularly, or subcutanously), in a concentration of at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The embodiments and definitions of terms described in the context of the means such as compounds according to Formula (I), Formula (II), Formula (III) and/or Formula (IV) of the invention are equally applicable to the methods and uses described herein, *mutatis mutandis.*

The present invention also envisions a method of treating in a subject a disease associated with kallikrein-like peptidase 6 overexpression, comprising administering to said subject an efficient amount of a compound according to Formula (I), Formula (II), Formula (III) and/or Formula (IV) or a pharmaceutically acceptable salt, solvate or hydrate thereof or a pharmaceutical composition comprising said compound. Said method may comprise further administering at least one additional pharmaceutically active compound. The above described aspects, embodiments, definitions, etc. are also applicable to said method of treatment, *mutatis mutandis.*

Further, the invention shall be explained in more detail by the following Examples.

## EXAMPLES

### 1) Determination of Biochemical Inhibition Values

The activity of the compounds was tested against KLK6 (produced in insect cells, according to the procedure reported in J. Biol. Chem. 2002, 277, 24562-24570). In addition, the activity of the compounds was tested against Trypsin (Polymum, TRY001-010), Thrombin (Biopur, 10-13-1104-01 1000U) and Factor Xa (Enzo Life Sciences, BML-SE362). Trypsin, Thrombin and Factor Xa were chosen since these enzymes are structurally similar to KLK6. However, Trypsin, Thrombin and Factor Xa are targets that are important for other biological processes. In particular, Trypsin, Thrombin and Factor Xa are involved in the blood coagulation cascade. Therefore, these enzymes are preferably not affected by the KLK6 inhibitors of the present invention.

*Procedure:*

In a 384 well plate, 5 μL of eleven concentrations of inhibitor in 8% DMSO (serial dilution 2:1) and a blank were incubated with an equal amount of enzyme (KLK6, Thrombin, Trypsin or Factor Xa) diluted in 50 mM Tris pH 7.5, 150 mM NaCl, 1 mM EDTA, 0.05% Tween20 assay buffer for 30 minutes. Then, 10 μL of the fluorogenic substrate FSR-AMC (Bachem, I-1400) were added to each well and the increase in fluorescence was measured with FLUOstar OPTIMA Microplate Reader (Ex: 355, Em: 440-10) for 30 minutes. Inhibition of the enzyme as a function of the inhibitor concentration was used to generate dose response curves, which allowed estimating $IC_{50}$ values, according to the following equation in GraphPad Prism:

$$y = y_{min} + (y_{max} - y_{min})/\left(1 + 10^{\left((\mathrm{LogIC}_{50} - x) * HillSlope\right)}\right)$$

*Assay final concentrations:*

KLK6: 5 nM

Trypsin: 0.88 ng/mL

Thrombin: $9.8 \cdot 10^{-5}$ NIH unit/mL

Factor Xa: 4 nM

FSR-AMC: 20 μM

The IC50 values are shown in the following Table 3.

**Table 3**

| Example Compound | KLK6 $IC_{50}$ (μM) | Thrombin $IC_{50}$ (μM) | Trypsin $IC_{50}$ (μM) | Factor Xa $IC_{50}$ (μM) |
|---|---|---|---|---|
| 1 | 0.176 | >200 | 39.4 | >200 |
| 2 | 1.25 | >200 | 71.5 | >200 |
| 3 | 0.371 | >200 | 65.7 | >200 |
| 4 | 2.89 | >200 | >200 | >200 |
| 5 | 0.172 | >200 | 51.0 | 50.0 |
| 6 | 0.439 | >200 | >200 | >200 |
| 7 | 0.598 | >200 | >200 | >200 |
| 8 | 0.258 | >200 | 126 | 139 |
| 9 | 0.267 | 197 | 67.1 | >200 |
| 10 | 0.994 | >200 | >200 | >200 |
| 11 | 14.5 | >200 | >200 | >200 |
| 12 | 6.84 | >200 | 139 | 135 |
| 13 | 6.47 | >200 | 133 | 162 |
| 14 | 16.2 | >200 | >200 | >200 |
| 15 | 4.72 | >200 | 144 | >200 |
| 16 | 0.158 | >200 | >200 | >200 |
| 17 | 109 | >200 | >200 | >200 |
| 18 | 11.6 | >200 | 149 | 50.5 |
| 19 | 0.264 | >200 | 21.3 | >200 |
| 20 | 0.240 | >200 | >200 | >200 |
| 21 | 112 | >200 | >200 | >200 |
| 22 | 0.486 | >200 | 105 | >200 |
| 23 | 0.375 | >200 | >200 | >200 |
| 24 | 9.96 | >50 | >50 | >50 |
| 25 | 30.6 | >50 | >50 | >50 |
| 26 | 17.5 | >50 | >50 | >50 |
| 28 | 26.0 | >50 | >50 | >50 |
| 30 | 1.01 | >50 | >50 | >50 |
| 31 | 1.20 | >50 | >50 | >50 |
| 32 | 3.36 | >50 | >50 | >50 |
| 33 | 6.49 | >50 | >50 | >50 |
| 34 | 11.3 | >50 | >50 | >50 |
| 35 | 1.41 | >50 | >50 | >50 |
| 36 | 0.433 | >50 | 29.3 | >50 |

(continued)

| Example Compound | KLK6 $IC_{50}$ (μM) | Thrombin $IC_{50}$ (μM) | Trypsin $IC_{50}$ (μM) | Factor Xa $IC_{50}$ (μM) |
|---|---|---|---|---|
| 37 | 1.48 | >50 | >50 | >50 |
| 38 | 0.081 | >50 | 27.4 | >50 |
| 39 | 6.98 | >50 | >50 | >50 |
| 40 | 30.4 | >50 | >50 | >50 |
| 41 | 31.3 | >50 | >50 | >50 |
| 42 | 10.3 | >50 | >50 | NA |
| 43 | 10.1 | >50 | >50 | NA |
| 44 | 12.2 | >50 | >50 | NA |
| 45 | 1.36 | >50 | >50 | NA |
| 46 | 29.4 | >50 | >50 | NA |
| 47 | 31.4 | >200 | >200 | >200 |
| 48 | 3.34 | >200 | >200 | >200 |
| 50 | 2.16 | >200 | >200 | >200 |
| 51 | 0.261 | >200 | >200 | >200 |
| 52 | 32.8 | >200 | >200 | >200 |
| 53 | 30.0 | >200 | >200 | >200 |
| 54 | 4.85 | >200 | >200 | >200 |
| 55 | 17.0 | >200 | >200 | >200 |
| 56 | 16.1 | >200 | >200 | >200 |
| 58 | 8.86 | >200 | >200 | >200 |
| 59 | 6.28 | >200 | >200 | >200 |
| 60 | 32.6 | >200 | >200 | >200 |
| 61 | 2.33 | >200 | >200 | >200 |
| 63 | 2.35 | >200 | >200 | >200 |
| 64 | 22.0 | >200 | >200 | >200 |
| 65 | 38.7 | >200 | >200 | >200 |
| 66 | 7.40 | >200 | >200 | >200 |
| 67 | 166 | >200 | >200 | >200 |
| 68 | 46.0 | >200 | >200 | >200 |
| 69 | 57.4 | >200 | >200 | >200 |
| 70 | 27.1 | >200 | >200 | >200 |
| 71 | 78.7 | >200 | >200 | >200 |
| 72 | 12.3 | 60.3 | 50.4 | 65.9 |
| 73 | 1.46 | >200 | 114 | 34.7 |
| 74 | 4.57 | >200 | >200 | >200 |
| 75 | 5.38 | >200 | >200 | >200 |
| 76 | 6.46 | >200 | >200 | >200 |
| 77 | 1.86 | >200 | 28.7 | 21.1 |

(continued)

| Example Compound | KLK6 $IC_{50}$ (μM) | Thrombin $IC_{50}$ (μM) | Trypsin $IC_{50}$ (μM) | Factor Xa $IC_{50}$ (μM) |
|---|---|---|---|---|
| 78 | 4.73 | >200 | >200 | >200 |
| 79 | 6.05 | >200 | >200 | >200 |
| 80 | 6.55 | >200 | >200 | >200 |
| 81 | 29.5 | >200 | >200 | >200 |
| 82 | 27.3 | >200 | >200 | >200 |
| 83 | 14.3 | >200 | >200 | >200 |
| 84 | 3.27 | 90.6 | 82.1 | 67.5 |
| 85 | 172 | >200 | >200 | 157 |
| 86 | 16.7 | 66.1 | >200 | 148 |
| 87 | 61.1 | >200 | >200 | >200 |
| 88 | 2.88 | 14.1 | 9.1 | 18.1 |

**2) Preparation of the Example Compounds**

*Abbreviations*

DCM: Dichloromethane

DMF: Dimethylformamide

EtOAC: Ethyl acetate

THF: Tetrahydrofuran

PE: Petroleum Ether

DCC: Dicyclohexyl carbodiimide

DCU: Dicyclohexylurea

DMAP: 4-dimethylaminopyridine

HOBT: 1-Hydroxybenzotriazole

EDCI: 1-Ethyl-3-(3-dimethylaminopropyl)carbodiimide

DMSO: Dimethylsulfoxide

*General*

Analytical thin layer chromatography (TLC) was carried out on Merck silica gel 60 $F_{254}$ TLC plates. TLC visualization was accomplished using 254 nm UV light or charring solutions of $KMnO_4$, cerric ammonium molybdate, or *p*-anisaldehyde. All reagents were purchased at the highest level of purity and were used without purification. Anhydrous $CH_2Cl_2$ and THF were prepared with an MBraun SPS800 Solvent Purification System. Flash column chromatography was performed using SiliCycle SiliaFlash® E60 silica gel (15-40 μm particle size). NMR spectra were recorded on a Bruker Ultrashield 400 or a Bruker Avance 600 at the NMR Structural Analysis Unit of the Deutsches Krebsforschungszentrum (German Cancer Research Center, DKFZ), Heidelberg. The residual solvent peak was used as internal ref-

erence ($^1H$ NMR: $CHCl_3$ (7.26 ppm); DMSO (2.50 ppm); $CHDCl_2$ (5.32); $CD_2HOD$ (3.31) $^{13}C$ NMR: $CHCl_3$ (77.16 ppm); DMSO (39.52 ppm); $CHDCl_2$ (5..84); $CD_2HOD$ (49.00)). Abbreviations (singlet = s, doublet = d, triplet = t, quartet = q, quintet = quin, sextet = sext, broad = br). High-resolution ESI-MS were recorded on a Bruker ApexQe FT-ICR instrument at the Organic Chemistry Institute of the University of Heidelberg.

*Examples Compound 1*

*2-((3,5-dimethyl-1-phenyl-1H-pyrazol-4-yl)amino)-2-oxoethyl 2-(2-methyl-H-indol-1-yl)acetate*

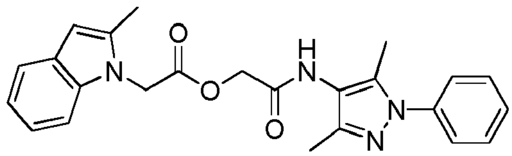

Step 1: 3,5-dimethyl-4-nitro-1-phenyl-1 H-pyrazole

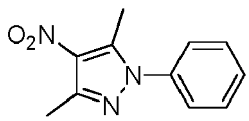

A mixture of 3,5-dimethyl-4-nitro-1 H-pyrazole (see international patent application WO 2003057673) (3.982 g, 28.22 mmol), iodobenzene (11.3 mL, 101.4 mmol), $K_2CO_3$ (8.19 g, 59.26 mmol), CuI (268.8 mg, 1.41 mmol), and trans-N,N'-di-methylcyclohexane-1,2-diamine (890 μL, 5.63 mmol) were heated to 180 °C in a microwave reactor for 4 h under argon. The reaction mixture was cooled to room temperature, diluted with EtOAc (250 mL), filtered through celite, and the product was purified by column chromatography (30→40→75% EtOAc in petroleum ether) to give 4.428 g (72%) of the title compound.

Step 2: 3,5-dimethyl-1-phenyl-1H-pyrazol-4-amine chlorohydrate

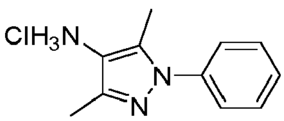

A mixture of 3,5-dimethyl-4-nitro-1-phenyl-1H-pyrazole (2.976 g, 13.70 mmol) and a spoon of 10% Pd/C was stirred in an autoclave under $H_2$ (4 bar) in EtOH (35 mL) and $CHCl_3$ (5 mL) at room temperature overnight. The mixture was filtered through celite, and the celite was repeatedly washed with EtOH (total of 800 mL). The filtrate was concentrated *in vacuo* to give 3.098 g (quantitative) of the title compound.

Step 3: 2-((3,5-dimethyl-1-phenyl-1 H-pyrazol-4-yl)amino)-2-oxoethyl acetate

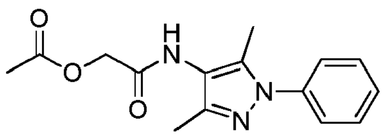

To a solution of 3,5-dimethyl-1-phenyl-1H-pyrazol-4-amine chlorohydrate (3.09 g, 13.8 mmol) in anhydrous

$CH_2Cl_2$ (60 mL) was added $Et_3N$ (4.20 mL, 30.3 mmol) under argon. The mixture was cooled to -10 °C and acetoxy acetylchloride (1.80 mL, 16.7 mmol) was added dropwise. The reaction mixture was allowed to warm to room temperature and after 3 h, the reaction mixture was filtered, diluted with EtOAc (250 mL), washed with $H_2O$ (100 mL), brine (100 mL), dried ($MgSO_4$), filtered, and concentrated *in vacuo.* Crystallization from toluene with a small amount of EtOAc provided 3.36 g (85%) of the title compound.

Step 4: N-(3,5-dimethyl-1-phenyl-1 H-pyrazol-4-yl)-2-hydroxyacetamide

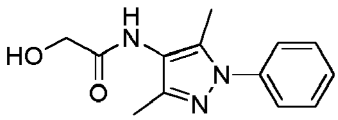

To a suspension of 2-((3,5-dimethyl-1-phenyl-1 H-pyrazol-4-yl)amino)-2-oxoethyl acetate (2.25 g, 7.83 mmol) in MeOH (20 mL) was added NaOMe (20 mg, 0.37 mmol) at room temperature. After 30 min, Amberlite resin was added to neutralize the base, and the mixture was filtered and concentrated *in vacuo.* The product was crystallized from a mixture of toluene/EtOAc to give 1.715 g (89%) of the title compound.

Step 5: 2-((3,5-dimethyl-1-phenyl-1 H-pyrazol-4-yl)amino)-2-oxoethyl 2-(2-methyl-1 H-indol-1-yl)acetate

To a solution of 2-(2-methyl-1 H-indol-1-yl)acetic acid (54.7 mg, 289 μmol) in anhydrous $CH_2Cl_2$ (4 mL) was added DCC (59.9 mg, 290 μmol), DMAP (8 mg, 65 μmol) and N-(3,5-dimethyl-1-phenyl-1 H-pyrazol-4-yl)-2-hydroxyacetamide (71.0 mg, 290 μmol) at 0 °C under argon. The mixture was allowed to warm to room temperature and for 18 h. The reaction mixture was filtered over cotton, washed with $NH_4Cl$ (2 mL), dried ($MgSO_4$), filtered, and concentrated *in vacuo.* Purification by column chromatography (75% EtOAc in petroleum ether) gave 98 mg (81%) of the title compound.
TLC: $R_f$ (5% MeOH in $CH_2Cl_2$): 0.15.
**$^1$H** NMR (400 MHz, DMSO-$d_6$) δ: 9.37 (bs, 1H), 7.48 - 7.54 (m, 4H), 7.36-7.45 (m, 3H), 7.04 (ddd, 1 H, *J* = 8.3, 7.1, 1.3 Hz), 6.98 (ddd, 1 H, *J* = 7.8, 7.2, 1.2 Hz), 6.24 (dq, *J* = 2.0, 0.9 Hz, 1 H), 5.21 (s, 2H), 4.79 (s, 2H), 2.37 (d, *J*= 0.8 Hz, 3H), 2.13 (s, 3H), 2.05 (s, 3H) ppm
**$^{13}$C** NMR (100 MHz, DMSO-$d_6$) δ: 168.9, 165.9, 144.7, 139.6, 137.1, 137.1, 134.4, 129.2, 127.7, 127.0, 123.8, 120.3, 119.3, 119.1, 116.6, 109.2, 100.1, 63.0, 44.0, 12.1, 11.3, 10.6 ppm
HRMS (ESI) m/z: $(M+H)^+$ calcd for $C_{24}H_{25}N_4O_3$: 417.1921; found: 417.1926

*Example Compound 2*

*2-oxo-2-((1,3,5-trimethyl-1H-pyrazol-4-yl)amino)ethyl 2-(2-methyl-1H-indol-1-yl)acetate*

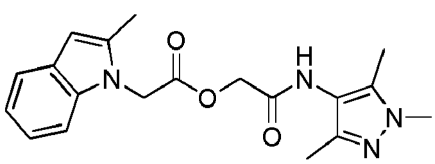

Step 1: 1,3,5-trimethyl-4-nitro-1H-pyrazole

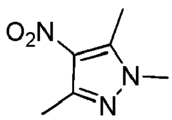

To a suspension of 3,5-dimethyl-4-nitro-1H-pyrazole (201 mg, 1.4 mmol) and $K_2CO_3$ (216 mg, 1.6 mmol) in dry DMF (1 mL), iodomethane (93 μL, 1.5 mmol) was added at room temperature. After 5 h, the reaction was heated

to 40 °C and additional iodomethane (90 μL) and $K_2CO_3$ (200 mg) was added. After 1.5 h, the reaction was quenched with $Et_3N$ (150 μL), diluted with $H_2O$ (15 mL) and EtOAc (15 mL). The two layers were separated and the aqueous layer was extracted with EtOAc (2 x 10 mL). The combined organics were dried ($MgSO_4$), filtered, and concentrated *in vacuo.* Purification by column chromatography (30% EtOAc in PE) yielded 206 mg (93%) of the title compound.

Step 2: 1,3,5-trimethyl-1H-pyrazol-4-amine

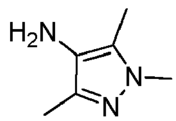

To a solution of the pyrazole from step 1 (194.8 mg, 1.3 mmol) in EtOH (5 mL) stirring under Ar, a spatula of palladium on carbon 10% was added, and then the Ar was replaced with $H_2$. After 7 h, more catalyst and new $H_2$ were added to the system, which was left stirring overnight. The crude was filtered through celite and concentrated to give 153 mg of the title compound (97%).

Step 3: 2-oxo-2-((1,3,5-trimethyl-1H-pyrazol-4-yl)amino)ethyl acetate

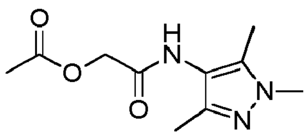

The title compound (220 mg, 85%) was prepared analogously to Example 1, step 3.

Step 4: 2-hydroxy-N-(1,3,5-trimethyl-1H-pyrazol-4-yl)acetamide

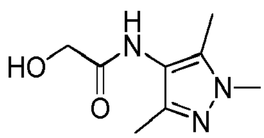

The title compound (27 mg, 15%) was prepared analogously to Example 1, step 4.

Step 5: 2-oxo-2-((1,3,5-trimethyl-1H-pyrazol-4-yl)amino)ethyl 2-(2-methyl-1 H-indol-1-yl)acetate

(2-Methyl-indol-1-yl)-acetic acid (24.7 mg, 0.1 mmol) was dissolved in dry DCM (6 mL) at 0 °C under Argon atmosphere. DCC (25.8 mg, 0.1 mmol) and DMAP (1.7 mg, 0.01 mmol) were added followed by the alcohol from step 4 (23.8 mg, 0.1 mmol). The mixture was allowed to warm to room temperature and stirred for 4.5 h. The suspension was filtered over cotton to remove the bulk of dicyclohexylurea (DCU) and washed with $NH_4Cl$ solution (semi-saturated). After drying with $MgSO_4$ the solvent was evaporated and the product was purified by column chromatography (100% EtOAc and then 5% MeOH in DCM) to yield 32 mg (73%) of the title compound.

**TLC:** $R_f$ (EtOAc): 0.11.

**$^1$H NMR** (400 MHz, $CD_2Cl_2$) δ: 7.45 (dd, 1H, *J* = 7.8, 0.8 Hz), 7.22 (ddd, *J* = 8.3, 1.5, 0.9 Hz, 1 H), 7.10 (ddd, *J* = 8.2, 7.2, 1.0 Hz, 1H), 7.01 (ddd, *J* = 7.9, 7.2, 0.8 Hz, 1 H), 6.30 (dq, *J* = 1.9, 0.9 Hz, 1 H), 4.97 (s, 2H), 4.64 (s, 2H), 3.64 (s, 3H), 2.43 (d, *J* = 0.7 Hz, 3H), 1.92 (s, 3H), 1.90 (s, 3H) ppm

**$^{13}$C NMR** (100 MHz, $CD_2Cl_2$) δ: 168.0, 165.8, 143.5, 137.8, 137.5, 135.6, 128.9, 121.9, 120.8, 120.6, 113.2, 108.9, 101.9, 64.1, 45.4, 36.8, 12.9, 11.3, 9.6 ppm

**HRMS** (ESI) m/z: $(M+H)^+$ calcd for $C_{19}H_{23}N_4O_3$: 355.1765; found: 355.1764

*Example Compound 3*

*2-((3,5-dimethyl-1-(m-tolyl)-1H-pyrazol-4-yl)amino)-2-oxoethyl 2-(2-methyl-1H-indol-1-yl)acetate*

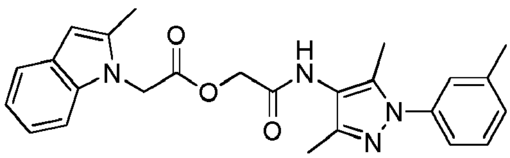

Step 1: 3,5-dimethyl-4-nitro-1-(m-tolyl)-1H-pyrazole

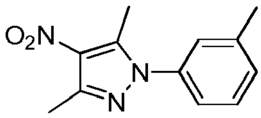

3,5-dimethyl-4-nitro-1 H-pyrazole (201 mg, 1.4 mmol), $K_2CO_3$ (411 mg, 3.0 mmol), CuI (13.6 mg, 0.07 mmol), trans-N,N-dimethylcyclohexyldiamine (44.8 μL, 0.3 mmol) were 3-iodotoluene (528 μL, 4.1 mmol) were combined in a microwave vial and irradiated for 4 h to 195 °C in the microwave. The reaction mixture was filtered through celite, washed with EtOAc, concentrated *in vacuo* and was purified by column chromatography (10% EtOAc in PE) to yield 233 mg (71%) of the title compound.

Step 2: 3,5-dimethyl-1-(m-tolyl)-1H-pyrazol-4-amine

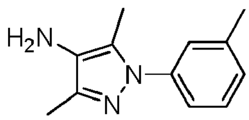

The title compound was prepared from the compound of step 1 (173 mg, 0.748 mmol) in quantitative yield (155 mg) analogously to Example 2, step 2.

Step 3: 2-((3,5-dimethyl-1-(m-tolyl)-1H-pyrazol-4-yl)amino)-2-oxoethyl acetate

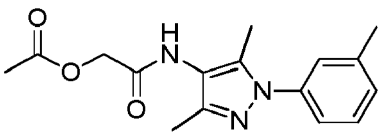

From the compound of step 2 (143 mg, 0.711 mmol) was prepared 199 mg (93%) of the title compound analogously to Example 1, step 3.

Step 4: N-(3,5-dimethyl-1-(m-tolyl)-1H-pyrazol-4-yl)-2-hydroxyacetamide

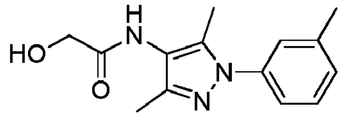

From the compound of step 3 (191 mg, 0.632 mmol), was prepared 163 mg (99%) of the title compound analogously to Example 1, step 4.

Step 5: 2-((3,5-dimethyl-1-(m-tolyl)-1H-pyrazol-4-yl)amino)-2-oxoethyl 2-(2-methyl-1H-indol-1-yl)acetate

(2-Methyl-indol-1-yl)-acetic acid (22.7 mg, 0.1 mmol) was dissolved in dry DCM (4 mL) at 0 °C under Argon atmosphere. DCC (24.0 mg, 0.1 mmol) and DMAP (1.7 mg, 0.01 mmol) were added followed by the alcohol from step 4 (31.4 mg, 0.1 mmol). The mixture was allowed to warm to room temperature and stirred for 4 h. The suspension was filtered over cotton to remove the bulk of DCU and washed with $NH_4Cl$ solution (semi-saturated). After drying with $MgSO_4$ the solvent was evaporated and the product was purified by column chromatography (60% EtOAc in PE) to yield 39 mg (78%) of the title compound.

**TLC:** $R_f$ (5% MeOH in DCM; 2x): 0.60

**$^1$H NMR** (400 MHz, MeOD) $\delta$: 7.35-7.43 (m, 2H), 7.24-7.30 (m, 3H), 7.18-7.23 (m, 1H), 7.06 (ddd, *J* = 8.2, 7.2, 1.0 Hz, 1 H), 6.98 (ddd, *J* = 7.9, 7.2, 0.8 Hz, 1 H), 6.25 (dq, *J* = 1.9, 1.0 Hz, 1 H), 5.12 (s, 2H), 4.82 (s, 2H), 2.41 (bs, 3H), 2.41 (d, *J* = 0.9 Hz, 3H), 2.12 (s, 3H), 2.08 (s, 3H) ppm

**$^{13}$C NMR** (100 MHz, MeOD) $\delta$: 170.4, 169.4, 146.6, 140.8, 140.6, 138.9, 138.2, 137.7, 130.1, 130.0, 129.8, 126.8, 123.3, 121.8, 120.7, 120.6, 116.7, 109.7, 101.7, 64.2, 48.4, 21.3, 12.5, 11.1, 10.6 ppm

**HRMS** (ESI) m/z: $(M+H)^+$ calcd for $C_{25}H_{27}N_4O_3$: 431.2078; found: 431.2082

*Example Compound 4*

*2-((3,5-dimethyl-1-(4-(trifluoromethyl)phenyl)-1H-pyrazol-4-yl)amino)-2-oxoethyl 2-(2-methyl-1H-indol-1-yl)acetate*

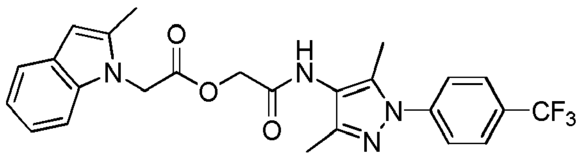

Step 1: 3,5-dimethyl-4-nitro-1-(4-(trifluoromethyl)phenyl)-1H-pyrazole

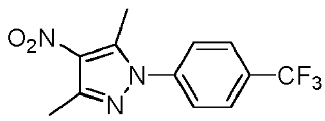

3,5-dimethyl-4-nitro-1 H-pyrazole (200 mg, 1.4 mmol) and potassium t-butanoate (175 mg, 1.5 mmol) were dissolved in DMSO (1 mL). After 15 minutes, 1-fluoro-4-(trifluoromethyl)benzene (189 $\mu$L, 1.5 mmol) was added, and the reaction mixture was heated to 150 °C and left stirring overnight. After 18 h, more 1-fluoro-4-(trifluoromethyl)benzene (189 $\mu$L, 1.5 mmol) was added. After an additional 24 h, the reaction mixture was diluted with $H_2O$ (20 mL) and DCM (20 mL), the phases were separated and the aqueous phase was extracted 3 times with toluene. The reunited organic phase was washed with brine, dried over $MgSO_4$, filtered and concentrated. The crude was purified by column chromatography (10% EtOAc in PE) yielding 168 mg (41%) of the title compound.

Step 2: 3,5-dimethyl-1-(4-(trifluoromethyl)phenyl)-1H-pyrazol-4-amine

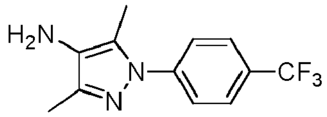

From the compound of step 1 (79 mg, 0.278 mmol) was prepared 65 mg (92%) of the title compound analogously to Example 2, step 2.

Step 3: 2-((3,5-dimethyl-1-(4-(trifluoromethyl)phenyl)-1 H-pyrazol-4-yl)amino)-2-oxoethyl acetate

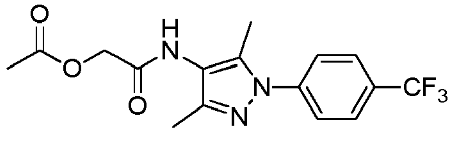

From the compound of step 2 (61 mg, 0.24 mmol) was prepared 56 mg (65%) of the title compound analogously to Example 1, step 3.

Step 4: N-(3,5-dimethyl-1-(4-(trifluoromethyl)phenyl)-1H-pyrazol-4-yl)-2-hydroxyacetamide

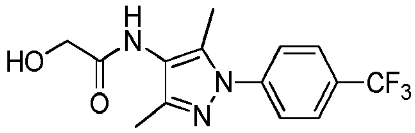

The title compound was obtained in quantitative yield (54 mg) analogously to Example 1, step 4.

Step 5: 2-((3,5-dimethyl-1-(4-(trifluoromethyl)phenyl)-1H-pyrazol-4-yl)amino)-2-oxoethyl 2-(2-methyl-1 H-indol-1-yl)acetate

(2-Methyl-indol-1-yl)-acetic acid (21.7 mg, 0.1 mmol) was dissolved in dry DCM (1 mL) at 0 °C under Argon atmosphere. DCC (23.4 mg, 0.1 mmol) and DMAP (1.6 mg, 0.01 mmol) were added followed by the alcohol from step 4 (35.0 mg, 0.1 mmol). The mixture was allowed to warm to room temperature and stirred for 8 h. The suspension was filtered over cotton to remove the bulk of DCU and washed with $NH_4Cl$ solution (semi-saturated). After drying with $MgSO_4$ the solvent was evaporated and the product was purified by column chromatography (75% EtOAc in PE) to give 30 mg (55%) of the title compound.

**TLC:** $R_f$ (75% EtOAc in PE): 0.55.

**$^1$H NMR** (400 MHz, $CDCl_3$) δ: 7.72 (d, 2H), 7.56 (d, 2H), 7.44 (dddd, *J* = 7.4, 2.3, 1.7, 0.8 Hz, 1H), 7.23 (ddd, *J* = 8.4, 1.7, 1.0 Hz, 1H), 7.10 (ddd, *J* = 8.2, 7.2, 1.0 Hz, 1H), 6.99 (ddd, *J*= 7.9, 7.2, 0.8 Hz, 1H), 6.32 (dq, *J* = 1.8, 0.9 Hz, 1H), 6.16 (bs, 1H), 4.98 (s, 2H), 4.68 (s, 2H), 2.47 (d, *J* = 1.0 Hz, 3H), 1.99 (s, 3H), 1.99 (s, 3H) ppm

**$^{13}$C NMR** (100 MHz, $CDCl_3$) δ: 167.2, 165.4, 146.3, 142.7, 137.2, 136.8, 135.5, 129.3 (q, $J_{C-F}$ = 31.4 Hz), 128.4, 126.5 (q, $J_{C-F}$ = 3.6 Hz), 124.2, 124.0 (q, $J_{C-F}$ = 273 Hz), 121.8, 120.7, 120.5, 115.7, 108.3, 102.0, 63.5, 45.3, 12.7, 11.2, 11.0 ppm

**HRMS** (ESI) m/z: $(M+H)^+$ calcd for $C_{25}H_{24}F_3N_4O_3$: 485.1795; found: 485.1815

*Example Compound 5*

*2-((3,5-dimethyl-1-(2-nitrophenyl)-1H-pyrazol-4-yl)amino)-2-oxoethyl 2-(2-methyl-1H-indol-1-yl)acetate*

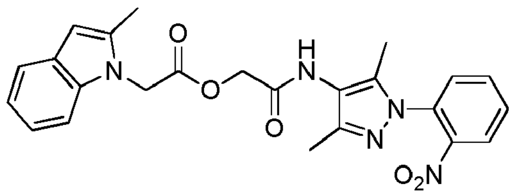

Step 1: 3,5-dimethyl-1H-pyrazol-4-amine

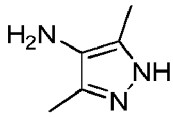

3,5-dimethyl-4-nitro-1 H-pyrazole (3.0 g, 21.3 mmol, see international patent application WO 2003057673) was dissolved in an autoclave in EtOH (50 mL) and added 10% Pd/C (1.0 g). Then 3 bar of $H_2$ were applied to the reaction, which was stirred for 6 h. The suspension was filtered over celite, washed with EtOH, and the filtrate was concentrated *in vacuo.* The product (2.0 g, 86%) was crystallized from an EtOH/EtOAc mixture.

Step 2: 3,5-dimethyl-1-(2-nitrophenyl)-1H-pyrazol-4-amine

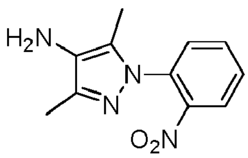

The amine from step 1 (202.2 mg, 1.8 mmol), and potassium t-butanoate (224.5 mg, 2.0 mmol) were dissolved in DMSO (2 mL). After 10 minutes, 1-fluoro-2-nitrobenzene (201.4 μL, 1.9 mmol) was added, and the reaction mixture was heated to 70 °C and left stirring for 3 h. The crude was then diluted with a saturated solution of $NaHCO_3$ and DCM, the phases were separated and the aqueous phase was extracted 3 times with toluene. The reunited organic phase was washed with brine, dried over $MgSO_4$, filtered and concentrated. The crude was purified by column chromatography (1→5% MeOH in DCM) yielding 282 mg (67%) of the title compound.

Step 3: 2-((3,5-dimethyl-1-(2-nitrophenyl)-1H-pyrazol-4-yl)amino)-2-oxoethyl acetate

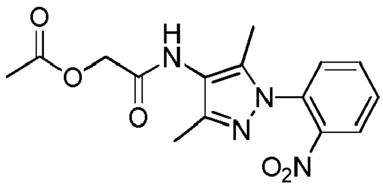

From the compound of step 2 (255 mg, 1.10 mmol) was prepared 290 mg (79%) of the title compound analogously to Example 1, step 3.

Step 4: N-(3,5-dimethyl-1-(2-nitrophenyl)-1H-pyrazol-4-yl)-2-hydroxyacetamide

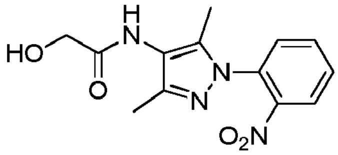

From the compound of step 3 (203 mg, 0.61 mmol) was prepared 197 mg (quantitative) of the title compound analogously to Example 1, step 4.

Step 5: 2-((3,5-dimethyl-1-(2-nitrophenyl)-1H-pyrazol-4-yl)amino)-2-oxoethyl 2-(2-methyl-1H-indol-1-yl)acetate

(2-Methyl-indol-1-yl)-acetic acid (23.1 mg, 0.1 mmol) was dissolved in dry DCM (2 mL) at 0 °C under Argon atmosphere. DCC (24.4 mg, 0.1 mmol) and DMAP (1.5 mg, 0.01 mmol) were added followed by the alcohol from step 4 (35.4 mg, 0.1 mmol). The mixture was allowed to warm to room temperature and stirred for 8 h. The suspension was filtered over cotton to remove the bulk of DCU and washed with $NH_4Cl$ solution (semi-saturated). After drying with $MgSO_4$ the solvent was evaporated and the product was purified twice by column chromatography (3% MeOH in DCM, and then 75% EtOAc in PE) to yield 49 mg (86%) of the title compound.

**TLC:** $R_f$ (75% EtOAc in PE): 0.25.

**$^1$H NMR** (400 MHz, $CD_2Cl_2$) δ: 7.98 (dd, *J* = 8.1, 1.3 Hz, 1 H), 7.72 (dt, *J* = 7.7, 1.5 Hz, 1 H), 7.60 (dt, *J* = 8.0, 1.4 Hz, 1H), 7.47 (dd, *J* = 8.0, 1.2 Hz, 1H), 7.46 (dd, *J* = 8.0, 1.2 Hz, 1H), 7.24 (d, *J* = 8.1 Hz, 1H), 7.13 (ddd, *J* = 8.1, 7.2, 1.2 Hz, 1H), 7.05 (ddd, *J* = 7.8, 7.2, 1.0 Hz, 1H), 6.53 (bs, 1 H), 6.32 (dq, *J* = 1.8, 0.9 Hz, 1 H), 4.99 (s, 2H), 4.67 (s, 2H), 2.44 (d, *J* = 0.8 Hz, 3H), 1.97 (s, 3H), 1.90 (s, 3H) ppm

**$^{13}$C NMR** (100 MHz, $CD_2Cl_2$) δ: 167.4, 165.3, 146.8, 146.3, 137.2, 137.0, 136.8, 133.3, 132.7, 129.4, 129.2, 128.4, 125.1, 121.4, 120.4, 120.1, 115.1, 108.3, 101.4, 63.4, 44.9, 12.3, 10.9, 9.6 ppm

**HRMS** (ESI) m/z: $(M+H)^+$ calcd for $C_{24}H_{24}N_5O_5$: 462.1772; found: 462.1775

*Example Compound 6*

*2-((1-(4-cyanophenyl)-3,5-dimethyl-1H-pyrazol-4-yl)amino)-2-oxoethyl 2-(2-methyl-1H-indol-1-yl)acetate*

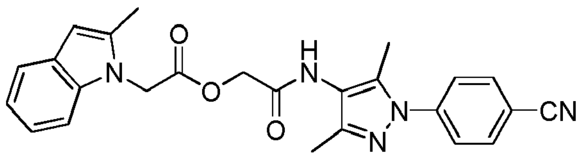

Step 1: 4-(3,5-dimethyl-4-nitro-1H-pyrazol-1-yl)benzonitrile

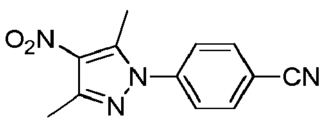

3,5-dimethyl-4-nitro-1H-pyrazole (199.5 mg, 1.4 mmol) and potassium t-butanoate (173.8 mg, 1.5 mmol) were dissolved in DMSO (1 mL). After 15 minutes, 4-fluorobenzonitrile (182.0 mg, 1.5 mmol) was added, and the reaction mixture was heated to 120 °C and left stirring for 5 h. The reaction mixture was then diluted with water and DCM, the phases were separated and the aqueous phase was extracted 3 times with DCM. The reunited organic phase was dried over $MgSO_4$, filtered and concentrated. The crude was purified by column chromatography (25% EtOAc in PE) yielding 263 mg (77%) of the title compound.

Step 2: 4-(4-amino-3,5-dimethyl-1H-pyrazol-1-yl)benzonitrile

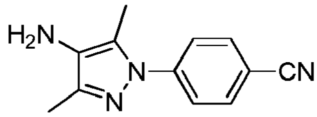

To the nitrile from step 1 (233.0 mg, 1.0 mmol) in DMF (3.5 mL), was added $SnCl_2$ (1.9 g, 8.7 mmol) and the solution was heated to 70 °C. After 3 h the reaction mixture was diluted with water and EtOAc and filtered through celite. The aqueous phase was extracted 3 times with EtOAc and the reunited organic phase was additionally washed with a saturated solution of $NaHCO_3$, dried over $MgSO_4$, filtered and concentrated. Column chromatography (5% MeOH in DCM) yielded the product in a 3:1 mixture with the corresponding acetylated byproduct (138 mg), which was used without further purification in the next step.

Step 3: 2-((1-(4-cyanophenyl)-3,5-dimethyl-1H-pyrazol-4-yl)amino)-2-oxoethyl acetate

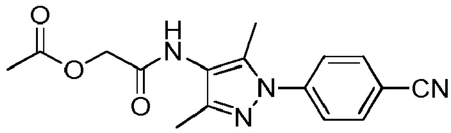

From the mixture of products of step 2 was prepared 141 mg (47% 2 steps) of the title compound analogously to Example 1, step 3.

Step 4: N-(1-(4-cyanophenyl)-3,5-dimethyl-1 H-pyrazol-4-yl)-2-hydroxyacetamide

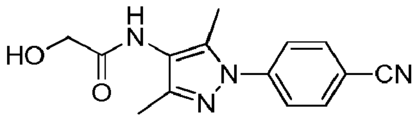

From the compound of step 3 (135 mg, 0.43 mmol) was prepared 109 mg (93%) of the title compound analogously to Example 1, step 4.

Step 5: 2-((3,5-dimethyl-1-(4-(trifluoromethyl)phenyl)-1H-pyrazol-4-yl)amino)-2-oxoethyl 2-(2-methyl-1 H-indol-1-yl)acetate

(2-Methyl-indol-1-yl)-acetic acid (25.0 mg, 0.1 mmol) was dissolved in dry DCM (3 mL) at 0 °C under Argon atmosphere. DCC (26.5 mg, 0.1 mmol) and DMAP (1.7 mg, 0.01 mmol) were added followed by the alcohol from step 4 (35.9 mg, 0.1 mmol). The mixture was allowed to warm to room temperature and stirred for 8 h. The suspension was filtered over cotton to remove the bulk of DCU and washed with $NH_4Cl$ solution (semi-saturated). After drying with $MgSO_4$ the solvent was evaporated and the product was purified by column chromatography (75% EtOAc in PE) to yield 43 mg (73%) of the title compound.

**TLC:** $R_f$ (5% MeOH in DCM. 2x): 0.53.

**$^1$H NMR** (400 MHz, $CD_2Cl_2$/MeOD) $\delta$: 7.72 (d, 2H), 7.56 (d, 2H), 7.44 (dddd, *J* = 7.9, 2.3, 1.2, 0.8 Hz, 1H), 7.23 (ddd, *J* = 8.4, 1.7, 1.0 Hz, 1H), 7.10 (ddd, *J* = 8.2, 7.2, 1.0 Hz, 1H), 6.99 (ddd, *J* = 7.9, 7.2, 0.8 Hz, 1H), 6.32 (dq, *J* = 1.8, 0.9 Hz, 1H), 6.16 (bs, 1H), 4.98 (s, 2H), 4.68 (s, 2H), 2.47 (d, *J* = 1.0 Hz, 3H), 1.99(s, 3H), 1.99 (s, 3H) ppm

**$^{13}$C NMR** (100 MHz, $CD_2Cl_2$/MeOD) $\delta$: 168.8, 167.1, 147.7, 143.7, 137.9, 137.7, 136.7, 133.9, 129.0, 124.8, 121.7, 120.6, 120.5, 118.9, 117.4, 111.1, 101.6, 63.8, 45.2, 12.7, 11.4, 11.3 ppm **HRMS** (ESI) m/z: $(M+H)^+$ calcd for $C_{25}H_{24}N_5O_3$: 442.1874; found: 442.1876

*Example Compound 7*

*2-((1-(4-methoxybenzyl)-3,5-dimethyl-1H-pyrazol-4-yl)amino)-2-oxoethyl 2-(2-methyl-1H-indol-1-yl)acetate*

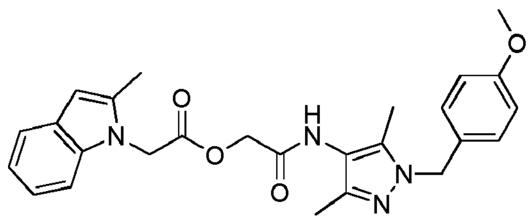

Step 1: 1-(4-methoxybenzyl)-3,5-dimethyl-4-nitro-1 H-pyrazole

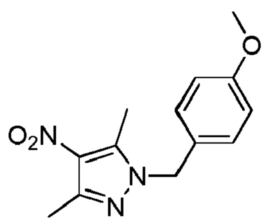

To a suspension of 3,5-dimethyl-4-nitro-1H-pyrazole (203.4 mg, 1.8 mmol, see international patent application WO 2003057673) and $K_2CO_3$ (278 mg, 2.0 mmol) in dry DMF (1 mL), 4-methoxybenzyl chloride (260.5 μL, 1.9 mmol) was added slowly after 15 minutes. The reaction was left stirring overnight The mixture was diluted with water and extracted 3 times with EtOAc. After drying over $MgSO_4$ and being filtered, the organic phase was concentrated and the crude was purified by column chromatography (30% EtOAc in PE) yielding 355 mg (96%) of the title compund.

Step 2: 1-(4-methoxybenzyl)-3,5-dimethyl-1 H-pyrazol-4-amine

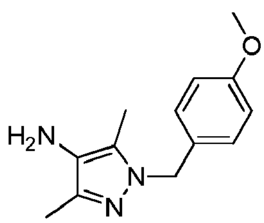

From the compound of step 1 (316 mg, 1.21 mmol) was prepared 297 mg (quantitative) of the title compound analogously to Example 2, step 2.

Step 3: 2-((1-(4-methoxybenzyl)-3,5-dimethyl-1H-pyrazol-4-yl)amino)-2-oxoethyl acetate

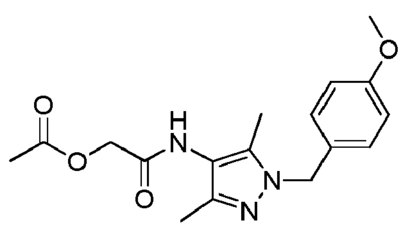

From the compound of step 2 (291 mg, 1.26 mmol) was prepared 356 mg (85%) of the title compound analogously to Example 1, step 3.

Step 4: 2-hydroxy-N-(1-(4-methoxybenzyl)-3,5-dimethyl-1H-pyrazol-4-yl)acetamide

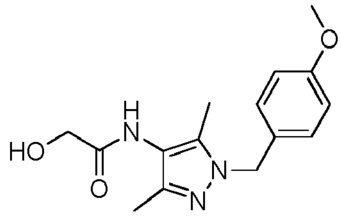

From the compound of step 3 (343 mg, 1.04 mmol) was prepared 287 mg (96%) of the title compound analogously to Example 1, step 4.

Step 5: 2-oxo-2-((1,3,5-trimethyl-1H-pyrazol-4-yl)amino)ethyl 2-(2-methyl-1 H-indol-1-yl)acetate

(2-Methyl-indol-1-yl)-acetic acid (40.0 mg, 0.2 mmol) was dissolved in dry DCM (4 mL) at 0 °C under Argon atmosphere. DCC (42.1 mg, 0.2 mmol) and DMAP (2.8 mg, 0.02 mmol) were added followed by the alcohol from step 4 (61.1 mg, 0.2 mmol). The mixture was allowed to warm to room temperature and stirred for 6.5 h. The suspension was filtered over cotton to remove the bulk of DCU and washed with $NH_4Cl$ solution (semi-saturated). After drying with $MgSO_4$ the solvent was evaporated and the product was purified by column chromatography (75% EtOAc in PE and then 5% MeOH in DCM) to yield 72 mg (71%) of the title compound.

**TLC:** $R_f$ (5% MeOH in DCM, 2x): 0.25.

**$^1$H NMR** (400 MHz, $CD_2Cl_2$) δ: 7.44 (d, *J*= 8.0 Hz, 1H), 7.21 (d, *J*= 8.3 Hz, 1H), 7.02-7.11 (m, 3H), 6.95 (ddd, *J* = 7.9, 7.2, 0.9 Hz, 1 H), 6.85-6.91 (m, 2H), 6.32 (bs, 1 H), 6.29 (dq, *J* = 1.8, 0.9 Hz, 1 H), 5.07 (s, 2H), 4.96 (s, 2H), 4.43 (s, 2H), 3.79 (s, 3H), 2.43 (d, *J* = 0.9 Hz, 3H), 1.93(s, 3H), 1.86 (s, 3H) ppm

**$^{13}$C NMR** (100 MHz, $CD_2Cl_2$) δ: 167.9, 165.8, 159.8, 144.1, 137.7, 137.5, 135.4, 133.9, 129.8, 128.9, 128.8, 124.8, 121.9, 120.8, 120.6, 114.6, 114.0, 108.9, 101.9, 64.0, 55.8, 45.5, 12.9, 11.4, 9.6 ppm

**HRMS** (ESI) m/z: $(M+H)^+$ calcd for $C_{26}H_{29}N_4O_4$: 461.2183; found: 461.2186

*Example Compound 8*

*2-((3,5-dimethyl-1-(2-(trifluoromethyl)phenyl)-1H-pyrazol-4-yl)amino)-2-oxoethyl 2-(2-methyl-1H-indol-1-yl)acetate*

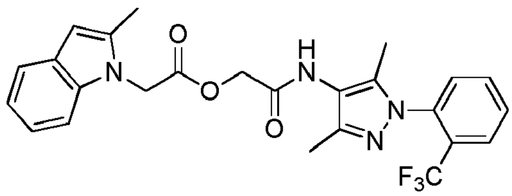

Step 1: 3,5-dimethyl-4-nitro-1-(2-(trifluoromethyl)phenyl)-1H-pyrazole

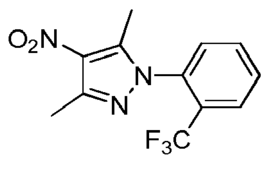

3,5-dimethyl-4-nitro-1H-pyrazole (199.6 mg, 1.4 mmol) and potassium t-butanoate (175.0 mg, 1.5 mmol) were dissolved in DMSO (1 mL). After 25 minutes, 1-fluoro-3-(trifluoromethyl)benzene (189 μL, 1.5 mmol) was added, and the reaction mixture was heated to 150 °C and left stirring overnight. The crude was then diluted with water and DCM, the phases were separated and the aqueous phase was extracted 3 times with DCM. The reunited organic phase was washed with brine, dried over $MgSO_4$, filtered and concentrated. The crude was purified by column chromatography (30% EtOAc in PE) yielding 279 mg (69%) of the title compound.

Step 2: 3,5-dimethyl-1-(2-(trifluoromethyl)phenyl)-1H-pyrazol-4-amine

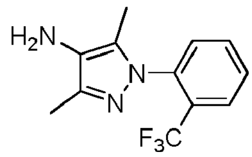

From the compound of step 1 (266 mg, 0.93 mmol) was prepared 238 mg (quantitative) of the title compound analogously to Example 2, step 2.

Step 3: 2-((3,5-dimethyl-1-(3-(trifluoromethyl)phenyl)-1 H-pyrazol-4-yl)amino)-2-oxoethyl acetate

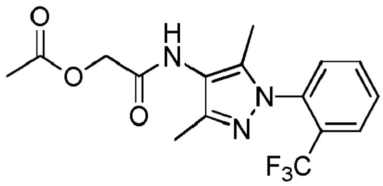

From the compound of step 2 (226 mg, 0.88 mmol) was prepared 285 mg (91%) of the title compound analogously to Example 1, step 3.

Step 4: N-(3,5-dimethyl-1-(2-(trifluoromethyl)phenyl)-1H-pyrazol-4-yl)-2-hydroxyacetamide

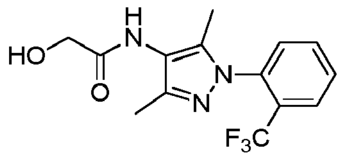

From the compound of step 3 (276 mg, 0.78 mmol) was prepared 205 mg (84%) of the title compound analogously to Example 1, step 4.

Step 5: 2-((3,5-dimethyl-1-(2-(trifluoromethyl)phenyl)-1H-pyrazol-4-yl)amino)-2-oxoethyl 2-(2-methyl-1 H-indol-1-yl)acetate

(2-Methyl-indol-1-yl)-acetic acid (25.1 mg, 0.1 mmol) was dissolved in dry DCM (1 mL) at 0 °C under Argon atmosphere. DCC (27.0 mg, 0.1 mmol) and DMAP (1.7 mg, 0.01 mmol) were added followed by the alcohol from step 4 (41.7 mg, 0.1 mmol). The mixture was allowed to warm to room temperature, added some more (2-Methyl-indol-1-yl)-acetic acid after 8 h (5 mg) and stirred overnight. The suspension was filtered over cotton to remove the bulk of DCU and washed with $NH_4Cl$ solution (semi-saturated). After drying with $MgSO_4$ the solvent was evaporated and the product was purified by column chromatography (75% EtOAc in PE) to yield 6 mg (9%) of the title compound. The low yield results from copolarity with DCU which could not be completely separated.

**TLC:** $R_f$ (5% MeOH in DCM, 2x): 0.25.

**$^1$H NMR** (400 MHz, $CD_2Cl_2$) δ: 7.83 (dd, *J* = 7.8, 1.3 Hz, 1H), 7.70 (dt, *J* = 7.8, 1.3 Hz, 1H), 7.64 (t, *J* = 7.5 Hz, 1 H), 7.45 (d, *J* = 7.8 Hz, 1 H), 7.36 (d, *J* = 7.7 Hz, 1 H), 7.24 (d, *J* = 8.2 Hz, 1 H), 7.11 (ddd, *J* = 8.2, 7.3, 1.1 Hz, 1H), 7.01 (ddd, *J* = 7.9, 7.2, 0.9 Hz, 1H), 6.32 (bs, 1H), 6.31 (bs, 1 H), 5.00 (s, 2H), 4.66 (s, 2H), 2.45 (d, *J*= 0.9 Hz, 3H), 1.93 (s, 3H), 1.76 (s, 3H) ppm

**$^{13}$C NMR** (101 MHz, DMSO-$d_6$) δ: 168.92, 165.68, 144.41, 137.12, 137.09, 136.83, 136.02, 133.71, 130.64, 130.15, 127.69, 127.42 (q, $J_{C-F}$ = 4.9 Hz), 126.61 (q, $J_{C-F}$ = 30.7 Hz), 124.4 (q,

$J_{C-F}$ = 273.3 Hz) 120.28, 119.29, 119.15, 115.43, 109.25, 100.10, 63.02, 44.03, 12.14, 11.34, 9.59 ppm

**HRMS** (ESI) m/z: $(M+H)^+$ calcd for $C_{25}H_{24}F_3N_4O_3$: 485.1795; found: 485.1797

*ExampleCompound 9*

*2-((3,5-dimethyl-1-(p-tolyl)-1H-pyrazol-4-yl)amino)-2-oxoethyl 2-(2-methyl-1H-indol-1-yl)acetate*

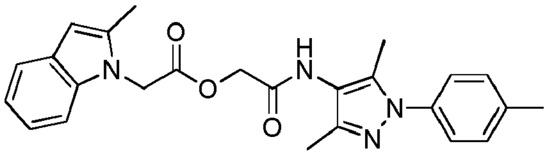

Step 1: 3,5-dimethyl-4-nitro-1-(p-tolyl)-1H-pyrazole

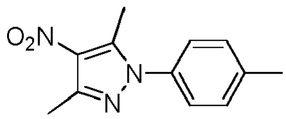

3,5-dimethyl-4-nitro-1 H-pyrazole (1.9 g, 13.5 mmol), $K_2CO_3$ (3.9 g, 28.2 mmol), CuI (13.6 mg, 0.071 mmol), trans-N,N-dimethylcyclohexyldiamine (425 μL, 2.7 mmol) and finally 4-iodotoluene (5 mL, 39.0 mmol) were added to a MW vial and irradiated for 4 h to 195 °C in the microwave. The reaction mixture was filtered through celite, washed with EtOAc, concentrated and purified by column chromatography (5% EtOAc in PE) to yield 2.49 g (80%) of the title compound.

Step 2: 3,5-dimethyl-1-(p-tolyl)-1 H-pyrazol-4-amine

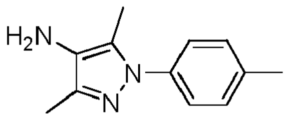

The title compound was prepared analogously to Example 2, step 2.

Step 3: 2-((3,5-dimethyl-1-(p-tolyl)-1H-pyrazol-4-yl)amino)-2-oxoethyl acetate

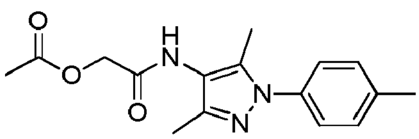

From the comound of step 2 (798 mg, 3.96 mmol) was prepared 598 mg (50%) of the title compound analogously to Example 1, step 3.

Step 4: N-(3,5-dimethyl-1-(p-tolyl)-1H-pyrazol-4-yl)-2-hydroxyacetamide

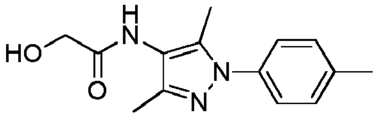

From the compound of step 3 (565 mg, 1.87 mmol) was prepared 488 mg (quantitative) of the title compound analogously to Example 1, step 4.

Step 5 2-((3,5-dimethyl-1-(p-tolyl)-1H-pyrazol-4-yl)amino)-2-oxoethyl 2-(2-methyl-1 H-indol-1-yl)acetate

(2-Methyl-indol-1-yl)-acetic acid (23.1 mg, 0.1 mmol) was dissolved in dry DCM (2 mL) at 0 °C under Argon atmosphere. DCC (26.5 mg, 0.1 mmol) and DMAP (1.7 mg, 0.01 mmol) were added followed by the alcohol from step 4 (31.6 mg, 0.1 mmol). The mixture was allowed to warm to room temperature and stirred for 8 h. The suspension was filtered over cotton to remove the bulk of DCU and washed with $NH_4Cl$ solution (semi-saturated). After drying with $MgSO_4$ the solvent was evaporated and the product was purified by column chromatography (2% MeOH in DCM) to yield 48 mg (92%) of the title compound.

**TLC:** $R_f$ (5% MeOH in DCM): 0.45.

**$^1$H NMR** (400 MHz, $CD_2Cl_2$) δ: 7.45 (ddd, *J* = 7.8, 1.3, 0.9 Hz, 1 H), 7.23-7.29 (m, 4H), 7.21 (ddd, *J*= 8.1, 1.7, 0.9 Hz, 1H), 7.10 (ddd, *J* = 8.3, 7.2, 1.2 Hz, 1H), 7.00 (ddd, *J* = 7.9, 7.2, 1.0 Hz, 1H), 6.41 (bs, 1H), 6.31 (dq, *J* = 1.9, 1.0 Hz, 1H), 4.95 (s, 2H), 4.67 (s, 2H), 2.45 (d, *J* = 1.0 Hz, 3H), 2.39 (s, 3H), 2.00 (s, 3H), 1.93 (s, 3H) ppm

**$^{13}$C NMR** (100 MHz, $CD_2Cl_2$) δ: 167.3, 165.1, 144.9, 137.6, 137.4, 137.2, 136.7, 135.3, 129.9, 128.4, 124.7, 121.7, 120.6, 120.4,, 114.5, 108.3, 101.8, 63.5, 45.1, 21.2, 12.7, 11.2, 10.6 ppm **HRMS** (ESI) m/z: $(M+H)^+$ calcd for $C_{25}H_{27}N_4O_3$: 431.2078; found: 431.2081

*Example Compound 10*

*2-((3,5-dimethyl-1-(4-nitrophenyl)-1H-pyrazol-4-yl)amino)-2-oxoethyl 2-(2-methyl-1H-indol-1-yl)acetate*

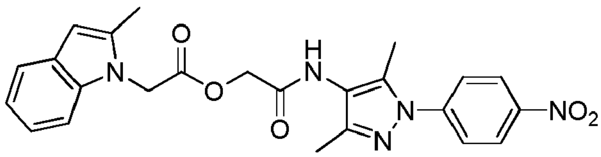

Step 1: 3,5-dimethyl-1-(4-nitrophenyl)-1H-pyrazol-4-amine

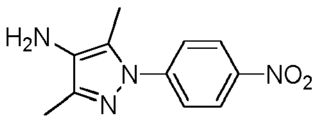

From the compound of example 5, step 1 (400 mg, 3.60 mmol) and 1-fluoro-4-nitrophenol (400 μL, 3.78 mmol), was prepared 512 mg (61%) of the title compound analogously as described in example 5, step 2.

Step 2: 2-((3,5-dimethyl-1-(4-nitrophenyl)-1H-pyrazol-4-yl)amino)-2-oxoethyl acetate

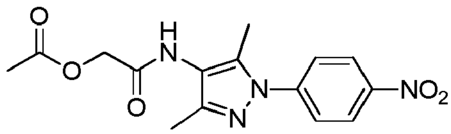

From the compound of step 1 (501 mg, 2.16 mmol), was prepared 531 mg (74%) of the title compound analogously as described in example 1, step 3.

Step 3: N-(3,5-dimethyl-1-(4-nitrophenyl)-1 H-pyrazol-4-yl)-2-hydroxyacetamide

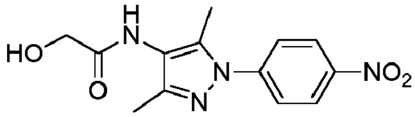

From the compound of step 2 (571 mg, 1.72 mmol), was prepared 336 mg (67%) of the title compound analogously as described in example 1, step 4.

Step 4: 2-((3,5-dimethyl-1-(4-nitrophenyl)-1H-pyrazol-4-yl)amino)-2-oxoethyl 2-(2-methyl-1H-indol-1-yl)acetate

From the compound of step 3 (35 mg, 0.12 mmol) and 2-(2-methyl-1 H-indol-1-yl)acetic acid (23.1 mg, 0.12 mmol) was prepared 12.0 mg (21%) of the title compound analogously as described in example 1, step 5.

**TLC:** $R_f$ (75% EtOAc in PE): 0.25.

**$^1$H NMR** (400 MHz, $CDCl_3$) δ: 8.30-8.35 (m, 2H), 7.61-7.66 (m, 2H), 7.44 (ddd, *J* = 7.9, 1.4, 0.8 Hz, 1H), 7.23 (ddd, *J* = 8.3, 1.7, 1.0 Hz, 1H), 7.10 (ddd, *J* = 7.9, 6.8, 1.1 Hz, 1H), 6.99 (ddd, *J* = 7.9, 7.0, 1.0 Hz, 1H), 6.32 (dq, *J* = 1.9, 1.1 Hz, 1H), 6.14 (bs, 1H), 4.98 (s, 2H), 4.69 (s, 2H), 2.48 (d, *J* = 0.9 Hz, 3H), 2.03 (s, 3H), 1.98 (s, 3H) ppm

**$^{13}$C NMR** (100 MHz, $CDCl_3$) δ: 167.1, 165.5, 147.2, 146.1, 144.8, 137.2, 136.8, 135.7, 128.5, 124.9, 123.7, 121.8, 120.7, 120.5, 116.7, 108.4, 102.0, 63.4, 45.3, 12.7, 11.4, 11.3 ppm **HRMS** (ESI) m/z: $(M+H)^+$ calcd for $C_{24}H_{24}N_5O_5$: 462.1772; found: 462.1777

*Example Compound 11*

*2-((3,5-dimethyl-1-phenyl-1H-pyrazol-4-yl)amino)-2-oxoethyl 2-(1H-indol-1-yl)acetate*

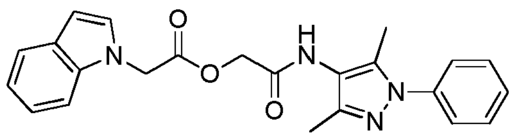

From N-(3,5-dimethyl-1-phenyl-1H-pyrazol-4-yl)-2-hydroxyacetamide (51 mg, 0.2 mmol, example 1, step 4) and 2-(1H-indol-1-yl)acetic acid (40.0 mg, 0.2 mmol) was prepared 66.5 mg (79%) of the title compound analogously as described in example 1, step 5.

**TLC:** $R_f$ (75% EtOAc in PE): 0.33.

**$^1$H NMR** (400 MHz, $CDCl_3$) δ: 7.58 (ddd, *J* = 8.0, 1.2, 0.8 Hz, 1 H), 7.32-7.48 (m, 5H), 7.30 (ddd, *J* = 8.3, 1.9, 1.0 Hz, 1H), 7.20 (dddd, *J* = 8.3, 7.1, 1.2, 0.2 Hz, 1H), 7.14 (d, *J* = 3.3 Hz, 1H), 7.06 (ddd, *J* = 8.0, 7.1, 1.0 Hz, 1H), 6.58 (dd, *J* = 3.2, 0.9 Hz, 1H), 6.40 (bs, 1H), 5.02 (s, 2H), 4.67 (s, 2H), 2.00 (s, 3H), 1.95 (s, 3H) ppm

**$^{13}$C NMR** (100 MHz, $CDCl_3$) δ: 168.5, 166.8, 146.6, 141.2, 137.9, 136.8, 130.6, 130.2, 130.1, 129.1, 126.2, 124.2, 123.0, 122.0, 116.1, 110.2, 104.8, 64.9, 46.7, 12.7, 12.1.ppm **HRMS** (ESI) m/z: $(M+H)^+$ calcd for $C_{23}H_{23}N_4O_3$: 403.1765; found: 403.1767

*Example Compound 12*

*2-((3,5-dimethyl-1-phenyl-1H-pyrazol-4-yl)amino)-2-oxoethyl 2-(1H-indol-3-yl)acetate*

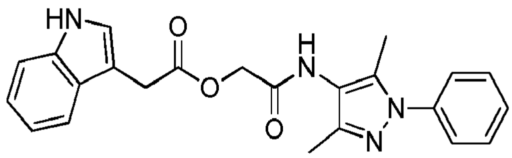

To a stirring solution of 2-(1H-indol-3-yl)acetic acid (79.1 mg, 0.4 mmol) in DCM (2 mL), DCC (93.0 mg, 0.4 mmol) was added slowly at 0 °C, then DMAP (5.8 mg, 0.05 mmol) and finally N-(3,5-dimethyl-1-phenyl-1 H-pyrazol-4-yl)-2-hydroxyacetamide (101.0 mg, 0.4 mmol), synthesized as reported in Example 1, step 4. The reaction mixture was stirred for 6 h and then filtered to remove the DCU precipitate. The product was purified by column chromatography (gradient of EtOAc in PE) to yield 124 mg (75%) of the title compound.

**TLC:** $R_f$ (5% MeOH in DCM): 0.38.

**$^1$H NMR** (400 MHz, $CDCl_3$) δ: 8.78 (bs, 1H), 7.64 (d, *J* = 7.8 Hz, 1H), 7.38-7.45 (m, 2H), 7.30-7.36 (m, 2H), 7.26 (d, *J* = 7.9 Hz, 1H), 7.19 (bs, 1H), 7.03-7.17 (m, 3H), 6.81 (bs, 1H), 4.78 (s, 2H), 3.91 (s, 2H), 2.43 (s, 3H), 1.97 (s, 3H), 1.89 (s, 3H) ppm

**$^{13}$C NMR** (100 MHz, $CDCl_3$) δ: 170.3, 166.5, 145.0, 139.6, 136.3, 135.3, 129.0, 127.5, 126.6, 124.6, 123.4, 122.4, 120.0, 118.1, 114.7, 111.6, 107.3, 63.0, 31.5, 10.9, 10.4 ppm **HRMS** (ESI) m/z: $(M+H)^+$ calcd for $C_{23}H_{23}N_4O_3$: 403.1765; found: 403.1766

*Example Compound 13*

*2-((3,5-dimethyl-1-(p-tolyl)-1H-pyrazol-4-yl)amino)-2-oxoethyl 2-(1H-indol-3-yl)acetate*

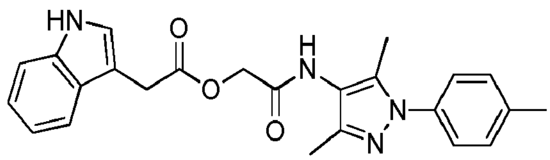

To a solution of 3-indolyl acetic acid (21.4 mg, 0.12 mmol) in anhydrous $CH_2Cl_2$ (2.0 mL) was added DCC (24 mg, 0.12 mmol), DMAP (1.5 mg, 0.012 mmol), and N-(3,5-dimethyl-1-(p-tolyl)-1 H-pyrazol-4-yl)-2-hydroxyacetamide (31.6 mg, 0.12 mmol) at 0 °C under argon. The suspension was stirred for 6 h and then filtered to remove the DCU precipitate. The filtrate was washed with aqueous $NH_4Cl$, the layers were separated, the organics were dried ($MgSO_4$), filtered, and concentrated *in vacuo.* The product was purified by column chromatography (3% MeOH in $CH_2Cl_2$) to yield 43 mg (85%) of the title compound.

**TLC:** $R_f$ (5% MeOH in DCM): 0.30.

**$^1$H NMR** (600 MHz, $CDCl_3$) δ: 8.65 (bs, 1 H), 7.69 (d, *J* = 8.0 Hz, 1 H), 7.31 (ddd, *J* = 7.8, 1.2, 0.8 Hz, 1H), 7.26 (bs, 4H), 7.19 (bs, 1H), 7.17 (ddd, *J* = 8.2, 7.2, 1.1 Hz, 1H), 7.12 (ddd, *J* = 7.8, 6.9, 1.0 Hz, 1 H), 6.79 (bs, 1 H), 4.78 (s, 2H), 3.96 (s, 2H), 2.43 (s, 3H), 1.97 (s, 3H), 1.92 (s, 3H) ppm

**$^{13}$C NMR** (151 MHz, $CDCl_3$) δ: 170.4, 166.5, 144.9, 137.6, 137.3, 136.4, 135.4, 129.7, 126.8, 124.7, 123.5, 122.7, 120.3, 118.3, 114.5, 111.7, 107.6, 63.2, 31.6, 21.2, 11.1, 10.5 ppm

**HRMS** (ESI) m/z: $(M+H)^+$ calcd for $C_{24}H_{25}N_4O_3$: 417.1921; found: 417.1929

*Example Compound 14*

*2-((3,5-dimethyl-1-phenyl-1H-pyrazol-4-yl)amino)-2-oxoethyl 2-(3-methyl-1H-indol-1-yl)acetate*

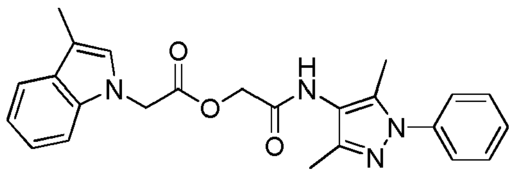

Step 1: methyl 2-(3-methyl-1 H-indol-1-yl)acetate

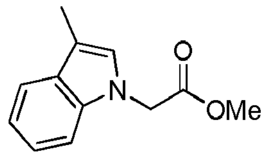

To a solution of 3-methylindole (260 mg, 1.98 mmol) in anhydrous DMF (3.5 mL) was added NaH (95 mg, 2.38 mmol) at 0 °C under argon. The mixture was allowed to warm to room temperature, and after 30 min, was cooled back to 0 °C. To the resulting solution was added methyl bromoacetate (0.24 mL, 2.4 mmol) and the mixture was warmed to room temperature. After 4 h, the reaction was quenched with aqueous $NH_4Cl$ (30 mL) and diluted with EtOAc (100 mL). The two layers were separated and the organic layer was washed with $H_2O$ (30 mL), aqueous $NaHCO_3$ (70 mL), and brine (30 mL). The organic layer was dried ($MgSO_4$), filtered, and concentrated *in vacuo.* Purification by column chromatography (20% EtOAc in petroleum ether) gave 208 mg (52%) of the title compound.

Step 2: 2-(3-methyl-1H-indol-1-yl)acetic acid

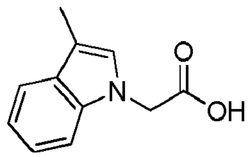

To a solution of methyl 2-(3-methyl-1H-indol-1-yl)acetate (185 mg, 0.91 mmol) in THF (3.0 mL) was added a solution of LiOH (1.1 mL, 1.1 mmol, 1.0 M in $H_2O$). After 45 min, the reaction mixture was diluted with EtOAc (100 mL), $H_2O$ (75 mL), and 1M HCl (10 mL). The two layers were separated and the aqueous layer was extracted with EtOAc (2 x 25 mL). The combined organics were washed with brine (30 mL), dried ($MgSO_4$), filtered, and concentrated *in vacuo* to give the title compound.

Step 3: 2-((3,5-dimethyl-1-phenyl-1 H-pyrazol-4-yl)amino)-2-oxoethyl 2-(3-methyl-1 H-indol-1-yl)acetate

To a solution of 2-(3-methyl-1 H-indol-1-yl)acetic acid (30.0 mg, 0.2 mmol) in anhydrous $CH_2Cl_2$ (1.0 mL), DCC (37.6 mg, 0.2 mmol) was added at 0 °C, then DMAP (2.0 mg, 0.02 mmol) and finally N-(3,5-dimethyl-1-phenyl-1H-pyrazol-4-yl)-2-hydroxyacetamide (37 mg, 0.2 mmol). The suspension was stirred for 5 h and then filtered to remove the DCU precipitate and washed with $NH_4Cl$ solution (semi-saturated) and brine. The product was purified by column chromatography (70% EtOAc in PE) to yield the title compound.

**TLC:** $R_f$ (75% EtOAc in PE): 0.38.

**$^1$H NMR** (400 MHz, $CDCl_3$) δ: 7.51 (ddd, *J* = 8.2, 1.2, 0.8 Hz, 1 H), 7.33-7.48 (m, 5H), 7.26 (ddd, *J*= 8.2, 1.2, 0.8 Hz, 1H), 7.19 (ddd, *J* = 7.8, 7.0, 1.1 Hz, 1H), 7.06 (ddd, *J* = 8.0, 7.0, 1.0 Hz, 1H), 6.90 (q, *J*= 1.1 Hz, 1H), 6.33 (bs, 1H), 4.96 (s, 2H), 4.67 (s, 2H), 2.28 (d, *J* = 1.1 Hz, 3H), 1.99 (s, 3H), 1.95 (s, 3H) ppm

**$^{13}$C NMR** (100 MHz, $CDCl_3$) δ: 168.8, 167.0, 146.6, 141.2, 138.3, 136.8, 130.7, 130.6, 129.1, 127.5, 126.2, 124.1, 121.4, 121.1, 116.2, 114.1, 110.8, 64.9, 49.5, 12.6, 12.1, 11.0 ppm

**HRMS** (ESI) m/z: $(M+H)^+$ calcd for $C_{24}H_{25}N_4O_3$: 417.1921; found: 417.1924

*Example Compound 15*

*2-((3,5-dimethyl-1-phenyl-1H-pyrazol-4-yl)amino)-2-oxoethyl 2-(2,3-dimethyl-1H-indol-1-yl)acetate*

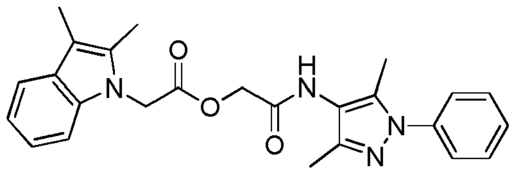

Step 1: methyl 2-(2,3-dimethyl-1 H-indol-1-yl)acetate

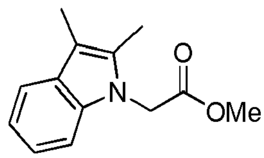

To a solution of 2,3-dimethylindole (287 mg, 1.98 mmol) in anhydrous DMF (1.5 mL) was added NaH (95 mg, 2.38 mmol) at 0 °C under argon. The mixture was allowed to warm to room temperature, and after 30 min, was cooled back to 0 °C. To the resulting solution was added methyl bromoacetate (0.24 mL, 2.4 mmol) and the mixture was warmed to room temperature. After 4 h, the reaction was quenched with aqueous $NH_4Cl$ (30 mL) and diluted with EtOAc (100 mL). The two layers were separated and the organic layer was washed with $H_2O$ (30 mL), aqueous $NaHCO_3$ (70 mL), and brine (30 mL). The organic layer was dried ($MgSO_4$), filtered, and concentrated *in vacuo.* Purification by column chromatography (5% EtOAc in petroleum ether) gave 149 mg (35%) of the title compound.

Step 2: 2-(2,3-dimethyl-1 H-indol-1-yl)acetic acid

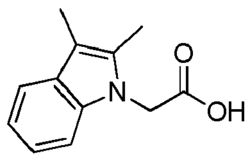

To a solution of methyl 2-(2,3-dimethyl-1H-indol-1-yl)acetate (135 mg, 0.62 mmol) in THF (2.0 mL) was added a solution of LiOH (0.75 mL, 0.75 mmol, 1.0 M in $H_2O$). After 45 min, the reaction mixture was diluted with EtOAc (100 mL), $H_2O$ (75 mL), and 1 M HCl (10 mL). The two layers were separated and the aqueous layer was extracted with EtOAc (2 x 25 mL). The combined organics were washed with brine (30 mL), dried ($MgSO_4$), filtered, and concentrated *in vacuo* to give the title compound.

Step 3: 2-((3,5-dimethyl-1-phenyl-1 H-pyrazol-4-yl)amino)-2-oxoethyl 2-(2,3-dimethyl-1 H-indol-1-yl)acetate

To a solution of 2-(2,3-dimethyl-1 H-indol-1-yl)acetic acid (41.5 mg, 0.20 mmol) in anhydrous $CH_2Cl_2$ (2.0 mL) was added oxalyl chloride (28 $\mu$L, 0.33 mmol) and anhydrous DMF (1 drop) at room temperature under argon. After 1.5 h, the reaction mixture was concentrated *in vacuo* and redissolved in anhydrous THF (2.0 mL). To this solution was added DMAP (spatula tip), *i*-$PrNEt_2$ (40 $\mu$L, 0.23 mmol) and N-(3,5-dimethyl-1-phenyl-1H-pyrazol-4-yl)-2-hydroxyacetamide (40 mg, 0.16 mmol) at room temperature under argon. After 18 h, the solution was quenched with 200 $\mu$L of MeOH and diluted with EtOAc (20 mL). After washing with aqueous $NH_4Cl$ (5 mL), sat. $NaHCO_3$ (5 mL), and brine (5 mL) the solution was dried ($MgSO_4$), filtered, and concentrated *in vacuo.* Purification by column chromatography (3% MeOH in $CH_2Cl_2$) gave 31 mg (44%) of the title compound.

**TLC:** $R_f$ (5% MeOH in DCM): 0.30

**$^1$H NMR** (400 MHz, $CDCl_3$) $\delta$: 7.33-7.50 (m, 6H), 7.22 (ddd, *J* = 8.0, 1.0, 0.8 Hz, 1 H), 7.12 (ddd, *J*= 8.0, 7.1, 1.1 Hz, 1H), 7.02 (ddd, *J* = 7.9, 7.1, 1.1 Hz, 1H), 6.12 (bs, 1H), 4.96 (s, 2H), 4.67 (s, 2H), 2.38 (bd, *J* = 0.6 Hz, 3H), 2.20

(dq, $J$ = 1.4, 0.7 Hz, 3H), 1.98 (s, 3H), 1.94 (s, 3H) ppm

**$^{13}$C NMR** (100 MHz, $CDCl_3$) δ: 167.3, 165.5, 145.2, 139.8, 136.4, 135.3, 132.2, 129.1, 129.0, 127.5, 124.7, 121.7, 120.0, 118.6, 114.6, 108.6, 107.9, 63.3, 45.3, 11.1, 10.5, 10.1, 8.8 ppm

**HRMS** (ESI) m/z: $(M+H)^+$ calcd for $C_{25}H_{27}N_4O_3$: 431.2078; found: 431.2082

*Example Compound 16*

*2-((3,5-dimethyl-1-phenyl-1H-pyrazol-4-yl)amino)-2-oxoethyl 2-(2-(trifluoromethyl)-1H-indol-1-yl)acetate*

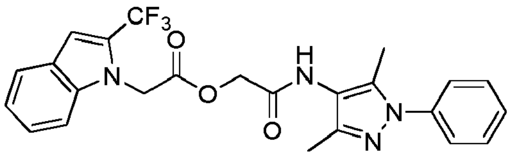

Step 1: ethyl 2-(2-(trifluoromethyl)-1 H-indol-1-yl)acetate

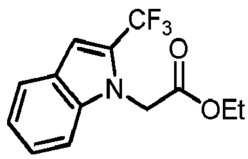

To a solution of 2-(trifluoromethyl)-1 H-indole (250 mg, 1.35 mmol) in anhydrous DMF (1.5 mL) was added NaH (60 mg, 1.49 mmol) at 0 °C under argon. The mixture was allowed to warm to room temperature, and after 30 min, was cooled back to 0 °C. To the resulting solution was added ethyl bromoacetate (0.18 mL, 1.6 mmol) and the mixture was warmed to room temperature. After 4 h, the reaction was quenched with aqueous $NH_4Cl$ (30 mL) and diluted with EtOAc (100 mL). The two layers were separated and the organic layer was washed with $H_2O$ (30 mL), aqueous $NaHCO_3$ (70 mL), and brine (30 mL). The organic layer was dried ($MgSO_4$), filtered, and concentrated *in vacuo.* Purification by column chromatography (5% EtOAc in petroleum ether) gave 350 mg (96%) of the title compound.

Step 2: 2-(2-(trifluoromethyl)-1 H-indol-1-yl)acetic acid

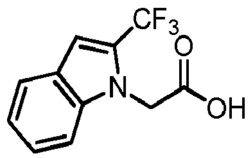

To a solution of ethyl 2-(2-(trifluoromethyl)-1H-indol-1-yl)acetate (340 mg, 1.25 mmol) in THF (5.0 mL) was added a solution of LiOH (1.5 mL, 1.5 mmol, 1.0 M in $H_2O$). After 45 min, the reaction mixture was diluted with EtOAc (100 mL), $H_2O$ (75 mL), and 1 M HCl (10 mL). The two layers were separated and the aqueous layer was extracted with EtOAc (2 x 25 mL). The combined organics were washed with brine (30 mL), dried ($MgSO_4$), filtered, and concentrated *in vacuo* to give the title compound.

Step 3: 2-((3,5-dimethyl-1-phenyl-1H-pyrazol-4-yl)amino)-2-oxoethyl 2-(2-(trifluoromethyl)-1 H-indol-1-yl)acetate

To a solution of 2-(2-Trifluoromethylindolyl)-acetic acid (51.5 mg, 0.21 mmol) in anhydrous $CH_2Cl_2$ (2.0 mL) was added oxalylchloride (36 µL, 0.42 mmol) and 2 drops of anhydrous DMF at 0 °C under argon. After 1.5 h at room temperature, the solution was evaporated *in vacuo* and redissolved in anhydrous $CH_2Cl_2$ (2.0 mL) at room remperature under argon. To this solution was added N-(3,5-dimethyl-1-phenyl-1 H-pyrazol-4-yl)-2-hydroxyacetamide (44 mg, 0.18 mmol) and anhydrous pyridine (17 µL, 0.21 mmol). After 18 h, the solution was quenched with aqueous $NaHCO_3$ (10

mL) and diluted with $CH_2Cl_2$ (15 mL). The two layers were separated and the organic layer was extracted with $CH_2Cl_2$ (2 x 10 mL). The combined organics were dried ($MgSO_4$), filtered, and concentrated *in vacuo.* Purification by column chromatography (3% MeOH in $CH_2Cl_2$) gave 39 mg (46%) of the title compound.

**TLC:** $R_f$ (3% MeOH in DCM): 0.15

**$^1$H NMR** (400 MHz, $CDCl_3$) δ: 7.67 (d, *J* = 8.0 Hz, 1 H), 7.47 - 7.32 (m, 8H), 7.19 (ddd, *J* = 8.0, 6.0, 2.0 Hz, 1 H), 7.07 - 7.02 (m, 1 H), 5.14 (s, 2H), 4.71 (s, 2H), 2.04 (s, 3H), 2.02 (s, 3H) ppm

**$^{13}$C NMR** (101 MHz, $CDCl_3$) δ: 166.4, 165.2, 145.1, 139.6, 138.5, 135.5, 129.1, 127.6, 126.8 (q, $J_{C-F}$ = 37.5 Hz), 125.8, 125.6, 123.7, 122.8, 121.8, 121.2 ($J_{C-F}$ = 268.9 Hz), 114.5, 109.4, 106.4 (q, $J_{C-F}$ = 4.0 Hz), 63.7, 45.8 (q, $J_{C-F}$ = 1.5 Hz), 11.0, 10.6 ppm

**HRMS** (ESI) m/z: $(M+H)^+$ calcd for $C_{24}H_{22}F_3N_4O_3$: 471.1639; found: 471.1647

*Example Compound 17*

*2-((3,5-dimethyl-1-phenyl-1H-pyrazol-4-yl)amino)-2-oxoethyl 2-(3,5-dimethyl-1H-pyrazol-1-yl)acetate*

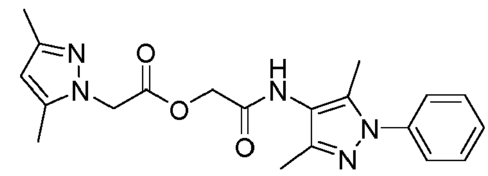

Step 1: methyl 2-(3,5-dimethyl-1 H-pyrazol-1-yl)acetate

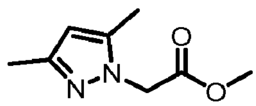

3,5-dimethylpyrazole (201.4 mg, 2.0 mmol) and $K_2CO_3$ (868.8 mg, 6.3 mmol) were suspended in dry DMF (2.1 mL) and after 30 min, methy 2-bromoacetate (397 μL, 4.2 mmol) was added. After 3 h, the reaction mixture was diluted with water and extracted 3 times with EtOAc. The organic layer was additionally washed with brine, dried ($MgSO_4$), filtered and concentrated. The title compound (241 mg, 68%) was obtained by column chromatography (45% EtOAc in PE).

Step 2: 2-(3,5-dimethyl-1H-pyrazol-1-yl)acetic acid

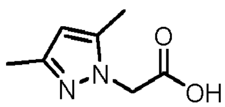

The ester from step 1 (226.3 mg, 1.3 mmol) was dissolved in THF (4 mL) and cooled to 0 °C, then 1 N aqueous lithium hydroxide (1.48 mL, 1.5 mmol) was added and the reaction was allowed to warm to room temperature. After 2.5 h, 1 M HCl was used to quench the reaction and the solution was concentrated to afford the title compound in quantitative yield.

Step 3: 2-((3,5-dimethyl-1-phenyl-1 H-pyrazol-4-yl)amino)-2-oxoethyl 2-(3,5-dimethyl-1H-pyrazol-1-yl)acetate

The acid from step 2 (31.8 mg, 0.2 mmol) was dissolved in DCM (2 mL) at 0 °C under Argon atmosphere. Oxalylchloride (25 μL, 0.3 mmol) and 2 drops of dry DMF was added and the mixture was stirred for 1.5 h at room temperature. The solution was evaporated *in vacuo* and redissolved in THF (2 mL). N-(3,5-dimethyl-1-phenyl-1H-pyrazol-4-yl)-2-hydroxyacetamide (30 mg, 122 μmol), ), synthesized as reported in Example 1, DMAP (0.1 eq) and $Et_3N$ (30 μL, 220 μmol, 1.8 eq) were added and the mixture was stirred overnight. The solution was quenched with $NH_4Cl$ solution (semi-saturated) and diluted with EtOAc. The organic phase was dried with $MgSO_4$ and the solvent was evaporated. Purification by column chromatography (3% → 5% MeOH in DCM) yielded 20 mg (43%) of the title compound.

**TLC:** $R_f$ (5% MeOH in DCM): 0.30

**$^1$H NMR** (400 MHz, $CDCl_3$) δ: 8.27 (bs, 1 H), 7.40-7.50 (m, 4H), 7.32-7.39 (m, 1 H), 5.33 (bs, 1 H), 4.87 (s, 4H), 2.29 (d, *J*= 0.6 Hz, 3H), 2.19 (s, 3H), 2.19 (s,3H), 2.02 (s, 3H) ppm

**$^{13}$C NMR** (101 MHz, $CDCl_3$) δ: 166.4, 166.2, 149.4, 145.5, 140.6, 139.8, 135.5, 129.2, 127.4, 123.7, 115.3, 106.0, 63.4, 50.1, 13.1, 11.3, 10.9, 10.8 ppm

**HRMS** (ESI) m/z: $(M+H)^+$ calcd for $C_{22}H_{26}N_2O_4$: 382.1887; found: 382.1873

*Example Compound 18*

*2-((3,5-dimethyl-1-phenyl-1H-pyrazol-4-yl)amino)-2-oxoethyl 2-(1-methyl-1H-indol-3-yl)acetate*

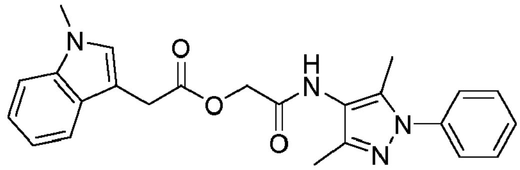

Step 1: 2-(1-methyl-1 H-indol-3-yl)acetic acid

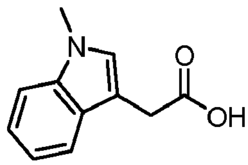

In an oven-dried flask under argon atmosphere, sodium hydride (228.2 mg, 5.7 mmol, 60% dispersion in mineral oil) was suspended in dry THF (15 mL) cooled to 0 °C and 3-indoleacetic acid (200.1 mg, 1.1 mmol) was added. After 30 minutes, no more bubbling was observed and iodomethane (235.0 μL, 3.8 mmol) was added slowly to the suspension, which was allowed to warm to room temperature and left stirring overnight. The crude mixture was cooled to 0 °C and quenched with MeOH, observing a clear solution afterwards. Water was added and diethyl ether was used to wash the aqueous phase which was then acidified to pH ~0 and extracted 3 times with DCM. The DCM phase was dried ($MgSO_4$), filtered and concentrated The title compound (157 mg, 73%) was obatine after purification by column chromatography (20% EtOAc in PE).

Step 2: 2-((3,5-dimethyl-1-phenyl-1 H-pyrazol-4-yl)amino)-2-oxoethyl 2-(1-methyl-1H-indol-3-yl)acetate

To a stirring solution of the acid from step 1 (39 mg, 0.2 mmol) in DCM (1 mL), DCC (40.8 mg, 0.2 mmol) was added slowly at 0 °C, then DMAP (2.7 mg, 0.02 mmol) and finally N-(3,5-dimethyl-1-phenyl-1H-pyrazol-4-yl)-2-hydroxyacetamide (50.1 mg, 0.2 mmol), synthesized as reported in Example 1. Additional acid (5 mg) was added after 5 h. The suspension was stirred overnight and then filtered to remove the DCU precipitate and washed with $NH_4Cl$ solution (semi-saturated) and brine. The product was purified twice by column chromatography (60% EtOAc in PE, and then 5%MeOH in DCM) to yield 80 mg (94%) of the title compound.

**TLC:** $R_f$ (5% MeOH in DCM): 0.30

**$^1$H NMR** (400 MHz, $CD_2Cl_2$) δ: 7.64 (d, *J* = 7.8 Hz; 1H), 7.44-7.50 (m, 2H), 7.34-7.42 (m, 3H), 7.29 (d, *J* = 8.2 Hz, 1H), 7.19 (t, *J*= 8.0 Hz, 1H), 7.11 (bs, 1H), 7.08 (t, *J* = 7.7 Hz, 1H), 6.76 (bs, 1 H), 4.70 (s, 2H), 3.93 (s, 2H), 3.73 (s, 3H), 1.96 (s, 3H), 1.94 (s, 3H) ppm

**$^{13}$C NMR** (100 MHz, $CD_2Cl_2$) δ: 170.5, 166.4, 145.8, 140.0, 137.2, 135.3, 129.1, 128.2, 127.4, 127.4, 124.5, 122.1, 119.6, 118.5, 115.2, 109.7, 106.2, 63.2, 32.7, 31.3, 10.9, 10.4 ppm

**HRMS** (ESI) m/z: $(M+H)^+$ calcd for $C_{24}H_{25}N_4O_3$: 417.1921; found: 417.1922

*Example Compound 19*

*2-((1-(4-((4-hydroxybutyl)amino)phenyl)-3,5-dimethyl-1H-pyrazol-4-yl)amino)-2-oxoethyl 2-(2-methyl-1H-indol-1-yl)acetate*

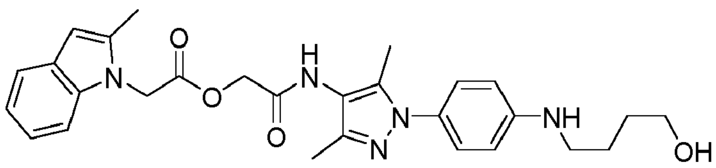

To a solution of 2-((3,5-dimethyl-1-(4-nitrophenyl)-1H-pyrazol-4-yl)amino)-2-oxoethyl 2-(2-methyl-1H-indol-1-yl)acetate from example 10 (10.0 mg, 0.022 mmol) in EtOAc/THF (1:1, 1.0 mL) was added a spatula tip of 10% Pd/C and the mixture was stirred under $H_2$ (1 atm). After 18 h, the mixture was filtered through celite, washed with EtOAc, and concentrated *in vacuo*. Purification by column chromatography (3% MeOH in $CH_2Cl_2$) gave 6 mg (54%) of the title compound.

**TLC:** $R_f$ (5% MeOH in DCM): 0.15

$^1$**H NMR** (400 MHz, MeOD) δ: 7.42 (d, *J* = 7.6 Hz, 1H), 7.28 (d, *J* = 8.0 Hz , 1H), 7.08-7.14 (m, 2H), 7.06 (t, *J* = 8.0 Hz, 1H), 6.98 (t, 1H, *J* = 7.6 Hz), 6.65-6.73 (m, 2H), 6.24 (bs, 1H), 5.13 (s, 2H), 4.82 (s, 2H), 3.60 (t, 2H, *J* = 6.0 Hz), 3.14 (t, 2H, *J* = 6.6 Hz), 2.41 (bs, 3H), 2.09 (s, 3H), 2.00 (s, 3H), 1.61-1.75 (m. 4H) ppm

$^{13}$**C NMR** (101 MHz, DMSO-$d_6$) δ: 168.9, 165.8, 148.4, 143.2, 137.1, 137.1, 134.1, 128.1, 127.7, 125.5, 120.3, 119.3, 119.1, 115.3, 111.5, 109.2, 100.1, 63.0, 60.5, 44.0, 42.8, 30.2, 25.3, 12.1, 11.3, 10.3 ppm

**HRMS** (ESI) m/z: $(M+H)^+$ calcd for $C_{28}H_{34}N_5O_4$: 504.2605; found: 504.2611

*Example Compound 20*

*2-((3,5-dimethyl-1-(p-tolyl)-1H-pyrazol-4-yl)amino)-2-oxoethyl 2-(2-(trifluoromethyl)-1H-indol-1-yl)acetate*

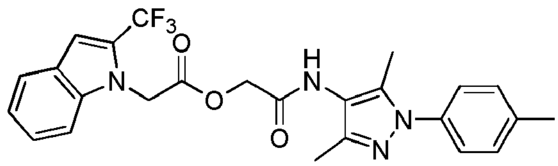

2-(2-(trifluoromethyl)-1 H-indol-1-yl)acetic acid (example 16, step 2, 30 mg, 0.1 mmol) was dissolved in DCM (2 mL) under Argon, cooled to 0 °C and added DCC (19.9 mg, 0.1 mmol) and DMAP (1.3 mg, 0.01 mmol) followed by N N-(3,5-dimethyl-1-(p-tolyl)-1 H-pyrazol-4-yl)-2-hydroxyacetamide (25.4 mg, 0.1 mmol), synthesized as reported in Example 9. The mixture was left stirring overnight and then diluted with water and extracted 3 times with DCM. The organic phase was washed with brine, dried over $MgSO_4$, filtered and concentrated. Purification by column chromatography was needed twice (5% MeOH in DCM and then 75% EtOAc in PE) to yield the title compound.

**TLC:** $R_f$ (5% MeOH in DCM): 0.31

$^1$**H NMR** (400 MHz, DMSO-$d_6$) δ: 9.36 (s, 1 H), 7.76 - 7.70 (m, 1 H), 7.66 - 7.61 (m, 1 H), 7.41 - 7.34 (m, 3H), 7.33 - 7.27 (m, 2H), 7.24 - 7.19 (m, 2H), 5.39 (s, 2H), 4.79 (s, 2H), 2.36 (s, 3H), 2.09 (s, 3H), 2.02 (s, 3H).

$^{13}$**C NMR** (101 MHz, DMSO-$d_6$) δ: 167.8, 165.7, 144.4, 138.6, 137.2, 136.5, 134.3, 129.6, 125.6 (q, $J_{C-F}$ = 38.3 Hz), 125.1, 124.9, 123.7, 122.2, 121.2, 121.2 (q, $J_{C-F}$ = 268.6 Hz), 116.3, 111.0, 105.8 (q, $J_{C-F}$ = 4.0 Hz), 63.2, 45.3, 20.6, 11.2, 10.5.

**HRMS** (ESI) m/z: $(M+H)^+$ calcd for $C_{25}H_{24}F_3N_4O_3$: 485.1795; found: 485.1799

(q, $J_{C-F}$ = 38.3 Hz)

*Example Compound 21*

*2-((3,5-dimethyl-1-(4-nitrophenyl)-1H-pyrazol-4-yl)amino)-2-oxoethyl 2-(2-(trifluoromethyl)-1H-indol-1-yl)acetate*

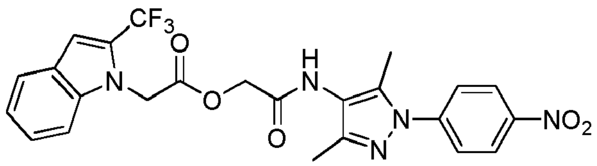

To a solution of 2-(2-(trifluoromethyl)-1 H-indol-1-yl)acetic acid (example 16, step 2, 46 mg, 0.16 mmol) in anhydrous $CH_2Cl_2$ (2.0 mL) was added oxalyl chloride (22 μL, 0.26 mmol) and one drop of anhydrous DMF at room temperature under argon. After 1 h, the reaction mixture was concentrated *in vacuo* and redissolved in $CH_2Cl_2$ (2.0 mL). To this solution was added N-(3,5-dimethyl-1-(4-nitrophenyl)-1 H-pyrazol-4-yl)-2-hydroxyacetamide (example 10, step 3, 37 mg, 0.13 mmol), *i*-$PrNEt_2$ (45 μL, 0.26 mmol) and DMAP (spatula tip) at room temperature. After 18 h, the solution was quenched with aqueous $NaHCO_3$ (10 mL) and diluted with $CH_2Cl_2$ (15 mL). The two layers were separated and the organic layer was extracted with $CH_2Cl_2$ (2 x 10 mL). The combined organics were dried ($MgSO_4$), filtered, and concentrated *in vacuo.* Purification by column chromatography (3% MeOH in $CH_2Cl_2$) gave 16 mg (24%) of the title compound.

**TLC:** $R_f$ (5% MeOH in DCM): 0.54

**$^1$H NMR** (600 MHz, $CDCl_3$/MeOD 1:1) δ: 8.29-8.34 (m, 2H), 7.62-7.67 (m, 3H), 7.38 (dd, 1H, *J* = 8.4, 0.9 Hz), 7.33 (ddd, 1H, *J* = 7.9, 6.7, 0.8 Hz), 7.17 (ddd, 1H, *J* = 7.9, 6.8, 0.7 Hz), 7.00 (bs, 1H), 5.19 (s, 2H), 4.78 (s, 2H), 2.18 (s, 3H), 2.12 (s, 3H) ppm

**$^{13}$C NMR** (125 MHz, $CDCl_3$/MeOD 1:1) δ: 167.8, 167.1, 147.6, 146.6, 144.8, 138.9, 136.6, 127.0 (q, $J_{C-F}$ = 37.3 Hz), 126.1, 125.5, 125.1, 124.3, 122.7, 121.8, 121.6 (q, $J_{C-F}$ = 268 Hz), 117.5, 110.0, 106.3 (q, $J_{C-F}$ = 4.0 Hz), 63.6, 45.6, 11.3, 11.1 ppm

**$^{19}$F NMR** (565 MHz, $CDCl_3$/MeOD 1:1) δ: 62.36.

**HRMS** (ESI) m/z: $(M+H)^+$ calcd for $C_{24}H_{21}F_3N_5O_5$: 516.1489; found: 516.1489

*Example Compound 22*

*2-((1-(4-aminophenyl)-3,5-dimethyl-1H-pyrazol-4-yl)amino)-2-oxoethyl 2-(2-methyl-1H-indol-1-yl)acetate*

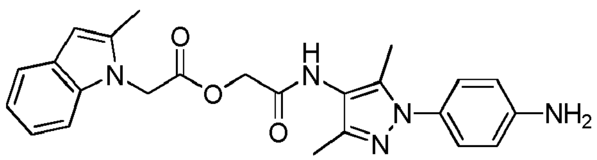

A suspension of 2-((3,5-dimethyl-1-(4-nitrophenyl)-1H-pyrazol-4-yl)amino)-2-oxoethyl 2-(2-methyl-1 H-indol-1-yl)acetate (2 mg, 0.004 mmol) and 10% Pd/C (spatula tip) in EtOH (0.5 mL) was stirred under $H_2$ (1 atm) for 18 h. The mixture was concentrated *in vacuo* and purified by column chromatography (3% MeOH in $CH_2Cl_2$) to give the title compound.

**TLC:** $R_f$ (5% MeOH in DCM): 0.18 **$^1$H NMR** (400 MHz, DMSO-$d_6$) δ: 9.27 (s, 1 H), 7.40 (ddd, *J* = 17.1, 7.8, 0.8 Hz, 2H), 7.08 - 7.00 (m, 3H), 7.01 - 6.95 (m, 1 H), 6.66 - 6.58 (m, 2H), 6.27 - 6.21 (m, 1 H), 5.30 (s, 2H), 5.20 (s, 2H), 4.77 (s, 2H), 2.36 (d, *J* = 1.0 Hz, 3H), 2.00 (s, 3H), 2.00 (s, 3H) ppm

**$^{13}$C NMR** (101 MHz, DMSO-$d_6$) δ: 168.9, 165.8, 148.2, 143.2, 137.1, 134.1, 128.4, 127.7, 125.4, 120.3, 119.3, 119.1, 115.3, 113.5, 109.2, 100.1, 63.1, 44.1, 29.1, 12.1, 11.2, 10.2 ppm

**HRMS** (ESI) m/z: $(M+H)^+$ calcd for $C_{24}H_{26}N_5O_3$: 432.2030; found: 432.2032

*Example Compound 23*

*2-((1-(2-aminophenyl)-3,5-dimethyl-1H-pyrazol-4-yl)amino)-2-oxoethyl 2-(2-methyl-1H-indol-1-yl)acetate*

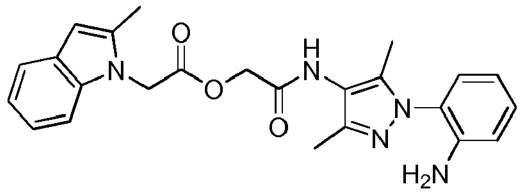

A suspension of 2-((3,5-dimethyl-1-(2-nitrophenyl)-1 H-pyrazol-4-yl)amino)-2-oxoethyl 2-(2-methyl-1 H-indol-1-yl)acetate (2.9 mg, 0.006 mmol) and 10% Pd/C (spatula tip) in EtOH (0.5 mL) was stirred under $H_2$ (1 atm) for 18 h. The mixture was concentrated *in vacuo* and purified by column chromatography (3% MeOH in $CH_2Cl_2$) to give 2.3 mg (85%) the title compound.

**TLC:** $R_f$ (5% MeOH in DCM): 0.29

$^1$**H NMR** (400 MHz, DMSO-$d_6$) δ: 9.33 (s, 1 H), 7.48 - 7.34 (m, 2H), 7.18 - 7.10 (m, 1 H), 7.07 - 6.96 (m, 3H), 6.91 - 6.80 (m, 1 H), 6.71 - 6.58 (m, 1 H), 6.24 (s, 1 H), 5.21 (s, 2H), 4.90 (s, 2H), 4.78 (s, 2H), 2.37 (s, 3H), 2.04 (s, 3H), 1.92 (s, 3H) ppm

$^{13}$**C NMR** (101 MHz, DMSO-$d_6$) δ: 168.9, 165.8, 144.4, 144.3, 137.2, 137.1, 135.3, 129.2, 127.7, 127.2, 124.2, 120.3, 119.3, 119.2, 116.1, 116.1, 115.3, 109.3, 100.1, 63.0, 44.1, 12.2, 11.5, 9.8 ppm

**HRMS** (ESI) m/z: $(M+H)^+$ calcd for $C_{24}H_{26}N_5O_3$: 432.2030; found: 432.2034

*Example Compound 24*

*2-oxo-2-(phenylamino)ethyl 2-(2-methyl-1 H-indol-1-yl)acetate*

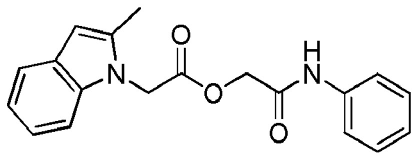

To a solution of 2-hydroxy-N-phenylacetamide (29 mg, 0.19 mmol) and 2-(2-methyl-1H-indol-1-yl)acetic acid (36 mg, 0.19 mmol) in anhydrous $CH_2Cl_2$ (5.0 mL) was added DCC (38 mg, 0.19 mmol) and DMAP (5 mg, 0.04 mmol) at room temperature under argon. After 4 h, the reaction mixture was filtered, the filter cake was washed with $CH_2Cl_2$ (20 mL), the combined organics were washed with aqueous $NH_4Cl$ (10 mL), dried ($MgSO_4$), filtered, and concentrated *in vacuo.* Purification by column chromatography (1% MeOH in $CH_2Cl_2$) gave 53.7 mg (88%) of the title compound.

**TLC:** $R_f$ (30% EtOAc in PE): 0.51

$^1$**H NMR** (400 MHz, DMSO-d6) δ: 10.10 (bs, 1H), 7.48-7.54 (m, 2H), 7.36-7.44 (m, 2H), 7.28-7.35 (m, 2H), 7.06-7.10 (m, 1H), 7.05 (ddd, 1H, $J_{4\text{-}5}$ = 8.1, 7.2, 1.3 Hz), 6.98 (ddd, 1H, $J$ = 7.7, 7.1, 1.2 Hz), 6.24 (t, 1H, $J$ = 0.9 Hz), 5.20 (s, 2H), 4.77 (s, 2H), 2.39 (d, 3H, $J$ = 0.8 Hz).

$^{13}$**C NMR** (100 MHz, DMSO-*d6*) δ: 168.9, 164.9, 138.3, 137.1, 128.8, 127.7, 123.6, 120.3, 119.3, 119.3, 109.2, 100.1, 63.0, 44.0, 12.1, 11.3, 10.6.

**HRMS** (ESI) m/z: $(M+H)^+$ calcd for $C_{19}H_{19}N_2O_3$: 323.1390; found: 323.1391

*Example Compound 25*

*2-((3,5-dimethyl-1-phenyl-1H-pyrazol-4-yl)amino)-2-oxoethyl 2-(4-fluoro-1H-indol-1-yl)acetate*

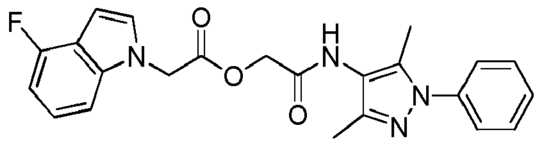

Step 1: ethyl 2-(4-fluoro-1H-indol-1-yl)acetate

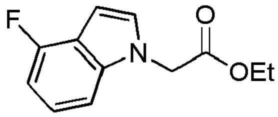

To a suspension of NaH (76 mg, 1.90 mmol, 60% in oil) in anhydrous THF (2.0 mL) was added a solution of 4-fluoroindole (225 mg, 1.67 mmol) in anhydrous DMF (3 mL + 2 mL rinse) dropwise via cannula at 0 °C under argon. The reaction mixture was allowed to warm to room temperature, and after 45 min, was cooled to 0 °C at which time ethyl bromoacetate (0.22 mL, 2.0 mmol) was added. The reaction was warmed to room temperature, and after 5 h, the reaction was quenched with aqueous $NH_4Cl$ (30 mL) and diluted with EtOAc (20 mL). The two layers were separated and the aqueous layer was extracted with EtOAc (2 x 10 mL). The combined organics were washed with brine (10 mL), dried ($MgSO_4$), filtered, and concentrated *in vacuo.* Purification by column chromatography (20% EtOAc in petroleum ether) gave 349 mg (94%) of the title compound.

Step 2: 2-(4-fluoro-1H-indol-1-yl)acetic acid

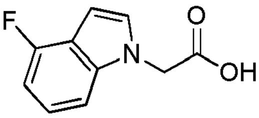

To a solution of ethyl 2-(4-fluoro-1 H-indol-1-yl)acetate (155 mg, 0.70 mmol) in THF was added a solution of NaOH (1.05 mL, 2.1 mmol, 2M in $H_2O$) at room temperature. After 5 h, the reaction was diluted with $Et_2O$ (25 mL) and $H_2O$ (25 mL). The two layers were separated and the aqueous layer was extracted with $Et_2O$ (10 mL). The aqueous phase was acidified with 3M HCl and extracted with $CH_2Cl_2$ (2 x 20 mL). The combined dichloromethane extracts were dried ($MgSO_4$), filtered, and concentrated *in vacuo* to give 125 mg (93%) of the title compound.

Step 3: 2-((3,5-dimethyl-1-phenyl-1 H-pyrazol-4-yl)amino)-2-oxoethyl 2-(4-fluoro-1 H-indol-1-yl)acetate

From 2-(4-fluoro-1 H-indol-1-yl)acetic acid (37 mg, 0.19 mmol) and N-(3,5-dimethyl-1-phenyl-1 H-pyrazol-4-yl)-2-hydroxyacetamide (47 mg, 0.19 mmol), 75 mg (94%) of the title compound was prepared analogously as described in example 1, step 5.

**TLC:** $R_f$ (5% MeOH in DCM): 0.36

**$^1$H NMR** (400 MHz, DMSO-d6) δ: 9.36 (s, 1 H), 7.54 - 7.47 (m, 4H), 7.44 - 7.35 (m, 2H), 7.34 - 7.27 (m, 1H), 7.11 (td, *J* = 8.0, 5.2 Hz, 1 H), 6.83 (ddd, *J* = 10.8, 7.8, 0.7 Hz, 1 H), 6.55 (dd, *J* = 3.3, 0.9 Hz, 1 H), 5.33 (s, 2H), 4.80 (s, 2H), 2.14 (s, 3H), 2.05 (s, 3H) ppm

**$^{13}$C NMR** (101 MHz, DMSO-d6) δ: 168.6, 165.9, 155.5 (d, $J_{C-F}$ = 244.1 Hz), 144.8, 139.6, 139.1 (d, $J_{C-F}$ = 11.7 Hz), 134.5, 130.0, 129.2, 127.1, 123.8, 121.9 (d, $J_{C-F}$ = 7.8 Hz), 116.7 (d, $J_{C-F}$ = 22.5 Hz), 116.6, 106.7 (d, $J_{C-F}$ = 3.4 Hz), 104.1 (d, $J_{C-F}$ = 18.4 Hz), 96.9, 63.1, 47.2, 11.3, 10.6 ppm

**HRMS** (ESI) m/z: $(M+H)^+$ calcd for $C_{23}H_{22}FN_4O_3$: 421.1671; found: 421.1674

*Example Compound 26*

*2-oxo-2-(o-tolylamino)ethyl 2-(2-methyl-1H-indol-1-yl)acetate*

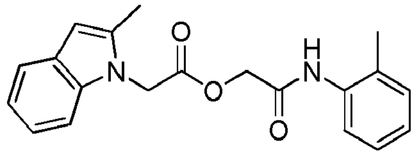

Step 1: 2-hydroxy-N-(o-tolyl)acetamide

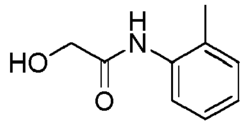

To a solution of 2-methylaniline (0.13 mL, 1.2 mmol) in $Et_2O$ (15 mL) was added acetoxyacetyl chloride (0.13 mL, 1.2 mmol) and a solution of NaOH (0.60 mL, 1.2 mmol, 2M in $H_2O$) at room temperature. After 30 min, an additional 1.2 mL of the NaOH solution was added. After 3 h, the reaction mixture was diluted with $Et_2O$ (35 mL) and 1 M aqueous $K_2CO_3$ (15 mL). The two layers were separated and the organic layer was washed with 1 M aqueous $K_2CO_3$ (2 x 10 mL). The combined aqueous washes were extracted with $Et_2O$ (20 mL) and the combined organics were dried ($MgSO_4$), filtered, and concentrated *in vacuo.* Purification by column chromatography (30→50% EtOAc in petroleum ether) to give 95 mg (50%) of the title compound.

Step 2: 2-oxo-2-(o-tolylamino)ethyl 2-(2-methyl-1H-indol-1-yl)acetate

From 2-hydroxy-N-(o-tolyl)acetamide (31 mg, 0.19 mmol) and 2-(2-methyl-1H-indol-1-yl)acetic acid (36 mg, 0.19 mmol), 46 mg (72%) of the title compound was prepared analogously as described in example 1, step 5.

**TLC:** $R_f$ (30% EtOAc in PE): 0.21 **$^1$H NMR** (400 MHz, DMSO-$d_6$) δ: 9.51 (s, 1 H), 7.49 - 7.31 (m, 3H), 7.29 - 7.08 (m, 3H), 7.09 - 6.94 (m, 2H), 6.26 - 6.23 (m, 1 H), 5.20 (s, 2H), 4.81 (s, 2H), 2.37 (d, $J$ = 1.0 Hz, 3H), 2.16 (s, 3H) ppm

**$^{13}$C NMR** (101 MHz, DMSO-$d_6$) δ: 168.88, 165.19, 137.13, 137.08, 135.38, 132.22, 130.36, 127.68, 126.05, 125.67, 125.28, 120.28, 119.27, 119.14, 109.22, 100.09, 63.04, 44.03, 17.71, 12.12 ppm

**HRMS** (ESI) m/z: $(M+H)^+$ calcd for $C_{20}H_{21}N_2O_3$: 337.1547; found: 337.1551

*Example Compound 27*

*2-((3,5-dimethyl-1-phenyl-1H-pyrazol-4-yl)amino)-2-oxoethyl 2-(6-chloro-1H-indol-1-yl)acetate*

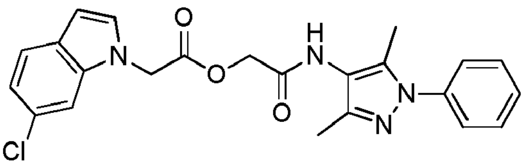

Step 1: ethyl 2-(6-chloro-1 H-indol-1-yl)acetate

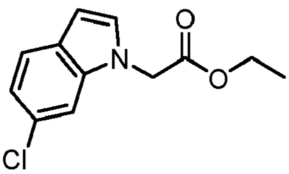

Sodium hydride (73.0 mg, 1.8 mmol, 60% dispersion in mineral oil) was suspended in a mixture of dry THF (2 mL) and dry DMF (5 mL) under Argon and cooled to 0 °C before adding 6-chloroindole (250.0 mg, 1.7 mmol). The stirring mixture was allowed to warm to room temperature and after 30 minutes it was cooled again to 0 °C before adding ethyl 2-bromoacetate (220.0 μL, 2.0 mmol) and letting it stir for 3 h. Then the mixture was quenched with $NH_4Cl$ (saturated solution), diluted with water and extracted with toluene 3 times. The organic layer was washed again with water, dried over $MgSO_4$, filtered and concentrated to give 375.0 mg of pure title compound (96%) after column chromatography purification (10% EtOAc in PE).

Step 2: 2-(6-chloro-1 H-indol-1-yl)acetic acid

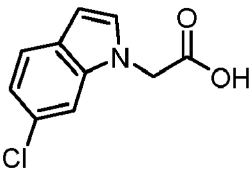

To a stirring solution of the ester from step 1 (150.6 mg, 0.6 mmol) in THF (3 mL), 2 M aqueous sodium hydroxide (950.0 μL, 1.9 mmol) was added dropwise. The reaction was stirred overnight and then the crude was diluted with water and washed with basified diethyl ether. The aqueous phase was acidified with 1 M HCl and extracted 3 times with DCM, dried over $MgSO_4$, filtered and concentrated. Yield: 124.5 mg (94%)

Step 3: 2-((3,5-dimethyl-1-phenyl-1H-pyrazol-4-yl)amino)-2-oxoethyl 2-(6-chloro-1H-indol-1-yl)acetate

The acid from step 2 (39.9 mg, 0.2 mmol) was dissolved in dry DCM (4.8 mL) under argon, and added DCC (38.0 mg, 0.2 mmol) and DMAP (5.2 mg, 0.04 mmol) at 0 °C, followed by N-(3,5-dimethyl-1-phenyl-1H-pyrazol-4-yl)-2-hydroxyacetamide (48.2 mg, 0.2 mmol), synthesized as reported in Example 1. The mixture was left stirring overnight. The suspension was filtered over cotton to remove the bulk of DCU and purified twice by column chromatography (75% EtOAc in hexane) to yield 71.0 mg of the title compound (85%).

**TLC:** $R_f$ (75% EtOAc in PE): 0.35

**$^1$H NMR** (400 MHz, DMSO-$d_6$) δ: 9.32 (s, 1 H), 7.60 - 7.56 (m, 1 H), 7.54 (d, *J* = 8.4 Hz, 1 H), 7.52 - 7.45 (m, 4H), 7.40 - 7.33 (m, 2H), 7.03 (dd, *J* = 8.4, 1.9 Hz, 1 H), 6.49 (dd, *J* = 3.2, 0.9 Hz, 1 H), 5.29 (s, 2H), 4.77 (s, 2H), 2.11 (s, 3H), 2.03 (s, 3H ppm

**$^{13}$C NMR** (101 MHz, DMSO-$d_6$) δ: 168.7, 166.0, 144.8, 139.6, 136.9, 134.5, 130.7, 129.2, 127.1, 126.9, 126.2, 123.8, 121.7, 119.6, 116.6, 110.0, 101.6, 63.1, 47.0, 11.3, 10.6 ppm **HRMS** (ESI) m/z: $(M+H)^+$ calcd for $C_{23}H_{22}ClN_4O_3$: 437.1375; found: 437.1380

*Example Compound 28*

*2-((3,5-dimethyl-1-phenyl-1H-pyrazol-4-yl)amino)-2-oxoethyl 2-(5-chloro-1H-indol-1-yl)acetate*

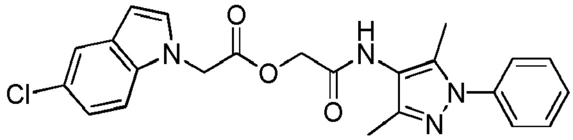

Step 1: ethyl 2-(5-chloro-1 H-indol-1-yl)acetate

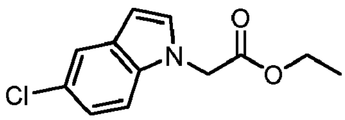

Sodium hydride (73.2 mg, 1.8 mmol, 60% dispersion in mineral oil) was suspended in a mixture of dry THF (2 mL) and dry DMF (5 mL) under Argon and cooled to 0 °C before adding 5-chloroindole (251.6 mg, 1.7 mmol). The stirring mixture was allowed to warm to room temperature and after 45 minutes it was cooled again to 0 °C before adding ethyl 2-bromoacetate (220.0 μL, 2.0 mmol) and letting it stir for 2 h. Then the mixture was quenched with $NH_4Cl$ (saturated solution), diluted with water and extracted with toluene 3 times. The organic layer was washed again with water, dried over $MgSO_4$, filtered and concentrated to give 192 mg of pure title compound (49%) after column chromatography purification (40% MTBE in PE).

Step 2: 2-(5-chloro-1H-indol-1-yl)acetic acid

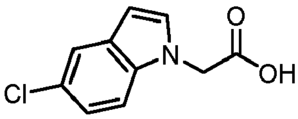

To a stirring solution of the ester from step 1 (208.9 mg, 0.9 mmol) in THF (3 mL), 2 M aqueous sodium hydroxide (1.3 mL, 2.6 mmol) was added dropwise. The reaction was stirred for 5h, after which the crude was diluted with water and washed with basified diethyl ether. The aqueous phase was acidified with 1 M HCl and extracted 3 times with DCM, dried over $MgSO_4$, filtered and concentrated. Yield: 148 mg (80%)

Step 3: 2-((3,5-dimethyl-1-phenyl-1H-pyrazol-4-yl)amino)-2-oxoethyl 2-(5-chloro-1H-indol-1-yl)acetate

The acid from step 2 (40.6 mg, 0.2 mmol) was dissolved in dry DCM (4.4 mL) under argon, and added DCC (37.9 mg, 0.2 mmol) and DMAP (5.2 mg, 0.04 mmol) at 0 °C, followed by N-(3,5-dimethyl-1-phenyl-1H-pyrazol-4-yl)-2-hydroxyacetamide (48.3 mg, 0.2 mmol), synthesized as reported in Example 1. The mixture was left stirring overnight. The suspension was filtered over cotton to remove the bulk of DCU and purified by column chromatography (75% EtOAc in hexane) to yield 70 mg of the title compound (82%).

**TLC:** $R_f$ (75% EtOAc in PE): 0.26

**$^1$H NMR** (400 MHz, DMSO-$d_6$) δ: 9.32 (s, 1H), 7.59 (dd, *J* = 2.1, 0.5 Hz, 1H), 7.51 - 7.44 (m, 5H), 7.41 (d, *J* = 3.2 Hz, 1 H), 7.40 - 7.33 (m, 1 H), 7.11 (dd, *J* = 8.7, 2.1 Hz, 1 H), 6.45 (dd, *J* = 3.2, 0.8 Hz, 1 H), 5.29 (s, 2H), 4.76 (s, 2H), 2.11 (s, 3H), 2.02 (s, 3H) ppm

**$^{13}$C NMR** (101 MHz, DMSO-$d_6$) δ: 168.6, 165.9, 144.8, 139.6, 134.9, 134.4, 131.3, 129.2, 129.1, 127.0, 124.1, 123.8, 121.2, 119.5, 116.6, 111.6, 101.1, 63.1, 47.0, 11.3, 10.6 ppm

**HRMS** (ESI) m/z: $(M+H)^+$ calcd for $C_{23}H_{22}ClN_4O_3$: 437.1375; found: 437.1379

*Example Compound 29*

*2-((3,5-dimethyl-1-phenyl-1H-pyrazol-4-yl)amino)-2-oxoethyl 2-(5-fluoro-1H-indol-1-yl)acetate*

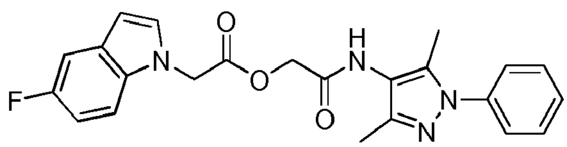

Step 1: ethyl 2-(5-fluoro-1 H-indol-1-yl)acetate

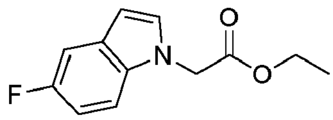

Sodium hydride (75.0 mg, 1.9 mmol, 60% dispersion in mineral oil) was suspended in a mixture of dry THF (2 mL) and dry DMF (5 mL) under Argon and cooled to 0 °C before adding 5-fluoroindole (225.6 mg, 1.7 mmol). The stirring mixture was allowed to warm to room temperature and after 45 minutes it was cooled again to 0 °C before adding ethyl 2-bromoacetate (220.0 μL, 2.0 mmol) and letting it stir for 2 h. Then the mixture was quenched with $NH_4Cl$ (saturated solution), diluted with water and extracted with toluene 3 times. The organic layer was washed again with water, dried over $MgSO_4$, filtered and concentrated to give 334 mg of pure title compound (91%) after column chromatography purification (20% EtOAc in PE).

Step 2: 2-(5-fluoro-1 H-indol-1-yl)acetic acid

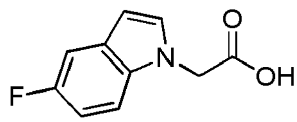

To a stirring solution of the ester from step 1 (154.8 mg, 0.7 mmol) in THF (3 mL), 2 M aqueous sodium hydroxide (1.1 mL, 2.1 mmol) was added dropwise. After 5 h, the reaction mixture was diluted with $H_2O$ (25 mL) and $Et_2O$ (25 mL). The two layers were separated, the aqueous layer was acidified with 3 M HCl, and extracted with $Et_2O$ (3 x 25 mL). The combined organics were dried ($MgSO_4$), filtered, and concentrated *in vacuo* to give 126 mg (93%) of the title compound.

Step 3: 2-((3,5-dimethyl-1-phenyl-1 H-pyrazol-4-yl)amino)-2-oxoethyl 2-(5-fluoro-1 H-indol-1-yl)acetate

The acid from step 2 (39.7 mg, 0.2 mmol) was dissolved in dry DCM (5 mL) under argon, and added DCC (42.3 mg, 0.2 mmol) and DMAP (5.1 mg, 0.04 mmol) at 0 °C, followed by N-(3,5-dimethyl-1-phenyl-1 H-pyrazol-4-yl)-2-hydroxyacetamide (52.8 mg, 0.2 mmol), synthesized as reported in Example 1, step. The mixture was left stirring overnight. The suspension was filtered over cotton to remove the bulk of DCU and purified by column chromatography (75% EtOAc in hexane) to yield 75 mg of the title compound (87%).

**TLC:** $R_f$ (75% EtOAc in PE): 0.30

**$^1$H NMR** (400 MHz, DMSO-$d_6$) δ: 9.36 (s, 1H), 7.54 - 7.47 (m, 4H), 7.48 - 7.36 (m, 3H), 7.32 (dd, $J$ = 9.8, 2.5 Hz, 1 H), 6.98 (td, $J$ = 9.2, 2.6 Hz, 1 H), 6.47 (dd, $J$ = 3.1, 0.8 Hz, 1 H), 5.30 (s, 2H), 4.79 (s, 2H), 2.14 (s, 3H), 2.05 (s, 3H) ppm

**$^{13}$C NMR** (101 MHz, DMSO-$d_6$) δ 168.7, 165.9, 144.8, 139.6, 134.5, 133.1, 131.4, 129.2, 128.37 (d, $J_{CF}$ = 10.5 Hz), 127.1, 123.8, 116.6, 111.0 (d, $J_{CF}$ = 97. Hz), 109.3 (d, $J_{CF}$ = 25.9 Hz), 105.0 (d, $J_{CF}$ = 23.2 Hz), 101.4 (d, $J_{CF}$ = 4.6 Hz), 63.1, 47.1, 11.3, 10.6 ppm

**HRMS** (ESI) m/z: $(M+H)^+$ calcd for $C_{23}H_{22}FN_4O_3$: 421.1671; found: 421.1676

*Example Compound 30*

*2-((3,5-dimethyl-1-phenyl-1H-pyrazol-4-yl)amino)-2-oxoethyl 2-(5-chloro-2-methyl-1H-indol-1-yl)acetate*

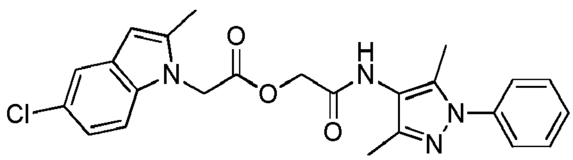

Step 1: ethyl 2-(5-chloro-2-methyl-1H-indol-1-yl)acetate

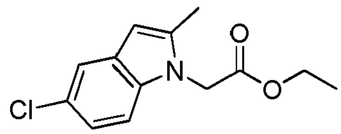

From 5-chloro-2-methylindole (273 mg, 1.65 mmol) was prepared 370 mg (89%) of the title compound analogously to example 29, step 1.

Step 2: 2-(5-chloro-2-methyl-1H-indol-1-yl)acetic acid

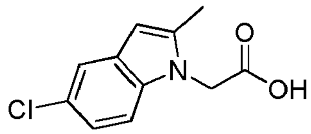

From the ester of step 1 (150 mg, 0.59 mmol) was prepared 124 mg (93%) of the title compound analogously to example 29, step 2.

Step 3: 2-((3,5-dimethyl-1-phenyl-1 H-pyrazol-4-yl)amino)-2-oxoethyl 2-(5-chloro-2-methyl-1 H-indol-1-yl)acetate

From the acid of step 2 (40.8 mg, 0.18 mmol) was prepared 70 mg (85%) of the title compound analogously to example 1, step 5.
**TLC:** $R_f$ (5% MeOH in DCM): 0.33
**$^1$H NMR** (400 MHz, DMSO-$d_6$) δ 9.37 (s, 1 H), 7.54-7.36 (m, 7H), 7.05 (dd, $J$ = 8.7, 2.1 Hz, 1 H), 6.26 (s,, 1 H), 5.24 (s, 2H), 4.79 (s, 2H), 2.36 (s, 3H), 2.13 (s, 3H), 2.04 (s, 3H) ppm
**$^{13}$C NMR** (101 MHz, DMSO-$d_6$) δ 168.7, 165.9, 144.7, 139.6, 139.2, 135.6, 134.4, 129.1, 128.9, 127.0, 123.9, 123.8, 120.1, 118.3, 116.6, 110.8, 99.9, 63.1, 44.2, 12.2, 11.3, 10.6 ppm
**HRMS** (ESI) m/z: $(M+H)^+$ calcd for $C_{24}H_{24}ClN_4O_3$: 451.1531; found: 451.1537

*Example Compound 31*

*2-((3,5-dimethyl-1-phenyl-1H-pyrazol-4-yl)amino)-2-oxoethyl 2-(7-chloro-1H-indol-1-yl)acetate*

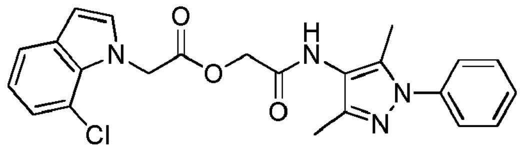

Step 1: ethyl 2-(7-chloro-1H-indol-1-yl)acetate

From 7-chloroindole (125 mg, 0.82 mmol) was prepared 178 mg (91%) of the title compound analogously to example 29, step 1.

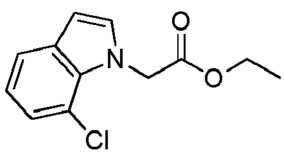

Step 2: 2-(7-chloro-1H-indol-1-yl)acetic acid

From the ester of step 1 (136 mg, 0.57 mmol) was prepared 120 mg (quantitative) of the title compound analogously to example 29, step 2.

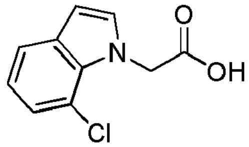

Step 3: 2-((3,5-dimethyl-1-phenyl-1H-pyrazol-4-yl)amino)-2-oxoethyl 2-(7-chloro-1H-indol-1-yl)acetate

From the acid of step 2 (40.0 mg, 0.19 mmol) was prepared 46 mg (55%) of the title compound analogously to example 1, step 5.

**TLC:** $R_f$ (5% MeOH in DCM): 0.33

**$^1$H NMR** (400 MHz, DMSO-$d_6$) $\delta$ 9.36 (s, 1 H), 7.55 (dd, *J* = 7.9, 1.0 Hz, 1 H), 7.52 - 7.48 (m, 4H), 7.42 (d, *J* = 3.2 Hz, 1 H), 7.41 - 7.35 (m, 1 H), 7.14 (dd, *J* = 7.6, 1.0 Hz, 1 H), 7.02 (t, *J* = 7.8 Hz, 1 H), 6.56 (d, *J* = 3.2 Hz, 1 H), 5.53 (s, 2H), 4.80 (s, 2H), 2.14 (s, 3H), 2.05 (s, 3H) ppm

**$^{13}$C NMR** (101 MHz, DMSO-$d_6$) $\delta$ 169.2, 165.7, 144.7, 139.6, 134.4, 132.5, 131.6, 131.1, 129.1, 127.0, 123.7, 122.8, 120.5, 119.9, 116.7, 115.7, 102.0, 63.0, 49.6, 11.3, 10.6 ppm

**HRMS** (ESI) m/z: $(M+H)^+$ calcd for $C_{23}H_{22}ClN_4O_3$: 437.1375; found: 437.1375

*Example Compound 32*

*2-((3,5-dimethyl-1-phenyl-1H-pyrazol-4-yl)amino)-2-oxoethyl 2-(2-isopropyl-1H-indol-1-yl)acetate*

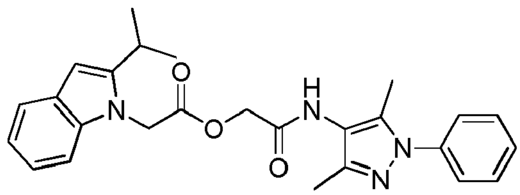

Step 1: 2-isopropyl-1 H-indole

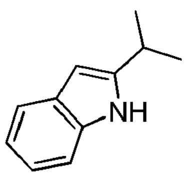

A mixture of phenylhydrazine (0.428 mL, 4.0 mmol) and 3-methyl-2-butanone (0.425 mL, 4.0 mmol) was heated to 70 °C. After 4 h, $H_2O$ (0.3 mL) and concentrated $H_2SO_4$ (1.3 mL) was added and this mixture was heated at 70 °C. After 14 h, the reaction mixture was quenched into 5% $Na_2CO_3$ (100 mL) and diluted with $Et_2O$ (100 mL). The two layers were separated and the aqueous layer was extracted with $Et_2O$ (100 mL). The combined organic layers were washed with brine (50 mL), dried ($MgSO_4$), filtered, and concentrated *in vacuo.* Purification by column chromatography (5% EtOAc in PE) gave 192 mg (30%) of the title compound.

Step 2: ethyl 2-(2-isopropyl-1 H-indol-1-yl)acetate

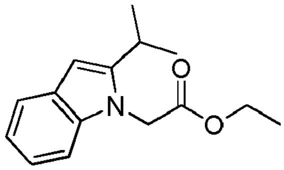

From the indole of step 1 (189 mg, 1.19 mmol) was prepared the title compound analogously to example 29, step 1. This compound was used without purification.

Step 3: 2-(2-isopropyl-1 H-indol-1-yl)acetic acid

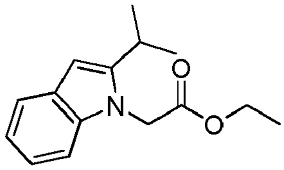

From the ester of step 2 (entire lot) was prepared 201 mg (78% over two steps) of the title compound analogously to example 20, step 2.

Step 4: 2-((3,5-dimethyl-1-phenyl-1H-pyrazol-4-yl)amino)-2-oxoethyl 2-(2-isopropyl-1 H-indol-1-yl)acetate

From the acid of step 3 (60 mg, 0.28 mmol) was prepared the title compound analogously to example 1, step 5.

**TLC:** $R_f$ (5% MeOH in DCM): 0.37

**$^1$H NMR** (400 MHz, DMSO-$d_6$) $\delta$ 9.35 (s, 1 H), 7.54 - 7.33 (m, 7H), 7.07 - 6.97 (m, 2H), 6.27 (t, $J$ = 0.8 Hz, 1 H), 5.22 (s, 2H), 4.79 (s, 2H), 3.07 (sept, $J$ = 6.8 Hz, 1 H), 2.13 (s, 3H), 2.05 (s, 3H), 1.26 (d, $J$ = 6.8 Hz, 6H) ppm

**$^{13}$C NMR** (101 MHz, DMSO-$d_6$) $\delta$ 168.9, 165.9, 147.5, 144.7, 139.6, 137.0, 134.4, 129.1, 127.6, 127.0, 123.7, 120.4, 119.4, 119.3, 116.6, 109.4, 96.8, 63.0, 44.0, 24.8, 22.8, 11.2, 10.5 ppm

**HRMS** (ESI) m/z: $(M+H)^+$ calcd for $C_{26}H_{29}N_4O_3$: 445.2234; found: 445.2238

*Example Compound 33*

*2-((3,5-dimethyl-1-phenyl-1H-pyrazol-4-yl)amino)-2-oxoethyl 2-(7-chloro-2-methyl-1H-indol-1-yl)acetate*

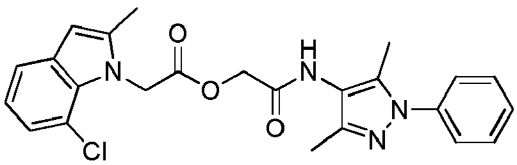

Step 1: 7-chloro-2-methyl-1 H-indole

To a solution of 2-chloronitrobenzene (403 mg, 2.56 mmol) in anhydrous THF (5.0 mL) was added a solution of isopropylmagesium bromide (15.2 mL, 7.6 mmol, 0.5 M in THF) at -40 °C under argon. After 20 min, the reaction was quenched with aqueous $NH_4Cl$ (50 mL) and diluted with $Et_2O$ (50 mL). The two layers were separated and the aqueous layer was extracted with $Et_2O$ (2 x 30 mL). The combined organics were dried ($MgSO_4$), filtered, and concentrated *in vacuo.* Purification by column chromatography (5→10% EtOAc in PE) gave 176 mg (42%) of the title compound.

Step 2: ethyl 2-(7-chloro-2-methyl-1H-indol-1-yl)acetate

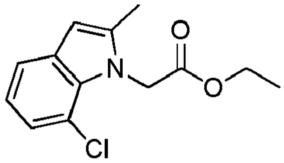

From the indole of step 1 (240 mg, 1.45 mmol) was prepared the title compound analogously to example 29, step 1. This compound was used without purification.

Step 3: 2-(7-chloro-2-methyl-1H-indol-1-yl)acetic acid

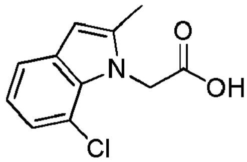

From the ester of step 2 (entire lot) was prepared 224 mg (69% over 2 steps) of the title compound analogously to example 29, step 2.

Step 4:

From the acid of step 3 (47 mg, 0.21 mmol) was prepared the title compound analogously to example 1, step 5.
**TLC:** $R_f$ (5% MeOH in DCM): 0.35
**$^1$H NMR** (400 MHz, DMSO-$d_6$) δ 9.37 (s, 1 H), 7.54 - 7.48 (m, 4), 7.42 (dd, *J* = 7.7, 1.1 Hz, 1 H), 7.41 - 7.34 (m, 1 H), 7.06 (dd, *J* = 7.7, 1.1 Hz, 1 H), 6.97 (t, *J* = 7.7 Hz, 1 H), 6.36 (q, *J* = 1.0 Hz, 1 H), 5.48 (s, 2H), 4.81 (s, 2H), 2.37 (d, *J* = 1.0 Hz, 3H), 2.14 (s, 3H), 2.05 (s, 3H) ppm
**$^{13}$C NMR** (101 MHz, DMSO-$d_6$) δ 169.0, 165.7, 144.6, 139.6, 139.3, 134.3, 131.6, 131.0, 129.1, 127.0, 123.7, 122.1, 120.3, 118.6, 116.6, 114.9, 101.3, 63.1, 46.0, 12.3, 11.2, 10.5 ppm
**HRMS** (ESI) m/z: $(M+H)^+$ calcd for $C_{24}H_{24}ClN_4O_3$: 451.1531 found: 451.1551

*Example Compound 34*

*2-((3,5-dimethyl-1-phenyl-1H-pyrazol-4-yl)amino)-2-oxoethyl 2-(2-methyl-1H-indol-1-yl)propanoate*

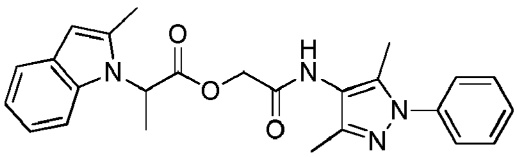

Step 1: ethyl 2-(2-methyl-1 H-indol-1-yl)propanoate

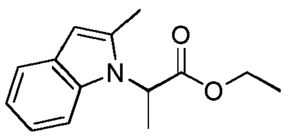

From 2-methylindole (101 mg, 0.78 mmol) and ethyl 2-bromopropionate (119 μL, 0.91 mmol) was prepared

the title compound analogously to example 29, step 1. The material could not be completely purified and was used "as is" in the next step.

Step 2: 2-(2-methyl-1 H-indol-1-yl)propanoic acid

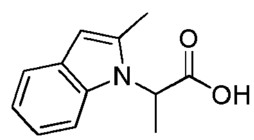

From the ester of step 1 (entire lot) was prepared 81 mg (51% over 2 steps) of the title compound analogously to example 29, step 2.

Step 3: 2-((3,5-dimethyl-1-phenyl-1 H-pyrazol-4-yl)amino)-2-oxoethyl 2-(2-methyl-1 H-indol-1-yl)propanoate

From the acid of step 2 (72 mg, 0.35 mmol) was prepared 19 mg (12%) of the title compound analogously to example 1, step 5.
**TLC:** $R_f$ (5% MeOH in DCM): 0.35
**$^1$H NMR** (400 MHz, DMSO-$d_6$) δ 9.26 (s, 1H), 7.54 - 7.47 (m, 4H), 7.44 - 7.36 (m, 2H), 7.36 - 7.31 (m, 1 H), 7.05 - 6.94 (m, 2H), 6.24 (bm, 1 H), 5.61 (q, *J* = 7.2 Hz, 1 H), 4.81 (d, *J* = 14.5 Hz, 1H), 4.71 (d, *J* = 14.5 Hz, 1H), 2.42 (d, *J* = 1.0 Hz, 3H), 2.13 (s, 3H), 2.05 (s, 3H), 1.70 (d, *J* = 7.2 Hz, 3H) ppm
**$^{13}$C NMR** (101 MHz, DMSO-$d_6$) δ 170.6, 165.7, 144.6, 139.6, 136.6, 135.7, 134.3, 129.1, 128.0, 127.0, 123.7, 120.2, 119.3, 119.1, 116.6, 110.1, 100.8, 63.2, 51.6, 16.5, 13.0, 11.2, 10.5 ppm
**HRMS** (ESI) m/z: $(M+H)^+$ calcd for $C_{25}H_{27}N_4O_3$: 431.2078; found: 431.2080

*Example Compound 35*

*2-((4-morpholinophenyl)amino)-2-oxoethyl 2-(2-methyl-1 H-indol-1-yl)acetate*

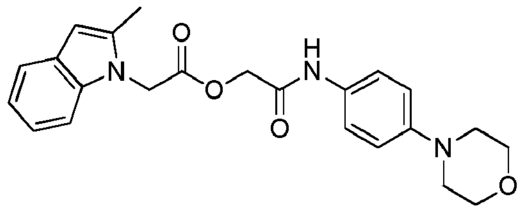

(2-Methyl-indol-1-yl)-acetic acid (61.0 mg, 0.3 mmol) was dissolved in DCM (3 mL), and added DCC (53.0 mg, 0.3 mmol) and a catalytic amount of DMAP followed by N-(3,5-dimethyl-1-phenyl-1 H-pyrazol-4-yl)-2-hydroxyacetamide (49.0 mg, 0.3 mmol), synthesized as reported in Example 1. The mixture was left stirring overnight. The suspension was filtered over cotton to remove the bulk of DCU and purified by column chromatography (gradient of EtOAc in PE) to yield 60.0 mg of the title compound (57%).
**TLC:** $R_f$ (40% EtOAc in PE): 0.08
**$^1$H NMR** (400 MHz, DMSO-$d_6$) δ: 9.88 (s, 1 H), 7.45 - 7.37 (m, 4H), 7.05 (ddd, *J* = 8.2, 7.1, 1.3 Hz, 1H), 6.98 (ddd, *J* = 7.6, 7.1, 1.1 Hz, 1H), 6.94 - 6.87 (m, 2H), 6.28 - 6.20 (m, 1H), 5.19 (s, 2H), 4.72 (s, 2H), 3.76 - 3.69 (m, 4H), 3.07 - 3.02 (m, 4H), 2.38 (d, *J* = 1.0 Hz, 3H) ppm
**$^{13}$C NMR** (101 MHz, DMSO-$d_6$) δ: 168.9, 164.3, 147.5, 137.1, 130.4, 27.7, 120.5, 120.3, 119.3, 119.1, 115.4, 115.4, 109.2, 100.1, 66.1, 63.1, 48.8, 44.0, 12.1 ppm
**HRMS** (ESI) m/z: $(M+H)^+$ calcd for $C_{23}H_{26}N_3O_4$: 408.1918; found: 408.1920

*Example Compound 36*

*2-((3,5-dimethyl-1-phenyl-1H-pyrazol-4-yl)amino)-2-oxoethyl 2-(2-ethyl-1H-indol-1-yl)acetate*

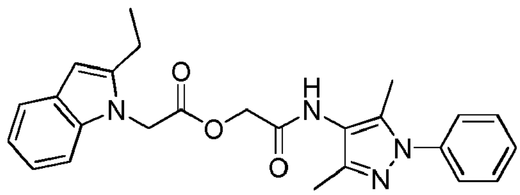

Step 1: ethyl 2-(2-ethyl-1 H-indol-1-yl)acetate

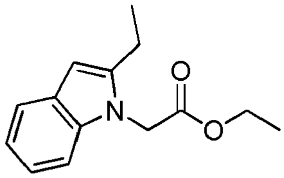

Sodium hydride (42.0 mg, 1.0 mmol, 60% dispersion in mineral oil) was suspended in a mixture of dry THF (2 mL) and dry DMF (3 mL) under Argon and cooled to 0 °C before adding 2-ethylindole (130.7 mg, 0.9 mmol, international patent application WO2006/082245). The stirring mixture was allowed to warm to room temperature and after 25 minutes it was cooled again to 0 °C before adding ethyl 2-bromoacetate (120 μL, 1.1 mmol) and letting it stir overnight. The day after it was heated to 60 °C for 4 h to go to completion, and then it was diluted with water and extracted with toluene 3 times. The organic layer was dried over $MgSO_4$, filtered and concentrated to give 163.5 mg of the title compound (78%) after column chromatography purification (10% EtOAc in PE).

Step 2: 2-(2-ethyl-1 H-indol-1-yl)acetic acid

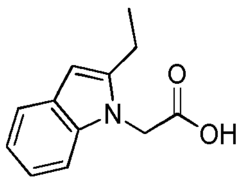

To a stirring solution of the ester from step 1 (163.5 mg, 0.7 mmol) in THF (3 mL), 2 M aqueous sodium hydroxide (1.1 mL, 2.1 mmol) was added dropwise. The reaction was stirred for 4h, after which the crude was diluted with water and extracted with diethyl ether. The aqueous phase was acidified with 3 M HCl and extracted 3 times with DCM, dried over $MgSO_4$, filtered and concentrated. Yield: 111.1 mg (77%)

Step 3: 2-((3,5-dimethyl-1-phenyl-1H-pyrazol-4-yl)amino)-2-oxoethyl 2-(2-ethyl-1H-indol-1-yl)acetate

The acid from step 2 (37.0 mg, 0.2 mmol) was dissolved in dry DCM (4 mL) under argon, and added DCC (38.3 mg, 0.2 mmol) and DMAP (5 mg, 0.04 mmol) at 0 °C, followed by N-(3,5-dimethyl-1-phenyl-1 H-pyrazol-4-yl)-2-hydroxyacetamide (45.5 mg, 0.2 mmol), synthesized as reported in Example 1. The mixture was left stirring overnight. The suspension was filtered over cotton to remove the bulk of DCU and purified by column chromatography (75% EtOAc in PE) to yield 56.0 mg of the title compound (71%).

**TLC:** $R_f$ (5% MeOH in DCM): 0.20

**$^1$H NMR** (400 MHz, DMSO-$d_6$) δ: 9.37 (s, 1 H), 7.50 (d, $J$ = 3.8 Hz, 4H), 7.47 - 7.44 (m, 1 H), 7.43 - 7.35 (m, 2H), 7.05 (ddd, $J$ = 8.2, 7.1, 1.3 Hz, 1 H), 6.99 (ddd, $J$ = 8.0, 7.1, 1.1 Hz, 1 H), 6.25 (d, $J$ = 0.9 Hz, 1H), 5.21 (s, 2H), 4.79 (s, 2H), 2.71 (dd, $J$ = 7.5, 1.1 Hz, 2H), 2.13 (s, 3H), 2.05 (s, 3H), 1.27 (t, $J$ = 7.4 Hz, 3H) ppm

**$^{13}$C NMR** (101 MHz, DMSO-$d_6$) δ: 168.9, 165.9, 144.7, 142.9, 139.6, 137.1, 134.4, 129.2, 127.6, 127.0, 123.8, 120.4, 119.3, 119.3, 116.6, 109.2, 98.2, 63.0, 43.9, 18.9, 12.2, 11.3, 10.6 ppm
**HRMS** (ESI) m/z: $(M+H)^+$ calcd for $C_{25}H_{27}N_4O_3$: 431.2078; found: 431.2080

*Example Compound 37*

*1-((3,5-dimethyl-1-phenyl-1H-pyrazol-4-yl)amino)-1-oxopropan-2-yl 2-(2-methyl-1H-indol-1-yl)acetate*

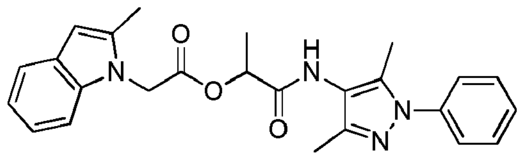

Step 1: N-(3,5-dimethyl-1-phenyl-1 H-pyrazol-4-yl)-2-hydroxypropanamide

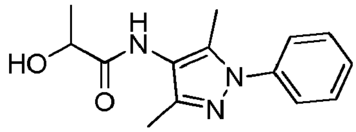

To a solution of racemic 2-hydroxypropanoic acid (123 μL, 1.6 mmol) in dry DCM (10 mL) uder argon, cooled to 0 °C, DCC (338 mg, 1.6 mmol) was added slowly, and then 3,5-dimethyl-4-nitro-1-phenyl-1 H-pyrazole (154.2 mg, 0.8 mmol), synthesized as reported in Example 1, step 2. The mixture was left stirring over the weekend. Then the suspension was filtered over cotton to remove the bulk of DCU and purified by column chromatography (5% MeOH in DCM) to yield 68.7 mg of the title compound (32%).

Step 2: 1-((3,5-dimethyl-1-phenyl-1H-pyrazol-4-yl)amino)-1-oxopropan-2-yl 2-(2-methyl-1H-indol-1-yl)acetate

(2-Methyl-indol-1-yl)-acetic acid (21.2 mg, 0.1 mmol) was dissolved in DCM (4 mL), and added DCC (22.4 mg, 0.1 mmol) and a catalytic amount of DMAP followed by the alcohol from step 1 (28.2 mg, 0.1 mmol). The mixture was left stirring for 6 h and then it was filtered over cotton to remove the bulk of DCU and washed with $NH_4Cl$, dried over $MgSO_4$, filtered and concentrated. Purification by column chromatography (75% EtOAc in PE) yielded 25.5 mg of pure title compound (54%).
**TLC:** $R_f$ (5% MeOH in DCM): 0.26
**$^1$H NMR** (400 MHz, DMSO-$d_6$) δ: 9.36 (s, 1 H), 7.50 (d, $J$ = 5.5 Hz, 4H), 7.45 - 7.33 (m, 3H), 7.06 - 6.95 (m, 2H), 6.27 - 6.21 (m, 1 H), 5.20 - 5.11 (m, 3H), 2.35 (d, $J$ = 1.0 Hz, 3H), 2.11 (s, 3H), 2.02 (s, 3H), 1.53 (d, $J$ = 6.8 Hz, 3H) ppm
**$^{13}$C NMR** (101 MHz, DMSO-$d_6$) δ: 169.3, 168.6, 144.7, 139.6, 137.1, 137.0, 134.4, 129.1, 127.6, 127.0, 123.7, 120.2, 119.2, 119.1, 116.6, 109.1, 100.0, 70.8, 44.0, 17.7, 12.1, 11.1, 10.4 ppm
**HRMS** (ESI) m/z: $(M+H)^+$ calcd for $C_{25}H_{27}N_4O_3$: 431.2078; found: 431.2080

*Example Compound 38*

*2-((3,5-dimethyl-1-phenyl-1H-pyrazol-4-yl)amino)-2-oxoethyl 2-(2-methyl-1H-indol-3-yl)acetate*

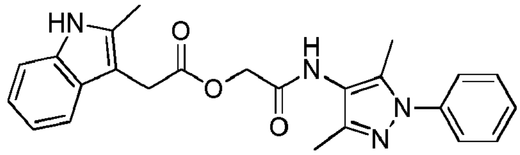

(2-Methyl-indol-3-yl)-acetic acid (70.9 mg, 0.4 mmol) was dissolved in DCM (6 mL), and added DCC (73.6 mg, 0.4 mmol) and DMAP (8.7 mg, 0.07 mmol) followed by N-(3,5-dimethyl-1-phenyl-1 H-pyrazol-4-yl)-2-hydroxyacetamide (91.2 mg, 0.4 mmol), synthesized as reported in Example 1. The mixture was left stirring for overnight and then filtered over cotton to remove the bulk of DCU and washed with $NH_4Cl$, dried over $MgSO_4$, filtered and concentrated. Purification by column chromatography (75% EtOAc in PE) yielded 139.4 mg of pure title compound (90%).

**TLC:** $R_f$ (5% MeOH in DCM): 0.24

**$^1$H NMR** (400 MHz, DMSO-$d_6$) δ: 10.83 (s, 1H), 9.24 (s, 1H), 7.48 - 7.45 (m, 4H), 7.42 - 7.32 (m, 2H), 7.23 - 7.18 (m, 1 H), 6.95 (ddd, *J* = 8.1, 7.0, 1.3 Hz, 1 H), 6.89 (ddd, *J* = 8.0, 7.1, 1.1 Hz, 1 H), 4.63 (s, 2H), 3.79 (s, 2H), 2.31 (s, 3H), 2.10 (s, 3H), 2.02 (s, 3H) ppm

**$^{13}$C NMR** (101 MHz, DMSO-$d_6$) δ: 171.2, 166.3, 144.7, 139.6, 135.0, 134.4, 133.4, 129.1, 128.2, 127.0, 123.7, 120.1, 118.3, 117.6, 116.8, 110.4, 102.9, 62.6, 29.2, 11.3, 10.6 ppm

**HRMS** (ESI) m/z: $(M+H)^+$ calcd for $C_{24}H_{25}N_4O_3$: 417.1921; found: 417.1931

*Example Compound 39*

*2-oxo-2-((1-phenyl-1H-pyrazol-4-yl)amino)ethyl 2-(2-methyl-1H-indol-3-yl)acetate*

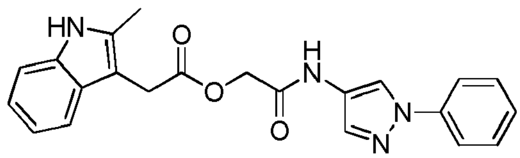

Step 1: 4-nitro-1-phenyl-1 H-pyrazole

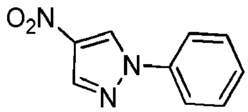

4-nitro-1 H-pyrazole (201.1 mg, 1.8 mmol), sodium hydroxide (71.2 g, 1.8 mmol), copper chloride (30.1 mg, 0.2 mmol), and finally phenylboronic acid (351.0 mg, 2.9 mmol) were added to a two-necked flask, letting air bubble into the solution with a siringe cannula. The mixture was heated to reflux overnight. The crude was concentrated and purified by column chromatography (10% EtOAc in PE) to yield 223 g (66%) of the product.

Step 2: 1-phenyl-1 H-pyrazol-4-amine

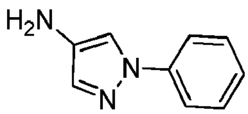

From the compound of step 1 (213.2 mg, 1.13 mmol) was prepared 227 mg (quantitative) of the title compound analogously to Example 1, step 2.

Step 3: 2-oxo-2-((1-phenyl-1 H-pyrazol-4-yl)amino)ethyl acetate

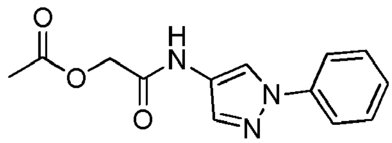

From the compound of step 2 (227 mg, 1.13 mmol) was prepared 191 mg (65%) of the title compound analogously to Example 1, step 3.

Step 4: 2-hydroxy-N-(1-phenyl-1 H-pyrazol-4-yl)acetamide

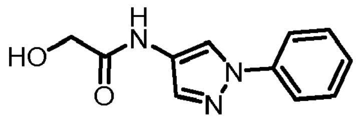

From the compound of step 3 (184 mg, 0.71 mmol) was prepared 149 mg (97%) of the title compound analogously to Example 1, step 4.

Step 5: 2-oxo-2-((1-phenyl-1 H-pyrazol-4-yl)amino)ethyl 2-(2-methyl-1 H-indol-3-yl)acetate

(2-Methyl-indol-3-yl)-acetic acid (70.3 mg, 0.4 mmol) was dissolved in DCM (6 mL), and added DCC (72.6 mg, 0.4 mmol) and DMAP (10.0 mg, 0.08 mmol) followed by N-(3,5-dimethyl-1-phenyl-1 H-pyrazol-4-yl)-2-hydroxyacetamide (80.0 mg, 0.4 mmol), synthesized as reported in Example 1. The mixture was left stirring for overnight and then filtered over cotton to remove the bulk of DCU and washed with $NH_4Cl$, dried over $MgSO_4$, filtered and concentrated. Purification by column chromatography (75% EtOAc in PE) yielded 79.4 mg of pure title compound (55%).
**TLC:** $R_f$ (5% MeOH in DCM): 0.31
**$^1$H NMR** (400 MHz, DMSO-$d_6$) δ: 10.88 (s, 1H), 10.29 (s, 1H), 8.52 (d, *J* = 0.7 Hz, 1H), 7.81 - 7.78 (m, 2H), 7.77 (d, *J* = 0.6 Hz, 1 H), 7.52 - 7.45 (m, 2H), 7.45 - 7.40 (m, 1 H), 7.32 - 7.23 (m, 2H), 7.00 (ddd, *J* = 8.0, 7.1, 1.3 Hz, 1 H), 6.94 (ddd, *J* = 7.6, 7.0, 1.2 Hz, 1 H), 4.66 (s, 2H), 3.82 (s, 2H), 2.36 (s, 3H).
**$^{13}$C NMR** (101 MHz, DMSO-$d_6$) δ: 171.2, 164.5, 139.6, 135.0, 133.5, 132.9, 129.6, 128.2, 126.1, 123.0, 120.2, 118.4, 118.0, 118.0, 117.5, 110.4, 102.9, 62.5, 29.3, 11.4 ppm
**HRMS** (ESI) m/z: $(M+H)^+$ calcd for $C_{22}H_{21}N_4O_3$: 389.1608; found: 389.1613

*Example Compound 40*

*1-((3,5-dimethyl-1-phenyl-1H-pyrazol-4-yl)carbamoyl)cyclopropyl 2-(2-methyl-1H-indol-1-yl)acetate*

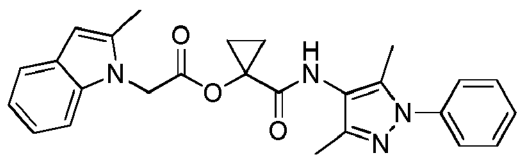

Step 1: N-(3,5-dimethyl-1-phenyl-1H-pyrazol-4-yl)-1-hydroxycyclopropanecarboxamide

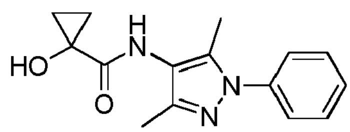

To a solution of 1-hydroxycyclopropanecarboxylic acid (30 mg, 0.29 mmol) and 3,5-dimethyl-1-phenyl-1 H-pyrazol-4-amine hydrochloride (66 mg, 0.29 mmol) in DMF (1.5 mL) was added EDCI (59 mg, 0.31 mmol), HOBT·$H_2O$ (45 mg, 0.29 mmol), and *i*-$Pr_2NEt$ (77 μL, 0.44 mmol) at room temperature. After 4 h, the reaction mixture was diluted with $H_2O$ and EtOAc. The two layers were separated and the aqueous layer was extracted with EtOAc (2 x). The combined organics were dried ($MgSO_4$), filtered, and concentrated *in vacuo.* The crude product was pure enough to be used "as is" in the next step.

Step 2: 1-((3,5-dimethyl-1-phenyl-1H-pyrazol-4-yl)carbamoyl)cyclopropyl 2-(2-methyl-1H-indol-1-yl)acetate

(2-Methyl-indol-1-yl)-acetic acid (56 mg, 0.29 mmol) was dissolved in DCM (6 mL), and added DCC (61 mg, 0.29 mmol) and DMAP (10.0 mg, 0.08 mmol) followed by the compound of step 1 (-0.29 mmol). The mixture was left stirring for overnight and then filtered over cotton to remove the bulk of DCU and washed with $NH_4Cl$, dried over $MgSO_4$, filtered and concentrated. Purification by column chromatography (75% EtOAc in PE) yielded the title compound.

**TLC:** $R_f$ (5% MeOH in DCM): 0.49

**$^1$H NMR** (400 MHz, DMSO-$d_6$) δ: 9.29 (s, 1 H), 7.57 - 7.46 (m, 4H), 7.47 - 7.36 (m, 3H), 7.07 - 6.96 (m, 2H), 6.25 (s, 1 H), 5.20 (s, 2H), 2.36 (s, 3H), 2.09 (s, 3H), 2.01 (s, 3H), 1.44 (t, *J* = 4.5 Hz, 2H), 1.22 (t, *J* = 4.1 Hz, 2H).

**$^{13}$C NMR** (101 MHz, DMSO-$d_6$) δ: 169.4, 168.6, 145.4, 139.6, 137.1, 137.0, 135.0, 129.2, 127.7, 127.0, 123.7, 120.3, 119.3, 119.1, 116.9, 109.3, 100.1, 58.7, 44.2, 14.3, 12.3, 11.1, 10.4.

**HRMS** (ESI) m/z: $(M+H)^+$ calcd for $C_{26}H_{27}N_4O_3$: 443.2078; found: 443.2087

*Example Compound 41*

*2-((3,5-dimethyl-1-phenyl-1H-pyrazol-4-yl)amino)-2-oxoethyl 2-(benzofuran-3-yl)acetate*

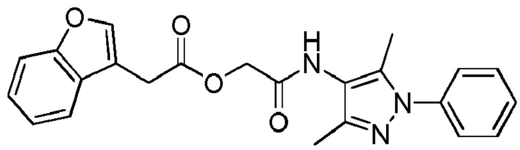

2-(benzofuran-3-yl)acetic acid (10.0 mg, 0.06 mmol) was dissolved under argon in dry DCM (1.5 mL), and added DCC (11.8 mg, 0.06mmol) and a catalytic amount of DMAP followed by N-(3,5-dimethyl-1-phenyl-1 H-pyrazol-4-yl)-2-hydroxyacetamide (14.0 mg, 0.06 mmol), synthesized as reported in Example 1. The mixture was left stirring for 2 h and then it was filtered over cotton to remove the bulk of DCU and washed with $NH_4Cl$, dried over $MgSO_4$, filtered and concentrated. Purification by column chromatography (75% EtOAc in PE) yielded 18.8 mg of the title compound (82%).

**TLC:** $R_f$ (5% MeOH in DCM): 0.35

**$^1$H NMR** (400 MHz, DMSO-$d_6$) δ: 9.37 (s, 1 H), 8.06 - 7.86 (m, 1 H), 7.67 - 7.64 (m, 1 H), 7.59 - 7.55 (m, 1 H), 7.50 (d, *J* = 5.0 Hz, 4H), 7.43 - 7.36 (m, 1 H), 7.32 (ddd, *J* = 8.3, 7.2, 1.4 Hz, 1 H), 7.28 - 7.23 (m, 1 H), 4.75 (s, 2H), 3.96 (d, *J* = 1.0 Hz, 2H), 2.14 (s, 3H), 2.06 (s, 3H) ppm

**$^{13}$C NMR** (101 MHz, DMSO-$d_6$) δ: 170.1, 166.2, 154.5, 144.7, 143.2, 139.6, 134.4, 129.1, 127.5, 127.0, 124.4, 123.7, 122.6, 120.2, 116.7, 113.3, 111.3, 62.8, 28.5, 11.3, 10.6 ppm

**HRMS** (ESI) m/z: $(M+H)^+$ calcd for $C_{23}H_{22}N_3NaO_4$: 404.1605; found: 404.1610

*Example Compound 42*

*2-((3,5-dimethyl-1-phenyl-1H-pyrazol-4-yl)amino)-2-oxoethyl 2-(naphthalen-1-yl)acetate*

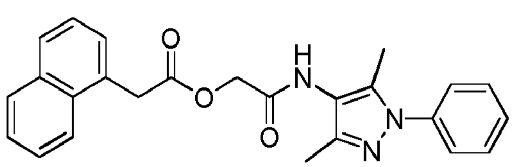

2-(1-naphthalenyl)acetic acid (34.3 mg, 0.2 mmol) was dissolved in dry DCM (3.6 mL), cooled to 0 °C and added DCC (36.4 mg, 0.2 mmol) and DMAP (4.5 mg, 0.04 mmol) followed by N-(3,5-dimethyl-1-phenyl-1 H-pyrazol-4-yl)-2-hydroxyacetamide (44.5 mg, 0.2 mmol), synthesized as reported in Example 1. The obtained suspension was filtered over cotton to remove the bulk of DCU and purified by column chromatography (75% EtOAc in PE) to yield 62.8 mg of the title compound (84%).

**TLC:** $R_f$ (5% MeOH in DCM): 0.20

**$^1$H NMR** (400 MHz, DMSO-$d_6$) δ: 9.34 (bs, 1H), 7.99-8.05 (m, 1H), 7.92-7.97 (m, 1H), 7.85-7.90 (m, 1H), 7.45-7.58 (m, 8H), 7.35-7.43 (m, 1H), 4.72 (s, 2H), 4.31 (s, 2H), 2.13 (s, 3H), 2.05 (s, 3H) ppm

**$^{13}$C NMR** (100 MHz, DMSO-$d_6$) δ: 171.0, 166.2, 144.8, 139.6, 134.4, 133.3, 131.8, 130.7, 129.2, 128.4, 128.2, 127.7, 127.0, 126.2, 125.8, 125.5, 124.1, 123.7, 116.7, 62.8, 37.6, 11.3, 10.6 ppm

**HRMS** (ESI) m/z: $(M+H)^+$ calcd for $C_{25}H_{24}N_3O_3$: 414.1812; found: 414.1816

*Example Compound 43*

*2-((3,5-dimethyl-1-phenyl-1H-pyrazol-4-yl)amino)-2-oxoethyl 2-phenylacetate*

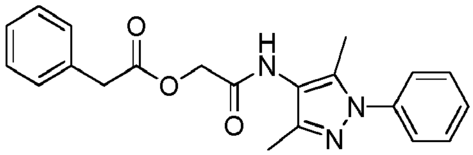

2-(phenyl)acetic acid (25.6 mg, 0.2 mmol) was dissolved in dry DCM (3.4 mL), cooled to 0 °C and added DCC (34.9 mg, 0.2 mmol) and DMAP (4.6 mg, 0.04 mmol) followed by N-(3,5-dimethyl-1-phenyl-1 H-pyrazol-4-yl)-2-hydroxyacetamide (41.4 mg, 0.2 mmol), synthesized as reported in Example 1. The obtained suspension was filtered over cotton to remove the bulk of DCU and purified by column chromatography (75% EtOAc in PE) to yield 57.7 mg of the title compound (94%)

**TLC:** $R_f$ (5% MeOH in DCM): 0.27

**$^1$H NMR** (400 MHz, DMSO-$d_6$) δ: 9.33 (bs, 1H), 7.47-7.54 (m, 4H), 7.35-7.42 (m, 1H), 7.29-7.36 (m, 4H), 7.23-7.30 (m, 1 H), 4.71 (s, 2H), 3.82 (s, 2H), 2.14 (s, 3H), 2.05 (s, 3H) ppm

**$^{13}$C NMR** (100 MHz, DMSO-$d_6$) δ: 170.9, 166.2, 144.7, 139.6, 134.4, 134.1, 129.4, 129.1, 128.3, 127.0, 126.8, 123.7, 116.7, 62.7, 39.9, 11.3, 10.6 ppm

**HRMS** (ESI) m/z: $(M+H)^+$ calcd for $C_{21}H_{22}N_3O_3$: 364.1656; found: 364.1658

*Example Compound 44*

*2-((3,5-dimethyl-1-phenyl-1H-pyrazol-4-yl)amino)-2-oxoethyl 2-(4-aminophenyl)acetate*

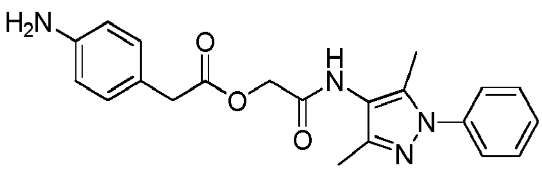

Step 1: 2-((3,5-dimethyl-1-phenyl-1 H-pyrazol-4-yl)amino)-2-oxoethyl 2-(4-nitrophenyl)acetate

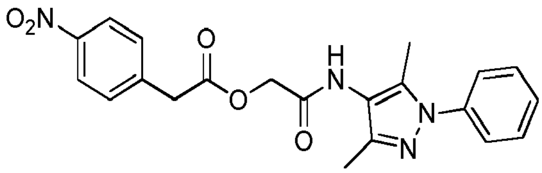

2-(4-nitrophenyl)acetic acid (31.2 mg, 0.2 mmol) was dissolved in dry DCM (3.4 mL), cooled to 0 °C and added DCC (34.3 mg, 0.2 mmol) and DMAP (4.1 mg, 0.03 mmol) followed by N-(3,5-dimethyl-1-phenyl-1 H-pyrazol-4-yl)-2-hydroxyacetamide (41.5 mg, 0.2 mmol), synthesized as reported in Example 1. The obtained suspension was filtered over cotton to remove the bulk of DCU and purified by column chromatography (75% EtOAc in PE) to yield 54.6 mg of the title compound (79%).

Step 2: 2-((3,5-dimethyl-1-phenyl-1H-pyrazol-4-yl)amino)-2-oxoethyl 2-(4-aminophenyl)acetate

The nitrophenylacetate ester from step 1 (20.8 mg, 0.1 mmol) was dissolved in EtOAc (3 mL), and added a spatula tip of 10% palladium on carbon. The mixture was left stirring under an atmosphere of $H_2$ overnight, then the suspension was filtered over celite to get 10.8 mg of the product (56%), which was used without further purification.

**TLC:** $R_f$ (5% MeOH in DCM, 2x): 0.34

**$^1$H NMR** (600 MHz, DMSO-$d_6$) δ: 9.28 (s, 1 H), 7.53 - 7.48 (m, 6H), 7.41 - 7.36 (m, 1 H), 6.94 (d, $J$ = 8.3 Hz, 2H), 6.50 (d, $J$ = 8.4 Hz, 2H), 4.66 (s, 2H), 3.58 (s, 2H), 2.14 (s, 3H), 2.05 (s, 3H) ppm

**$^{13}$C NMR** (151 MHz, DMSO-$d_6$) δ: 171.6, 166.3, 147.5, 144.7, 139.6, 134.4, 129.8, 129.2, 127.0, 123.7, 120.8, 116.8, 113.8, 104.5, 62.6, 40.0, 11.3, 10.6 ppm

**HRMS** (ESI) m/z: $(M+H)^+$ calcd for $C_{21}H_{23}N_4O_3$: 379.1781; found: 379.1773

*Example Compound 45*

*2-((3,5-dimethyl-1-phenyl-1H-pyrazol-4-yl)amino)-2-oxoethyl 2-(2-chlorophenyl)acetate*

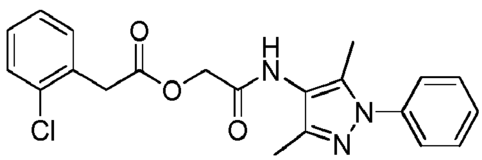

From 2-(2-chlorophenyl)acetic acid (30 mg, 0.18 mmol) was obtained the title compound analogously to example 1, step 5.

**TLC:** $R_f$ (5% MeOH in DCM): 0.35

**$^1$H NMR** (400 MHz, DMSO-$d_6$) δ: 9.34 (s, 1 H), 7.52 - 7.49 (m, 4H), 7.48 - 7.45 (m, 2H), 7.41 - 7.36 (m, 1 H), 7.36 - 7.30 (m, 2H), 4.73 (s, 2H), 3.97 (s, 2H), 2.14 (s, 3H), 2.05 (s, 3H).

**$^{13}$C NMR** (101 MHz, DMSO-$d_6$) δ: 169.9, 166.1, 144.7, 139.6, 134.4, 133.7, 132.4, 132.2, 129.2, 129.1, 129.1, 127.3, 127.1, 123.8, 116.8, 62.9, 38.0, 11.3, 10.6 ppm

**HRMS** (ESI) m/z: $(M+H)^+$ calcd for $C_{21}H_{21}ClN_3O_3$: 398.1266; found: 398.1269

*Example Compound 46*

*2-((3,5-dimethyl-1-phenyl-1H-pyrazol-4-yl)amino)-2-oxoethyl 2-(4-cyanophenyl)acetate*

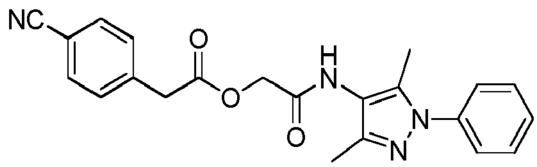

2-(4-cyanophenyl)acetic acid (31.2 mg, 0.2 mmol) was dissolved in dry DCM (4 mL), cooled to 0 °C and added DCC (39.0 mg, 0.4 mmol) and DMAP (4.6 mg, 0.04 mmol) followed by N-(3,5-dimethyl-1-phenyl-1 H-pyrazol-4-yl)-2-hydroxyacetamide (46.1 mg, 0.2 mmol), synthesized as reported in Example 1. The obtained suspension was filtered over cotton to remove the bulk of DCU and purified by column chromatography (75% EtOAc in PE) to yield 27.7 mg of the title compound (38%).

**TLC:** $R_f$ (5% MeOH in DCM): 0.23

**$^1$H NMR** (400 MHz, DMSO-$d_6$) δ: 9.34 (bs, 1 H), 7.82 (d, *J* = 8.3 Hz, 2H), 7.54 (d, *J* = 8.2 Hz, 2H), 7.47-7.53 (m, 4H), 7.35-7.42 (m, 1 H), 4.73 (s, 2H), 3.97 (s, 2H), 2.14 (s, 3H), 2.05 (s, 3H) ppm

**$^{13}$C NMR** (101 MHz, DMSO-$d_6$) δ: 170.2, 166.0, 144.7, 140.0, 139.6, 134.3, 132.1, 132.1, 130.6, 130.6, 129.1, 127.0, 123.7, 118.7, 116.7, 109.8, 62.9, 39.7, 11.3, 10.6 ppm

**HRMS** (ESI) m/z: $(M+H)^+$ calcd for $C_{22}H_{21}N_4O_3$: 389.1608; found: 389.1612

*Example Compound 47*

*2-((3,5-dimethyl-1-phenyl-1H-pyrazol-4-yl)amino)-2-oxoethyl 2-(3-acetamidophenyl)acetate*

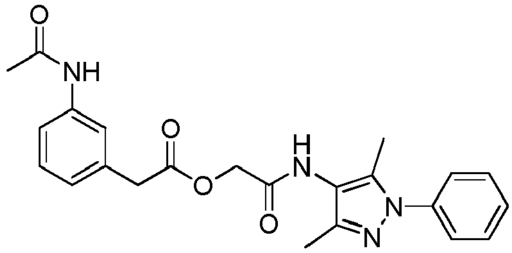

Step 1: 2-((3,5-dimethyl-1-phenyl-1 H-pyrazol-4-yl)amino)-2-oxoethyl 2-(4-nitrophenyl)acetate

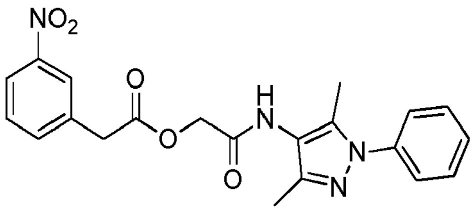

2-(3-nitrophenyl)acetic acid (81.6 mg, 0.4 mmol) was dissolved in dry DCM (9 mL), cooled to 0 °C and added DCC (85.6 mg, 0.4 mmol) and DMAP (11.0 mg, 0.09 mmol) followed by N-(3,5-dimethyl-1-phenyl-1 H-pyrazol-4-yl)-2-hydroxyacetamide (101.5 mg, 0.4 mmol), synthesized as reported in Example 1. The obtained suspension was filtered over cotton to remove the bulk of DCU and purified by column chromatography (75% EtOAc in PE) to yield 144.0 mg of the title compound (85%).

Step 2: 2-((3,5-dimethyl-1-phenyl-1H-pyrazol-4-yl)amino)-2-oxoethyl 2-(3-aminophenyl)acetate

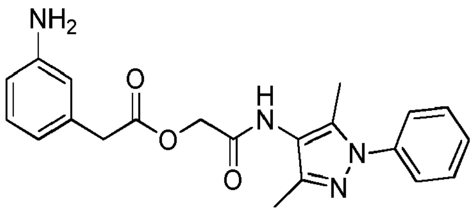

The nitrophenylacetate ester from step 1 (140 mg, 0.3 mmol) was dissolved in EtOAc (5 mL), and added a spatula of 10% palladium on carbon. The mixture was left stirring under an atmosphere of $H_2$ for 72 h, then the suspension

was filtered over celite, and the product was purified by column chromatography (5% MeOH in DCM) to afford 81.5 mg of the title compound (63%)

Step 3: 2-((3,5-dimethyl-1-phenyl-1 H-pyrazol-4-yl)amino)-2-oxoethyl 2-(3-acetamidophenyl)acetate

The aniline from step 2 (31.1 mg, 0.1 mmol) was dissolved in dry DCM (1 mL) and cooled to 0 °C. Then acetic anhydride (15.6 μL, 0.1 mmol) and pyridine (33.2 μL, 0.4 mmol) were added and the mixture was allowed to stir at room temperature for 3 h, after which it was diluted with DCM and washed with a saturated solution of $NH_4Cl$ and a saturated solution of $NaHCO_3$. The organic phase was dried over $MgSO_4$, filtered and concentrated and 30.2 mg (87%) of the title compound were obtained by column chromatography (5% MeOH in DCM).

**TLC:** $R_f$ (5% MeOH in DCM, 2x): 0.24

**$^1$H NMR** (400 MHz, DMSO-$d_6$) δ: 9.92 (s, 1 H), 9.31 (s, 1 H), 7.56 - 7.44 (m, 6H), 7.43 - 7.36 (m, 1 H), 7.24 (t, *J* = 7.8 Hz, 1 H), 6.98 (dt, *J* = 7.7, 1.3 Hz, 1 H), 4.70 (s, 2H), 3.78 (s, 2H), 2.14 (s, 3H), 2.05 (s, 3H), 2.03 (s, 3H) ppm

**$^{13}$C NMR** (101 MHz, DMSO-$d_6$) δ: 170.8, 168.3, 166.2, 144.7, 139.6, 139.3, 134.5, 134.4, 129.1, 128.6, 127.0, 124.1, 123.7, 120.0, 117.6, 116.7, 62.7, 40.0, 24.0, 11.3, 10.6 ppm

**HRMS** (ESI) m/z: $(M+H)^+$ calcd for $C_{23}H_{25}N_4O_4$: 421.1870; found: 421.1873

*Example Compound 48*

*2-((3,5-dimethyl-1-phenyl-1H-pyrazol-4-yl)amino)-2-oxoethyl 2-(2-bromophenyl)acetate*

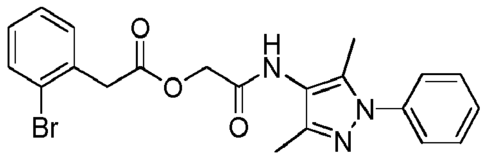

2-(2-bromophenyl)acetic acid (27.0 mg, 0.1 mmol) was dissolved in DCM (2.5 mL) and added DCC (25.5 mg, 0.1 mmol) and DMAP (3.5 mg, 0.03 mmol) followed by N-(3,5-dimethyl-1-phenyl-1 H-pyrazol-4-yl)-2-hydroxyacetamide (30.5 mg, 0.1 mmol), synthesized as reported in Example 1. After stirring overnight, the obtained suspension was filtered over cotton to remove the bulk of DCU and purified by column chromatography (75% EtOAc in PE) to yield 52.3 mg of the title compound (95%).

**TLC:** $R_f$ (5% MeOH in DCM): 0.39

**$^1$H NMR** (400 MHz, DMSO-$d_6$) δ: 9.33 (s, 1 H), 7.64 (dd, *J* = 7.9, 1.3 Hz, 1 H), 7.51 - 7.49 (m, 4H), 7.47 (dd, *J* = 7.8, 1.9 Hz, 1 H), 7.41 - 7.35 (m, 2H), 7.25 (td, *J* = 7.7, 1.8 Hz, 1 H), 4.73 (s, 2H), 3.97 (s, 2H), 2.14 (s, 3H), 2.06 (s, 3H) ppm

**$^{13}$C NMR** (101 MHz, DMSO-$d_6$) δ: 169.8, 166.0, 144.7, 139.6, 134.4, 134.1, 132.4, 132.2, 129.3, 129.1, 127.8, 127.0, 124.5, 123.7, 116.7, 62.8, 40.5, 11.3, 10.6 ppm

**HRMS** (ESI) m/z: $(M+H)^+$ calcd for $C_{21}H_{21}BrN_3O_3$: 442.0761; found: 442.0767

*Example Compound 49*

*2-((3,5-dimethyl-1-phenyl-1H-pyrazol-4-yl)amino)-2-oxoethyl 2-(m-tolyl)acetate*

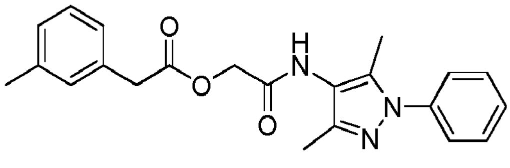

2-(3-tolyl)acetic acid (26.8 mg, 0.2 mmol) was dissolved in DCM (3.4 mL) and added DCC (34.1 mg, 0.2 mmol) and DMAP (4.0 mg, 0.03 mmol) followed by N-(3,5-dimethyl-1-phenyl-1 H-pyrazol-4-yl)-2-hydroxyacetamide (38.2 mg, 0.2 mmol), synthesized as reported in Example 1. The obtained suspension was filtered over cotton to remove the bulk of DCU and purified by column chromatography (75% EtOAc in PE) to afford 59 mg (99%) of the title compound.

**TLC:** $R_f$ (5% MeOH in DCM): 0.29

**$^1$H NMR** (400 MHz, DMSO-$d_6$) δ: 9.32 (bs, 1 H), 7.46-7.55 (m, 4H), 7.34-7.43 (m, 1 H),7.12 (t, *J* = 7.5 Hz, 1H), 7.05-7.14 (*m*, 3H), 4.70 (s, 2H), 3.77 (s, 2H), 2.29 (s, 3H), 2.14 (s, 3H), 2.06 (s, 3H) ppm

**$^{13}$C NMR** (101 MHz, DMSO-$d_6$) δ: 171.0, 166.2, 144.7, 139.6, 137.4, 134.4, 134.0, 130.1, 129.1, 128.2, 127.5, 127.0, 126.5, 123.7, 116.8, 62.7, 39.9, 20.9, 11.3, 10.6 ppm

**HRMS** (ESI) m/z: $(M+H)^+$ calcd for $C_{22}H_{24}N_3O_3$: 378.1812; found: 378.1817

*Example Compound 50*

*2-((3,5-dimethyl-1-phenyl-1H-pyrazol-4-yl)amino)-2-oxoethyl 2-(o-tolyl)acetate*

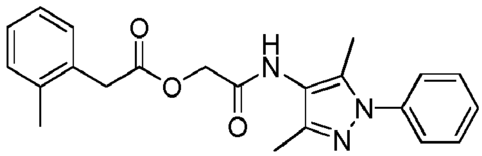

2-(2-tolyl)acetic acid (26.3 mg, 0.2 mmol) was dissolved in DCM (3.4 mL) and added DCC (34.0 mg, 0.2 mmol) and DMAP (4.0 mg, 0.03 mmol) followed by N-(3,5-dimethyl-1-phenyl-1 H-pyrazol-4-yl)-2-hydroxyacetamide (38.0 mg, 0.2 mmol), synthesized as reported in Example 1. The obtained suspension was filtered over cotton to remove the bulk of DCU and purified by column chromatography (75% EtOAc in PE) to afford 60 mg (99%) of the title compound.

**TLC:** $R_f$ (5% MeOH in DCM): 0.26

**$^1$H NMR** (400 MHz, DMSO-$d_6$) δ: 9.33 (bs, 1H), 7.47-7.54 (m, 4H), 7.35-7.42 (m, 1H), 7.21-7.26 (m, 1H), 7.11-7.20 (m, 3H), 4.71 (s, 2H), 3.82 (s, 2H), 2.26 (s, 3H), 2.14 (s, 3H), 2.05 (s, 3H) ppm

**$^{13}$C NMR** (101 MHz, DMSO-$d_6$) δ: 170.8, 166.2, 144.7, 139.6, 136.9, 134.3, 132.9, 130.3, 129.9, 129.1, 127.1, 127.0, 125.8, 123.7, 116.8, 62.7, 38.1, 19.1, 11.3, 10.6 ppm

**HRMS** (ESI) m/z: $(M+H)^+$ calcd for $C_{22}H_{24}N_3O_3$: 378.1812; found: 378.1817

*Example Compound 51*

*2-((3,5-dimethyl-1-phenyl-1H-pyrazol-4-yl)amino)-2-oxoethyl 2-(2-iodophenyl)acetate*

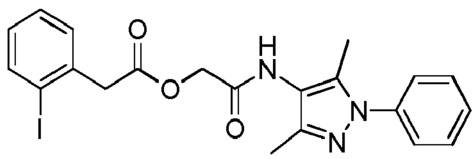

2-(2-iodophenyl)acetic acid (25.9 mg, 0.1 mmol) was dissolved in dry DCM (2 mL), cooled to 0 °C and added DCC (20.2 mg, 0.1 mmol) and DMAP (2.8 mg, 0.02 mmol) followed by N-(3,5-dimethyl-1-phenyl-1 H-pyrazol-4-yl)-2-hydroxyacetamide (24.0 mg, 0.1 mmol), synthesized as reported in Example 1. The obtained suspension was filtered over cotton to remove the bulk of DCU and purified by column chromatography (75% EtOAc in PE) to yield 44 mg of the title compound (92%).

**TLC:** $R_f$ (5% MeOH in DCM): 0.31

**$^1$H NMR** (400 MHz, DMSO-$d_6$) δ: 9.33 (bs, 1 H), 7.87 (dd, *J* = 7.9, 1.1 Hz, 1 H), 7.47-7.56 (m, 4H), 7.35-7.46 (m, 1 H), 7.04 (ddd, *J* = 7.9, 7.3, 1.9 Hz, 1 H), 4.73 (s, 2H), 3.96 (s, 2H), 2.14 (s, 3H), 2.06 (s, 3H) ppm

**$^{13}$C NMR** (101 MHz, DMSO-$d_6$) δ: 169.8, 166.0, 144.7, 139.6, 139.0, 137.6, 134.3, 131.2, 129.1, 128.4, 127.0, 123.7, 116.7, 101.6, 62.9, 45.0, 11.3, 10.7 ppm

**HRMS** (ESI) m/z: $(M+H)^+$ calcd for $C_{21}H_{21}IN_3O_3$: 490.0622; found: 490.0634

*Example Compound 52*

*2-((3,5-dimethyl-1-phenyl-1H-pyrazol-4-yl)amino)-2-oxoethyl 2-(4-methoxyphenyl)acetate*

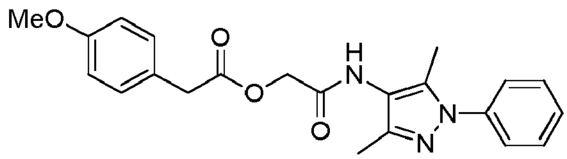

2-(4-methoxyphenyl)acetic acid (26.7 mg, 0.2 mmol) was dissolved in dry DCM (3 mL), cooled to 0 °C and added DCC (31.0 mg, 0.2 mmol) and DMAP (4.2 mg, 0.03 mmol) followed by N-(3,5-dimethyl-1-phenyl-1 H-pyrazol-4-yl)-2-hydroxyacetamide (35.7 mg, 0.1 mmol), synthesized as reported in Example 1. The obtained suspension was filtered over cotton to remove the bulk of DCU and purified by column chromatography (75% EtOAc in PE) to yield 56 mg of the title compound (97%).

**TLC:** $R_f$ (5% MeOH in DCM, 2x): 0.47

**$^1$H NMR** (400 MHz, DMSO-$d_6$) δ: 9.31 (bs, 1 H), 7.47-7.56 (m, 4H), 7.35-7.43 (m, 1 H), 7.23 (d, *J* = 8.6 Hz, 1 H), 6.89 (d, *J* = 8.6 Hz, 1 H), 4.69 (s, 2H), 3.74 (s, 2H), 3.73 (s, 3H, C), 2.14 (s, 3H), 2.05 (s, 3H) ppm

**$^{13}$C NMR** (101 MHz, DMSO-$d_6$) δ: 171.2, 166.2, 158.2, 144.7, 139.6, 134.4, 130.5, 129.1, 127.0, 126.0, 123.7, 116.8, 113.7, 62.7, 55.0, 39.0, 11.3, 10.6 ppm

**HRMS** (ESI) m/z: $(M+H)^+$ calcd for $C_{22}H_{24}N_3O_4$: 394.1761; found: 394.1767

*Example Compound 53*

*2-((3,5-dimethyl-1-phenyl-1H-pyrazol-4-yl)amino)-2-oxoethyl 2-(3-methoxyphenyl)acetate*

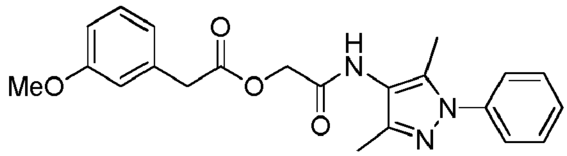

2-(3-methoxyphenyl)acetic acid (27.3 mg, 0.2 mmol) was dissolved in dry DCM (3 mL), cooled to 0 °C and added DCC (30.6 mg, 0.1 mmol) and DMAP (3.9 mg, 0.03 mmol) followed by N-(3,5-dimethyl-1-phenyl-1 H-pyrazol-4-yl)-2-hydroxyacetamide (35.2 mg, 0.1 mmol), synthesized as reported in Example 1. The obtained suspension was filtered over cotton to remove the bulk of DCU and purified by column chromatography (75% EtOAc in PE) to yield 52.6 mg of the title compound (93%).

**TLC:** $R_f$ (5% MeOH in DCM): 0.24.

**$^1$H NMR** (400 MHz, DMSO-$d_6$) δ: 9.33 (bs, 1H), 7.47-7.56 (m, 4H), 7.35-7.43 (m, 1H), 7.23 (ddd, *J* = 8.1, 7.5, 0.3 Hz, 1 H), 6.86-6.92 (m, 2H), 6.84 (ddd, *J* = 8.2, 2.6, 0.9 Hz, 1H), 4.71 (s, 2H), 3.79 (s, 2H), 3.74 (s, 3H), 2.14 (s, 3H), 2.05 (s, 3H) ppm

**$^{13}$C NMR** (101 MHz, DMSO-$d_6$) δ: 170.8, 166.2, 159.2, 144.7, 139.6, 135.6, 134.4, 129.3, 129.1, 127.0, 123.7, 121.7, 116.8, 115.1, 112.3, 62.7, 55.0, 39.9, 11.3, 10.6 ppm

**HRMS** (ESI) m/z: $(M+H)^+$ calcd for $C_{22}H_{24}N_3O_4$: 394.1761; found: 394.1769

*Example Compound 54*

*2-((3,5-dimethyl-1-phenyl-1H-pyrazol-4-yl)amino)-2-oxoethyl 2-(2-methoxyphenyl)acetate*

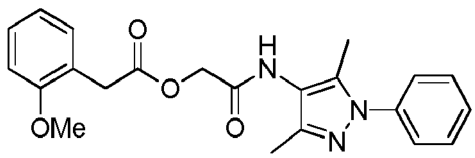

2-(2-methoxyphenyl)acetic acid (25.3 mg, 0.1 mmol) was dissolved in dry DCM (3 mL), cooled to 0 °C and added DCC (30.4 mg, 0.1 mmol) and DMAP (3.7 mg, 0.03 mmol) followed by N-(3,5-dimethyl-1-phenyl-1 H-pyrazol-4-yl)-2-hydroxyacetamide (35.3 mg, 0.1 mmol), synthesized as reported in Example 1. The obtained suspension was

filtered over cotton to remove the bulk of DCU and purified by column chromatography (75% EtOAc in PE) to yield 56 mg of the title compound (99%).

**TLC:** $R_f$ (5% MeOH in DCM): 0.24

**$^1$H NMR** (400 MHz, DMSO-$d_6$) δ: 9.28 (bs, 1H), 7.47-7.55 (m, 4H), 7.35-7.42 (m, 1H), 7.22-7.30 (m, 2H), 6.99 (ddd, *J* = 8.2, 0.8 Hz, 1 H), 6.91 (dt, *J* = 7.4, 1.1 Hz, 1 H), 4.69 (s, 2H), 3.76 (s, 3H), 3.74 (s, 2H), 2.14 (s, 3H), 2.06 (s, 3H) ppm

**$^{13}$C NMR** (101 MHz, DMSO-$d_6$) δ: 170.8, 166.2, 157.2, 144.7, 139.6, 134.4, 131.0, 129.1, 128.5, 127.0, 123.7, 122.6, 120.2, 116.8, 110.8, 62.6, 55.4, 34.8, 11.3, 10.6 ppm

**HRMS** (ESI) m/z: $(M+H)^+$ calcd for $C_{22}H_{24}N_3O_4$: 394.1761; found: 394.1766

*Example Compound 55*

*2-((3,5-dimethyl-1-phenyl-1H-pyrazol-4-yl)amino)-2-oxoethyl 2-(3-aminophenyl)acetate*

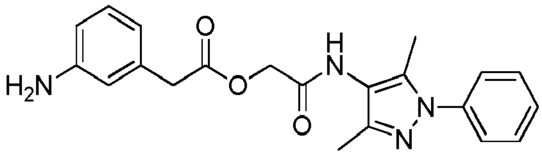

The title compound was synthesized as reported in Example 47, step 1 and 2.

**TLC:** $R_f$ (5% MeOH in DCM): 0.31

**$^1$H NMR** (400 MHz, DMSO-$d_6$) δ: 9.30 (s, 1 H), 7.52 - 7.47 (m, 4H), 7.43 - 7.34 (m, 1 H), 6.95 (t, *J* = 7.7 Hz, 1 H), 6.51 - 6.41 (m, 3H), 5.04 (s, 2H), 4.68 (s, 2H), 3.61 (s, 2H), 2.14 (s, 3H), 2.06 (s, 3H) ppm

**$^{13}$C NMR** (101 MHz, DMSO-$d_6$) δ: 171.0, 166.2, 148.7, 144.7, 139.6, 134.4, 134.4, 129.1, 128.8, 127.0, 123.7, 116.8, 114.8, 112.5, 62.6, 40.3, 11.3, 10.6 ppm

**HRMS** (ESI) m/z: $(M+H)^+$ calcd for $C_{21}H_{23}N_4O_3$: 379.1765; found: 379.1770

*Example Compound 56*

*2-((3,5-dimethyl-1-phenyl-1H-pyrazol-4-yl)amino)-2-oxoethyl 2-(3-cyanophenyl)acetate*

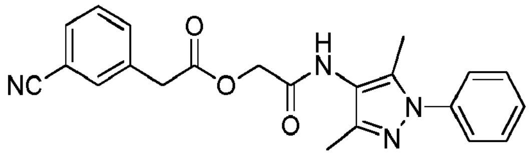

2-(3-cyanophenyl)acetic acid (33.0 mg, 0.2 mmol) was dissolved in DCM (4 mL), and added DCC (40.0 mg, 0.2 mmol) and DMAP (5.0 mg, 0.04 mmol) followed by N-(3,5-dimethyl-1-phenyl-1 H-pyrazol-4-yl)-2-hydroxyacetamide (49.8 mg, 0.2 mmol), synthesized as reported in Example 1. The obtained suspension was filtered over cotton to remove the bulk of DCU and purified by column chromatography (75% EtOAc in PE) to yield 53.1 mg of the title compound (67%).

**TLC:** $R_f$ (5% MeOH in DCM): 0.31

**$^1$H NMR** (400 MHz, DMSO-$d_6$) δ: 9.34 (bs, 1 H), 7.79-7.82 (m, 1 H), 7.76 (ddd, *J* = 7.7, 1.6, 1.2 Hz, 1 H), 7.68 (ddd, *J* = 7.9, 1.7, 1.3 Hz, 1 H), 7.47-7.53 (m, 4H), 7.34-7.42 (m, 1 H), 6.84 (dt, *J* = 7.8, 0.4 Hz, 1 H), 4.73 (s, 2H), 3.94 (s, 2H), 2.14 (s, 3H), 2.05 (s, 3H) ppm

**$^{13}$C NMR** (101 MHz, DMSO-$d_6$) δ: 170.4, 166.1, 144.7, 139.6, 135.9, 134.7, 134.4, 133.1, 130.7, 129.5, 129.1, 127.0, 123.7, 118.7, 116.7, 111.2, 62.9, 39.0, 11.3, 10.6 ppm

**HRMS** (ESI) m/z: $(M+H)^+$ calcd for $C_{22}H_{21}N_4O_3$: 389.1608; found: 389.1613

*Example Compound 57*

*2-((3,5-dimethyl-1-phenyl-1H-pyrazol-4-yl)amino)-2-oxoethyl 2-(pyridin-3-yl)acetate*

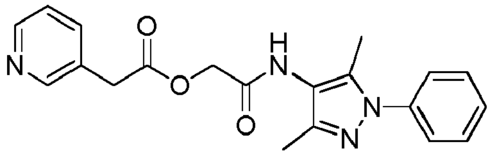

2-(pyridin-3-yl)acetic acid hydrochloride (32.5 mg, 0.2 mmol) was suspended in DCM (4 mL) under Argon, cooled to 0 °C and added trimethylamine (26 μL, 0.2 mmol). After 30 minutes a clear solution is observed and EDCI (35.9 mg, 0.2 mmol) and DMAP (4.8 mg, 0.04 mmol) followed by N-(3,5-dimethyl-1-phenyl-1H-pyrazol-4-yl)-2-hydroxyacetamide (44.1 mg, 0.2 mmol), synthesized as reported in Example 1. The mixture was left stirring overnight and then diluted with water and extracted 3 times with DCM. The organic phase was washed with brine, dried over $MgSO_4$, filtered and concentrated. Purification by column chromatography (5% MeOH in DCM) yielded 60.6 mg of the title compound (92%).

**TLC:** $R_f$ (5% MeOH in DCM, 2x): 0.38

**$^1$H NMR** (400 MHz, DMSO-$d_6$) δ: 9.35 (bs, 1 H), 8.53 (dd, *J* = 2.3, 0.6 Hz, 1 H), 8.48 (dd, *J* = 4.8, 1.6 Hz, 1 H), 7.74-7.78 (m, 1 H), 7.46-7.54 (m, 4H), 7.34-7.42 (m, 2H), 4.73 (s, 2H), 3.90 (s, 2H), 2.14 (s, 3H), 2.05 (s, 3H) ppm

**$^{13}$C NMR** (101 MHz, DMSO-$d_6$) δ: 170.6, 166.1, 150.2, 147.9, 144.7, 139.6, 137.3, 134.4, 130.1, 129.1, 127.0, 123.7, 123.4, 116.7, 62.9, 36.9, 11.3, 10.6 ppm

**HRMS** (ESI) m/z: $(M+H)^+$ calcd for $C_{20}H_{21}N_4O_3$: 365.1608; found: 365.1612

*Example Compound 58*

*2-((3,5-dimethyl-1-phenyl-1H-pyrazol-4-yl)amino)-2-oxoethyl 2-(pyridin-2-yl)acetate*

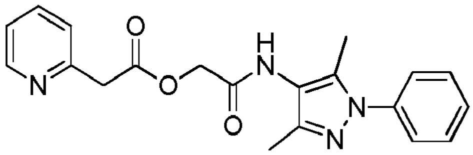

Step 1: 2-((3,5-dimethyl-1-phenyl-1H-pyrazol-4-yl)amino)-2-oxoethyl 2-(pyridin-4-yl)acetate

2-(pyridin-2-yl)acetic acid hydrochloride (30.4 mg, 0.2 mmol) was suspended in DCM (4 mL) under Argon, cooled to 0 °C and added trimethylamine (24 μL, 0.2 mmol). After 20 minutes a clear solution is observed and EDCI (33.1 mg, 0.2 mmol) and DMAP (4.1 mg, 0.03 mmol) followed by N-(3,5-dimethyl-1-phenyl-1H-pyrazol-4-yl)-2-hydroxyacetamide (42.3 mg, 0.2 mmol), synthesized as reported in Example 1. The mixture was left stirring overnight and then diluted with water and extracted 3 times with DCM. The organic phase was washed with brine, dried over $MgSO_4$, filtered and concentrated. Purification by column chromatography (5% MeOH in DCM) yielded 54.5 mg of the title compound (87%).

**TLC:** $R_f$ (5% MeOH in DCM): 0.40

**$^1$H NMR** (400 MHz, DMSO-$d_6$) δ: 9.30 (s, 1H), 7.81 - 7.74 (m, 2H), 7.55 - 7.42 (m, 6H), 7.35 (ddd, *J* = 5.7, 4.6, 2.5 Hz, 1 H), 4.69 (s, 2H), 3.94 (s, 2H), 2.10 (s, 3H), 2.02 (s, 3H) ppm

**$^{13}$C NMR** (101 MHz, DMSO-$d_6$) δ: 170.1, 166.0, 144.7, 140.0, 139.6, 134.3, 132.1, 130.6, 129.1, 127.0, 123.7, 118.7, 116.7, 109.8, 62.8, 11.2, 10.6 ppm

**HRMS** (ESI) m/z: $(M+H)^+$ calcd for $C_{20}H_{21}N_4O_3$: 365.1608; found: 365.1613

*Example Compound 59*

*2-((3,5-dimethyl-1-phenyl-1H-pyrazol-4-yl)amino)-2-oxoethyl 2-(thiophen-2-yl)acetate*

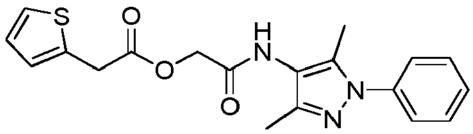

From 2-(thiophen-2-yl)acetic acid (20 mg, 0.14 mmol) was prepared 49 mg (94%) of the title compound analogously to example 1, step 5.

**TLC:** $R_f$ (5% MeOH in DCM): 0.46

**$^1$H NMR** (400 MHz, DMSO-$d_6$) δ: 9.34 (bs, 1H), 7.47-7.54 (m, 4H), 7.43 (dd, *J* = 5.1, 1.3 Hz, 1H), 7.35-7.42 (m, 1 H), 7.01-7.03 (m, 1 H), 6.99 (dd, *J* = 5.1, 3.5 Hz, 1 H), 4.73 (s, 2H), 4.08 (s, 2H), 2.15 (s, 3H), 2.06 (s, 3H) ppm

**$^{13}$C NMR** (101 MHz, DMSO-$d_6$) δ: 170.0, 166.1, 144.7, 139.6, 135.0, 134.4, 129.1, 127.2, 127.0, 126.7, 125.5, 123.7, 116.7, 62.9, 34.2, 11.3, 10.6 ppm

**HRMS** (ESI) m/z: $(M+H)^+$ calcd for $C_{19}H_{20}N_3O_3S$: 370.1220; found: 370.1220

*Example Compound 60*

*2-((3,5-dimethyl-1-phenyl-1H-pyrazol-4-yl)amino)-2-oxoethyl 2-(5-chlorothiophen-2-yl)acetate*

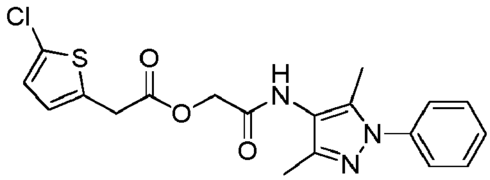

The substance (5-chloro-2-thienyl)acetic acid (30.3 mg, 0.2 mmol) was dissolved in DCM (3 mL), and added DCC (35.0 mg, 0.2 mmol) and DMAP (4.3 mg, 0.04 mmol) followed by N-(3,5-dimethyl-1-phenyl-1 H-pyrazol-4-yl)-2-hydroxyacetamide (41.9 mg, 0.2 mmol), synthesized as reported in Example 1. The mixture was left stirring overnight. The suspension was filtered over cotton to remove the bulk of DCU and purified by column chromatography (75% EtOAc in PE) to yield 25.0 mg of the title compound (86%).

**TLC:** $R_f$ (5% MeOH in DCM): 0.36

**$^1$H NMR** (400 MHz, DMSO-$d_6$) δ: 9.31 (bs, 1 H), 7.45-7.56 (m, 4H), 7.34-7.43 (m, 1 H), 6.95-7.02 (m, 1 H), 6.86-6.92 (m, 1 H), 4.74 (s, 2H), 4.07 (s, 2H), 2.14 (s, 3H), 2.06 (s, 3H) ppm

**$^{13}$C NMR** (100 MHz, DMSO-$d_6$) δ: 169.7, 166.0, 144.7, 139.6, 134.6, 134.4, 129.2, 127.1, 127.0, 126.2, 123.8, 116.7, 63.0, 34.5, 11.3, 10.6 ppm

**HRMS** (ESI) m/z: $(M+H)^+$ calcd for $C_{19}H_{19}ClN_3O_3S$: 404.0830; found: 404.0836

*Example Compound 61*

*2-((3,5-dimethyl-1-phenyl-1H-pyrazol-4-yl)amino)-2-oxoethyl 2-(2,5-dichlorothiophen-3-yl)acetate*

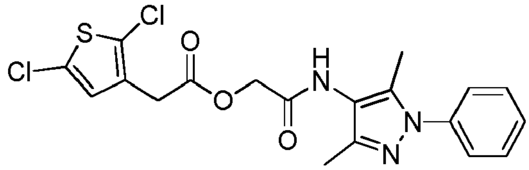

The substance (2,5-dichloro-3-thienyl)acetic acid from step 1 (59.9 mg, 0.3 mmol, see *Bioorg. Med. Chem. Lett.* **2010,** *vol. 20*, page 2809) was dissolved in DCM (5 mL), and added DCC (60.0 mg, 0.3 mmol) and DMAP (6.9 mg, 0.06 mmol) followed by N-(3,5-dimethyl-1-phenyl-1 H-pyrazol-4-yl)-2-hydroxyacetamide (69.3 mg, 0.3 mmol), synthesized as reported in Example 1. The obtained suspension was filtered over cotton to remove the bulk of DCU and purified by column chromatography (75% EtOAc in PE) to yield 106.7 mg of the title compound (86%).

**TLC:** $R_f$ (5% MeOH in DCM): 0.24

**$^1$H NMR** (400 MHz, DMSO-$d_6$) δ: 9.35 (bs, 1 H), 7.47-7.54 (m, 4H), 7.35-7.42 (m, 1 H), 7.14 (s, 1 H), 4.74 (s, 2H), 3.80 (s, 2H), 2.15 (s, 3H), 2.06 (s, 3H) ppm

**$^{13}$C NMR** (100 MHz, DMSO-$d_6$) δ: 169.0, 166.0, 144.7, 139.6, 134.4, 131.8, 129.2, 128.8, 127.0, 124.5, 123.8, 123.2, 116.7, 63.0, 32.6, 11.3, 10.6 ppm

**HRMS** (ESI) m/z: $(M+H)^+$ calcd for $C_{19}H_{18}Cl_2N_3O_3S$: 438.0440; found: 438.0447

*Example Compound 62*

*2-((3,5-dimethyl-1-phenyl-1H-pyrazol-4-yl)amino)-2-oxoethyl 2-(pyridin-4-yl)acetate*

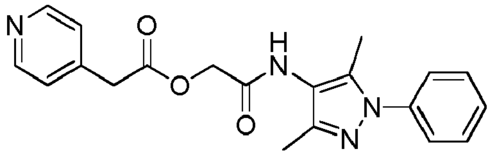

2-(pyridin-4-yl)acetic acid hydrochloride (31.6 mg, 0.2 mmol) was suspended in DCM (4 mL) under Argon, cooled to 0 °C and added trimethylamine (24 µL, 0.2 mmol). After 20 minutes a clear solution is observed and EDCI (35.0 mg, 0.2 mmol) and DMAP (4.6 mg, 0.04 mmol) followed by N-(3,5-dimethyl-1-phenyl-1H-pyrazol-4-yl)-2-hydroxyacetamide (42.8 mg, 0.2 mmol), synthesized as reported in Example 1. The mixture was left stirring overnight and then diluted with water and extracted 3 times with DCM. The organic phase was washed with brine, dried over $MgSO_4$, filtered, concentrated and purified twice by column chromatography (5% MeOH in DCM) to yield 30.8 mg of the title compound (48%).

**TLC:** $R_f$ (5% MeOH in DCM, 3x): 0.30

**$^1$H NMR** (400 MHz, DMSO-$d_6$) δ: 9.35 (s, 1 H), 8.58 - 8.48 (m, 2H), 7.51 - 7.50 (m, 2H), 7.50 - 7.48 (m, 2H), 7.39 (d, $J$ = 5.5 Hz, 1 H), 7.37 - 7.34 (m, 2H), 4.74 (s, 2H), 3.90 (s, 2H), 2.14 (s, 3H), 2.05 (s, 3H) ppm

**$^{13}$C NMR** (101 MHz, DMSO-$d_6$) δ: 169.5, 166.1, 149.5, 149.5, 144.7, 143.0, 139.6, 134.4, 129.1, 127.0, 124.9, 124.9, 123.7, 116.7, 62.9, 39.1, 11.3, 10.6 ppm

**HRMS** (ESI) m/z: $(M+H)^+$ calcd for $C_{20}H_{21}N_4O_3$: 365.1608; found: 365.1610

*Example Compound 63*

*2-((3,5-dimethyl-1-phenyl-1H-pyrazol-4-yl)amino)-2-oxoethyl 2-(2-(trifluoromethyl)phenyl)acetate*

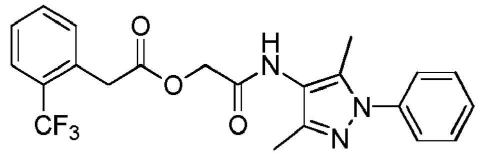

2-(o-trifluorophenyl)acetic acid (31.8 mg, 0.2 mmol) was dissolved in DCM (3 mL), and added DCC (32.0 mg, 0.2 mmol) and a catalytic amount of DMAP followed by N-(3,5-dimethyl-1-phenyl-1 H-pyrazol-4-yl)-2-hydroxyacetamide (38.1 mg, 0.2 mmol), synthesized as reported in Example 1. The mixture was left stirring overnight. The suspension was filtered over cotton to remove the bulk of DCU and purified by column chromatography (75% EtOAc in PE) to yield 56.9 mg of the title compound (85%).

**TLC:** $R_f$ (5% MeOH in DCM): 0.29.

**$^1$H NMR** (400 MHz, DMSO-d6) δ: 9.34 (bs, 1H), 7.74 (bd, $J$ = 8.0 Hz, 1 H), 7.67 (bt, $J$ = 7.5 Hz, 1 H), 7.58 (bd, $J$ = 7.7 Hz, 1 H), 7.46-7.55 (m, 5H), 7.34-7.43 (m, 1 H), 4.72 (s, 2H), 4.03 (s, 2H), 2.14 (s, 3H), 2.05 (s, 3H) ppm

**$^{13}$C NMR** (101 MHz, DMSO-d6) δ: 170.1, 166.0, 144.7, 139.6, 134.4, 133.2, 132.6, 132.3 (q, $J_{C-F}$ = 1.8 Hz), 129.1, 127.6 (q, $J_{C-F}$ = 30.5 Hz), 127.0, 126.8, 125.8 (q, $J_{C-F}$ = 5.7 Hz), 124.4 (q, $J_{C-F}$ = 273 Hz), 123.7, 116.7, 62.9, 37.2, 11.3, 10.6 ppm

**HRMS** (ESI) m/z: $(M+H)^+$ calcd for $C_{22}H_{21}F_3N_3O_3$: 432.1530; found: 432.1534

*Example Compound 64*

*2-((3,5-dimethyl-1-phenyl-1H-pyrazol-4-yl)amino)-2-oxoethyl 2-(3-fluorophenyl)acetate*

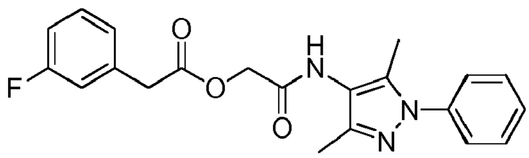

2-(m-fluorophenyl)acetic acid (30.0 mg, 0.2 mmol) was dissolved in DCM (3 mL), and added DCC (40.7 mg, 0.2 mmol) and a catalytic amount of DMAP followed by N-(3,5-dimethyl-1-phenyl-1 H-pyrazol-4-yl)-2-hydroxyacetamide (46.7 mg, 0.2 mmol), synthesized as reported in Example 1. The mixture was left stirring overnight. The suspension was filtered over cotton to remove the bulk of DCU and purified by column chromatography (75% EtOAc in PE) to yield 67.1 mg of the title compound (92%).

**TLC:** $R_f$ (5% MeOH in DCM): 0.32

**$^1$H NMR** (400 MHz, DMSO-$d_6$) δ: 9.34 (bs, 1H), 7.46-7.56 (m, 4H), 7.33-7.43 (m, 2H), 7.14-7.22 (m, 2H), 7.07-7.14 (m, 1H), 4.72 (s, 2H), 3.87 (s, 2H), 2.14 (s, 3H), 2.06 (s, 3H) ppm

**$^{13}$C NMR** (101 MHz, DMSO-$d_6$) δ: 170.5, 166.1, 162.0 (d, $J_{C-F}$ = 240 Hz), 144.7, 139.6, 136.6 (d, $J_{C-F}$ = 8.2 Hz), 134.4, 130.2 (d, $J_{C-F}$ = 8.2 Hz), 129.1, 127.0, 125.7 (d, $J_{C-F}$ = 2.7 Hz), 123.7, 116.7, 116.3 (d, $J_{C-F}$ = 21.6 Hz), 113.7 (d, $J_{C-F}$ = 20.5 Hz), 62.8, 39.4 (d, $J_{C-F}$ = 1.7 Hz), 11.3, 10.6 ppm

**HRMS** (ESI) m/z: $(M+H)^+$ calcd for $C_{21}H_{21}FN_3O_3$: 382.1562; found: 382.1564

*Example Compound 65*

*2-((2-(allyloxy)phenyl)amino)-2-oxoethyl 2-(2-iodophenyl)acetate*

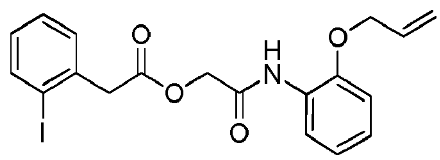

Step 1: 2-(2-acetoxyacetamido)phenyl 2-acetoxyacetate

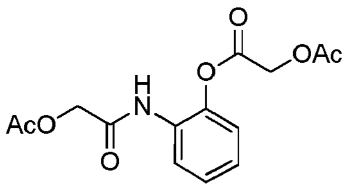

To a solution of 2-aminophenol (412.7 mg, 3.782 mmol) in DCM (35 mL) was added $Et_3N$ (1.05 mL, 7.53 mmol) and acetoxyacetyl chloride (815 μL, 7.58 mmol) at -10 °C under argon. After 5 min, the reaction was allowed to warm to room temperature, and after 72 h, the reaction mixture was quenched with aqueous $NH_4Cl$ (50 mL) and diluted with DCM (50 mL). the two layers were separated and the aqueous layer was extracted with DCM (2 x 30 mL). The combined organics were dried ($MgSO_4$), filtered, and concentrated *in vacuo.* Purification by column chromatography (3→5% MeOH in DCM) provided 1.104 g (94%) of the title compound.

Step 2: 2-hydroxy-N-(2-hydroxyphenyl)acetamide

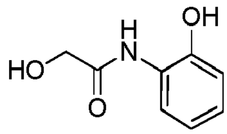

To a solution of the compound from step 1 (1.074 g, 3.473 mmol) in MeOH (15 mL) was added NaOMe (58 mg, 1.07 mmol) at room temperature. After 24 h, the reaction was quenched with 1 M aqueous HCl (4 drops) and the reaction mixture was concentrated *in vacuo.* Purification by column chromatography (10% MeOH in DCM) provided 521 mg (90%) of the title compound.

Step 3: N-(2-(allyloxy)phenyl)-2-hydroxyacetamide

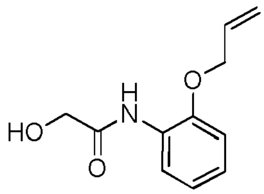

To a solution of the diol from step 2 (102 mg, 0.61 mmol) in DMF (1.5 mL) was added $K_2CO_3$ (101 mg, 0.73 mmol) and allyl bromide (184 μL, 2.13 mmol) at 0 °C. After 18 h, the reaction was quenched with aqueous $NH_4Cl$ (20 mL) and diluted with EtOAc (20 mL). The two layers were separated and the aqueous layer was extracted with EtOAc (2 x 10 mL). The combined organics were washed with aqueous $NaHCO_3$ (10 mL), brine (10 mL), dried ($MgSO_4$), filtered, and concentrated *in vacuo* to give 129 mg of the title compound which was used as is in the next step.

Step 4: 2-((2-(allyloxy)phenyl)amino)-2-oxoethyl 2-(2-iodophenyl)acetate

2(2-iodophenyl-)acetic acid (35.8 mg, 0.137 mmol) was dissolved in DCM (4 mL), and added DCC (29.1 mg, 0.141 mmol) and a catalytic amount of DMAP followed by the alcohol from step 3 (30.1 mg, 0.145 mmol). The mixture was left stirring overnight. The suspension was filtered over cotton to remove the bulk of DCU and purified by column chromatography (40% to 75% EtOAc in PE) to yield 60.3 mg of the title compound (98%).

**TLC:** $R_f$ (5% MeOH in DCM): 0.70

**$^1$H NMR** (400 MHz, DMSO-$d_6$) δ: 9.22 (bs, 1 H), 7.94 (bd, *J* = 7.9 Hz, 1 H), 7.87 (ddd, *J* = 7.9, 1.2, 0.4 Hz, 1H), 7.44 (ddd, *J* = 7.5, 1.9, 0.4 Hz, 1H), 7.39 (dt, *J* = 7.3, 1.3 Hz, 1H), 7.01-7.12 (m, 3H), 6.92 (ddd, *J* = 7.9, 6.4, 2.4 Hz, 1 H), 6.08 (ddt, *J* = 17.3, 10.6, 5.2 Hz, 1 H), 5.44 (dq, *J* = 17.4, 1.8 Hz, 1 H), 5.28 (dq, *J* = 10.6, 1.6 Hz, 1H), 4.78 (s, 2H), 4.65 (dt, *J* = 5.1, 1.6 Hz, 2H), 3.95 (s, 2H) ppm

**$^{13}$C NMR** (101 MHz, DMSO-$d_6$) δ: 169.6, 166.2, 148.4, 138.9, 137.5, 133.5, 131.2, 129.2, 128.4, 126.7, 124.7, 121.8, 120.5, 117.6, 112.7, 101.6, 68.9, 63.1, 45.0 ppm

**HRMS** (ESI) m/z: $(M+Na)^+$ calcd for $C_{19}H_{18}INNaO_4$: 474.0173; found: 474.0176

*Example Compound 66*

*2-(3,5-dimethyl-1-phenyl-1H-pyrazol-4-ylamino)-2-oxoethyl 2-(2-chlorothiophen-3-yl)acetate*

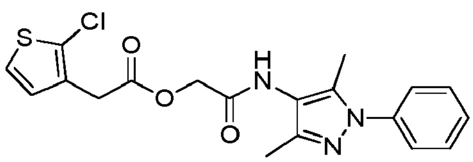

Step 1: 2-(2-chlorothiophen-3-yl)acetic acid

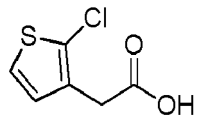

2-(thiophen-3-yl)acetic acid (150 mg, 1.06 mmol) was dissolved in acetic acid/benzene (1:1, 2 mL) and stirred under nitrogene atmosphere. *N*-chlorosuccinimide (141.0 mg, 1.06 mmol, 1 eq) was added and the reaction mixture was heated to 80°C. After 1 h the solution was poured into ice water and extracted with DCM (3x20 mL). The combined organic layers were dried ($MgSO_4$), the solvent evaporated in vacuo and purified by column chromatography (1% MeOH, 1% acetic acid in DCM) to yield 188 mg (1.06 mmol, quant.) of the title compound.

TLC: $R_f$ (1 % MeOH, 1 % AcOH in DCM): 0.25

Step 2: 2-(3,5-dimethyl-1-phenyl-1H-pyrazol-4-ylamino)-2-oxoethyl 2-(2-chlorothiophen-3-yl)acetate

The acid from step 1 (20.0 mg, 0.1 mmol) was dissolved in DCM (2 mL) at 0°C. HOBt*$H_2O$ (15.0 mg, 0.1 mmol) was added followed by EDCI (21.0 mg, 0.1 mmol). After stirring for 10 min, N-(3,5-dimethyl-1-phenyl-1 H-pyrazol-4-yl)-2-hydroxyacetamide (27.0 mg, 0.1 mmol) was added and the mixture was allowed to warm to room temperature. After stirring for 10 h the solution was diluted with DCM (30 mL) and extracted with semi-sat. $NH_4Cl$ solution, sat. $NaHCO_3$ solution and brine. The organic layer was dried (MgSO4), concentrated to dryness and purified by column chromatography (1% $\rightarrow$ 5% MeOH in DCM) to give 8.0 mg of the title compound (18%).

**TLC:** $R_f$ (5% MeOH in DCM): 0.35

**$^1$H NMR** (400 MHz, DMSO-$d_6$) $\delta$: 9.34 (bs, 1 H), 7.47-7.54 (m, 4H), 7.45 (d, *J* = 5.7 Hz, 1 H), 7.36-7.41 (m, 1H), 7.06 (d, *J* = 5.6 Hz, 1H), 4.73 (s, 2H), 3.81 (s, 2H), 2.14 (s, 3H), 2.06 (s, 3H) ppm

**$^{13}$C NMR** (100 MHz, DMSO-$d_6$) $\delta$: 169.4, 166.1, 144.7, 139.6, 134.4, 131.3, 129.1, 129.0, 127.0, 125.6, 123.9, 123.7, 116.7, 62.9, 32.6, 11.3, 10.6 ppm

**HRMS** (ESI) m/z: $(M+H)^+$ calcd for $C_{19}H_{19}ClN_3O_3S$: 404.0830; found: 404.0838

*Example Compound 67*

*2-(tert-butylamino)-2-oxoethyl 2-(2-methyl-1H-indol-3-yl)acetate*

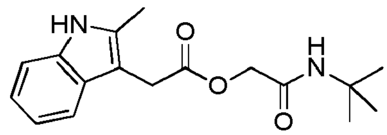

Step 1: benzyl 2-(2-(2-methyl-1 H-indol-3-yl)acetoxy)acetate

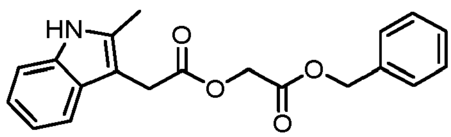

To a stirring solution of 2-(2-methyl-1 H-indol-3-yl)acetic acid (1.0 g, 5.3 mmol) in dry DCM (18 mL) cooled at 0°C, DCC (1.1 g, 5.2 mmol) was added slowly, then the benzyl 2-hydroxyacetate (0.7 mL, 5.1 mmol) and finally DMAP (143.6 mg, 1.2 mmol). After 10 minutes, the mixture was allowed to warm to room temperature. After 3h the reaction looked complete by TLC, and the crude suspension was filtered to remove the DCU precipitate and then concentrated at 40 °C. The product was purified by column chromatography (20% EtOAc in PE). 1.6 g of the title compound was

obtained (91%).

Step 2: 2-(2-(2-methyl-1H-indol-3-yl)acetoxy)acetic acid

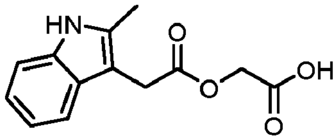

A mixture of the ester from step 1 (1.6 g, 4.7 mmol) and 10% Pd/C (158.3 mg) was stirred under an atmosphere of $H_2$ in EtOAc overnight. The reacting mixture was filtered over celite under vacuum then concentrated to give pure title compound (1.1g, 96%)

Step 3: 2-(tert-butylamino)-2-oxoethyl 2-(2-methyl-1H-indol-3-yl)acetate

To a stirring solution of the acid from step 2 (66.0 mg, 0.3 mmol) in dry THF (3 mL) cooled at 0 °C, EDCI (57.7 mg, 0.4 mmol) was added slowly, then HOBt (36.5 mg, 0.3 mmol), then 2-methylpropan-2-amine (28 μL, 0.3 mmol) and finally DIPEA (50 μL, 0.3 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (30% EtOAc in PE). Yield: 59.3 mg (73%).

**TLC:** $R_f$ (30% EtOAc in PE): 0.15

**$^1$H NMR** (400 MHz, DMSO-$d_6$) δ: 10.87 (s, 1H), 7.42 - 7.37 (m, 1H), 7.27 - 7.21 (m, 2H), 6.99 (ddd, *J* = 8.1, 7.1, 1.3 Hz, 1 H), 6.93 (ddd, *J* = 8.0, 7.0, 1.2 Hz, 1 H), 4.35 (s, 2H), 3.73 (s, 2H), 2.34 (s, 3H), 1.19 (s, 9H) ppm

**$^{13}$C NMR** (101 MHz, DMSO-$d_6$) δ: 170.9, 165.7, 135.0, 133.3, 128.2, 120.1, 118.4, 117.5, 110.4, 102.9, 62.5, 50.1, 29.4, 28.4, 11.3 ppm

**HRMS** (ESI) m/z: $(M+H)^+$ calcd for $C_{17}H_{23}N_2O_3$: 303.1703; found: 303.1700

*Example Compound 68*

*2-((3,5-dimethyl-1-phenyl-1H-pyrazol-4-yl)amino)-2-oxoethyl 2-(furan-2-yl)acetate*

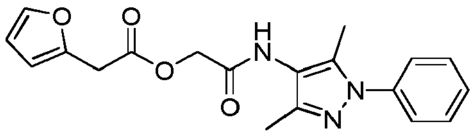

2-furanylacetic acid (30.3 mg, 0.2 mmol) was dissolved in DCM (4 mL), and added DCC (48.6 mg, 0.2 mmol) and DMAP (4.4 mg, 0.04 mmol) followed by N-(3,5-dimethyl-1-phenyl-1 H-pyrazol-4-yl)-2-hydroxyacetamide (56.3 mg, 0.2 mmol), synthesized as reported in Example 1. The mixture was left stirring overnight. The suspension was filtered over cotton to remove the bulk of DCU and purified by column chromatography (75% EtOAc in PE) to yield the title compound 82 mg (99%).

**TLC:** $R_f$ (5% MeOH in DCM): 0.13

**$^1$H NMR** (400 MHz, DMSO-$d_6$) δ: 9.34 (s, 1 H), 7.59 (dd, *J* = 1.9, 0.9 Hz, 1H), 7.54 - 7.47 (m, 4H), 7.44 - 7.34 (m, 1H), 6.41 (dd, *J* = 3.2, 1.9 Hz, 1 H), 6.33 (dd, *J* = 3.2, 0.8 Hz, 1 H), 4.72 (s, 2H), 3.95 - 3.90 (m, 2H), 2.15 (s, 3H), 2.06 (s, 3H) ppm

**$^{13}$C NMR** (101 MHz, DMSO-$d_6$) δ: 168.9, 166.0, 147.7, 144.7, 142.4, 139.6, 134.4, 129.1, 127.0, 123.7, 116.7, 110.6, 108.3, 62.8, 33.0, 11.3, 10.6 ppm

**HRMS** (ESI) m/z: $(M+H)^+$ calcd for $C_{19}H_{20}N_3O_4$: 354.1449; found: 354.1451

*Example Compound 69*

*2-((3,5-dimethyl-1-phenyl-1H-pyrazol-4-yl)amino)-2-oxoethyl 2-(2-(benzyloxy)phenyl)acetate*

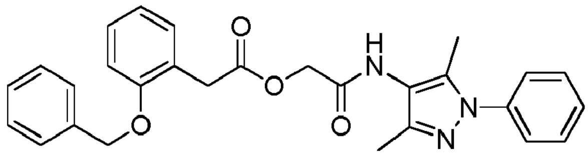

2-(2-(benzyloxy)phenyl)acetic acid (402.8 mg, 1.7 mmol) was dissolved in DCM (35 mL), and added DCC (339.0 mg, 1.6 mmol) and DMAP (40.5 mg, 0.3 mmol) followed by N-(3,5-dimethyl-1-phenyl-1 H-pyrazol-4-yl)-2-hydroxyacetamide (401.9 mg, 1.6 mmol), synthesized as reported in Example 1. The mixture was left stirring overnight. The suspension was filtered over cotton to remove the bulk of DCU and purified by column chromatography (75% EtOAc in PE) to yield 99.9 mg of the clean title compound (13%, low yield due to co-polarity of DCU).

**TLC:** $R_f$ (5% MeOH in DCM): 0.31

**$^1$H NMR** (400 MHz, DMSO-$d_6$) δ: 9.25 (s, 1 H), 7.54 - 7.47 (m, 4H), 7.46 - 7.41 (m, 2H), 7.41 - 7.35 (m, 3H), 7.34 - 7.22 (m, 3H), 7.10 - 7.04 (m, 1 H), 6.95 - 6.89 (m, 1 H), 5.12 (s, 2H), 4.66 (s, 2H), 3.80 (s, 2H), 2.12 (s, 3H), 2.05 (s, 3H) ppm

**$^{13}$C NMR** (101 MHz, DMSO-$d_6$) δ: 170.8, 166.1, 156.2, 144.7, 139.6, 137.1, 134.3, 131.1, 129.1, 128.4, 128.3, 128.3, 127.6, 127.0, 127.0, 123.7, 123.0, 120.4, 116.7, 112.1, 69.2, 62.5, 35.2, 11.3, 10.6 ppm

**HRMS** (ESI) m/z: $(M+H)^+$ calcd for $C_{28}H_{28}N_3O_4$: 470.2074; found: 470.2082

*Example Compound 70*

*2-((3,5-dimethyl-1-phenyl-1H-pyrazol-4-yl)amino)-2-oxoethyl 2-(thiophen-3-yl)acetate*

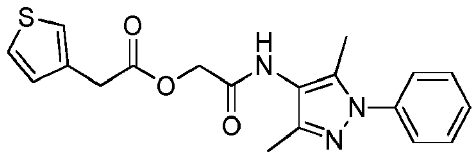

Thiophene-3-acetic acid (33.0 mg, 0.2 mmol) was dissolved in DCM (4 mL), and added DCC (44.0 mg, 0.2 mmol) and DMAP (5.0 mg, 0.04 mmol) followed by N-(3,5-dimethyl-1-phenyl-1 H-pyrazol-4-yl)-2-hydroxyacetamide (50.8 mg, 0.2 mmol), synthesized as reported in Example 1. The mixture was left stirring overnight. The suspension was filtered over cotton to remove the bulk of DCU and purified by column chromatography (75% EtOAc in PE) to yield 71.4 mg of the title compound (93%).

**TLC:** $R_f$ (5% MeOH in DCM): 0.34

**$^1$H NMR** (400 MHz, DMSO-$d_6$) δ: 9.34 (s, 1H), 7.54 - 7.47 (m, 5H), 7.42 - 7.35 (m, 2H), 7.08 (dd, $J$ = 4.9, 1.3 Hz, 1 H), 4.72 (s, 2H), 3.84 (d, $J$ = 0.9 Hz, 2H), 2.14 (s, 3H), 2.06 (s, 3H) ppm

**$^{13}$C NMR** (101 MHz, DMSO-$d_6$) δ: 170.5, 166.2, 144.7, 139.6, 134.4, 133.8, 129.1, 128.9, 127.0, 125.9, 123.7, 123.2, 116.7, 62.7, 34.6, 11.3, 10.6 ppm

**HRMS** (ESI) m/z: $(M+H)^+$ calcd for $C_{19}H_{20}N_3O_3S$: 370.1220; found: 370.1224

*Example Compound 71*

*2-(cyclopentylamino)-2-oxoethyl 2-(2-methyl-1H-indol-3-yl)acetate*

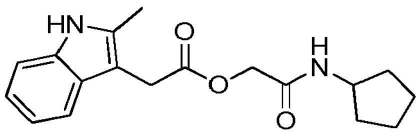

To a stirring solution of 2-(2-(2-methyl-1 H-indol-3-yl)acetoxy)acetic acid (62.3 mg, 0.3 mmol, example 67, step 2) in dry THF (3 mL) cooled at 0 °C, EDCI (55.6 mg, 0.4 mmol) was added slowly, then HOBt (34.9 mg, 0.3 mmol), then cyclopentylamine (25 μL, 0.3 mmol) and finally DIPEA (50 μL, 0.3 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (40% EtOAc in PE). Yield: 50.9 mg (64%).

**TLC:** $R_f$ (50% EtOAc in PE): 0.27

**$^1$H NMR** (400 MHz, DMSO-$d_6$) δ: 10.88 (s, 1H), 7.61 (d, *J* = 7.4 Hz, 1H), 7.43 - 7.37 (m, 1H), 7.24 (dt, *J* = 8.0, 1.0 Hz, 1 H), 6.99 (ddd, *J* = 8.0, 7.0, 1.3 Hz, 1 H), 6.93 (ddd, *J* = 8.0, 7.0, 1.2 Hz, 1 H), 4.40 (s, 2H), 4.06 - 3.88 (m, 1 H), 3.75 (s, 2H), 2.33 (s, 3H), 1.82 - 1.70 (m, 2H), 1.65 - 1.54 (m, 2H), 1.53 - 1.42 (m, 2H), 1.32 - 1.21 (m, 2H) ppm

**$^{13}$C NMR** (101 MHz, DMSO-$d_6$) δ: 171.0, 165.9, 135.0, 133.4, 128.1, 120.1, 118.4, 117.5, 110.4, 102.9, 62.3, 50.0, 32.1, 29.3, 23.4, 11.3 ppm

**HRMS** (ESI) m/z: $(M+H)^+$ calcd for $C_{18}H_{23}N_2O_3$: 315.1703; found: 315.1701

*Example Compound 72*

*2-((5-fluoropyridin-2-yl)amino)-2-oxoethyl 2-(2-methyl-1H-indol-3-yl)acetate*

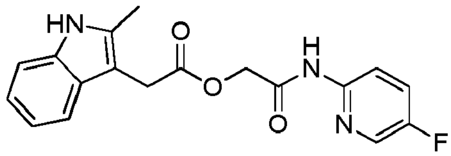

To a stirring solution of 2-(2-(2-methyl-1 H-indol-3-yl)acetoxy)acetic acid (63.7 mg, 0.3 mmol, example 67, step 2) in dry THF (3 mL) cooled at 0 °C, EDCI (50.5 mg, 0.3 mmol) was added slowly, then HOBt (35.5 mg, 0.3 mmol), then 5-fluoropyridin-2-amine (25.7 mg, 0.3 mmol) and finally DIPEA (50 μL, 0.3 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified twice by column chromatography (30% EtOAc in PE). Yield: 13.3 mg (17%).

**TLC:** $R_f$ (30% EtOAc in PE): 0.20

**$^1$H NMR** (400 MHz, DMSO-$d_6$) δ: 10.87 (s, 1H), 10.74 (s, 1H), 8.33 (d, *J* = 3.1 Hz, 1H), 8.14 - 8.02 (m, 1H), 7.75 (ddd, *J* = 9.1, 8.2, 3.1 Hz, 1H), 7.44 - 7.38 (m, 1H), 7.24 (dt, *J* = 8.0, 0.9 Hz, 1H), 6.99 (ddd, *J* = 8.0, 7.0, 1.3 Hz, 1H), 6.93 (ddd, *J* = 8.1, 7.1, 1.2 Hz, 1 H), 4.71 (s, 2H), 3.78 (s, 2H), 2.35 (s, 3H) ppm

**$^{13}$C NMR** (101 MHz, DMSO-$d_6$) δ: 171.2, 166.1, 148.0, 135.5, 135.3, 135.0, 133.4, 128.2, 125.4 (d, $J_{CF}$ = 19.5 Hz), 120.1, 118.4, 117.5, 114.6 (d, $J_{c\text{-}F}$ = 4.3 Hz), 110.4, 102.9, 62.5, 29.2, 11.3 ppm

**$^{19}$F NMR** (376 MHz, DMSO-$d_6$) δ: -135.97 ppm

**HRMS** (ESI) m/z: $(M+H)^+$ calcd for $C_{18}H_{17}FN_3O_3$: 342.1249; found: 342.1246

*Example Compound 73*

*2-((2-chlorophenyl)amino)-2-oxoethyl 2-(2-methyl-1H-indol-3-yl)acetate*

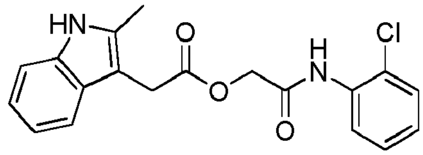

Step 1: 2-chloro-2-oxoethyl 2-(2-methyl-1 H-indol-3-yl)acetate

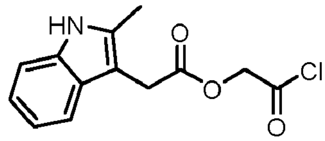

To a stirring solution of 2-(2-(2-methyl-1H-indol-3-yl)acetoxy)acetic acid (102 mg, 0.4 mmol, example 67, step 2) in dry DCM (5 mL), oxalyl chloride (105 μL, 1.2 mmol) was added slowly, and then two drops of DMF. Vigourous bubbling was observed and after 1 h, the crude reaction was concentrated to be directly used for the following step.

Step 2: 2-((2-chlorophenyl)amino)-2-oxoethyl 2-(2-methyl-1 H-indol-3-yl)acetate

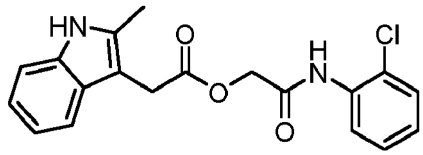

To a stirring solution of 2-chloroaniline (172 μL, 1.6 mmol) in dry DCM (7 mL) cooled at 0 °C, triethylamine (125 μL, 0.9 mmol) was added, and finally the entire lot from step 1. After 3 h, the reaction was stopped and the concentrated mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified twice by column chromatography (25% EtOAc in PE). Yield: 25.3 mg (17%).

**TLC:** $R_f$ (20% EtOAc in PE): 0.18

**$^1$H NMR** (400 MHz, DMSO-$d_6$) δ: 10.88 (s, 1 H), 9.60 (s, 1 H), 7.72 (dd, *J* = 8.0, 1.6 Hz, 1 H), 7.50 (dd, *J* = 8.0, 1.5 Hz, 1 H), 7.44 - 7.39 (m, 1 H), 7.38 - 7.29 (m, 1 H), 7.29 - 7.18 (m, 2H), 6.99 (ddd, *J* = 8.1, 7.0, 1.3 Hz, 1 H), 6.92 (ddd, *J* = 8.0, 7.1, 1.1 Hz, 1 H), 4.73 (s, 2H), 3.81 (s, 2H), 2.35 (s, 3H) ppm

**$^{13}$C NMR** (101 MHz, DMSO-$d_6$) δ: 171.1, 166.0, 135.0, 134.1, 133.4, 129.5, 128.2, 127.5, 126.6, 125.9, 120.1, 118.3, 117.5, 110.4, 102.8, 62.5, 29.2, 11.3 ppm

**HRMS** (ESI) m/z: $(M+H)^+$ calcd for $C_{19}H_{18}ClN_2O_3$: 357.1001; found: 357.0998

*Example Compound* 74

*2-oxo-2-(p-tolylamino)ethyl 2-(2-methyl-1H-indol-3-yl)acetate*

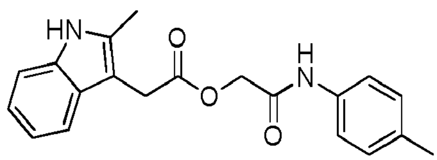

To a stirring solution of 2-(2-(2-methyl-1H-indol-3-yl)acetoxy)acetic acid (93 mg, 0.4 mmol, example 67, step 2) in dry DCM (4 mL) cooled at 0 °C, EDCI (83.2 mg, 0.4 mmol) was added slowly, and then p-toluidine (39.2 mg, 0.4 mmol). After 10 minutes, the mixture was allowed to warm to room temperature. After 4 h, the reaction mixture was

diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane). Yield: 78.5 mg (64%).

**TLC:** $R_f$ (30% EtOAc in PE): 0.20

**$^1$H NMR** (400 MHz, DMSO-$d_6$) δ: 10.84 (s, 1 H), 9.86 (s, 1 H), 7.38 (dd, *J* = 8.5, 1.9 Hz, 3H), 7.23 - 7.19 (m, 1 H), 7.11 - 7.06 (m, 2H), 6.96 (ddd, *J* = 8.0, 7.0, 1.3 Hz, 1H), 6.89 (ddd, *J* = 8.1, 7.0, 1.2 Hz, 1 H), 4.59 (s, 2H), 3.76 (s, 2H), 2.31 (s, 3H), 2.21 (s, 3H) ppm

**$^{13}$C NMR** (101 MHz, DMSO-$d_6$) δ: 171.2, 165.2, 135.9, 135.0, 133.4, 132.4, 129.1, 128.2, 120.1, 119.3, 118.4, 117.5, 110.4, 102.9, 62.7, 29.2, 20.4, 11.3 ppm

**HRMS** (ESI) m/z: $(M+H)^+$ calcd for $C_{20}H_{21}N_2O_3$: 337.1547; found: 337.1545

*Example Compound 75*

*2-oxo-2-(m-tolylamino)ethyl 2-(2-methyl-1H-indol-3-yl)acetate*

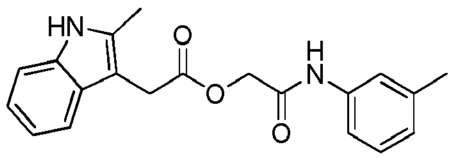

To a stirring solution of 2-(2-(2-methyl-1 H-indol-3-yl)acetoxy)acetic acid (92.2 mg, 0.4 mmol, example 67, step 2) in dry DCM (4 mL) cooled at 0 °C, EDCI (83.8 mg, 0.4 mmol) was added slowly, and then m-toluidine (39 μL, 0.4 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane). Yield: 74.4 mg (61%).

**TLC:** $R_f$ (20% EtOAc in PE): 0.16

**$^1$H NMR** (400 MHz, DMSO-$d_6$) δ: 10.86 (s, 1 H), 9.86 (s, 1 H), 7.45 - 7.40 (m, 1 H), 7.37 - 7.30 (m, 2H), 7.25 (dt, *J* = 8.0, 0.9 Hz, 1 H), 7.18 (t, *J* = 7.8 Hz, 1 H), 7.00 (ddd, *J* = 8.0, 7.0, 1.3 Hz, 1 H), 6.93 (ddd, *J* = 8.0, 7.1, 1.1 Hz, 1 H), 6.90 - 6.86 (m, 1 H), 4.63 (s, 2H), 3.80 (s, 2H), 2.35 (s, 3H), 2.27 (d, *J* = 0.8 Hz, 3H) ppm

**$^{13}$C NMR** (101 MHz, DMSO-$d_6$) δ: 171.1, 165.3, 138.2, 137.9, 135.0, 133.4, 128.5, 128.2, 124.2, 120.1, 119.8, 118.3, 117.5, 116.5, 110.4, 102.8, 62.6, 29.2, 21.1, 11.3 ppm

**HRMS** (ESI) m/z: $(M+H)^+$ calcd for $C_{20}H_{21}N_2O_3$: 337.1547; found: 337.1545

*Example Compound 76*

*2-((4-morpholinophenyl)amino)-2-oxoethyl 2-(2-methyl-1H-indol-3-yl)acetate*

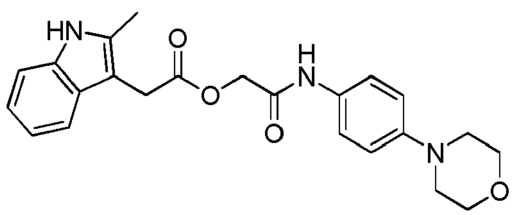

To a stirring solution of 2-(2-(2-methyl-1 H-indol-3-yl)acetoxy)acetic acid (104.6 mg, 0.4 mmol, example 67, step 2) in dry DCM (4 mL) cooled at 0 °C, EDCI (85.2 mg, 0.4 mmol) was added slowly, and then 4-morpholinoaniline (65.2 mg, 0.4 mmol). A dark-blue solution was observed, and after 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (50% EtOAc in hexane) and successively crystallized in EtOAc to give the pure product as a white powder (85.8 mg, 58%).

**TLC:** $R_f$ (50% EtOAc in PE): 0.56

**$^1$H NMR** (400 MHz, DMSO-$d_6$) δ: 10.87 (s, 1H), 9.78 (s, 1H), 7.44 - 7.37 (m, 3H), 7.24 (dt, $J$ = 8.0, 0.9 Hz, 1 H), 6.99 (ddd, $J$ = 8.0, 7.0, 1.3 Hz, 1 H), 6.95 - 6.87 (m, 3H), 4.60 (s, 2H), 3.79 (s, 2H), 3.76 - 3.70 (m, 4H), 3.09 - 2.98 (m, 4H), 2.34 (s, 3H) ppm

**$^{13}$C NMR** (101 MHz, DMSO-$d_6$) δ: 171.1, 164.7, 147.4, 135.0, 133.3, 130.5, 128.2, 120.5, 120.1, 118.3, 117.5, 115.3, 110.3, 102.9, 66.0, 62.6, 48.8, 29.2, 11.3 ppm

**HRMS** (ESI) m/z: $(M+H)^+$ calcd for $C_{23}H_{26}N_3O_4$: 408.1918; found: 408.1916

*Example Compound 77*

*2-((2-methoxyphenyl)amino)-2-oxoethyl 2-(2-methyl-1H-indol-3-yl)acetate*

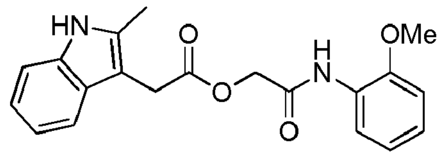

To a stirring solution of 2-(2-(2-methyl-1 H-indol-3-yl)acetoxy)acetic acid (103.1 mg, 0.4 mmol, example 67, step 2) in dry DCM (4 mL) cooled at 0 °C, EDCI (84.9 mg, 0.4 mmol) was added slowly, and then 2-methoxyaniline (42 µL, 0.4 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane) Yield: 110.6 mg (84%).

**TLC:** $R_f$ (40% EtOAc in PE): 0.41

**$^1$H NMR** (400 MHz, DMSO-$d_6$) δ: 10.85 (s, 1 H), 9.18 (s, 1 H), 7.97 - 7.87 (m, 1 H), 7.42 - 7.35 (m, 1 H), 7.21 (dt, $J$ = 8.0, 1.0 Hz, 1 H), 7.12 - 6.99 (m, 2H), 6.96 (ddd, $J$ = 8.0, 7.0, 1.3 Hz, 1 H), 6.92 - 6.83 (m, 2H), 4.67 (s, 2H), 3.79 (s, 3H), 3.76 (s, 2H), 2.32 (s, 3H) ppm

**$^{13}$C NMR** (101 MHz, DMSO-$d_6$) δ: 171.0, 165.5, 149.3, 135.0, 133.4, 128.2, 126.5, 124.6, 121.5, 120.3, 120.1, 118.4, 117.5, 111.1, 110.4, 102.8, 62.8, 55.7, 29.3, 11.3 ppm

**HRMS** (ESI) m/z: $(M+H)^+$ calcd for $C_{20}H_{21}N_2O_4$: 353.1496; found: 353.1494

*Example Compound 78*

*2-oxo-2-(o-tolylamino)ethyl 2-(2-methyl-1H-indol-3-yl)acetate*

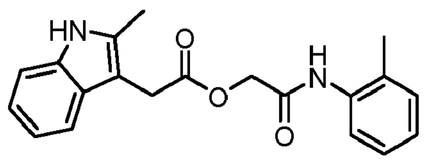

To a stirring solution of 2-(2-(2-methyl-1 H-indol-3-yl)acetoxy)acetic acid (90.2 mg, 0.4 mmol, example 67, step 2) in dry DCM (4 mL) cooled at 0 °C, EDCI (84.2 mg, 0.4 mmol) was added slowly, and then o-toluidine (39 µL, 0.4 mmol). After 10 minutes, the mixture was allowed to warm to room temperature. After completion, After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane) Yield: 69.9 mg (57%).

**TLC:** $R_f$ (30% EtOAc in PE): 0.59

**$^1$H NMR** (400 MHz, DMSO-$d_6$) δ: 10.87 (s, 1H), 9.40 (s, 1H), 7.44 - 7.39 (m, 1H), 7.38 - 7.33 (m, 1 H), 7.26 - 7.20 (m, 2H), 7.17 (td, $J$ = 7.3, 1.4 Hz, 1 H), 7.11 (td, $J$ = 7.4, 1.5 Hz, 1 H), 6.99 (ddd, $J$ = 8.1, 7.0, 1.3 Hz, 1 H), 6.92 (ddd, $J$ = 8.0, 7.1, 1.1 Hz, 1 H), 4.68 (s, 2H), 3.81 (s, 2H), 2.35 (s, 3H), 2.16 (s, 3H) ppm

**$^{13}$C NMR** (101 MHz, DMSO-$d_6$) δ: 171.2, 165.6, 135.5, 135.0, 133.4, 132.1, 130.3, 128.2, 126.0, 125.5, 125.2, 120.1, 118.3, 117.5, 110.4, 102.9, 62.6, 29.2, 17.7, 11.3 ppm

**HRMS** (ESI) m/z: $(M+H)^+$ calcd for $C_{20}H_{21}N_2O_3$: 337.1547; found: 337.1545

*Example Compound 79*

*2-((3-methoxyphenyl)amino)-2-oxoethyl 2-(2-methyl-1H-indol-3-yl)acetate*

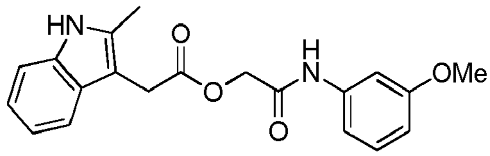

To a stirring solution of 2-(2-(2-methyl-1 H-indol-3-yl)acetoxy)acetic acid (92.7 mg, 0.4 mmol, example 67, step 2) in dry DCM (4 mL) cooled at 0 °C, EDCI (83.1 mg, 0.4 mmol) was added slowly, and then 3-methoxyaniline (41 μL, 0.4 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane) Yield: 84.8 mg (66%).

**TLC:** $R_f$ (30% EtOAc in PE): 0.27

**$^1$H NMR** (400 MHz, DMSO-$d_6$) δ: 10.88 (s, 1H), 10.01 (s, 1 H), 7.44 - 7.40 (m, 1 H), 7.27 - 7.18 (m, 3H), 7.11 - 7.05 (m, 1 H), 6.99 (ddd, *J* = 8.0, 7.1, 1.3 Hz, 1 H), 6.93 (ddd, *J* = 8.0, 7.1, 1.1 Hz, 1 H), 6.65 (ddd, *J* = 8.3, 2.5, 0.9 Hz, 1 H), 4.64 (s, 2H), 3.80 (s, 2H), 3.72 (s, 3H), 2.35 (s, 3H) ppm

**$^{13}$C NMR** (101 MHz, DMSO-$d_6$) δ: 171.2, 165.4, 159.5, 139.6, 135.0, 133.4, 129.6, 128.2, 120.1, 118.4, 117.5, 111.5, 110.4, 108.9, 105.1, 102.9, 62.7, 55.0, 29.2, 11.3 ppm

**HRMS** (ESI) m/z: $(M+H)^+$ calcd for $C_{20}H_{21}N_2O_4$: 353.1496; found: 353.1494

*Example Compound 80*

*2-((4-methoxyphenyl)amino)-2-oxoethyl 2-(2-methyl-1H-indol-3-yl)acetate*

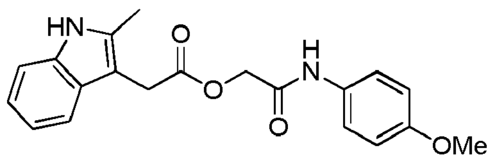

To a stirring solution of 2-(2-(2-methyl-1 H-indol-3-yl)acetoxy)acetic acid 2 (91.1 mg, 0.4 mmol, example 67, step 2) in dry DCM (4 mL) cooled at 0 °C, EDCI (83.1 mg, 0.4 mmol) was added slowly, and then 4-methoxyaniline (45.1 mg, 0.4 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (25% EtOAc in hexane) Yield: 88.5 mg (69%).

**TLC:** $R_f$ (30% EtOAc in PE): 0.25.

**$^1$H NMR** (400 MHz, DMSO-$d_6$) δ: 10.87 (s, 1H), 9.85 (s, 1H), 7.48 - 7.39 (m, 3H), 7.28 - 7.23 (m, 1 H), 6.99 (ddd, *J* = 8.0, 7.0, 1.3 Hz, 1 H), 6.93 (ddd, *J* = 7.6, 7.0, 1.2 Hz, 1 H), 6.91 - 6.86 (m, 2H), 4.62 (s, 2H), 3.80 (s, 2H), 3.72 (s, 3H), 2.35 (s, 3H) ppm

**$^{13}$C NMR** (101 MHz, DMSO-$d_6$) δ: 171.2, 164.9, 155.4, 135.0, 133.4, 131.5, 128.2, 120.9, 120.1, 118.4, 117.5, 113.9, 110.4, 102.9, 62.5, 55.2, 29.3, 11.3 ppm

**HRMS** (ESI) m/z: $(M+H)^+$ calcd for $C_{20}H_{21}N_2O_4$: 353.1496; found: 353.1494

*Example Compound 81*

*2-((3,5-dimethyl-1-phenyl-1H-pyrazol-4-yl)amino)-2-oxoethyl 2-(2-methyl-1H-indol-3-yl)propanoate*

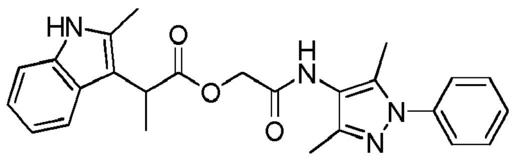

Step 1: 2-bromo-1-(2-methyl-1 H-indol-3-yl)propan-1-one

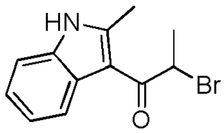

To a solution of 2-methylindole (2.5 g, 19.1 mmol) and pyridine (1.5 mL, 19.1 mmol) in toluene (250 mL) heated to 55 °C, 2-bromopropanoyl bromide (2.0 mL, 19.1 mmol) was added slowly over 1 hour. After an additional hour of stirring, the reaction was let cooling to room temperature and then poured into water (300 mL) cooled to 0 °C. Then the phases were separated, and the water phase was extracted 3 times with toluene. The reunited organic phase was dried over $MgSO_4$, filtered, concentrated and finally purified by column chromatography (30% EtOAc in PE) to obtain 4.0 g of the final product as a purple powder (79%).

Step 2: 2-(2-methyl-1H-indol-3-yl)propanoic acid

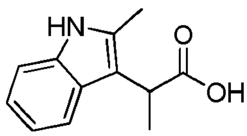

A solution of 4.8 M sodium hydroxide in 80% EtOH was carefully prepared and heated to 40 °C. Then the $\alpha$-bromoketone from step 1 (2.0 g, 7.5 mmol) was added in portions over 1 hour. After 4 h, the reaction was cooled to 0 °C and concentrated HCl was added to reach pH 7. The crude was concentrated and diluted with a saturated solution of $NaHCO_3$ and $Et_2O$. The aqueous phase was acidified with HCl and extracted 3 times with diethyl ether. The reunited organic phase was dried over $MgSO_4$, filtered, concentrated and finally purified by column chromatography (gradient of EtOAc in PE) to obtain 419.5 mg of the final product as a brown oil (27%).

Step 3: 2-((3,5-dimethyl-1-phenyl-1 H-pyrazol-4-yl)amino)-2-oxoethyl 2-(2-methyl-1 H-indol-3-yl)propanoate

To a stirring solution of the acid from step 2 (29.5 mg, 0.1 mmol) in DCM (3 mL), DCC (29.3 mg, 0.1 mmol) was added slowly, then DMAP (3.6 mg, 0.03 mmol) and finally N-(3,5-dimethyl-1-phenyl-1 H-pyrazol-4-yl)-2-hydroxyacetamide (35.0 mg, 0.1 mmol), synthesized as reported in Example 1. The crude suspension was filtered to remove the DCU precipitate and then concentrated at 40 °C. The product was purified by column chromatography (75% EtOAc in PE). 47.1 mg of the final product were obtained (77%).

**TLC:** $R_f$ (5% MeOH in DCM): 0.30 **$^1$H NMR** (400 MHz, DMSO-$d_6$) $\delta$: 10.86 (s, 1H), 9.14 (s, 1H), 7.55 - 7.43 (m, 6H), 7.38 (ddt, *J* = 6.9, 5.4, 2.5 Hz, 1 H), 7.28 - 7.20 (m, 1 H), 6.97 (ddd, *J* = 8.1, 7.0, 1.3 Hz, 1 H), 6.90 (ddd, *J* = 8.0, 7.0, 1.2 Hz, 1H), 4.69 (d, *J* = 14.6 Hz, 1 H), 4.53 (d, *J* = 14.6 Hz, 1 H), 2.37 (s, 3H), 2.11 (s, 3H), 2.03 (s, 3H), 1.49 (d, *J* = 7.2 Hz, 3H) ppm

**$^{13}$C NMR** (101 MHz, DMSO-$d_6$) $\delta$: 174.1, 166.3, 144.7, 139.6, 135.1, 134.3, 132.2, 129.2, 127.0, 126.8, 123.7,

120.0, 118.3, 118.0, 116.7, 110.6, 109.0, 62.6, 35.4, 17.3, 11.5, 11.3, 10.6 ppm

**HRMS** (ESI) m/z: $(M+H)^+$ calcd for $C_{25}H_{27}N_4O_3$: 431.2078; found: 431.2076

*Example Compound 82*

*2-((3,5-dimethyl-1-phenyl-1H-pyrazol-4-yl)amino)-2-oxoethyl 2-(2-azidophenyl)acetate*

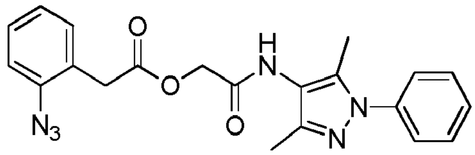

Step 1: potassium 2-(2-aminophenyl)acetate

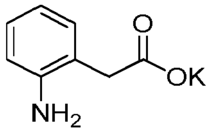

2-(2-nitrophenyl)acetic acid (1.0 g, 5.5 mmol) was dissolved in EtOH (15 mL) and then $K_2CO_3$ (763 mg, 5.5 mmol) and 10% Pd/C (101 mg, 10% p/p) were added. The reaction mixture was left stirring overnight under an atmosphere of $H_2$. The suspension was filtered with EtOH over celite, to afford quantitative yield of the title compound, which was used in the next step without further purification (1.1 g)

Step 2: 2-(2-azidophenyl)acetic acid

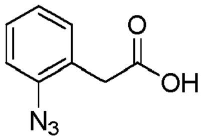

The salt from step 1 (250.7 mg, 1.3 mmol) was dissolved in water (1.8 mL) and cooled to 0 °C. Then, concentrated $H_2SO_4$ (360 μL, 6.8 mmol) and successively a solution of sodium nitrite (95.7 mg, 1.4 mmol) in water (480 μL) were added. After 20 minutes at 0 °C, a solution of sodium azide (90.3 mg, 1.4 mmol) in water (480 μL) was also added and the reaction looked completed by TLC after 15 min. The crude was diluted with water and extracted 3 times with EtOAc, and the reunited organic phase was dried over $MgSO_4$, filtered and concentrated to give 200.8 mg of the product, which was used as is in the next step.

Step 3: 2-((3,5-dimethyl-1-phenyl-1H-pyrazol-4-yl)amino)-2-oxoethyl 2-(2-azidophenyl)acetate

To a stirring solution of the acid from step 2 (32.7 mg, 0.2 mmol) in DCM (3.8 mL), DCC (37.3 mg, 0.2 mmol) was added slowly, then DMAP (4.5 mg, 0.04 mmol) and finally N-(3,5-dimethyl-1-phenyl-1H-pyrazol-4-yl)-2-hydroxyacetamide (44.3 mg, 0.2 mmol), synthesized as reported in Example 1. After 4 h, the crude suspension was filtered to remove the DCU precipitate and then concentrated *in vacuo*. The product was purified by column chromatography (75% EtOAc in PE). 55.9 mg of the final product were obtained (76%).

**TLC:** $R_f$ (5% MeOH in DCM): 0.32

**$^1$H NMR** (400 MHz, DMSO-$d_6$) δ: 9.32 (s, 1 H), 7.55 - 7.47 (m, 4H), 7.42 - 7.30 (m, 4H), 7.17 (td, $J$ = 7.5, 1.3 Hz, 1 H), 4.71 (s, 2H), 3.78 (s, 2H), 2.14 (s, 3H), 2.06 (s, 3H) ppm

**$^{13}$C NMR** (101 MHz, DMSO-$d_6$) δ: 170.3, 166.1, 144.7, 139.6, 138.2, 134.3, 131.8, 129.2, 128.9, 127.0, 125.6,

124.9, 123.7, 118.5, 116.8, 62.8, 35.6, 11.3, 10.6 ppm

**HRMS** (ESI) m/z: $(M+H)^+$ calcd for $C_{21}H_{21}N_6O_3$: 405.1670; found: 405.1669

*Example Compound 83*

*2-((3,5-dimethyl-1-phenyl-1H-pyrazol-4-yl)amino)-2-oxoethyl 2-(1H-indol-3-yl)propanoate*

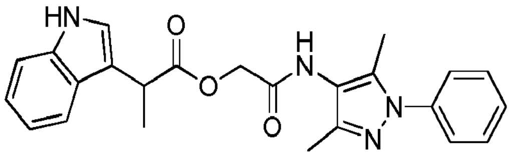

Step 1: methyl 3-(2-methoxy-2-oxoethyl)-1H-indole-1-carboxylate

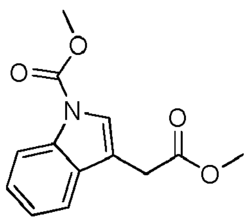

Methyl 2-(1H-indol-3-yl)acetate (1.1 g, 5.8 mmol) and tetrabutylammonium iodide (15.8 mg, 0.04 mmol) were dissolved in a mixture of 30% NaOH and DCM 1:1 (35.2 mL), which was cooled to - 4 °C to add methylchloroformate (670 μL, 8.7 mmol). After 15 min, the reaction was left stirring for 2.5h at 0 °C. The phases were separated and the aqueous phase was extracted 3 times with DCM. The reunited organic phase was washed with brine, dried over $MgSO_4$, filtered and concentrated to give the title compound in quantitative yield (1.48 g).

Step 2: methyl 3-(1-methoxy-1-oxopropan-2-yl)-1H-indole-1-carboxylate

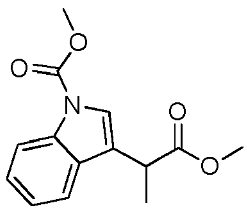

A solution of the carbamate from step 1 (1.48g, 5.8 mmol) in dry THF (8 mL) under Ar was cooled to -78 °C, and added of freshly prepared LDA (12.7 mL, 0.685 M). After 1 h, a solution of iodomethane (540.4 μL, 8.7 mmol) in dry THF (5 mL) was added, and the mixture was left stirring at -78 °C for about an hour, after which the reaction was quenched at room temperature with a saturated solution of $NH_4Cl$. The aqueous phase was extracted 3 times with EtOAc, and the reunited organic phase was dried over $MgSO_4$, filtered and concentrated. 501.6 mg (33%) of the product was obtained after column chromatography (25% EtOAc in PE).

Step 3: 2-(1 H-indol-3-yl)propanoic acid

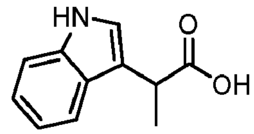

To a stirring solution of the ester from step 2 (489.3 mg, 1.9 mmol) in MeOH (20 mL), aqueous potassium hydroxide (13 mL, 11.3 mmol) was added dropwise. The reaction was stirred for 1.5h at 70 °C, after which the crude was concentrated and acidified with 1 M HCl to pH = 1. A suspension was observed, which was filtered. The filtrate was additionally extracted 3 times with EtOAc, and the organic phase was dried over $MgSO_4$, filtered and concentrated. The precipitate and the organic phase contained the title compound in quantitative yield.

Step 4: 2-((3,5-dimethyl-1-phenyl-1H-pyrazol-4-yl)amino)-2-oxoethyl 2-(1 H-indol-3-yl)propanoate

To a stirring solution of the acid from step 3 (33.8 mg, 0.2 mmol) in DCM (3 mL), DCC (36.2 mg, 0.2 mmol) was added slowly, then DMAP (3.2 mg, 0.03 mmol) and finally N-(3,5-dimethyl-1-phenyl-1 H-pyrazol-4-yl)-2-hydroxyacetamide (42.8 mg, 0.2 mmol), synthesized as reported in Example 1. After 6h the reaction looked complete by TLC, and the crude suspension was filtered to remove the DCU precipitate and then concentrated at 40 °C. The product was purified by column chromatography (75% EtOAc in PE). 70.5 mg of the final product were obtained (95%).

**TLC:** $R_f$ (5% MeOH in DCM): 0.26

**$^1$H** NMR (400 MHz, DMSO-$d_6$) δ: 11.06 - 10.88 (m, 1 H), 9.23 (s, 1 H), 7.63 - 7.57 (m, 1 H), 7.54 - 7.47 (m, 4H), 7.43 - 7.32 (m, 2H), 7.32 - 7.27 (m, 1H), 7.07 (ddd, *J* = 8.2, 7.0, 1.2 Hz, 1H), 6.98 (ddd, *J* = 8.0, 7.0, 1.1 Hz, 1 H), 4.71 (d, *J* = 14.6 Hz, 1 H), 4.62 (d, *J* = 14.6 Hz, 1 H), 4.20 - 4.10 (m, 1 H), 2.13 (s, 3H), 2.05 (s, 3H), 1.55 (d, *J* = 7.2 Hz, 3H) ppm

**$^{13}$C NMR** (101 MHz, DMSO-$d_6$) δ: 174.1, 166.2, 144.7, 139.6, 136.2, 134.3, 129.2, 127.0, 126.1, 123.7, 122.7, 121.1, 118.7, 118.5, 116.8, 113.5, 111.5, 62.6, 36.1, 17.8, 11.3, 10.6 ppm

**HRMS** (ESI) m/z: $(M+H)^+$ calcd for $C_{24}H_{25}N_4O_3$: 417.1921; found: 417.1922

*Example Compound 84*

*2-((3-carbamoylphenyl)amino)-2-oxoethyl 2-(2-methyl-1H-indol-3-yl)acetate*

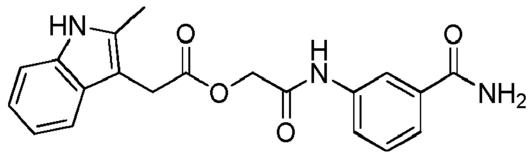

To a stirring solution of 2-(2-(2-methyl-1 H-indol-3-yl)acetoxy)acetic acid (103.5 mg, 0.4 mmol, example 67, step 2) in dry DCM (4 mL) cooled at 0 °C, EDCI (85.4 mg, 0.4 mmol) was added slowly, and then 3-aminobenzamide (50.5 mg, 0.4 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (5% MeOH in DCM) and successively crystallized in EtOAc/Toluene to give the pure product as a white powder (52.3 mg, 39%).

**TLC:** $R_f$ (5% MeOH in DCM): 0.24

**$^1$H NMR** (400 MHz, DMSO-$d_6$) δ: 10.88 (s, 1H), 10.17 (s, 1H), 8.03 (s, 0H), 7.94 (s, 1H), 7.59-7.53 (m, 1 H), 7.45 - 7.32 (m, 3H), 7.29 - 7.20 (m, 2H), 7.20 - 7.11 (m, 1 H), 6.99 (ddd, *J* = 8.0, 7.0, 1.3 Hz, 1 H), 6.93 (ddd, *J* = 8.1, 7.1, 1.2 Hz, 1 H), 4.66 (s, 2H), 3.81 (s, 2H), 2.35 (s, 3H) ppm

**$^{13}$C NMR** (101 MHz, DMSO-$d_6$) δ: 171.2, 167.7, 165.6, 138.4, 135.0, 133.4, 128.9, 128.6, 128.2, 122.3, 122.0, 120.1, 118.9, 118.4, 117.5, 110.4, 102.9, 62.7, 29.2, 11.3 ppm

**HRMS** (ESI) m/z: $(M+H)^+$ calcd for $C_{20}H_{20}N_3O_4$: 366.1448; found: 366.1442

*Example Compound 85*

*2-(((1H-imidazol-2-yl)methyl)amino)-2-oxoethyl 2-(2-methyl-1H-indol-3-yl)acetate*

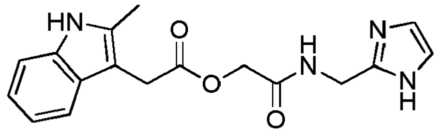

To a stirring solution of 2-(2-(2-methyl-1 H-indol-3-yl)acetoxy)acetic acid (148.5 mg, 0.6 mmol, example 67, step 2) in dry DCM (6 mL) cooled at 0 °C, EDCI (126.7 mg, 0.7 mmol) was added slowly, then HOBt (82.1 mg, 0.6 mmol), then N-((1H-imidazol-2-yl)methyl)-λ5-chloranimine (92.9 mg, 0.5 mmol) and finally dry DIPEA (110 μL, 0.6 mmol). After 10 minutes, the mixture was allowed to warm to room temperature. After 4 h, the reaction mixture was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified twice by column chromatography (5% MeOH in DCM) and successively crystallized in EtOAc/Toluene to give the pure product as a white powder (63.7 mg, 36%).

**TLC:** $R_f$ (30% EtOAc in PE): 0.20

**$^1$H NMR** (400 MHz, DMSO-$d_6$) δ: 11.78 (s, 1H), 10.86 (s, 1 H), 8.52 - 8.44 (m, 1 H), 7.39 (d, *J* = 7.7 Hz, 1 H), 7.24 (d, *J* = 7.9 Hz, 1 H), 6.95 (dt, *J* = 26.0, 7.3 Hz, 3H), 4.50 (s, 2H), 4.31 (d, *J* = 5.4 Hz, 2H), 3.77 (s, 2H), 2.33 (s, 4H) ppm

**$^{13}$C NMR** (101 MHz, DMSO-$d_6$) δ: 171.1, 166.8, 144.4, 135.0, 133.4, 128.9, 128.2, 125.3, 120.1, 118.4, 117.6, 110.4, 102.9, 62.3, 36.2, 29.3, 11.3 ppm

**HRMS** (ESI) m/z: $(M+H)^+$ calcd for $C_{17}H_{19}N_4O_3$: 327.1452; found: 327.1452

*Example Compound 86*

*2-oxo-2-(pyrimidin-5-ylamino)ethyl 2-(2-methyl-1H-indol-3-yl)acetate*

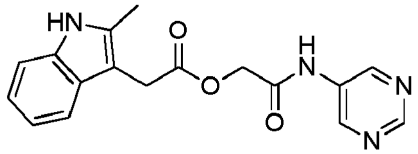

To a stirring solution of 2-(2-(2-methyl-1 H-indol-3-yl)acetoxy)acetic acid (81.8 mg, 0.3 mmol, example 67, step 2) in dry DCM (3 mL) cooled at 0 °C, DCC (68.9 mg, 0.3 mmol) was added slowly, then DMAP (9.6 mg, 0.1 mmol) and finally 5-aminopyrimidine (30 mg, 0.3 mmol). After 10 minutes, the mixture was allowed to warm to room temperature. After 3h the reaction looked complete by TLC, and the crude suspension was filtered to remove the DCU precipitate and then concentrated at 40 °C. The product was purified by column chromatography (75% EtOAc in hexane). 52.1 g of the final product were obtained (51%).

**TLC:** $R_f$ (75% EtOAc in PE): 0.25

**$^1$H NMR** (400 MHz, DMSO-$d_6$) δ: 10.88 (s, 1H), 10.53 (s, 1H), 8.97 (s, 2H), 8.91 (s, 1H), 7.46 - 7.36 (m, 1 H), 7.24 (dt, *J* = 8.0, 0.9 Hz, 1 H), 6.99 (ddd, *J* = 8.1, 7.0, 1.3 Hz, 1H), 6.93 (ddd, *J* = 8.0, 7.0, 1.1 Hz, 1 H), 4.72 (s, 2H), 3.81 (s, 2H), 2.35 (s, 3H) ppm

**$^{13}$C NMR** (101 MHz, DMSO-$d_6$) δ: 171.2, 166.6, 153.4, 147.3, 135.0, 133.8, 133.5, 128.2, 120.2, 118.4, 117.5, 110.4, 102.8, 62.5, 29.2, 11.3 ppm

**HRMS** (ESI) m/z: $(M+H)^+$ calcd for $C_{17}H_{17}N_4O_3$: 325.1295; found: 325.1299

*Example Compound 87*

*2-((3,5-dimethyl-1-phenyl-1H-pyrazol-4-yl)amino)-2-oxoethyl 3-(2-methyl-1H-indol-3-yl)propanoate*

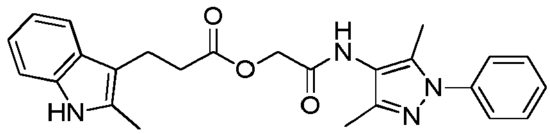

Step 1: 3-(2-methyl-1 H-indol-3-yl)propanoic acid

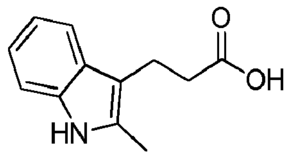

To a stirring solution of 5-oxohexanoic acid (735 μL, 6.2 mmol) in AcOH (15 mL), was added phenylhydrazine (605 μL, 6.1 mmol), zinc chloride (1.6 g, 11.6 mmol) and then heated to 70 °C. The crude mixture was concentrated and diluted with EtOAc. The organic phase was washed four times with water, and once with brine. Finally, it was dried over $MgSO_4$, filtered and concentrated to give the crude product, which was purified by column chromatography (40% EtOAc in PE). Yield: 870.7 mg (70%)

Step 2: 2-((3,5-dimethyl-1-phenyl-1H-pyrazol-4-yl)amino)-2-oxoethyl 3-(2-methyl-1H-indol-3-yl)propanoate

To a stirring solution of the acid from step 1 (50 mg, 0.2 mmol) in DCM (3 mL), DCC (51.3 mg, 0.2 mmol) was added slowly, then DMAP (5 mg, 0.04 mmol) and finally N-(3,5-dimethyl-1-phenyl-1H-pyrazol-4-yl)-2-hydroxyacetamide (49.2 mg, 0.2 mmol), synthesized as reported in Example 1. The reaction was left stirring overnight. The crude suspension was filtered to remove the DCU precipitate and then concentrated at 40 °C. The product was purified by column chromatography (50% and then 75% EtOAc in hexane). 74.4 mg of the final product were obtained (86%).

**TLC:** $R_f$ (75% EtOAc in PE): 0.11

**$^1$H NMR** (400 MHz, DMSO-$d_6$) δ: 10.70 (s, 1H), 9.24 (s, 1H), 7.56 - 7.47 (m, 4H), 7.44 - 7.34 (m, 2H), 7.25 - 7.20 (m, 1 H), 7.00 - 6.95 (m, 1 H), 6.92 (ddd, *J* = 7.6, 7.0, 1.2 Hz, 1 H), 4.64 (s, 2H), 2.95 (t, *J* = 7.3 Hz, 2H), 2.69 (d, *J* = 7.6 Hz, 2H), 2.33 (s, 3H), 2.13 (s, 3H), 2.05 (s, 3H) ppm

**$^{13}$C NMR** (101 MHz, DMSO-$d_6$) δ: 172.3, 166.4, 144.8, 139.6, 135.2, 134.4, 131.8, 129.2, 127.8, 127.0, 123.7, 119.9, 118.1, 117.2, 116.8, 110.4, 108.5, 62.4, 34.6, 19.2, 11.3, 11.2, 10.6 ppm

**HRMS** (ESI) m/z: $(M+H)^+$ calcd for $C_{25}H_{27}N_4O_3$: 431.2078; found: 431.2081

*Example Compound 88*

*2-((3,5-dimethyl-1-phenyl-1H-pyrazol-4-yl)(methyl)amino)-2-oxoethyl 2-(2-methyl-1H-indol-3-yl)acetate*

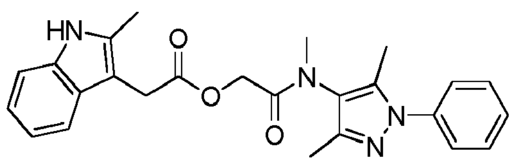

Step 1: N-(3,5-dimethyl-1-phenyl-1 H-pyrazol-4-yl)-2-hydroxy-N-methylacetamide

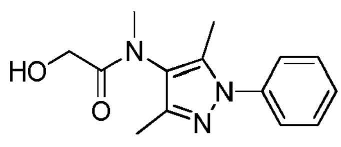

In an oven-dried schlenk flask under Ar, N-(3,5-dimethyl-1-phenyl-1H-pyrazol-4-yl)-2-hydroxyacetamide (299.9 mg, 1.2 mmol), synthesized as reported in Example 1, was dissolved in dry DMF (4 mL) and then sodium hydride (55.1 mg, 1.4 mmol, 60% dispersion in mineral oil) was added. Bubbles were observed for about 30 min, then iodomethane (83.8 μL, 1.3 mmol) was added to the mixture, which was left stirring for about 3 h. The crude was diluted in EtOAc and washed with a saturated solution of $NH_4Cl$. The aqueous solution was extracted 3 times with EtOAc and the reunited organic phase was dried over $MgSO_4$, filtered and concentrated. The crude was used in the following step without further purification.

Step 2: 2-((3,5-dimethyl-1-phenyl-1H-pyrazol-4-yl)(methyl)amino)-2-oxoethyl 2-(2-methyl-1H-indol-3-yl)acetate

To a stirring solution of 2-(2-methyl-1 H-indol-3-yl)acetic acid (116.3 mg, 0.6 mmol, example 67, step 2) in DCM (6 mL), DCC (104.8 mg, 0.5 mmol) was added slowly, then DMAP (12.3 mg, 0.1 mmol) and finally the alcohol from step 1 (130.3 mg, 0.5 mmol). The crude suspension was filtered to remove the DCU precipitate and then concentrated *in vacuo.* The product was purified by column chromatography (75% EtOAc in hexane). 145 mg of the final product were obtained (67%).

**TLC:** $R_f$ (3% MeOH in DCM): 0.32

**$^1$H NMR** (600 MHz, DMSO-$d_6$) δ: 10.84 (s, 1 H), 7.53 - 7.48 (m, 4H), 7.44 - 7.36 (m, 2H), 7.26 - 7.20 (m, 1 H), 6.98 (ddd, *J* = 8.1, 7.0, 1.2 Hz, 1 H), 6.92 (ddd, *J* = 8.0, 7.0, 1.1 Hz, 1 H), 4.43 - 4.31 (m, 2H), 3.72 (s, 2H), 3.07 (s, 3H), 2.33 (s, 3H), 2.20 (s, 3H), 2.14 (s, 3H) ppm

**$^{13}$C NMR** (151 MHz, DMSO-$d_6$) δ: 171.1, 167.0, 144.7, 139.3, 136.2, 134.9, 133.3, 129.1, 128.2, 127.4, 124.0, 121.3, 120.0, 118.3, 117.4, 110.3, 102.9, 61.3, 35.7, 29.2, 11.2, 10.6, 9.9 ppm

**HRMS** (ESI) m/z: $(M+H)^+$ calcd for $C_{25}H_{27}N_4O_3$: 431.2078; found: 431.2081

*Example Compound 89*

*2-((3,5-dimethyl-1-phenyl-1H-pyrazol-4-yl)amino)-2-oxoethyl 2-(1,2-dimethyl-1H-indol-3-yl)acetate*

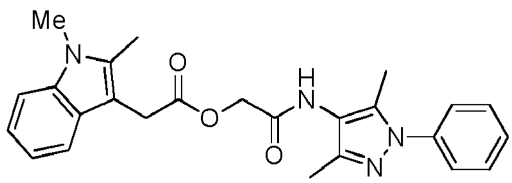

Step 1: 2-(1,2-dimethyl-1 H-indol-3-yl)acetic acid

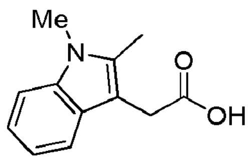

A stirring solution of 2-(2-methyl-1 H-indol-3-yl)acetic acid (252.6 mg, 1.3 mmol, example 67, step 2) in dry THF (15 mL) cooled at 0 °C, was added of sodium hydride (158.5 mg, 4.0 mmol, 60% dispersion in mineral oil), and left stirring for about 30 minutes. After iodomethane (275 μl; 4:4 mmol) was added, the mixture was allowed to warm to room temperature and left stirring overnight. The reaction was quenched with MeOH at 0 °C, diuted with water and extracted with $Et_2O$. The acqueous phase was acidified to pH 1-2 and extracted three times with DCM. The reunited organic phase was dried over $MgSO_4$, filtered and concentrated to give the crude product, which was purified by column chromatography (40% EtOAc in PE). Yield: 166.4 mg (61%)

Step 2: 2-((3,5-dimethyl-1-phenyl-1H-pyrazol-4-yl)amino)-2-oxoethyl 2-(1,2-dimethyl-1H-indol-3-yl)acetate

To a stirring solution of the acid from step 1 (133.12 mg, 0.7 mmol) in DCM (8 mL) cooled to 0 °C, DCC (173.9 mg, 0.8 mmol) was added slowly, then DMAP (25.6 mg, 0.2 mmol) and finally N-(3,5-dimethyl-1-phenyl-1 H-pyrazol-4-yl)-2-hydroxyacetamide (202.4 mg, 0.8 mmol), synthesized as reported in Example 1. The crude suspension was filtered to remove the DCU precipitate and then concentrated at 40 °C. The product was purified by column chromatography (3% MeOH in DCM). 108.5 mg of the final product were obtained (38%).

**TLC:** $R_f$ (3% MeOH in DCM): 0.33

**$^1$H NMR** (400 MHz, DMSO-$d_6$) δ: 9.27 (s, 1 H), 7.52 - 7.49 (m, 4H), 7.48 - 7.45 (m, 1 H), 7.41 - 7.34 (m, 2H), 7.07 (ddd, $J$ = 8.2, 7.0, 1.2 Hz, 1 H), 6.97 (ddd, $J$ = 8.0, 7.1, 1.0 Hz, 1 H), 4.66 (s, 2H), 3.87 (s, 2H), 3.66 (s, 3H), 2.37 (s, 3H), 2.13 (s, 3H), 2.05 (s, 3H) ppm

**$^{13}$C NMR** (101 MHz, DMSO-$d_6$) δ: 171.2, 166.3, 144.8, 139.6, 136.1, 134.9, 134.4, 129.2, 127.3, 127.0, 123.7, 120.3, 118.6, 117.7, 116.8, 109.0, 102.9, 62.6, 29.4, 29.4, 11.3, 10.6, 10.0 ppm

**HRMS** (ESI) m/z: $(M+H)^+$ calcd for $C_{25}H_{27}N_4O_3$: 431.2078; found: 431.2086

*Example Compound 90*

*2-((3,5-dimethyl-1-phenyl-1H-pyrazol-4-yl)amino)-2-oxoethyl 2-(2-methylpyridin-3-yl)acetate*

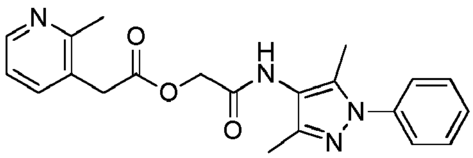

Step 1: ethyl 2-(2-methylpyridin-3-yl)acetate

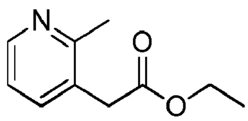

To an oven-dried schlenk flask under argon atmosphere, 3-bromo-2-methylpyridine (670 μL, 5.8 mmol), $Pd_2(dba)_3$ (14 mg, 0.5 mol%), tri-tert-butylphosphonium tetrafluoroborate (20.8 mg, 0.11 mol%), $K_2CO_3$ (1.2 g, 8.8 mmol), potassium bicarbonate (896.8 mg, 8.8 mmol), were added sequentially and finally ethyl malonate (6 mL, 39.5 mmol) The mixture was heated to 160 °C and left stirring overnight. The crude mixture was diluted with EtOAc and washed with water, saturated solution of $NaHCO_3$ and brine. Finally, the organic phase was dried over $MgSO_4$, filtered and concentrated to give the crude product, which was purified by column chromatography (30% EtOAc in PE). Yield: 329.1 mg (32%)

Step 2: 2-(2-methylpyridin-3-yl)acetic acid

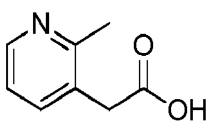

To a stirring solution of the ester from step 1 (328 mg, 1.8 mmol) in EtOH (20 mL), 2M aqueous sodium hydroxide (5 mL, 10 mmol) was added dropwise. The reaction was stirred for 3h at 80 °C, after which the crude was concentrated and then triturated with EtOH. Yield: 266 mg (96%)

Step 3: benzyl 2-(2-(2-methylpyridin-3-yl)acetoxy)acetate

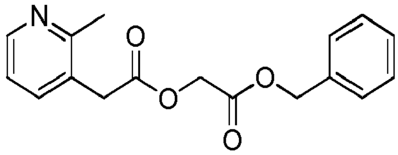

To a stirring solution of the acid from step 2 (150 mg, 1.0 mmol) in DCM (3 mL), DCC (206.5 mg, 1.0 mmol) was added slowly, then DMAP (26.9 mg, 0.2 mmol) and finally the benzyl 2-hydroxyacetate (140.8 μL, 1.0 mmol). The mixture was left stirring overnight. The crude suspension was filtered to remove the DCU precipitate and then concentrated *in vacuo.* The product was purified by column chromatography (60% EtOAc in PE). 75.3 mg of the title compound was obtained (25%).

Step 4: 2-(2-(2-methylpyridin-3-yl)acetoxy)acetic acid

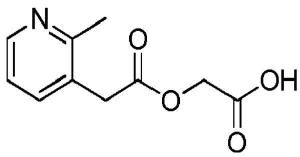

To a stirring solution of the ester from step 3 (74 mg, 0.25 mmol) in EtOAc (3 mL), a spatula tip of palladium on carbon 10% was added, and then the mixture was stirred under 1 $H_2$ atmosphere. After reaction completion, the crude was filtered through celite and concentrated to give 39.7 mg of the title compound (77%).

Step 5: 2-((3,5-dimethyl-1-phenyl-1H-pyrazol-4-yl)amino)-2-oxoethyl 2-(2-methylpyridin-3-yl)acetate

To a stirring solution of the acid from step 4 (39.7 mg, 0.2 mmol) in DCM (3 mL), EDCI (48.6 mg, 0.3 mmol) was added slowly, then 3,5-dimethyl-1-phenyl-1H-pyrazol-4-amine chlorohydrate (42.4 mg, 0.2 mmol) synthesized as reported in Example 1, and finally DIPEA, (39.6 μL, 0.2 mmol). The reaction was left stirring overnight. The crude was diluted with EtOAc and washed with a saturated solution of $NH_4Cl$, then $NaHCO_3$ (saturated solution) and finally with brine. The separated organic layer was dried over $MgSO_4$, filtered and concentrated to give the crude product, which was purified by column chromatography (5% MeOH in DCM). 36.4 mg of the final product were obtained (51%).

**TLC:** $R_f$ (5% MeOH in DCM): 0.24

**$^1$H NMR** (400 MHz, DMSO-$d_6$) δ: 9.35 (s, 1 H), 8.35 (dd, *J* = 4.8, 1.7 Hz, 1H), 7.63 (dd, *J* = 7.7, 1.5 Hz, 1H), 7.52 - 7.48 (m, 4H), 7.41 - 7.35 (m, 1H), 7.19 (ddd, *J* = 7.7, 4.9, 0.7 Hz, 1 H), 4.73 (s, 2H), 3.89 (s, 2H), 2.45 (s, 3H), 2.13 (s, 3H), 2.05 (s, 3H) ppm

**$^{13}$C NMR** (101 MHz, DMSO-$d_6$) δ: 170.4, 166.1, 157.3, 147.5, 144.7, 139.6, 137.9, 134.3, 129.2, 128.3, 127.0, 123.7, 121.3, 116.7, 62.8, 37.4, 22.2, 11.3, 10.6 ppm

HRMS (ESI) m/z: $(M+H)^+$ calcd for $C_{21}H_{23}N_4O_3$: 379.1765; found: 379.1767

*Example Compound 91*

*3,5-dimethyl-1-phenyl-4-(2-((3,3,3-trifluoro-2-(2-methyl-1H-indol-3-yl)propanoyl)oxy)acetamido)-1H-pyrazol-1-ium 2,2,2-trifluoroacetate*

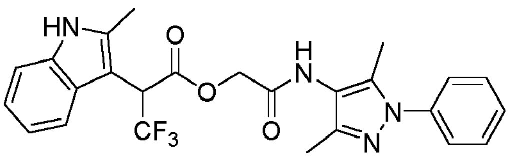

Step 1: 3,3,3-trifluoro-2-(2-methyl-1 H-indol-3-yl)propanoic acid

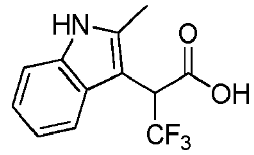

A mixture of concentrated HCl (850 μL) and acetic acid (5 mL) was prepared at 0 °C and added of methyl 3,3,3-trifluoro-2-(2-methyl-1H-indol-3-yl)propanoate (150 mg, 0.55 mmol, cf. Tsyshchuk et al. *Eur. J. Org. Chem.* **2014,** *2014,* 2480.). The reaction mixture was left stirring under vigorous reflux overnight, then concentrated to remove the AcOH excess and diluted with EtOAc. The organic phase was extracted 3 times with water, then washed with brine, dried over $MgSO_4$, filtered and concentrated to give a quantitative yield of crude product, which was used for the following step without further purification.

Step 2: 3,5-dimethyl-1-phenyl-4-(2-((3,3,3-trifluoro-2-(2-methyl-1H-indol-3-yl)propanoyl)oxy)acetamido)-1H-pyrazol-1-ium2,2,2-trifluoroacetate

To a stirring solution of the acid from step 1 (146.1 mg, 0.6 mmol) in dry DCM (8 mL), DCC (123.7 mg, 0.6 mmol) was added slowly, then a spatula tip of DMAP and finally N-(3,5-dimethyl-1-phenyl-1H-pyrazol-4-yl)-2-hydroxyacetamide (92 mg, 0.4 mmol), synthesized as reported in Example 1. The crude suspension was stirred overnight and then filtered to remove the DCU precipitate. The product was first purified by column chromatography (75% EtOAc in hexane) and then by HPLC to yield the title compound as its trifluoroacetate salt (34%)

**TLC:** $R_f$ (5% MeOH in DCM): 0.25

**$^1$H NMR** (600 MHz, $CD_3OD$) δ: 7.55 - 7.51 (m, 2H), 7.51 - 7.48 (m, 1H), 7.47 - 7.45 (m, 1H), 7.45 - 7.42 (m, 2H), 7.26 (dt, *J* = 8.1, 0.9 Hz, 1 H), 7.03 (ddd, *J* = 8.2, 7.1, 1.2 Hz, 1 H), 6.97 (ddd, *J* = 8.1, 7.1, 1.1 Hz, 1 H), 5.09 (q, *J* = 9.1 Hz, 1 H), 4.83 (d, *J* = 14.9 Hz, 1 H), 4.70 (d, *J* = 14.9 Hz, 1 H), 2.47 (s, 3H), 2.07 (s, 3H), 2.05 (s, 3H) ppm

**$^{13}$C NMR** (151 MHz, $CD_3OD$) 169.2, 168.0, 146.6, 140.6, 137.7, 137.3, 136.9, 130.4, 129.4, 128.3, 126.3 (q, *J* = 278.7 Hz), 126.3, 122.2, 120.6, 119.1, 116.7, 111.8, 100.0, 64.2, 47.6 (q, *J* = 30.2 Hz), 11.5, 11.0, 10.5 ppm

**$^{19}$F NMR** (376 MHz, $CD_3OD$) δ: -71.85 ppm

**HRMS** (ESI) m/z: $(M+H)^+$ calcd for $C_{25}H_{24}F_3N_4O_3$: 485.1795; found: 485.1801

**3) Stability Assay**

The stability of the synthesized Example Compounds 1, 38 and 81 in cell culture medium has been investigated.

*Procedure:*

In order to measure the stability of the synthesized Example Compounds in cell culture medium, an NMR-based assay has been developed. A solution of the Example Compound (~8mM) in DMSO-d6 is diluted 1:3 with the cell buffer (i.e. DMEM (Dulbecco's Modified Eagle Medium) + 10% Fetal Calf Serum (FCS), + 1% Penicillin/Streptomycin, + 1mM c-Glutamine), and observed by NMR at 37°C for a total of 16 hours. The stability of the compounds can be conveniently monitored by comparing the integration of the starting material peak and the corresponding appearing hydrolysis product peak. Also, the absorbance of the starting material peak has been used as a double check. The expected hydrolysis reaction of the ester moiety is reported below for Example Compound 1, and is the same for all compounds:

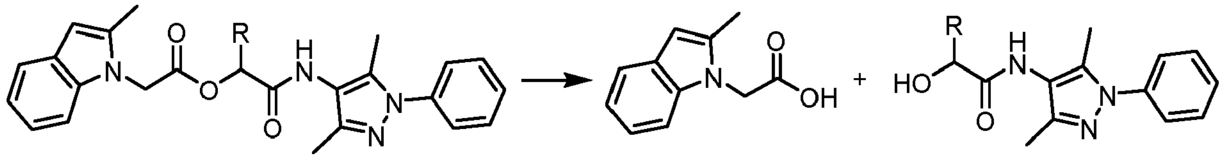

**Example 1** **Acid** **Alcohol**

The results are depicted in Figures 1, 2 and 3. As shown by the results depicted in Figures 1, 2 and 3, the stability of the compounds was totally enhanced from Example Compound 1 to Example Compounds bearing the 3-substituted indole, such as Example Compound 38. Moreover, the presence of a methyl group in α-position to the ester (i.e. Example Compound 81) supplies further stabilizing properties, although such a modification results in loss of inhibitory potency.

It will be clear that the invention may be practiced otherwise than as particularly described in the foregoing description and examples. Numerous modifications and variations of the present invention are possible in light of the above teachings and, therefore, are within the scope of the appended claims.

## Claims

1. A compound for use in the treatment of a disease associated with kallikrein-like peptidase 6 overexpression, said compound having a structure according to Formula I

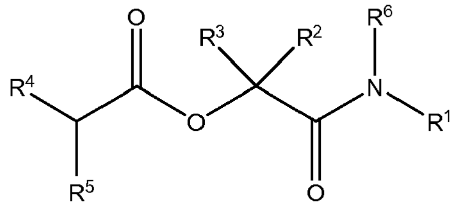

(I)

wherein,

$R^1$ is selected from the group consisting of substituted or unsubstituted alkyl, substituted or unsubstituted cycloalkyl, substituted or unsubstituted aryl, substituted or unsubstituted heteroaryl and substituted or unsubstituted heteroarylalkyl;
$R^2$ and $R^3$ are independently selected from the group consisting of hydrogen and substituted or unsubstituted alkyl; or $R^2$ and $R^3$ are substituted or unsubstituted alkyl and join together with the carbon atom to which they are attached to form a carbocyclic ring which is optionally substituted;
$R^4$ is selected from the group consisting of substituted or unsubstituted aryl, substituted or unsubstituted heteroaryl and substituted or unsubstituted heteroarylalkyl;
$R^5$ is hydrogen or substituted or unsubstituted alkyl;
$R^6$ is hydrogen or substituted or unsubstituted alkyl;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

2. A pharmaceutical composition for use in the treatment of a disease associated with kallikrein-like peptidase 6 overexpression, wherein said composition comprises a compound having a structure according to Formula I

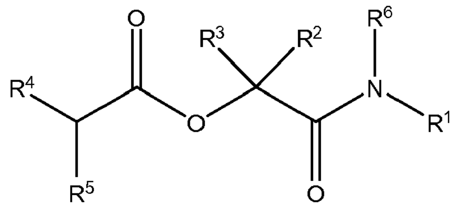

(I)

wherein,

$R^1$ is selected from the group consisting of substituted or unsubstituted alkyl, substituted or unsubstituted cycloalkyl, substituted or unsubstituted aryl, substituted or unsubstituted heteroaryl and substituted or unsubstituted heteroarylalkyl;
$R^2$ and $R^3$ are independently selected from the group consisting of hydrogen and substituted or unsubstituted alkyl; or $R^2$ and $R^3$ are substituted or unsubstituted alkyl and join together with the carbon atom to which they are attached to form a carbocyclic ring which is optionally substituted;
$R^4$ is selected from the group consisting of substituted or unsubstituted aryl, substituted or unsubstituted heteroaryl and substituted or unsubstituted heteroarylalkyl;
$R^5$ is hydrogen or substituted or unsubstituted alkyl;
$R^6$ is hydrogen or substituted or unsubstituted alkyl;
or a pharmaceutically acceptable salt, solvate or hydrate thereof;
with the proviso that

(i) $R^5$ is hydrogen, when one of $R^2$ and $R^3$ is substituted or unsubstituted alkyl, or $R^2$ and $R^3$ are substituted or unsubstituted alkyl and join together with the carbon atom to which they are attached to form a carbocyclic ring;
(ii) $R^2$ and $R^3$ are hydrogen, when $R^5$ is substituted or unsubstituted alkyl.

**3.** The compound for use according to claim 1 or the pharmaceutical composition for use according to claim 2, wherein the compound having a structure according to Formula (I) is **characterized in that**,
$R^1$ is selected from the group consisting of substituted or unsubstituted $(C_1\text{-}C_6)$alkyl, substituted or unsubstituted $(C_3\text{-}C_8)$cycloalkyl, substituted or unsubstituted $(C_6\text{-}C_{14})$aryl, substituted or unsubstituted $(C_3\text{-}C_{14})$heteroaryl and substituted or unsubstituted $(C_3\text{-}C_{14})$heteroaryl$(C_1\text{-}C_6)$alkyl;
$R^2$ and $R^3$ are independently selected from the group consisting of hydrogen and substituted or unsubstituted $(C_1\text{-}C_6)$alkyl; or $R^2$ and $R^3$ are substituted or unsubstituted $(C_1\text{-}C_6)$alkyl and join together with the carbon atom to which they are attached to form a carbocyclic ring which is optionally substituted;
$R^4$ is selected from the group consisting of substituted or unsubstituted $(C_6\text{-}C_{14})$aryl, substituted or unsubstituted $(C_3\text{-}C_{14})$heteroaryl and substituted or unsubstituted $(C_3\text{-}C_{14})$heteroaryl$(C_1\text{-}C_6)$alkyl;
$R^5$ is hydrogen or substituted or unsubstituted $(C_1\text{-}C_6)$alkyl, preferably hydrogen; and
$R^6$ is hydrogen or substituted or unsubstituted $(C_1\text{-}C_6)$alkyl, preferably hydrogen.

**4.** The compound for use or the pharmaceutical composition for use according to any one of the preceding claims, wherein the compound having a structure according to Formula (I) is **characterized in that**,
$R^1$ and $R^4$ are independently selected from the group consisting of substituted or unsubstituted $(C_6\text{-}C_{14})$aryl and substituted or unsubstituted $(C_3\text{-}C_{14})$ heteroaryl.

**5.** The compound for use or the pharmaceutical composition for use according to any one of the preceding claims, wherein the compound of Formula (I) is

(a) a compound having a structure according to Formula (II)

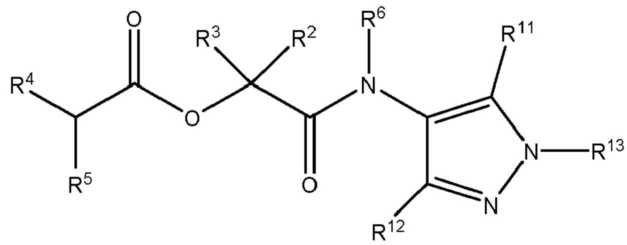

(II)

wherein,

$R^2$ and $R^3$ are independently selected from the group consisting of hydrogen and substituted or unsubstituted $(C_1\text{-}C_6)$alkyl; or $R^2$ and $R^3$ are substituted or unsubstituted $(C_1\text{-}C_6)$alkyl and join together with the carbon atom to which they are attached to form a carbocyclic ring which is optionally substituted;
$R^4$ is selected from the group consisting of substituted or unsubstituted $(C_6\text{-}C_{14})$aryl and substituted or unsubstituted $(C_3\text{-}C_{14})$ heteroaryl;
$R^5$ is hydrogen or substituted or unsubstituted $(C_1\text{-}C_6)$alkyl, preferably hydrogen;
$R^6$ is hydrogen or substituted or unsubstituted $(C_1\text{-}C_6)$alkyl, preferably hydrogen;
$R^{11}$ and $R^{12}$ are independently selected from the group consisting of hydrogen and substituted or unsubstituted $(C_1\text{-}C_6)$alkyl;
$R^{13}$ is selected from the group consisting of substituted or unsubstituted $(C_1\text{-}C_6)$alkyl, substituted or unsubstituted $(C_6\text{-}C_{14})$aryl, substituted or unsubstituted $(C_3\text{-}C_{14})$heteroaryl$(C_1\text{-}C_6)$alkyl and substituted or unsubstituted $(C_6\text{-}C_{14})$aryl$(C_1\text{-}C_6)$alkyl;

or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl; or

(b) a compound having a structure according to Formula (III)

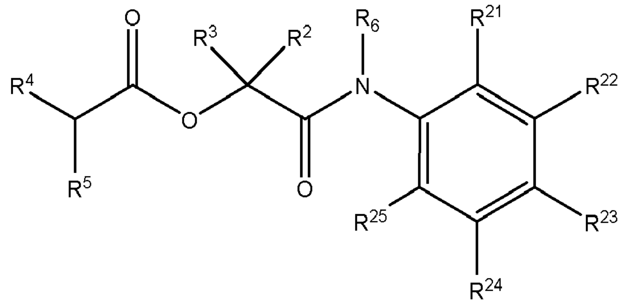

(III)

wherein,

$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of substituted or unsubstituted ($C_6$-$C_{14}$)aryl and substituted or unsubstituted ($C_3$-$C_{14}$) heteroaryl;
$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{21}$, $R^{22}$, $R^{23}$, $R^{24}$ and $R^{25}$ are independently selected from the group consisting of hydrogen, substituted or unsubstituted ($C_3$-$C_{14}$)heterocyclyl, substituted or unsubstituted amino, substituted or unsubstituted ($C_1$-$C_6$)alkyl, -$OR^{26}$, halogen and -C(O)$NR^{27}R^{28}$;
$R^{26}$ is selected from the group consisting of hydrogen, substituted or unsubstituted ($C_1$-$C_6$)alkyl and substituted or unsubstituted ($C_2$-$C_6$)alkenyl;
$R^{27}$ and $R^{28}$ are independently selected from the group consisting of hydrogen and substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

**6.** The compound for use or the pharmaceutical composition for use according to any one of the preceding claims, wherein the compound of Formula (I) is a compound having a structure according to Formula (IV)

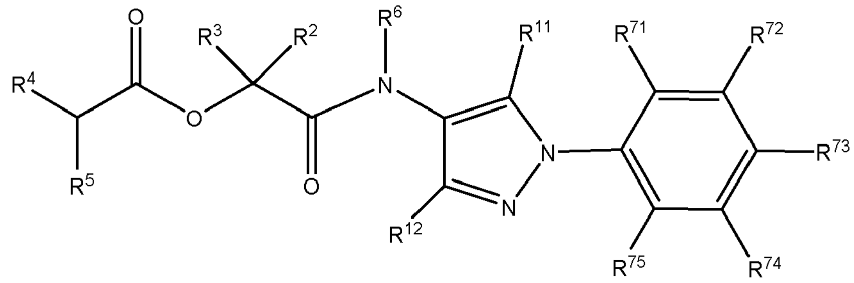

(IV)

wherein

$R^2$ and $R^3$ are independently selected from the group consisting of hydrogen and substituted or unsubstituted $(C_1\text{-}C_6)$alkyl; or $R^2$ and $R^3$ are substituted or unsubstituted $(C_1\text{-}C_6)$alkyl and join together with the carbon atom to which they are attached to form a carbocyclic ring which is optionally substituted;
$R^4$ is selected from the group consisting of substituted or unsubstituted $(C_6\text{-}C_{14})$aryl and substituted or unsubstituted $(C_3\text{-}C_{14})$ heteroaryl;
$R^5$ is hydrogen or substituted or unsubstituted $(C_1\text{-}C_6)$alkyl, preferably hydrogen;
$R^6$ is hydrogen or substituted or unsubstituted $(C_1\text{-}C_6)$alkyl, preferably hydrogen;
$R^{11}$ and $R^{12}$ are independently selected from the group consisting of hydrogen and substituted or unsubstituted $(C_1\text{-}C_6)$alkyl;
$R^{71}$, $R^{72}$, $R^{73}$, $R^{74}$, $R^{75}$ and $R^{76}$ are independently selected from the group consisting of hydrogen, $(C_1\text{-}C_6)$alkyl, $(C_1\text{-}C_6)$haloalkyl, $-NO_2$, halogen, amino, $-N(R^{78})((C_1\text{-}C_6)$alkyl-$OR^{77})$, cyano and - $OR^{77}$;
$R^{77}$ and $R^{78}$ are each independently selected from the group consisting of hydrogen, $(C_1\text{-}C_6)$haloalkyl and substituted or unsubstituted $(C_1\text{-}C_6)$alkyl, preferably hydrogen and/or substituted or unsubstituted $(C_1\text{-}C_6)$alkyl;
or a pharmaceutically acceptable salt, solvate or hydrate thereof;
with the proviso that

(i) $R^5$ is hydrogen, when one of $R^2$ and $R^3$ is substituted or unsubstituted $(C_1\text{-}C_6)$alkyl, or $R^2$ and $R^3$ are substituted or unsubstituted $(C_1\text{-}C_6)$alkyl and join together with the carbon atom to which they are attached to form a carbocyclic ring;
(ii) $R^2$ and $R^3$ are hydrogen, when $R^5$ is substituted or unsubstituted $(C_1\text{-}C_6)$alkyl.

7. The compound for use or the pharmaceutical composition for use according to any one of the preceding claims, wherein the compound having a structure according to Formula (I), Formula (II), Formula (III) or Formula (IV) is **characterized in that**,
$R^4$ is selected from the group consisting of substituted or unsubstituted phenyl, substituted or unsubstituted naphthyl, substituted or unsubstituted indolyl, substituted or unsubstituted thiophenyl, substituted or unsubstituted benzofuranyl and substituted or unsubstituted pyridyl.

8. The compound for use or the pharmaceutical composition for use according to claim 7, wherein the compound having a structure according to Formula (I), Formula (II), Formula (III) or Formula (IV) is **characterized in that**,
$R^4$ is selected from the group consisting of

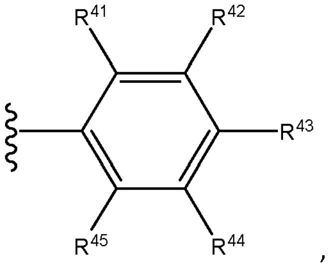
,
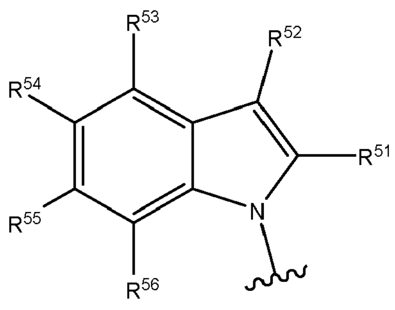
and
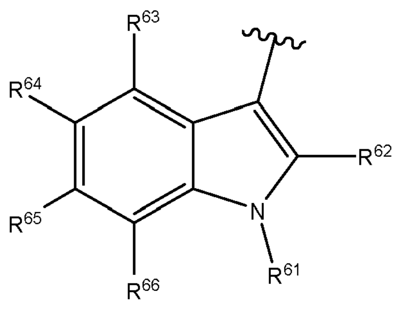
,

wherein,

$R^{41}$, $R^{42}$, $R^{43}$, $R^{44}$ and $R^{45}$ are independently selected from the group consisting of hydrogen, substituted or unsubstituted ($C_1$-$C_6$)alkyl, ($C_1$-$C_6$)haloalkyl, halogen, azido, -CN, - N($R^{46}$)C(=O)$R^{47}$, amino and $OR^{48}$;
$R^{46}$, $R^{47}$ and $R^{48}$ are independently selected from the group consisting of hydrogen, substituted or unsubstituted ($C_6$-$C_{14}$)aryl($C_1$-$C_6$)alkyl, ($C_1$-$C_6$)haloalkyl and substituted or unsubstituted ($C_1$-$C_6$)alkyl, preferably hydrogen and substituted or unsubstituted ($C_1$-$C_6$)alkyl;
$R^{51}$, $R^{52}$, $R^{53}$, $R^{54}$, $R^{55}$ and $R^{56}$ are independently selected from the group consisting of hydrogen, substituted or unsubstituted ($C_1$-$C_6$)alkyl, halogen and ($C_1$-$C_6$)haloalkyl, preferably substituted or unsubstituted ($C_1$-$C_6$)alkyl and ($C_1$-$C_6$)haloalkyl;
$R^{61}$, $R^{62}$, $R^{63}$, $R^{64}$, $R^{65}$ and $R^{66}$ are independently selected from the group consisting of hydrogen, substituted or unsubstituted ($C_1$-$C_6$)alkyl, halogen and ($C_1$-$C_6$)haloalkyl, preferably hydrogen, ($C_1$-$C_6$)haloalkyl and substituted or unsubstituted ($C_1$-$C_6$)alkyl.

**9.** The compound for use or the pharmaceutical composition for use according to any one of the preceding claims, wherein the disease associated with kallikrein-like peptidase 6 overexpression is selected from the group consisting of cancer, immune disease and neuron injury, preferably wherein cancer is selected from the group consisting of Melanoma, Glioblastoma, Astrocytoma, Ovarian cancer, Colon carcinoma, Lung cancer, Gastric cancer, Gastroesophageal junction adenocarcinoma, Pancreatic ductal adenocarcinoma, Breast cancer and Laryngeal adenocarcinoma, preferably wherein the immune disease is an autoimmune disease, more preferably wherein the autoimmune disease is selected from the group consisting of Multiple Sclerosis, Psoriasis, Morbus Crohn and Colitis ulcerosa, even more preferably Multiple Sclerosis and Psoriasis, still more preferably Multiple Sclerosis, preferably wherein the neuron injury is Spinal Cord Injury (SCI).

**10.** A compound having a structure according to Formula (IV)

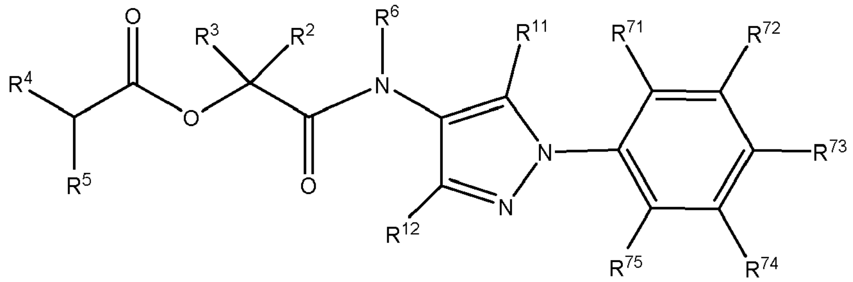

(IV)

wherein

$R^2$ and $R^3$ are independently selected from the group consisting of hydrogen and substituted or unsubstituted ($C_1$-$C_6$)alkyl; or $R^2$ and $R^3$ are substituted or unsubstituted ($C_1$-$C_6$)alkyl and join together with the carbon atom to which they are attached to form a carbocyclic ring which is optionally substituted;
$R^4$ is selected from the group consisting of

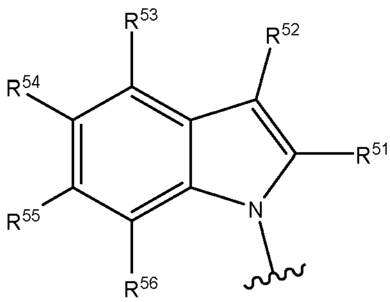

and

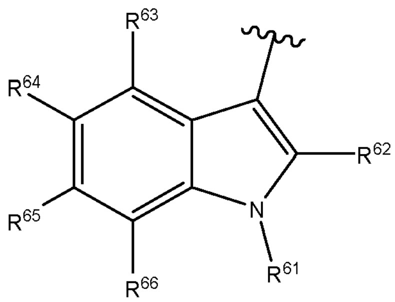

;

$R^5$ is hydrogen or substituted or unsubstituted ($C_1$-$C_6$)alkyl, preferably hydrogen;
$R^6$ is hydrogen or substituted or unsubstituted ($C_1$-$C_6$)alkyl, preferably hydrogen;
$R^{11}$ and $R^{12}$ are independently selected from the group consisting of hydrogen and substituted or unsubstituted ($C_1$-$C_6$)alkyl;
$R^{51}$, $R^{52}$, $R^{53}$, $R^{54}$, $R^{55}$ and $R^{56}$ are independently selected from the group consisting of hydrogen, substituted

or unsubstituted ($C_1$-$C_6$)alkyl, halogen and ($C_1$-$C_6$)haloalkyl, preferably substituted or unsubstituted ($C_1$-$C_6$)alkyl and ($C_1$-$C_6$)haloalkyl;
$R^{61}$, $R^{62}$, $R^{63}$, $R^{64}$, $R^{65}$ and $R^{66}$ are independently selected from the group consisting of hydrogen, substituted or unsubstituted ($C_1$-$C_6$)alkyl, halogen and ($C_1$-$C_6$)haloalkyl, preferably hydrogen, ($C_1$-$C_6$)haloalkyl and substituted or unsubstituted ($C_1$-$C_6$)alkyl;
$R^{71}$, $R^{72}$, $R^{73}$, $R^{74}$, $R^{75}$ and $R^{76}$ are independently selected from the group consisting of ($C_1$-$C_6$)haloalkyl, -$NO_2$, halogen, amino, -N($R^{78}$)(($C_1$-$C_6$)alkyl-O$R^{77}$), cyano and -O$R^{77}$;
$R^{77}$ and $R^{78}$ are each independently selected from the group consisting of hydrogen, ($C_1$-$C_6$)haloalkyl and substituted or unsubstituted ($C_1$-$C_6$)alkyl, preferably substituted or unsubstituted ($C_1$-$C_6$)alkyl;
or a pharmaceutically acceptable salt, solvate or hydrate thereof;
with the proviso that

(i) $R^5$ is hydrogen, when one of $R^2$ and $R^3$ is substituted or unsubstituted ($C_1$-$C_6$)alkyl, or $R^2$ and $R^3$ are substituted or unsubstituted ($C_1$-$C_6$)alkyl and join together with the carbon atom to which they are attached to form a carbocyclic ring;
(ii) $R^2$ and $R^3$ are hydrogen, when $R^5$ is substituted or unsubstituted ($C_1$-$C_6$)alkyl.

**11.** A compound selected from the group consisting of 2-((3,5-dimethyl-1-(m-tolyl)-1H-pyrazol-4-yl)amino)-2-oxoethyl 2-(2-methyl-1 H-indol-1-yl)acetate, 2-((3,5-dimethyl-1-(4-(trifluoromethyl)phenyl)-1 H-pyrazol-4-yl)amino)-2-oxoethyl 2-(2-methyl-1H-indol-1-yl)acetate, 2-((3,5-dimethyl-1-(2-nitrophenyl)-1H-pyrazol-4-yl)amino)-2-oxoethyl 2-(2-methyl-1H-indol-1-yl)acetate, 2-((1-(4-cyanophenyl)-3,5-dimethyl-1H-pyrazol-4-yl)amino)-2-oxoethyl 2-(2-methyl-1H-indol-1-yl)acetate, 2-((1-(4-methoxybenzyl)-3,5-dimethyl-1H-pyrazol-4-yl)amino)-2-oxoethyl 2-(2-methyl-1H-indol-1-yl)acetate, 2-((3,5-dimethyl-1-(2-(trifluoromethyl)phenyl)-1H-pyrazol-4-yl)amino)-2-oxoethyl 2-(2-methyl-1H-indol-1-yl)acetate, 2-((3,5-dimethyl-1-(p-tolyl)-1H-pyrazol-4-yl)amino)-2-oxoethyl2-(2-methyl-1H-indol-1-yl)acetate, 2-((3,5-dimethyl-1-(4-nitrophenyl)-1H-pyrazol-4-yl)amino)-2-oxoethyl 2-(2-methyl-1H-indol-1-yl)acetate, 2-((3,5-dimethyl-1-phenyl-1H-pyrazol-4-yl)amino)-2-oxoethyl 2-(1H-indol-1-yl)acetate, 2-((3,5-dimethyl-1-(p-tolyl)-1H-pyrazol-4-yl)amino)-2-oxoethyl 2-(1H-indol-1-yl)acetate, 2-((3,5-dimethyl-1-phenyl-1H-pyrazol-4-yl)amino)-2-oxoethyl 2-(3-methyl-1H-indol-1-yl)acetate, 2-((3,5-dimethyl-1-phenyl-1H-pyrazol-4-yl)amino)-2-oxoethyl 2-(2,3-dimethyl-1H-indol-1-yl)acetate, 2-((3,5-dimethyl-1-phenyl-1H-pyrazol-4-yl)amino)-2-oxoethyl 2-(2-(trifluoromethyl)-1H-indol-1-yl)acetate, 2-((3,5-dimethyl-1-phenyl-1H-pyrazol-4-yl)amino)-2-oxoethyl 2-(1-methyl-1H-indol-3-yl)acetate, 2-((1-(4-((3-hydroxypropyl)amino)phenyl)-3,5-dimethyl-1H-pyrazol-4-yl)amino)-2-oxoethyl 2-(2-methyl-1H-indol-1-yl)acetate, 2-((3,5-dimethyl-1-(p-tolyl)-1H-pyrazol-4-yl)amino)-2-oxoethyl 2-(2-(trifluoromethyl)-1H-indol-1-yl)acetate, 2-((3,5-dimethyl-1-(4-nitrophenyl)-1 H-pyrazol-4-yl)amino)-2-oxoethyl 2-(2-(trifluoromethyl)-1H-indol-1-yl)acetate, 2-((1-(4-aminophenyl)-3,5-dimethyl-1H-pyrazol-4-yl)amino)-2-oxoethyl 2-(2-methyl-1 H-indol-1-yl)acetate, 2-((1-(2-aminophenyl)-3,5-dimethyl-1H-pyrazol-4-yl)amino)-2-oxoethyl 2-(2-methyl-1 H-indol-1-yl)acetate, 2-((3,5-dimethyl-1-phenyl-1H-pyrazol-4-yl)amino)-2-oxoethyl 2-(4-fluoro-1H-indol-1-yl)acetate, 2-((3,5-dimethyl-1-phenyl-1H-pyrazol-4-yl)amino)-2-oxoethyl 2-(6-chloro-1H-indol-1-yl)acetate, 2-((3,5-dimethyl-1-phenyl-1H-pyrazol-4-yl)amino)-2-oxoethyl 2-(5-chloro-1H-indol-1-yl)acetate, 2-((3,5-dimethyl-1-phenyl-1H-pyrazol-4-yl)amino)-2-oxoethyl 2-(5-fluoro-1H-indol-1-yl)acetate, 2-((3,5-dimethyl-1-phenyl-1H-pyrazol-4-yl)amino)-2-oxoethyl 2-(5-chloro-2-methyl-1 H-indol-1-yl)acetate, 2-((3,5-dimethyl-1-phenyl-1 H-pyrazol-4-yl)amino)-2-oxoethyl 2-(7-chloro-1 H-indol-1-yl)acetate, 2-((3,5-dimethyl-1-phenyl-1 H-pyrazol-4-yl)amino)-2-oxoethyl 2-(2-isopropyl-1 H-indol-1-yl)acetate, 2-((3,5-dimethyl-1-phenyl-1 H-pyrazol-4-yl)amino)-2-oxoethyl 2-(7-chloro-2-methyl-1 H-indol-1-yl)acetate, 2-((3,5-dimethyl-1-phenyl-1 H-pyrazol-4-yl)amino)-2-oxoethyl 2-(2-methyl-1 H-indol-1-yl)propanoate, 2-((3,5-dimethyl-1-phenyl-1H-pyrazol-4-yl)amino)-2-oxoethyl 2-(2-ethyl-1H-indol-1-yl)acetate, 1-((3,5-dimethyl-1-phenyl-1H-pyrazol-4-yl)amino)-1-oxopropan-2-yl 2-(2-methyl-1H-indol-1-yl)acetate, 2-((3,5-dimethyl-1-phenyl-1 H-pyrazol-4-yl)amino)-2-oxoethyl 2-(2-methyl-1 H-indol-3-yl)acetate, 2-oxo-2-((1-phenyl-1H-pyrazol-4-yl)amino)ethyl 2-(2-methyl-1 H-indol-3-yl)acetate, 1-((3,5-dimethyl-1-phenyl-1H-pyrazol-4-yl)carbamoyl)cyclopropyl 2-(2-methyl-1H-indol-1-yl)acetate, 2-((3,5-dimethyl-1-phenyl-1H-pyrazol-4-yl)amino)-2-oxoethyl 2-(benzofuran-3-yl)acetate, 2-((3,5-dimethyl-1-phenyl-1H-pyrazol-4-yl)amino)-2-oxoethyl 2-(4-aminophenyl)acetate, 2-((3,5-dimethyl-1-phenyl-1H-pyrazol-4-yl)amino)-2-oxoethyl 2-(2-chlorophenyl)acetate, 2-((3,5-dimethyl-1-phenyl-1H-pyrazol-4-yl)amino)-2-oxoethyl 2-(4-cyanophenyl)acetate, 2-((3,5-dimethyl-1-phenyl-1H-pyrazol-4-yl)amino)-2-oxoethyl 2-(3-acetamidophenyl)acetate, 2-((3,5-dimethyl-1-phenyl-1H-pyrazol-4-yl)amino)-2-oxoethyl 2-(2-bromophenyl)acetate, 2-((3,5-dimethyl-1-phenyl-1H-pyrazol-4-yl)amino)-2-oxoethyl 2-(m-tolyl)acetate, 2-((3,5-dimethyl-1-phenyl-1H-pyrazol-4-yl)amino)-2-oxoethyl 2-(2-iodophenyl)acetate, 2-((3,5-dimethyl-1-phenyl-1H-pyrazol-4-yl)amino)-2-oxoethyl 2-(3-aminophenyl)acetate, 2-((3,5-dimethyl-1-phenyl-1H-pyrazol-4-yl)amino)-2-oxoethyl 2-(3-cyanophenyl)acetate, 2-((3,5-dimethyl-1-phenyl-1H-pyrazol-4-yl)amino)-2-oxoethyl 2-(pyridin-3-yl)acetate, 2-((3,5-dimethyl-1-phenyl-1H-pyrazol-4-yl)amino)-2-oxoethyl 2-(pyridin-2-yl)acetate, 2-((3,5-dimethyl-1-phenyl-1H-pyrazol-4-yl)amino)-2-oxoethyl 2-(5-chlorothiophen-2-yl)acetate, 2-((3,5-dimethyl-1-phenyl-1H-pyrazol-4-yl)amino)-2-oxoethyl 2-(2,5-dichlorothiophen-3-yl)acetate, 2-((3,5-dimethyl-1-phenyl-1H-pyrazol-4-yl)amino)-2-oxoethyl 2-(pyridin-4-yl)acetate, 2-((3,5-dimethyl-1-phenyl-1H-pyrazol-4-yl)amino)-2-oxoethyl 2-(2-(trifluoromethyl)phenyl)acetate, 2-((3,5-dimethyl-1-phenyl-1H-pyrazol-4-yl)amino)-2-oxoethyl 2-(3-fluorophenyl)acetate, 2-((3,5-dimethyl-1-phenyl-1H-pyrazol-4-yl)amino)-2-oxoethyl 2-(2-chlorothiophen-3-yl)acetate, 2-((3,5-dimethyl-1-phenyl-1H-pyrazol-4-yl)amino)-2-oxoethyl 2-(furan-2-yl)acetate, 2-((3,5-dimethyl-1-phenyl-1H-pyrazol-4-yl)amino)-2-oxoethyl 2-(2-(benzyloxy)phenyl)acetate, 2-((3,5-dimethyl-1-phenyl-1H-pyrazol-4-yl)amino)-2-oxoethyl 2-(thiophen-3-yl)acetate, 2-((3,5-dimethyl-1-phenyl-1H-pyrazol-4-yl)amino)-2-oxoethyl 2-(2-methyl-1 H-indol-3-yl)propanoate, 2-((3,5-dimethyl-1-phenyl-1H-pyrazol-4-yl)amino)-2-oxoethyl 2-(2-azidophenyl)acetate, 2-((3,5-dimethyl-1-phenyl-1H-pyrazol-4-yl)amino)-2-oxoethyl 2-(1H-indol-3-yl)propanoate, 2-((3,5-dimethyl-1-phenyl-1H-pyrazol-4-yl)amino)-2-oxoethyl 3-(2-methyl-1 H-indol-3-yl)propanoate, 2-((3,5-dimethyl-1-phenyl-1H-pyrazol-4-yl)(methyl)amino)-2-oxoethyl 2-(2-methyl-1 H-indol-3-yl)acetate, 2-(tert-butylamino)-2-oxoethyl 2-(2-methyl-1 H-indol-3-yl)acetate, 2-(cyclopentylamino)-2-oxoethyl 2-(2-methyl-1 H-indol-3-yl)acetate, 2-((3,5-dimethyl-1-phenyl-1 H-pyrazol-4-yl)amino)-2-oxoethyl 2-(3,5-dimethyl-1 H-pyrazol-1-yl)acetate, 2-((5-fluoropyridin-2-yl)amino)-2-oxoethyl 2-(2-methyl-1 H-indol-3-yl)acetate, 2-(((1H-imidazol-2-yl)methyl)amino)-2-oxoethyl 2-(2-methyl-1 H-indol-3-yl)acetate, 2-oxo-2-(pyrimidin-5-ylamino)ethyl 2-(2-methyl-1 H-indol-3-yl)acetate, 2-oxo-2-(phenylamino)ethyl 2-(2-methyl-1 H-indol-1-yl)acetate, 2-oxo-2-(o-tolylamino)ethyl 2-(2-methyl-1 H-indol-1-yl)acetate, 2-((4-morpholinophenyl)amino)-2-oxoethyl 2-(2-methyl-1 H-indol-1-yl)acetate, 2-((2-(allyloxy)phenyl)amino)-2-oxoethyl 2-(2-iodophenyl)acetate, 2-((2-chlorophenyl)amino)-2-oxoethyl 2-(2-methyl-1 H-indol-3-yl)acetate, 2-oxo-2-(p-tolylamino)ethyl 2-(2-methyl-1 H-indol-3-yl)acetate, 2-oxo-2-(m-tolylamino)ethyl 2-(2-methyl-1 H-indol-3-yl)acetate, 2-((4-morpholinophenyl)amino)-2-oxoethyl 2-(2-methyl-1 H-indol-3-yl)acetate, 2-((2-methoxyphenyl)amino)-2-oxoethyl 2-(2-methyl-1 H-indol-3-yl)acetate, 2-oxo-2-(o-tolylamino)ethyl 2-(2-methyl-1 H-indol-3-yl)acetate, 2-((3-methoxyphenyl)amino)-2-oxoethyl 2-(2-methyl-1 H-indol-3-yl)acetate, 2-((4-methoxyphenyl)amino)-2-oxoethyl 2-(2-methyl-1 H-indol-3-yl)acetate, 2-((3-carbamoylphenyl)amino)-2-oxoethyl 2-(2-methyl-1 H-indol-3-yl)acetate, 2-((3,5-dimethyl-1-phenyl-1 H-pyrazol-4-yl)amino)-2-oxoethyl 2-(1H-indol-3-yl)acetate, 2-((3,5-dimethyl-1-phenyl-1 H-pyrazol-4-yl)amino)-2-oxoethyl 2-(1,2-dimethyl-1 H-indol-3-yl)acetate, 2-((3,5-dimethyl-1-phenyl-1 H-pyrazol-4-yl)amino)-2-oxoethyl 2-(2-methylpyridin-3-yl)acetate and 2-((3,5-dimethyl-1-phenyl-1 H-pyrazol-4-yl)amino)-2-oxoethyl 3,3,3-trifluoro-2-(2-methyl-1H-indol-3-yl)propanoate
or a pharmaceutically acceptable salt, solvate or hydrate thereof.

**12.** A pharmaceutical composition comprising a compound according to claim 10 or 11 and at least one pharmaceutically acceptable carrier.

**13.** A compound according to claim 10 or 11 for use in medicine.

**14.** A compound according to any one of claims 10 or 11 or the pharmaceutical composition according to claim 12 for use in the treatment of a disease associated with kallikrein-like peptidase 6 overexpression, preferably wherein the disease associated with kallikrein-like peptidase 6 overexpression is selected from the group consisting of cancer, immune disease and neuron injury, preferably wherein cancer is selected from the group consisting of Melanoma, Glioblastoma, Astrocytoma, Ovarian cancer, Colon carcinoma, Lung cancer, Gastric cancer, Gastroesophageal junction adenocarcinoma, Pancreatic ductal adenocarcinoma, Breast cancer and Laryngeal adenocarcinoma, preferably wherein the immune disease is an autoimmune disease, more preferably wherein the autoimmune disease is selected from the group consisting of Multiple Sclerosis, Psoriasis, Morbus Crohn and Colitis ulcerosa, even more preferably Multiple Sclerosis and Psoriasis, still more preferably Multiple Sclerosis, preferably wherein the neuron injury is Spinal Cord Injury (SCI).

**15.** A kit comprising a compound according to any one of claims 10-11 or a pharmaceutical composition according to claim 12 and at least one pharmaceutically acceptable carrier.

Figure 1

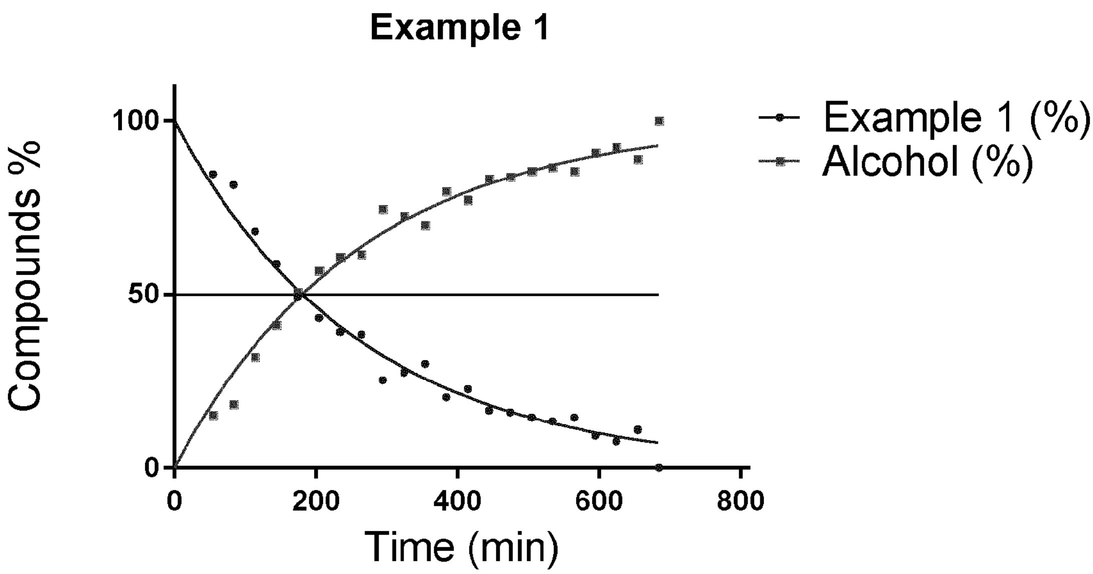


$t_{1/2}$ = 180.7 min ~ 3h

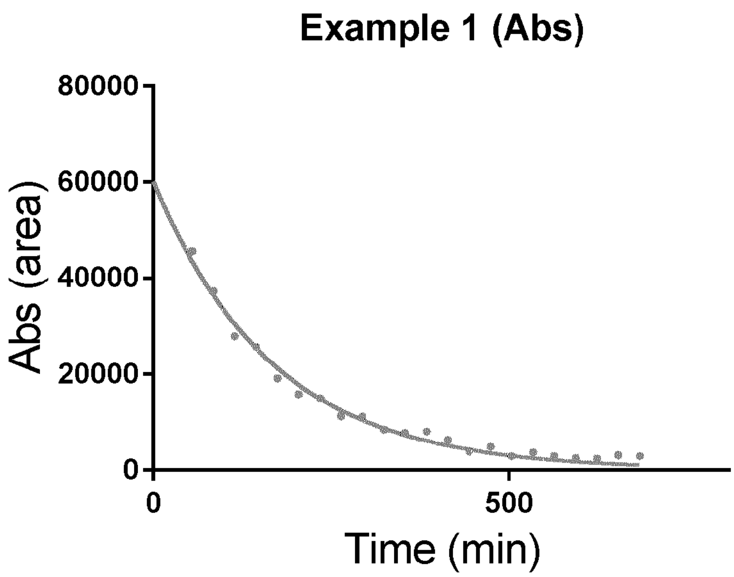


$t_{1/2}$ = 116.1min ~ 2h

Figure 2

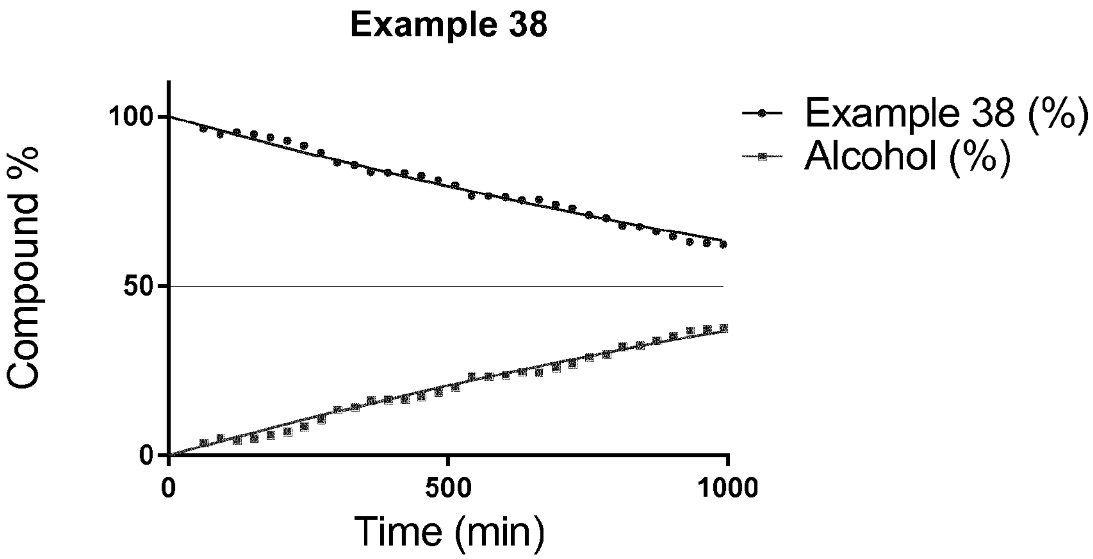


$t_{1/2}$ = 1502 min ~ 25h

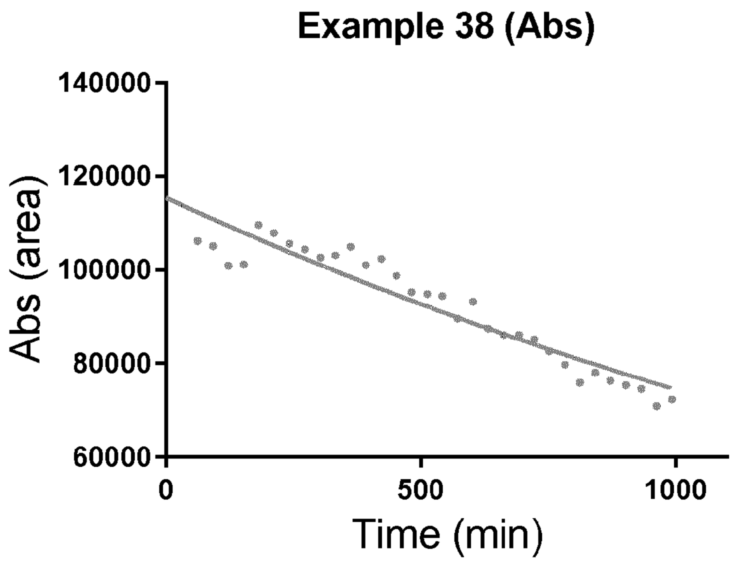


$t_{1/2}$ = 1580 min ~ 26h

Figure 3

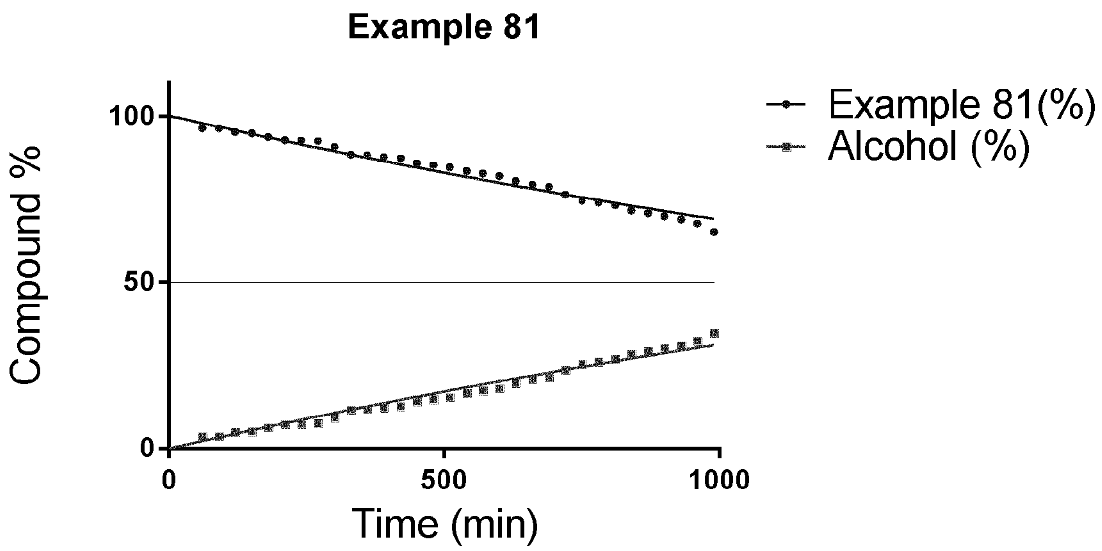


$t_{1/2}$ = 1847 min ~ 30-31h

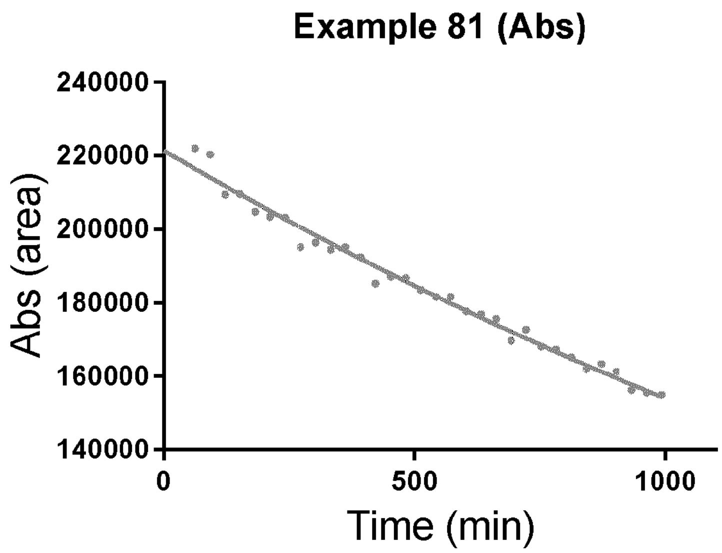


$t_{1/2}$ = 1907 min ~ 32h

Europäisches Patentamt
European Patent Office
Office européen des brevets

# EUROPEAN SEARCH REPORT

Application Number
EP 16 19 2835

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | WO 2015/112079 A1 (SIXERA PHARMA AB [SE]) 30 July 2015 (2015-07-30)<br>* claims 1, 10, 11, 14 *<br>* table 1 *<br>* examples 4, 5, 6, etc. *<br>----- | 1-15 | INV.<br>C07D403/12<br>C07D231/40<br>C07D209/08<br>C07D401/12<br>C07D405/12<br>C07D409/12<br>A61K31/4155<br>A61K31/405<br>A61K31/4439<br>A61K31/4178<br>A61K31/506<br>C07C235/00 |
| X | US 6 388 122 B1 (KIDO HIROSHI [JP] ET AL) 14 May 2002 (2002-05-14)<br>* claims 1, 6, 13 *<br>* compound 2 *<br>----- | 1-3,9 | |
| X | WO 2007/136607 A2 (MERCK & CO INC [US]; COLANDREA VINCENT J [US]; HAGMANN WILLIAM K [US];) 29 November 2007 (2007-11-29)<br>* claims 1, 8 *<br>* claim 5; compounds 45, 53 *<br>* page 19, line 33 - line 36 *<br>----- | 1-3,9 | |
| X | FLORA BARSOUM ET AL: "Anti-inflammatory Activity and PGE2 Inhibitory Properties of Novel Phenylcarbamoylmethyl Ester-Containing Compounds", MOLECULES, vol. 14, no. 2, 11 February 2009 (2009-02-11), pages 667-681, XP055316108, DOI: 10.3390/molecules14020667<br>* compounds 3a-d, 5a-d *<br>* table 1 *<br>* page 668, line 24 - line 25 *<br>----- | 1-5,7-9 | TECHNICAL FIELDS SEARCHED (IPC)<br>C07D<br>C07C |
| X | US 2013/059852 A1 (TRENT JOHN O [US] ET AL) 7 March 2013 (2013-03-07)<br>* page 7; compounds I-34 *<br>* claims 1, 3, 9 *<br>* paragraph [0009] *<br>-----<br>-/-- | 1-3,9 | |

The present search report has been drawn up for all claims

2

| Place of search | Date of completion of the search | Examiner |
|---|---|---|
| Munich | 4 November 2016 | Brandstetter, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 16 19 2835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KHANNA S ET AL: "Evaluation of glycolamide esters of indomethacin as potential cyclooxygenase-2 (COX-2) inhibitors", BIOORGANIC & MEDICINAL CHEMISTRY, PERGAMON, GB, vol. 14, no. 14, 15 July 2006 (2006-07-15), pages 4820-4833, XP027992753, ISSN: 0968-0896 [retrieved on 2006-07-15] * paragraph [5.1.1] - paragraph [5.1.55] * * table 1 * ----- | 1-5,7-9 | |
| X | WEIDLICH I E ET AL: "Inhibitors of human tyrosyl-DNA phospodiesterase (hTdp1) developed by virtual screening using ligand-based pharmacophores", BIOORGANIC & MEDICINAL CHEMISTRY, PERGAMON, GB, vol. 18, no. 1, 1 January 2010 (2010-01-01), pages 182-189, XP026810708, ISSN: 0968-0896 [retrieved on 2009-11-11] * compound 15 * * table 2 * * last paragraph in left column of page 185 * ----- | 1-5,7-9 | TECHNICAL FIELDS SEARCHED (IPC) |
| X | WO 2005/033102 A2 (AMPHORA DISCOVERY CORP [US]; HODGE CARL NICHOLAS [US]; JANZEN WILLIAM) 14 April 2005 (2005-04-14) * claims 41, 52, 55, 57 * * compound 2.109 * ----- -/-- | 1-4,7,9 | |

The present search report has been drawn up for all claims

2

| Place of search | Date of completion of the search | Examiner |
|---|---|---|
| Munich | 4 November 2016 | Brandstetter, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 5

Europäisches Patentamt
European Patent Office
Office européen des brevets

# EUROPEAN SEARCH REPORT

Application Number
EP 16 19 2835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/037779 A2 (INST MEDIZINTECHNOLOGIE MAGDEB [DE]; KEYNEUROTEK AG [DE]; ANSORGE SIEG) 28 April 2005 (2005-04-28)<br>* claims 5, 6, 29, 58, 63 *<br>* page 167; claim 6; compound D3.103 *<br>----- | 1-4,7,9 | |
| X | WO 2009/136175 A1 (MEDICAL RES COUNCIL [GB]; FERSHT ALAN [GB]; BOECKLER FRANK [DE]; JOERG) 12 November 2009 (2009-11-12)<br>* page 52; compound PK118 *<br>* claim 1 *<br>* page 27, line 28 - line 35 *<br>----- | 1-4,9 | |
| A | DATABASE Pubchem Compound [Online] NCBI;<br>30 May 2009 (2009-05-30),<br>XP002763834,<br>Database accession no. CID 41583852<br>* abstract *<br>----- | 10 | |
| A | DATABASE Pubchem Compound [Online] NCBI;<br>29 May 2009 (2009-05-29),<br>XP002763835,<br>Database accession no. CID 33964917<br>* abstract *<br>----- | 10 | TECHNICAL FIELDS SEARCHED (IPC) |
| X | DATABASE Pubchem Compound [Online] NCBI;<br>29 July 2006 (2006-07-29),<br>XP002763836,<br>Database accession no. CID 7665603 | 11,12,15 | |
| A | * abstract *<br>-----<br>-/-- | 10 | |

The present search report has been drawn up for all claims

| Place of search | Date of completion of the search | Examiner |
|---|---|---|
| Munich | 4 November 2016 | Brandstetter, T |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 2835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br>A | DATABASE Pubchem Compound [Online] NCBI; 29 July 2006 (2006-07-29), XP002763837, Database accession no. CID 7663788<br>* abstract * | 11,12,15<br><br><br><br><br>10 | |
| X | DATABASE Pubchem Compound [Online] NCBI; 30 July 2006 (2006-07-30), XP002763838, Database accession no. CID 8333076<br>* abstract * | 11,12,15 | |
| X | DATABASE Pubchem Compound [Online] NCBI; 29 July 2006 (2006-07-29), XP002763839, Database accession no. CID 7765533<br>* abstract * | 11,12,15 | |
| A,D | GUYAN LIANG ET AL: "Virtual Screening and X-ray Crystallography for Human Kallikrein 6 Inhibitors with an Amidinothiophene P1 Group", ACS MEDICINAL CHEMISTRY LETTERS, vol. 3, no. 2, 9 February 2012 (2012-02-09), pages 159-164, XP055315898, United States ISSN: 1948-5875, DOI: 10.1021/ml200291e<br>* the whole document * | 1-15 | TECHNICAL FIELDS SEARCHED (IPC) |

-/--

The present search report has been drawn up for all claims

2

| Place of search | Date of completion of the search | Examiner |
|---|---|---|
| Munich | 4 November 2016 | Brandstetter, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 2835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | GUYAN LIANG ET AL: "Human kallikrein 6 inhibitors with a-amidobenzylanmine P1 group identified through virtual screening", BIOORGANIC & MEDICINAL CHEMISTRY LETTERS, PERGAMON, AMSTERDAM, NL, vol. 22, no. 7, 6 February 2012 (2012-02-06), pages 2450-2455, XP028471743, ISSN: 0960-894X, DOI: 10.1016/J.BMCL.2012.02.014 [retrieved on 2012-02-14] * the whole document * ----- | 1-15 | |

TECHNICAL FIELDS SEARCHED (IPC)

2

The present search report has been drawn up for all claims

| Place of search | Date of completion of the search | Examiner |
|---|---|---|
| Munich | 4 November 2016 | Brandstetter, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 19 2835

This annex lists the patent family members relating to the patent documents cited in the above-mentioned European search report. The members are as contained in the European Patent Office EDP file on The European Patent Office is in no way liable for these particulars which are merely given for the purpose of information.

04-11-2016

| Patent document cited in search report | Publication date | Patent family member(s) | Publication date |
|---|---|---|---|
| WO 2015112079 A1 | 30-07-2015 | AU 2015209759 A1 | 07-07-2016 |
| | | CA 2934024 A1 | 30-07-2015 |
| | | EP 3097084 A1 | 30-11-2016 |
| | | WO 2015112079 A1 | 30-07-2015 |
| US 6388122 B1 | 14-05-2002 | EP 0893437 A1 | 27-01-1999 |
| | | KR 20000005312 A | 25-01-2000 |
| | | US 6388122 B1 | 14-05-2002 |
| | | WO 9737969 A1 | 16-10-1997 |
| WO 2007136607 A2 | 29-11-2007 | AU 2007254361 A1 | 29-11-2007 |
| | | CA 2652259 A1 | 29-11-2007 |
| | | EP 2024334 A2 | 18-02-2009 |
| | | JP 2009545518 A | 24-12-2009 |
| | | US 2012135975 A1 | 31-05-2012 |
| | | WO 2007136607 A2 | 29-11-2007 |
| US 2013059852 A1 | 07-03-2013 | US 2013059852 A1 | 07-03-2013 |
| | | US 2014303170 A1 | 09-10-2014 |
| | | WO 2011127333 A2 | 13-10-2011 |
| WO 2005033102 A2 | 14-04-2005 | EP 1670791 A2 | 21-06-2006 |
| | | JP 2007507530 A | 29-03-2007 |
| | | US 2005085531 A1 | 21-04-2005 |
| | | WO 2005033102 A2 | 14-04-2005 |
| WO 2005037779 A2 | 28-04-2005 | AU 2004281959 A1 | 28-04-2005 |
| | | CA 2542807 A1 | 28-04-2005 |
| | | CN 1889960 A | 03-01-2007 |
| | | DE 10348022 A1 | 25-05-2005 |
| | | EP 1675594 A2 | 05-07-2006 |
| | | JP 2008500270 A | 10-01-2008 |
| | | US 2007037785 A1 | 15-02-2007 |
| | | WO 2005037779 A2 | 28-04-2005 |
| WO 2009136175 A1 | 12-11-2009 | CN 102015639 A | 13-04-2011 |
| | | EP 2274282 A1 | 19-01-2011 |
| | | JP 2011519908 A | 14-07-2011 |
| | | US 2011059953 A1 | 10-03-2011 |
| | | WO 2009136175 A1 | 12-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 4522811 A
- US 5374548 A
- US 5399331 A
- US 5416016 A
- WO 2003057673 A
- WO 2006082245 A


### Non-patent literature cited in the description


- Kallikrein-Related Peptidases. De Gruyter, 2012, vol. 1 and 2
- **HERNANDEZ et al.** *Cancer,* 2004, vol. 101, 894
- **SCHMITT et al.** *Thromb. Haemost.,* 2009, vol. 101, 222
- **PRASSAS et al.** *Nat. Rev. Drug Discov.,* 2015, vol. 14, 183
- **DIAMANDIS et al.** *Clin. Biochem.,* 2000, vol. 33, 369
- **YOUSEF et al.** *Clin. Chim. Acta,* 2003, vol. 329, 1
- **SHAW et al.** *Clin. Chem.,* 2007, vol. 53, 1423
- **BLABER et al.** *Biochemistry,* 2007, vol. 46, 5209
- **KIM et al.** *Cancer,* 2011, vol. 117, 2608
- **KRENZER et al.** *J. Invest. Dermatol.,* 2011, vol. 131, 2281
- **LIANG et al.** *Bioorg. Med. Chem. Lett.,* 2012, vol. 22, 2450
- **LIANG et al.** *ACS Med. Chem. Lett.,* 2012, vol. 3, 159
- **BERGE, S. M. et al.** *J. Pharm. Sci.,* 1977, vol. 66, 1-19
- **MICHAEL B. SMITH ; JERRY MARCH.** March's Advanced Organic Chemistry. Wiley-Interscience, 2000
- **CAMILE G. WERMUTH.** The Practice of Medicinal Chemistry. Academia Press, 2003
- **THEODORA W. GREENE ; PETER G.M. WUTS.** Protective Groups in Organic Synthesis. John Wiley & Sons Inc, 1999
- **BERGE et al.** Pharmaceutical Salts. *J. Pharm. Sci.,* 1977, vol. 66, 1-19
- **REMINGTON.** The Science and Practice of Pharmacy. Pharmaceutical Sciences, September 2012
- **ANSEL et al.** Pharmaceutical Dosage Forms and Drug Delivery Systems. Lippincott Williams & Wilkins Publishers, 1999
- Sustained and Controlled Release Drug Delivery Systems. Marcel Dekker, Inc, 1978
- **STREJAN et al.** *J. Neuroimmunol.,* 1984, vol. 7, 27
- **V.V. RANADE.** *J. Clin. Pharmacol.,* 1989, vol. 29, 685
- **UMEZAWA et al.** *Biochem. Biophys. Res. Commun.,* 1988, vol. 153, 1038
- **P.G. BLOEMAN et al.** *FEBS Lett.,* 1995, vol. 357, 140
- **M. OWAIS et al.** *Antimicrob. Agents Chemother.,* 1995, vol. 39, 180
- **BRISCOE et al.** *Am. J. Physiol.,* 1995, vol. 1233, 134
- **KRENZER, S. ; PETERZIEL, H. ; MAUCH, C. ; BLABER, S. I. ; BLABER, M. ; ANGEL, P. ; HESS, J.** Expression and Function of the Kallikrein-Related Peptidase 6 in the Human Melanoma Microenvironment. *J. Invest. Dermatol.,* 2011, vol. 131 (11), 2281-2288
- **DRUCKER, K. L. ; PAULSEN, A. R. ; GIANNINI, C. ; DECKER, P. A. ; BLABER, S. I. ; BLABER, M. ; UHM, J. H. ; O'NEILL, B. P. ; JENKINS, R. B. ; SCARISBRICK, I. A.** Clinical significance and novel mechanism of action of kallikrein 6 in glioblastoma. *Neuro-Oncology,* 2013, vol. 15 (3), 305-318

- **DRUCKER, K. L. ; GIANINNI, C. ; DECKER, P. A. ; DIAMANDIS, E. P. ; SCARISBRICK, I. A.** Prognostic significance of multiple kallikreins in high-grade astrocytoma. *BMC Cancer,* 2015, vol. 15, 565
- **SHAN, S. J. C. ; SCORILAS A. ; KATSAROS, D. ; DIAMANDIS, E. P.** Transcriptional upregulation of human tissue kallikrein 6 in ovarian cancer: clinical and mechanistic aspects. *British Journal of Cancer,* 2007, vol. 96, 362-372
- **HENKHAUS, R. S. ; GERNER, E. W. ; IGNATENKO, N. A.** Kallikrein 6 is a mediator of K-RAS-dependent migration of colon carcinoma cells. *Biol Chem.,* 2008, vol. 389 (6), 757-764
- **HEUZÉ-VOURC'H, N. ; PLANQUE, C. ; GUYETANT, S. ; COCO, C. ; BRILLET, B. ; BLECHET, C. ; PARENT, C. ; LAURENT, B. ; REVERDIAU, P. ; JOURDAN, M-L.** High kallikrein-related peptidase 6 in non-small cell lung cancer cells: an indicator of tumour proliferation and poor prognosis. *J. Cell. Mol. Med.,* 2009, vol. 13 (9B), 4014-4022
- **HU, C. J. ; CHEN, K. X. ; ZHENG, J. F. ; CHEN, Y. J.** Expression and biological significance of human kallikrein 6 in gastric cancer tissues. *Contemp. Oncol.,* 2013, vol. 17 (1), 64-67
- **NAGAHARA, H. ; MIMORI, K. ; UTSUNOMIYA, T. ; BARNARD, G. F. ; OHIRA, M. ; HIRAKAWA, K. ; MORI, M.** Clinicopathologic and Biological Significance of Kallikrein 6 Overexpression in Human Gastric Cancer. *Clin Cancer Res.,* 2005, vol. 11 (19), 6800-6806
- **GRIN, A. ; SAMAAN, S ; TRIPATHI, M. ; ROTONDO, F. ; KOVACS, K. ; BASSILY, M. N. ; YOUSEF, G. M.** Evaluation of human tissue kallikrein-related peptidases 6 and 10 expression in early gastroesophageal adenocarcinoma. *Human Pathology,* 2015, vol. 46, 541-548
- **RÜCKERT, F. ; HENNIG, M. ; PETRAKI, C. D. ; WEHRUM, D. ; DISTLER, M. ; DENZ, A. ; SCHRÖDER, M. ; DAWELBAIT, G. ; KALTHOFF4, H. ; SAEGER, H-D.** Co-expression of KLK6 and KLK10 as prognostic factors for survival in pancreatic ductal adenocarcinoma. *British Journal of Cancer,* 2008, vol. 99, 1484-1492
- **SIDIROPOULOSA, K. G. ; DINGA, Q. ; PAMPALAKISC, G. ; WHITEA, N. M. A. ; BOULOSA, P. ; SOTIROPOULOUC, G. ; YOUSEF G. M.** KLK6-regulated miRNA networks activate oncogenic pathways in breast cancer subtypes. *Mol. Oncology,* 2016
- **ZHANG, Y. ; ZHANG, Z. ; YANG, L. ; XU, B. ; LI, W. ; TANG, P. ; ZHANG, Z. ; HAN, N. ; GAO, Y. ; CHENG, S.** Identification of Human Tissue Kallikrein 6 as a Potential Marker of Laryngeal Cancer Based on the Relevant Secretory/Releasing Protein Database. *Disease Markers,* 2014, 8
- **SCARISBRICK, I. A. ; YOON, H. ; PANOS, M. ; LARSON, N. ; BLABER, S. I. ; BLABER, M. ; RODRIGUES, M.** Kallikrein 6 Regulates Early CNS Demyelination in a Viral Model of Multiple Sclerosis. *Brain Pathol.,* 2012, vol. 22 (5), 709-722
- **LUNDBERG, K. C. ; FRITZ, Y. ; JOHNSTON, A. ; FOSTER, A. M. ; BALIWAG, J. ; GUDJONSSON, J. E. ; SCHLATZER, D. ; GOKULRANGAN, G. ; MCCORMICK, T. S. ; CHANCE, M. R.** Proteomics of Skin Proteins in Psoriasis: From Discovery and Verification in a Mouse Model to Confirmation in Humans. *Mol. And Cell. Proteomics,* 2015, vol. 14 (1), 109-119
- **KOMATSU, N. ; SAIJOH, K. ; KUK, C. ; SHIRASAKI, F. ; TAKEHARA, K. ; DIAMANDIS, E. P.** Aberrant human tissue kallikrein levels in the stratum corneum and serum of patients with psoriasis: dependence on phenotype, severity and therapy. *British Journal of Dermatology,* 2007, vol. 156, 875-883
- **YOON, H. ; RADULOVIC, M. ; WU, J. ; BLABER, S. I. ; BLABER, M. ; FEHLINGS, M. G. ; SCARISBRICK, I. A.** Kallikrein 6 Signals through PAR1 and PAR2 to Promote Neuron Injury and Exacerbate Glutamate Neurotoxicity. *J Neurochem,* 2013, vol. 127 (2), 283-298
- **SCARISBRICK, I. A. ; SABHARWAL, P. ; CRUZ, H. ; LARSEN, N. ; VANDELL,A. G. ; BLABER, S. I. ; AMEENUDDIN, S. ; PAPKE, L. M. ; FEHLINGS, M. G. ; REEVES, R. K.** Dynamic role of kallikrein 6 in traumatic spinal cord injury. *European Journal of Neuroscience,* 2006, 1457-1469
- **XI, Y. ; ZHANG, Y. ; FANG, J. ; WILSON, J. J. ; LUO, S. ; HUANG, R-P.** Development of Monoclonal Antibodies and Characterization of an ELISA Platform Against Kallikrein-Related Peptidase 6 as a Tumor Biomarker. *Monoclonal Antibodies in Immunodiagnosis and Immunotherapy,* 2015, vol. 34 (5), 346-353
- **SCARISBRICK, I. A. ; ASAKURA, K. ; BLABER, S. ; BLABER, M. ; ISACKSON, P. J. ; BIETO, T. ; RODRIGUES, M. ; WINDEBANK, A.** Preferential expression of myelencephalon specific protease by oligodendrocytes of the adult rat spinal cord white matter. *Glia,* 2000, vol. 30, 219-230
- *J. Biol. Chem.,* 2002, vol. 277, 24562-24570